# EUROPEAN PATENT APPLICATION

(11) **EP 4 011 886 A1**
(43) Date of publication of application: **15.06.2022**
(21) Application number: 20850862.2
(22) Date of filing: 03.08.2020
(51) Int. Cl.: C07D 498/14, C07D 498/22, A61P 35/00, A61K 31/519

(54) **TETRACYCLIC COMPOUND, PREPARATION METHOD THEREFOR AND USE THEREOF**

(30) Priority: 02.08.2019 CN 201910712388; 23.09.2019 CN 201910899724; 22.11.2019 CN 201911157922; 17.01.2020 CN 202010054185
(71) Applicant: Shanghai Jemincare Pharmaceuticals Co., Ltd., Pudong New Area, Shanghai 201203 (CN); Jiangxi Jemincare Group Co., Ltd., Nanchang, Jiangxi 330096 (CN)
(72) Inventor: GUO, Shuchun, Shanghai 201203 (CN); FAN, Jun, Shanghai 201203 (CN); LIU, Yang, Shanghai 201203 (CN); PENG, Jianbiao, Shanghai 201203 (CN); GUO, Haibing, Shanghai 201203 (CN)
(74) Representative: Mewburn Ellis LLP
(86) International application number: PCT/CN2020/106573
(87) International publication number: WO 2021/023154

(57) **Abstract**

Disclosed are a compound as shown in formula (I), an optical isomer thereof and a pharmaceutically acceptable salt thereof, and the use of the compound as a KRAS inhibitor.

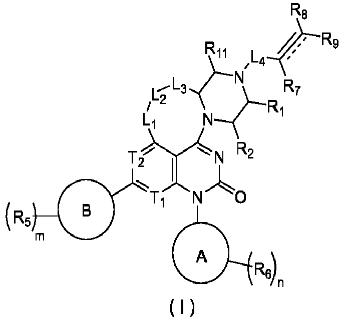

## Description

The present disclosure claims the priority to:
Chinese Patent Application No. CN201910712388.0 filed on Aug. 2, 2019;
Chinese Patent Application No. CN201910899724.7 filed on Sept. 23, 2019;
Chinese Patent Application No. CN201911157922.2 filed on Nov. 22, 2019; and
Chinese Patent Application No. CN202010054185.X filed on Jan. 17, 2020.

### Technical Field

The present disclosure relates to a compound of formula (I), an optical isomer thereof and a pharmaceutically acceptable salt thereof, and use of the compound as a KRAS inhibitor.

### Background

Cancer has been the first cause of death in China for 31 years. Among its types, lung cancer is one of the most common tumors, and more than 80% of lung cancers are non-small cell lung cancer. Besides, the incidence of lung cancer is high, with a variety of mutations present. In order to enrich the research and development pipeline of the company, focusing on unmet medical needs and developing innovative drugs for treating cancer are very necessary for the long-term development of the company and are of great economic and social significance.

Mutations in RAS genes are found in about 30% of cancer patients. In the research of oncogenes, scientists found that RAS genes were key genes to cancers including lung cancer, colorectal cancer and pancreatic cancer over 20 years ago.

In the United States, RAS mutations are also the most common in the three types of cancer with the highest mortality (pancreatic, colorectal and lung cancers), and are found in 95%, 52% and 31% of patients with these three types of cancer, respectively. KRAS mutations predominate in pancreatic, colorectal and lung cancers, NRAS mutations are common in melanoma and acute myeloid leukemia, and HRAS mutations are common in bladder and head and neck cancers.

The mutation rate of KRAS genes in Asian population is 10-15%. KRAS genes mutate in many cancers, and are one of the main oncogenes. KRAS mutant tumors are the most potentially targeted molecular subtype of non-small cell lung cancer (NSCLC), with a mutation rate of about 15% to 25% in NSCLC. In NSCLC cases, KRAS mutations mainly occur at codons 12 and 13. KRAS-G12C mutations, the most common codon mutations, are found in approximately 39% of KRAS mutant NSCLCs.

In lung adenocarcinoma, the probability of positive KRAS genes is 1/5 to 1/4, second only to that of positive EGFR mutations. The lack of targeted inhibitors leads to great difficulty in both treatment and prognosis of KRAS positive non-small cell lung cancer patients. NCCN Clinical Practice Guidelines for Non-Small Cell Lung Cancer (2013) specifically states that: KRAS gene testing must be done prior to the treatment of a lung cancer patient with EGFR-TKIs, and whether to use targeted drugs EGFR-TKIs as clinical treatment measures is determined based on the testing results. If KRAS genes have mutated, molecular targeted therapies with EGFR-TKIs are not recommended for the patient.

According to the Thomson Reuters Competitive Intelligence drug database (Cortellis for CI), there are 162 drugs directly associated with RAS genes/proteins (data acquired on Aug. 18, 2016), among which are 18 KRAS small molecule drugs, including 10 KRAS GTP enzyme inhibitors, 4 KRAS gene inhibitors, 2 KRAS GTP enzyme regulators and 2 KRAS gene regulators; currently, there are 1 of such drugs in clinical research. In addition, the first KRAS inhibitor Antroquinonol developed by Taiwan corporation has entered the U.S. FDA phase II clinical trials, and the inhibitor selumetinib targeting MEK in the KRAS downstream pathway developed by AstraZeneca is also in phase II clinical trials. KRAS mutations are the most important tumor driver genes. The mutation cases account for a certain proportion in patients with pancreatic cancer, lung cancer and colorectal cancer. At present, no specifically targeted drug acting on this target is available. Therefore, the project has important medical research value and clinical application value, and has greater medical value for people in China. In the development of a KRAS-G12C small molecule drug, its molecular mechanism has been substantially elucidated, and the molecular structure and efficacy of the drug are verified under the existing test conditions, showing that it has high activity and druggability.

### Content of the present invention

The present disclosure provides a compound of formula (I), an optical isomer and a pharmaceutically acceptable salt thereof, 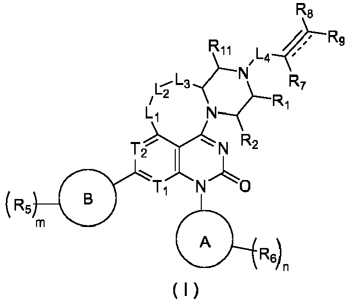 wherein,
R₁, R₂, and R₁₁ are each independently selected from H, halogen, OH, NH₂, CN, C₁₋₆ alkyl, C₁₋₆ alkoxy and C₁₋₆ alkylamino, wherein the C₁₋₆ alkyl, C₁₋₆ alkoxy or C₁₋₆ alkylamino is optionally substituted with 1, 2 or 3 R;
T₁ is selected from N and C(R₃);
T₂ is selected from N and C(R₄);
R₃ and R₄ are each independently selected from H, halogen, OH, NH₂, CN, C₂₋₄ alkenyl, C₂₋₄ alkynyl, C₁₋₆ alkyl, C₁₋₆ alkoxy, C₁₋₆ alkylamino, C₃₋₆ cycloalkyl and 3-6 membered heterocycloalkyl, wherein the C₂₋₄ alkenyl, C₂₋₄ alkynyl, C₁₋₆ alkyl, C₁₋₆ alkoxy, C₁₋₆ alkylamino, C₃₋₆ cycloalkyl or 3-6 membered heterocycloalkyl is optionally substituted with 1, 2 or 3 R;
R₅ is independently selected from H, halogen, OH, NH₂, CN, C₁₋₆ alkyl, C₁₋₆ alkoxy, C₁₋₆ alkylamino, C₁₋₆ alkyl-OC(=O)-, C₃₋₆ cycloalkyl, 3-6 membered heterocycloalkyl, C₆₋₁₀ aryl and 5-10 membered heteroaryl, wherein the C₁₋₆ alkyl, C₁₋₆ alkoxy, C₁₋₆ alkylamino, C₁₋₆ alkyl-OC(=O)-, C₃₋₆ cycloalkyl, 3-6 membered heterocycloalkyl, C₆₋₁₀ aryl or 5-10 membered heteroaryl is optionally substituted with 1, 2 or 3 R;
R₆ is independently selected from H, halogen, OH, NH₂, CN, C₁₋₆ alkyl, C₁₋₆ alkoxy, C₁₋₆ alkylamino, C₁₋₆ alkyl-OC(=O)-, C₃₋₆ cycloalkyl and 3-6 membered heterocycloalkyl, wherein the C₁₋₆ alkyl, C₁₋₆ alkoxy, C₁₋₆ alkylamino, C₁₋₆ alkyl-OC(=O)-, C₃₋₆ cycloalkyl or 3-6 membered heterocycloalkyl is optionally substituted with 1, 2 or 3 R;
ring A is selected from C₃₋₆ cycloalkyl, 3-6 membered heterocycloalkyl, C₆₋₁₀ aryl and 5-10 membered heteroaryl;
ring B is selected from C₆₋₁₀ aryl, 5-10 membered heteroaryl, benzo 5-6 membered heterocycloalkyl and 5-6 membered heteroaryl-fused 5-6 membered heterocycloalkyl;
R₇ is selected from H, halogen, CN, C₁₋₆ alkyl, C₁₋₆ alkoxy and C₁₋₆ alkylamino, wherein the C₁₋₆ alkyl, C₁₋₆ alkoxy or C₁₋₆ alkylamino is optionally substituted with 1, 2 or 3 R;
R₈ and R₉ are each independently selected from H, halogen, CN, C₁₋₆ alkyl, C₃₋₆ cycloalkyl and 3-6 membered heterocycloalkyl, wherein the C₁₋₆ alkyl, C₁₋₆ heteroalkyl, C₁₋₆ alkylamino, C₃₋₆ cycloalkyl or 3-6 membered heterocycloalkyl is optionally substituted with 1, 2 or 3 R; 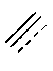 represents 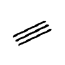 or 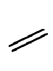 , and when 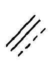 is 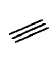 , then R₇ and R₉ are absent;
L₁ is selected from a single bond, CH₂, O, S, S(=O), C(=O), S(=O)₂ and N(R₁₀);
L₂ is selected from CH₂, O, S and C(=O);
L₃ is selected from a single bond, C(R₁₂R₁₂) and C(=O);
L₄ is selected from S(=O), S(=O)₂ and C(=O);
m is selected from 1, 2, 3 and 4;
n is selected from 1, 2, 3 and 4;
R₁₀ is selected from H, C₁₋₆ alkyl, C₃₋₆ cycloalkyl and 3-6 membered heterocycloalkyl, wherein the C₁₋₆ alkyl, C₃₋₆ cycloalkyl or 3-6 membered heterocycloalkyl is optionally substituted with 1, 2 or 3 R;
R₁₂ is independently selected from H, F, Cl, Br, I, OH, NH₂, CN, Me and CF₃;
R is independently selected from H, halogen, OH, NH₂, CN, 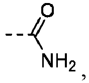 C₁₋₆ alkyl, C₁₋
₆ alkyl-OC(=O)-, C₁₋₆ alkoxy, C₁₋₆ alkylthio, C₁₋₆ alkylamino and 5-6 membered heterocycloalkyl, wherein the C₁₋₆ alkyl, C₁₋₆ alkyl-OC(=O)-, C₁₋₆ alkoxy, C₁₋₆ alkylthio, C₁₋₆ alkylamino or 5-6 membered heterocycloalkyl is optionally substituted with 1, 2 or 3 R';
R' is selected from F, Cl, Br, I, OH, NH₂ and CH₃;
the 3-6 membered heterocycloalkyl, 5-6 membered heterocycloalkyl, 5-10 membered heteroaryl or C₁₋₆ heterocycloalkyl contains 1, 2 or 3 heteroatoms or heteroatom groups independently selected from O, NH, S, C(=O)O, S(=O), S(=O)₂ and N.

In some embodiments of the present disclosure, R is independently selected from H, halogen, OH, NH₂, CN, 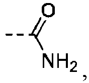 C₁₋₃ alkyl, C₁₋₃ alkoxy, C₁₋₃ alkylthio, C₁₋₃ alkylamino and 5-6 membered heterocycloalkyl, wherein the C₁₋₃ alkyl, C₁₋₃ alkoxy, C₁₋₃ alkylthio, C₁₋₃ alkylamino, or 5-6 membered heterocycloalkyl is optionally substituted with 1, 2, or 3 R', and the other variables are as defined in the present disclosure.

In some embodiments of the present disclosure, R is independently selected from H, F, Cl, Br, I, OH, NH₂, CN, Me, 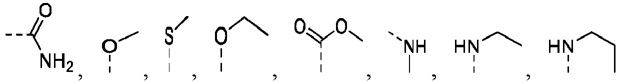 and 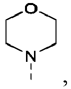 wherein Me, 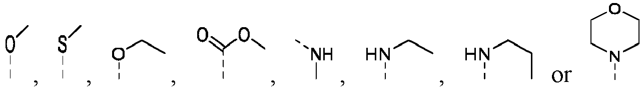 is optionally substituted with 1, 2 or 3 R', and the other variables are as defined in the present disclosure.

In some embodiments of the present disclosure, R is independently selected from H, F, Cl, Br, I, OH, NH₂, CN, Me, 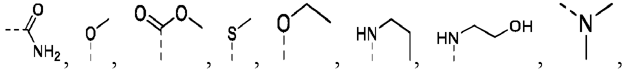 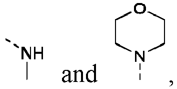 and the other variables are as defined in the present disclosure.

In some embodiments of the present disclosure, R₁, R₂ and R₁₁ are each independently selected from H, halogen, OH, NH₂, CN, C₁₋₃ alkyl, C₁₋₃ alkoxy, and C₁₋₃ alkylamino, wherein C₁₋₃ alkyl, C₁₋₃ alkoxy or C₁₋₃ alkylamino is optionally substituted with 1, 2, or 3 R, and the other variables are as defined in the present disclosure.

In some embodiments of the present disclosure, R₁, R₂ and R₁₁ are each independently selected from H, F, Cl, Br, I, OH, NH₂, CN, Me, CF₃, 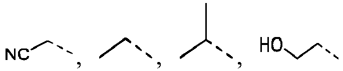 and 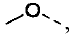 and the other variables are as defined in the present disclosure.

In some embodiments of the present disclosure, R₃ and R₄ are each independently selected from H, F, Cl, Br, I, OH, NH₂, CN, Me, CF₃, 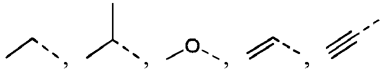 and 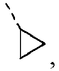 and the other variables are as defined in the present disclosure.

In some embodiments of the present disclosure, R₅ is selected from H, F, Cl, Br, I, OH, NH₂, CN, Me, CF₃, 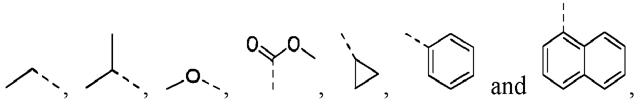 and the other variables are as defined in the present disclosure.

In some embodiments of the present disclosure, R₆ is selected from H, F, Cl, Br, I, OH, NH₂, CN, Me, CF₃, N(CH₃)₂, 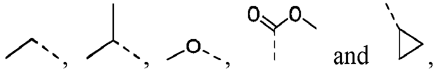 and the other variables are as defined in the present disclosure.

In some embodiments of the present disclosure, ring A is selected from C₃₋₆ cycloalkyl, 3-6 membered heterocycloalkyl, phenyl, naphthyl, thienyl, pyrazolyl, thiazolyl, imidazolyl, pyridyl, pyrimidinyl, indazolyl and indolyl, and the other variables are as defined in the present disclosure.

In some embodiments of the present disclosure, the moiety 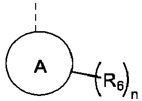 is selected from 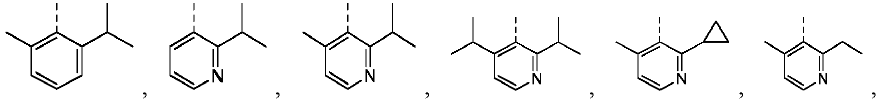 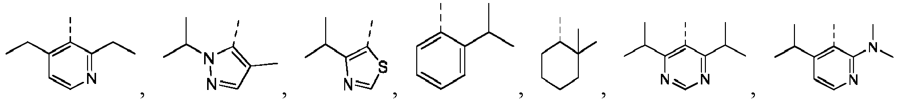 and 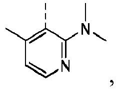 and the other variables are as defined in the present disclosure.

In some embodiments of the present disclosure, ring B is selected from phenyl, naphthyl, thienyl, pyridyl, pyrimidinyl, indazolyl, indolyl, 1*H*-benzo[*d*][1,2,3]triazolyl, 1,3-dihydro-2*H*-benzo[*d*]imidazol-2-onyl, benzo[d]oxazol-2(3*H*)-onyl, 1*H*-pyrazolo[3,4-b]pyridyl, isoquinolin-1(2*H*)-onyl and 1*H*-benzo[d]imidazolyl, and the other variables are as defined in the present disclosure.

In some embodiments of the present disclosure, the moiety 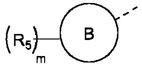 is selected from 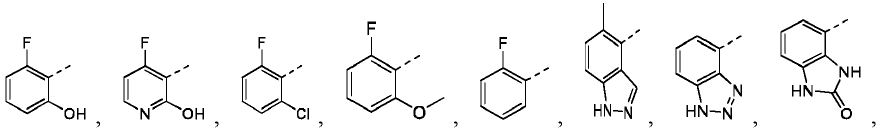 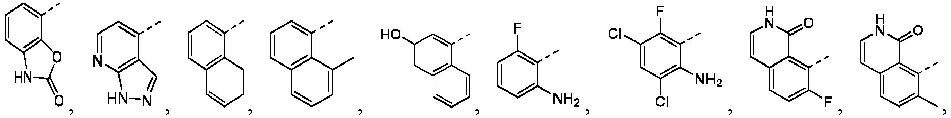 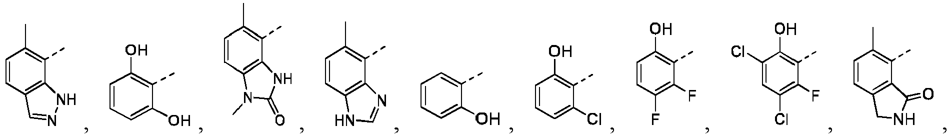 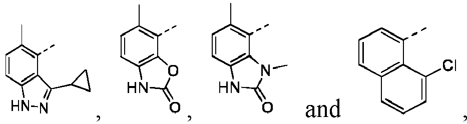 and the other variables are as defined in the present disclosure.

In some embodiments of the present disclosure, the moiety 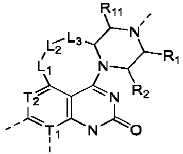 is selected from 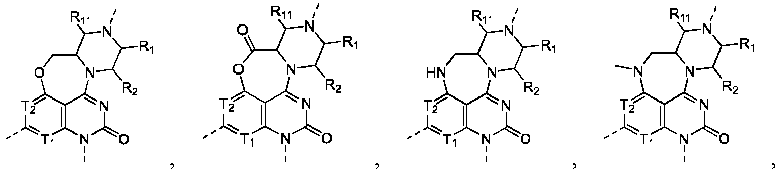 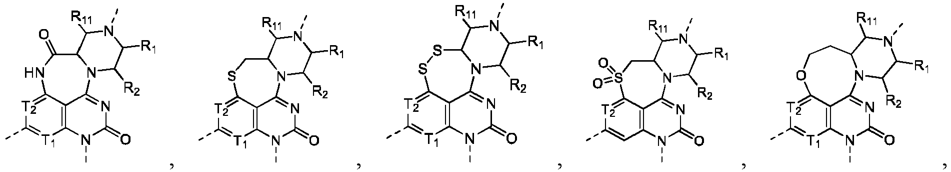 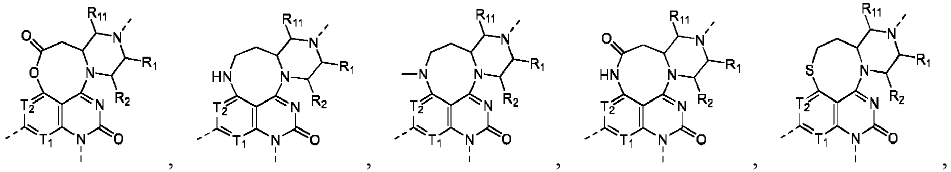 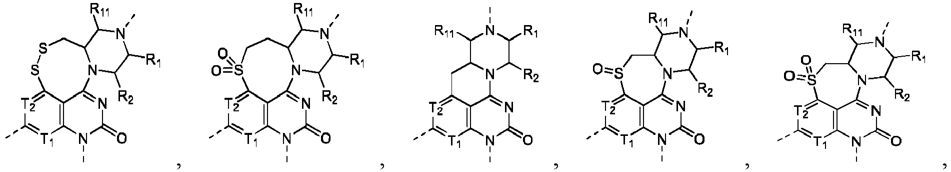 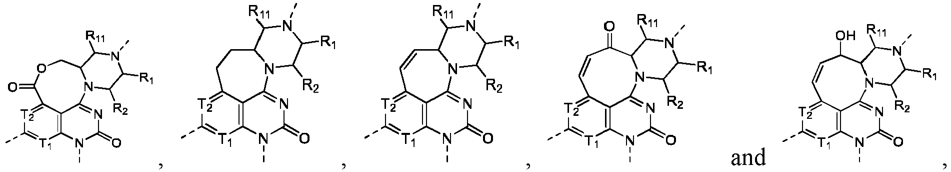 and the other variables are as defined in the present disclosure.

In some embodiments of the present disclosure, the moiety 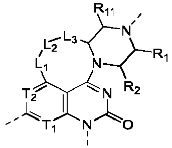 is selected from 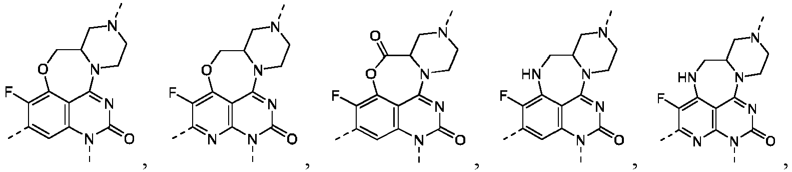 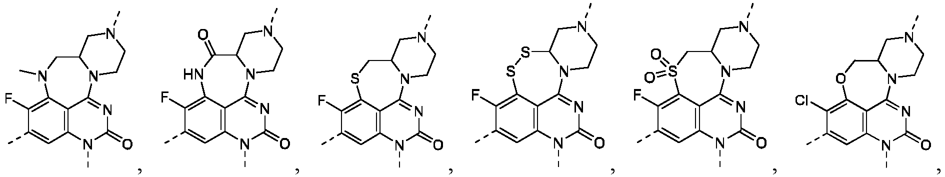 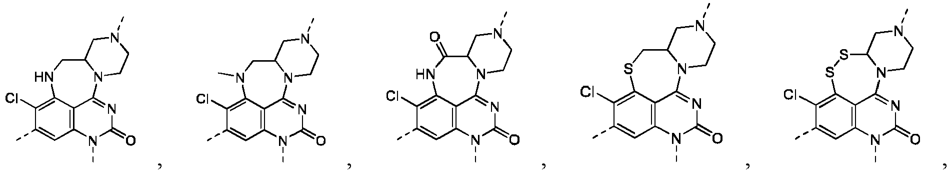 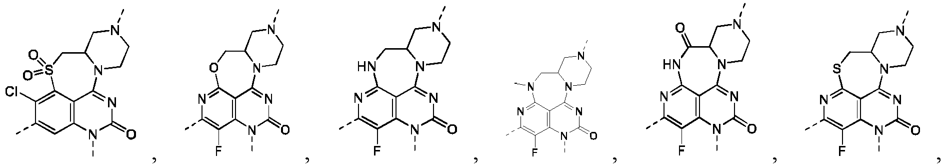 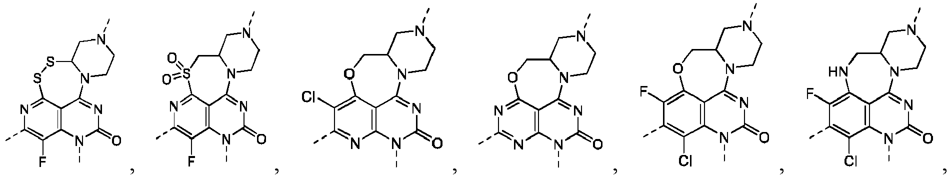 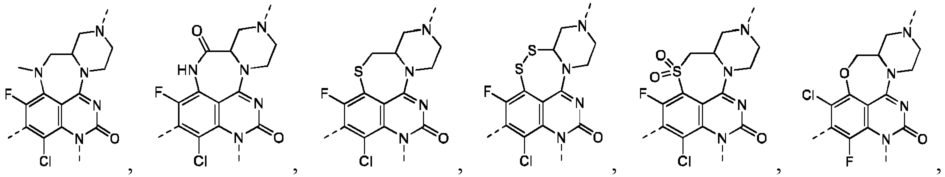 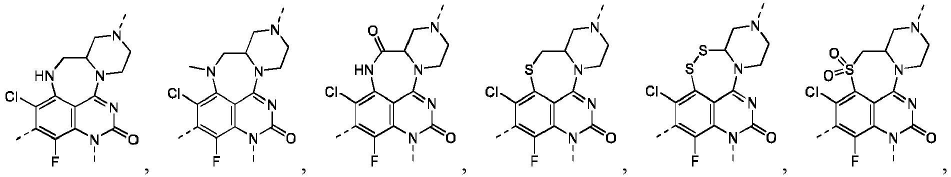 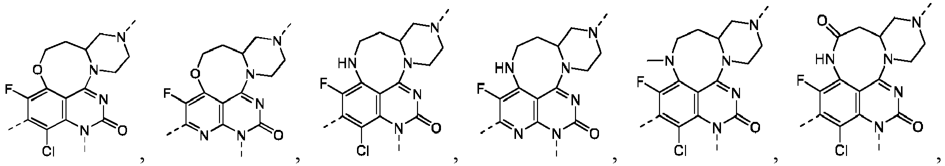 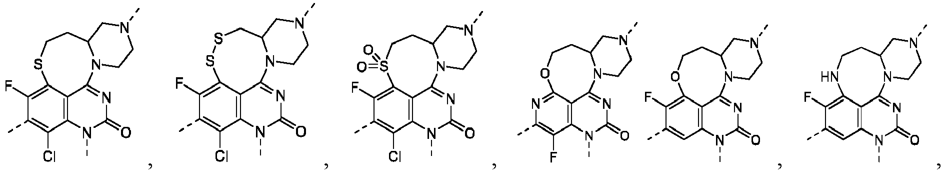 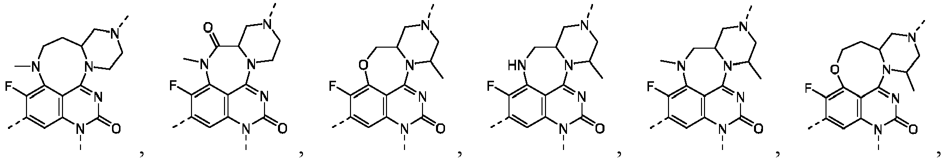 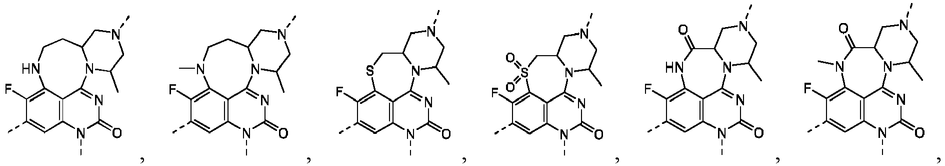 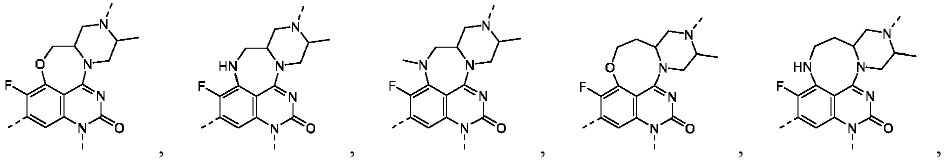 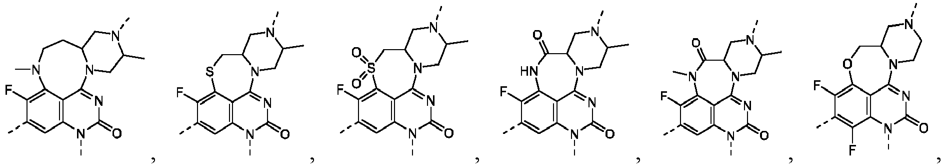 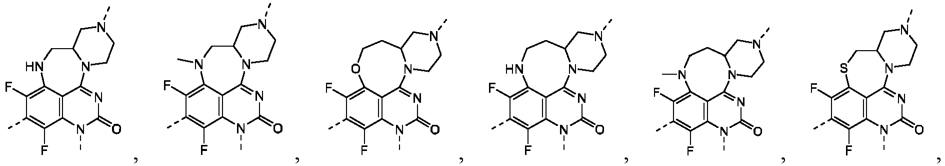 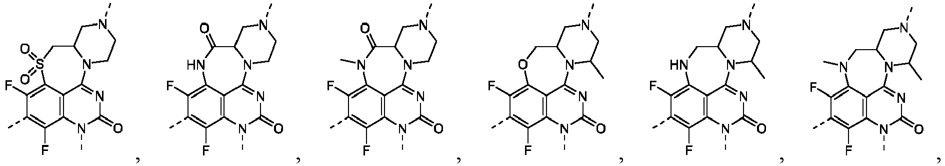 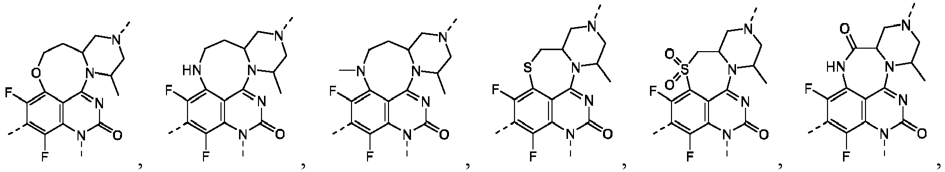 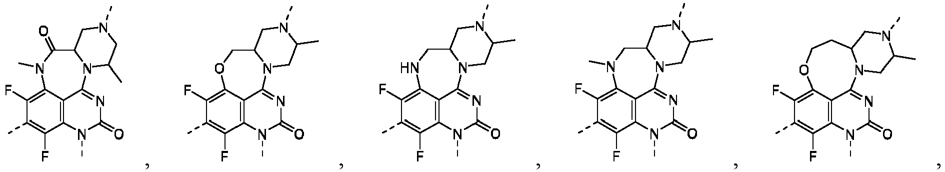 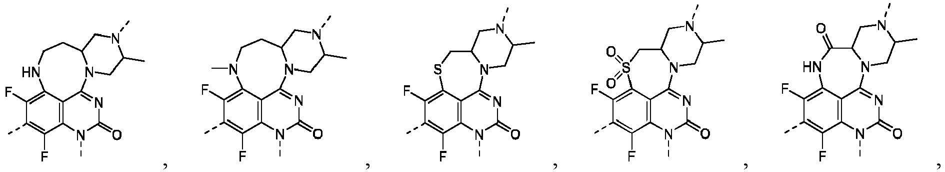 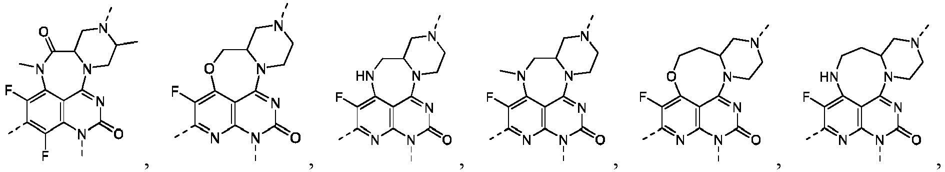 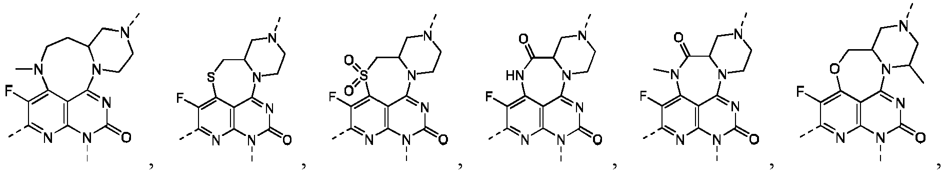 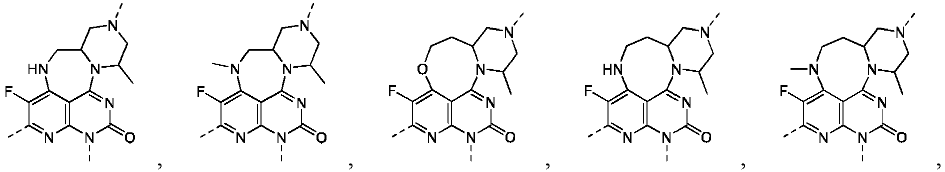 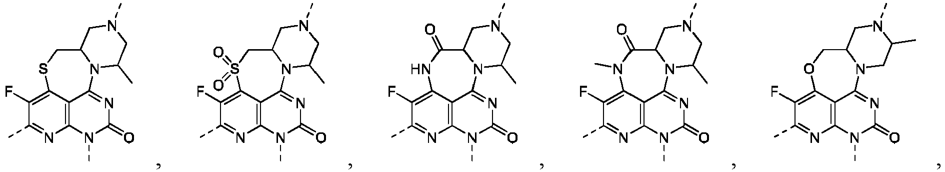 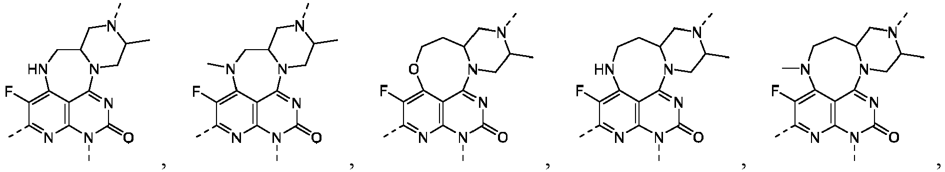 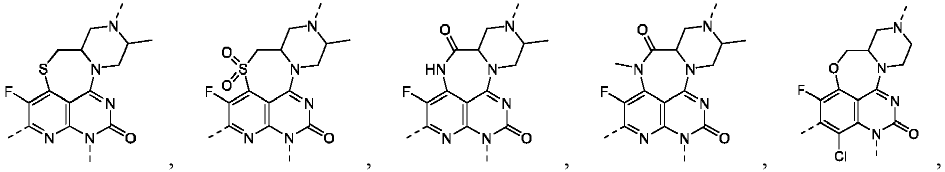 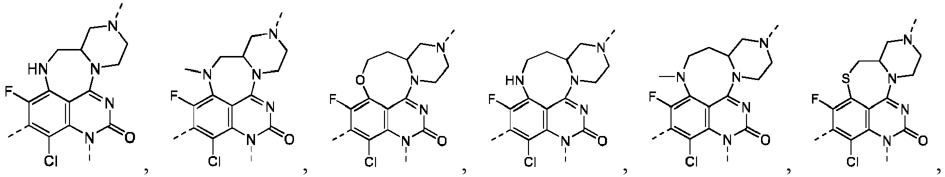 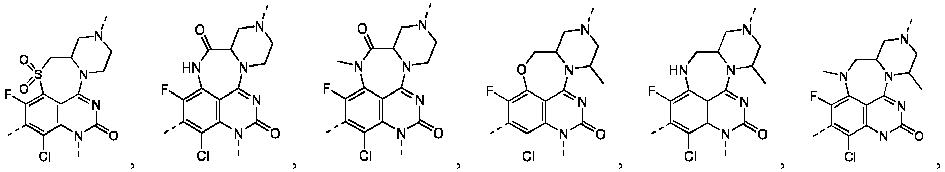 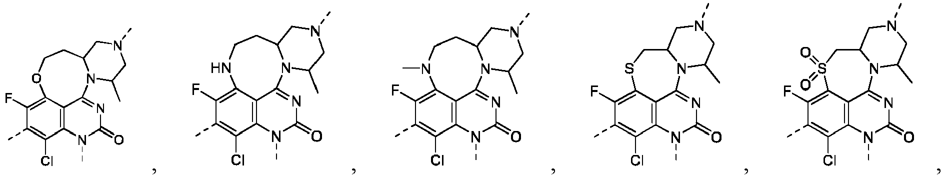 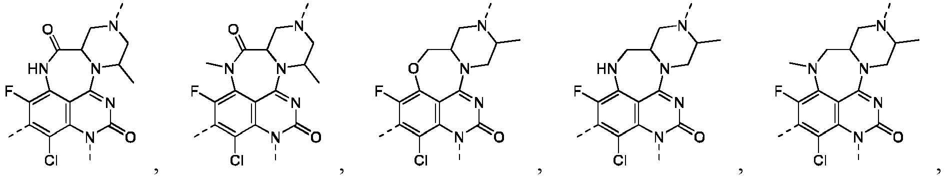 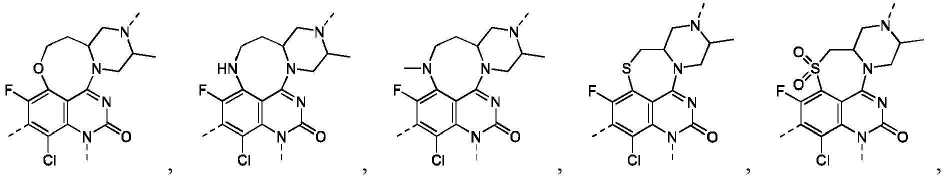 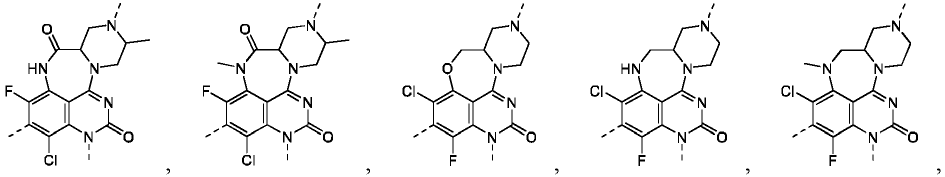 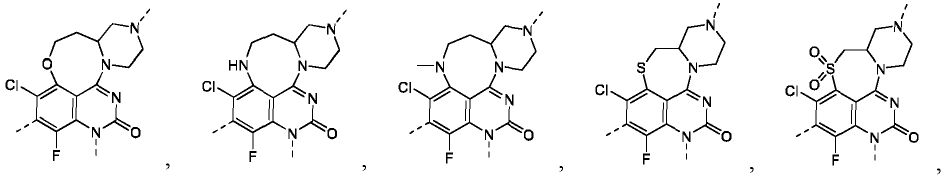 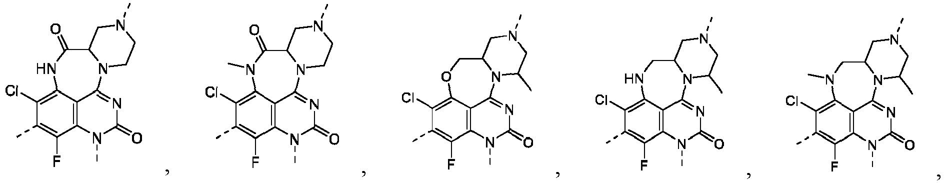 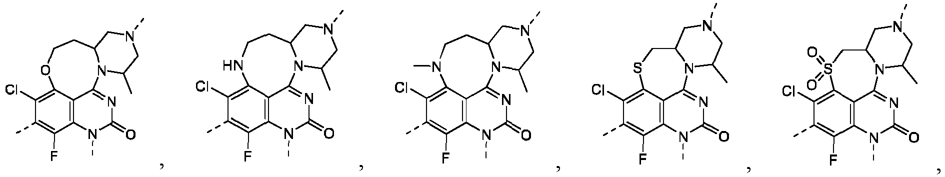 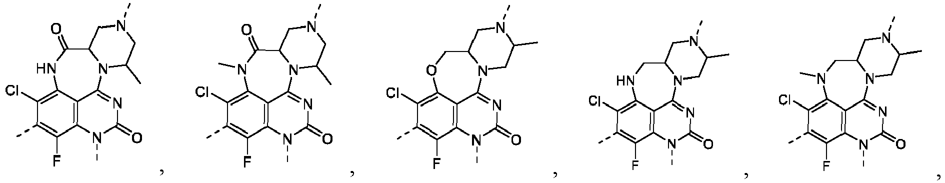 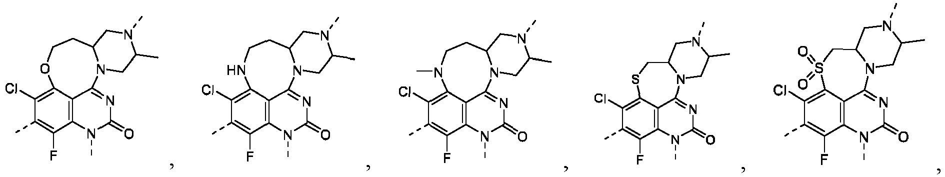 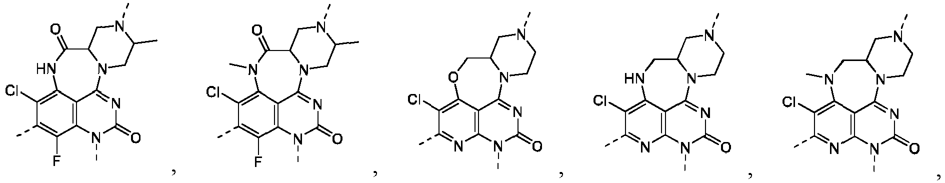 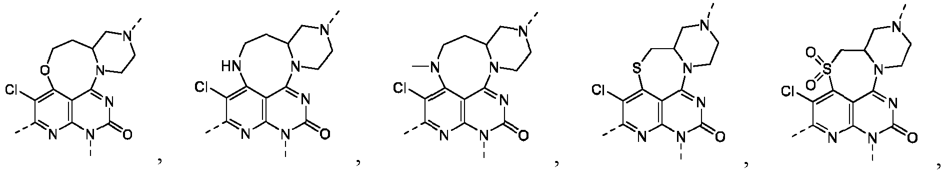 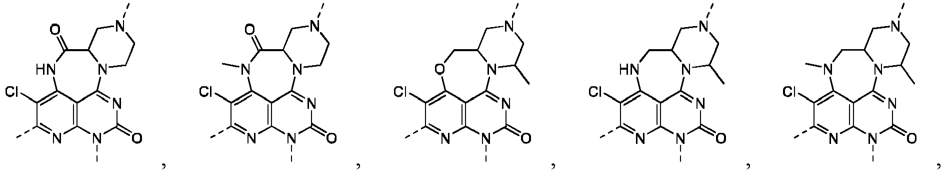 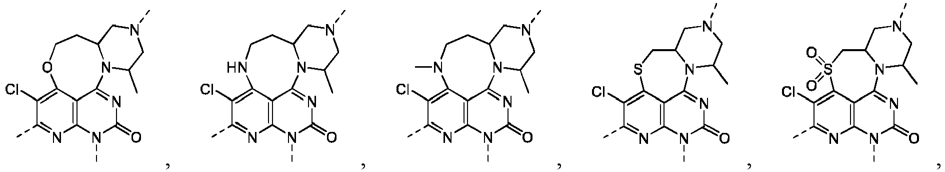 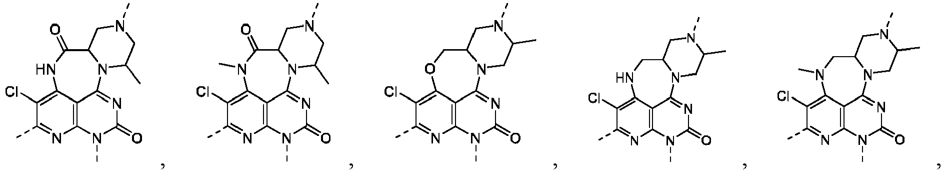 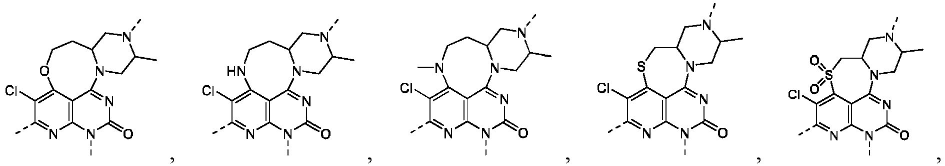 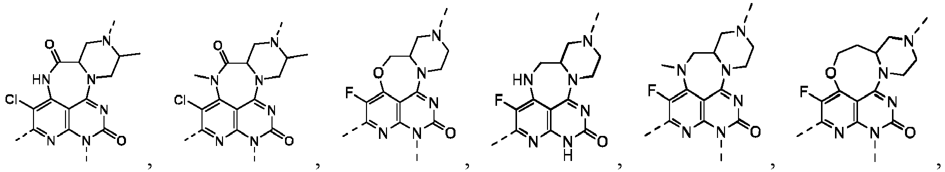 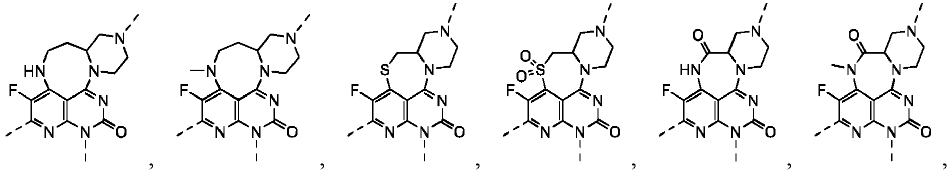 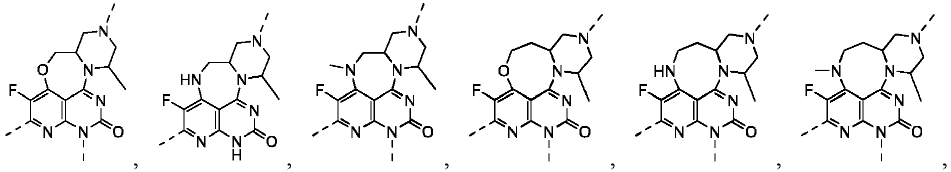 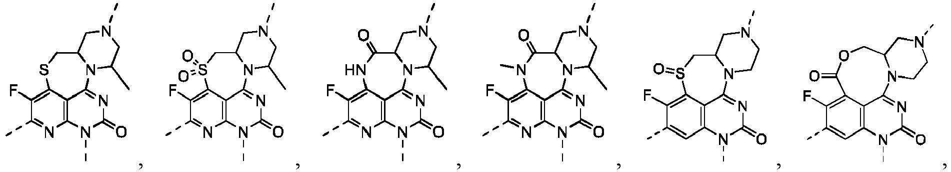 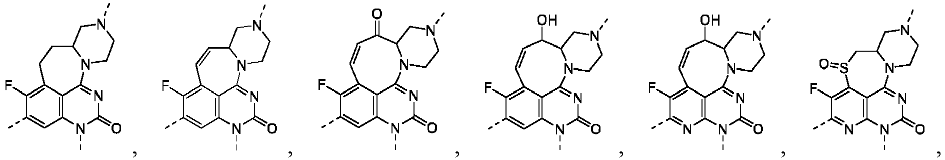 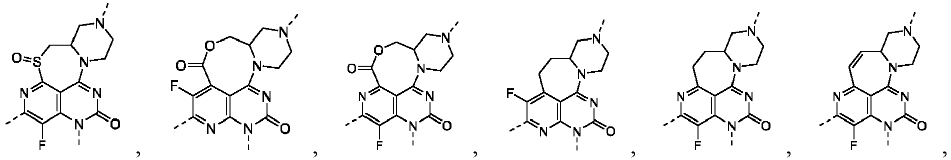 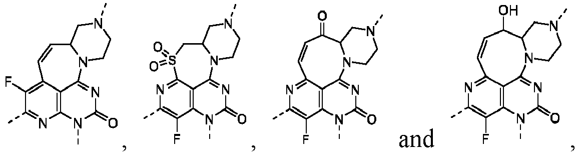 and the other variables are as defined in the present disclosure.

In some embodiments of the present disclosure, R₇ is selected from H, F, Cl, Br, I, CN, Me, CF₃, 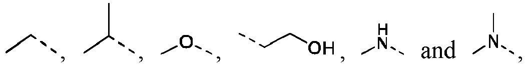 and the other variables are as defined in the present disclosure.

In some embodiments of the present disclosure, R₈ and R₉ are each independently selected from H, F, Cl, Br, I, CN, Me, CF₃, 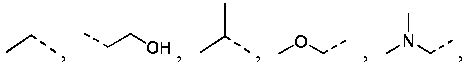 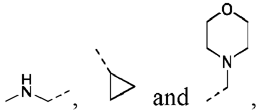 the other variables are as defined in the present disclosure.

In some embodiments of the present disclosure, the moietys 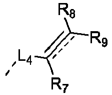 is selected from 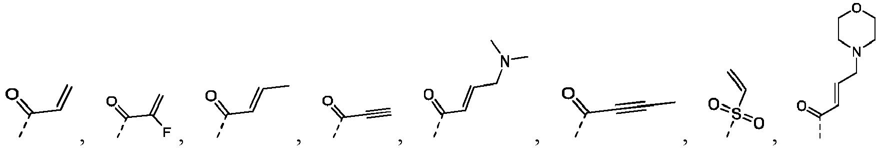 and 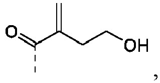 the other variables are as defined in the present disclosure.

In some embodiments of the present disclosure, the compound, the optical isomer thereof and the pharmaceutically acceptable salt thereof are selected from 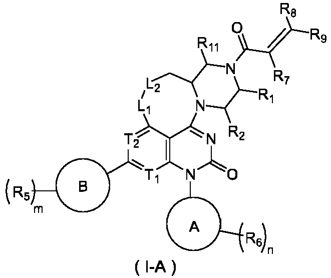 wherein,
R₁, R₂, L₁, L₂, T, T₂, R₅, R₆, ring A, ring B, R₇, R₈ and R₉ are as defined above.

In some embodiments of the present disclosure, the compound, the optical isomer thereof and the pharmaceutically acceptable salt thereof are selected from 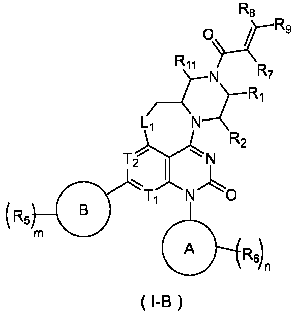 wherein,
R₁, R₂, L₁, T, T₂, R₅, R₆, ring A, ring B, R₇, R₈ and R₉ are as defined above.

In some embodiments of the present disclosure, the compound, the optical isomer thereof and the pharmaceutically acceptable salt thereof are selected from 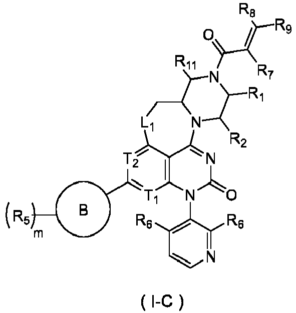 wherein,
R₁, R₂, L₁, T, T₂, R₅, R₆, ring A, ring B, R₇, R₈ and R₉ are as defined above.

The present disclosure also provides a compound of the formula below, an optical isomer thereof and a pharmaceutically acceptable salt thereof, which are selected from

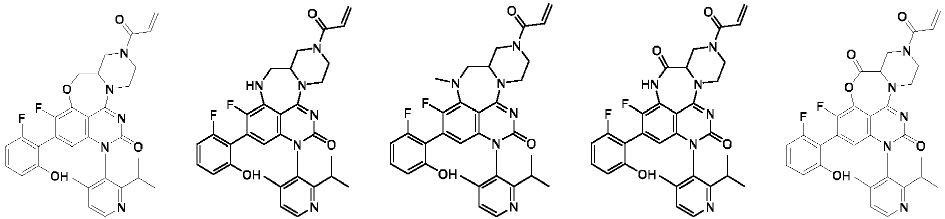

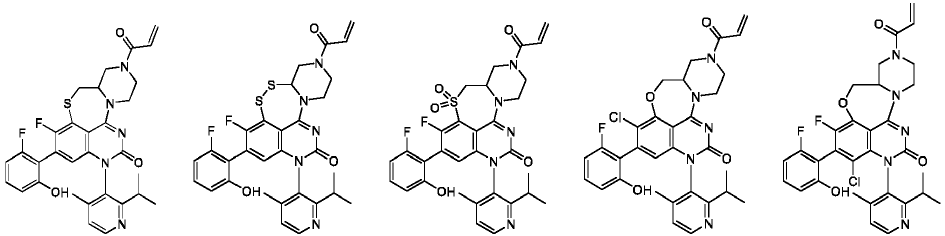

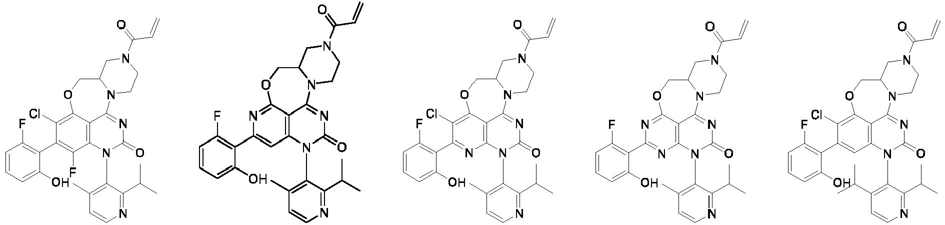

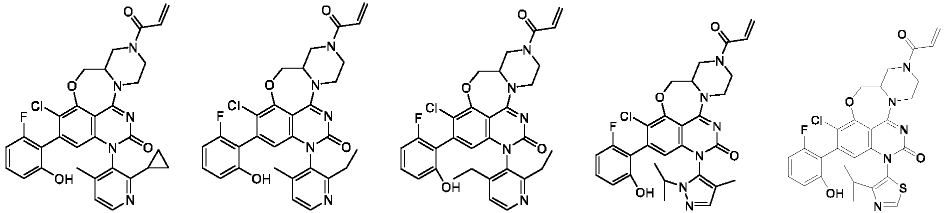

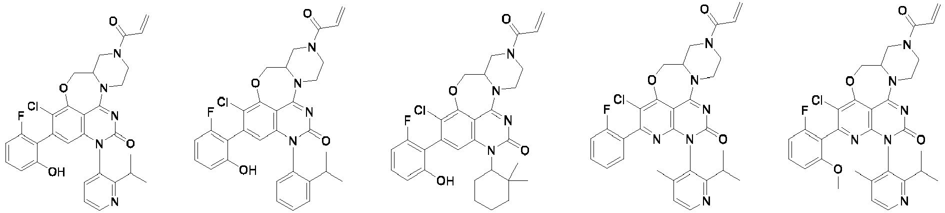

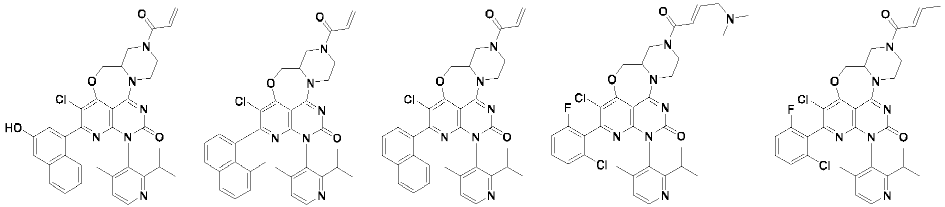

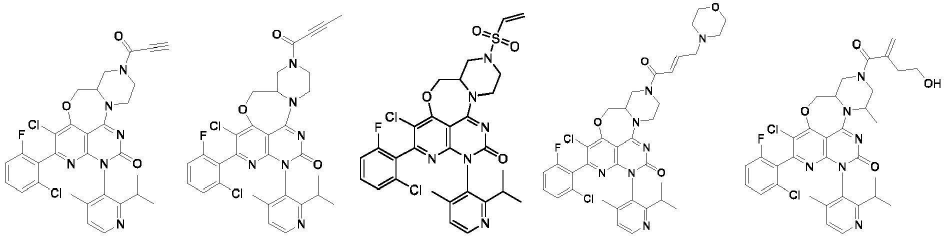

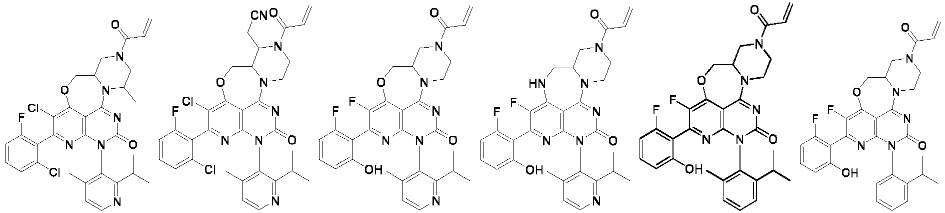

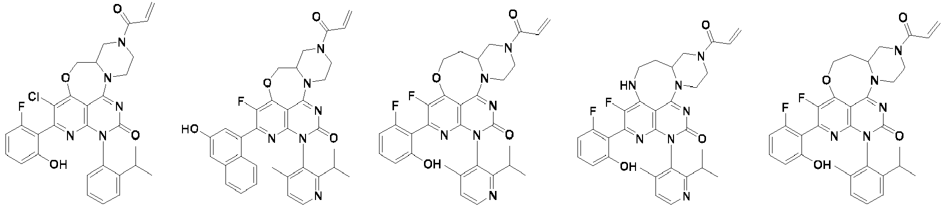

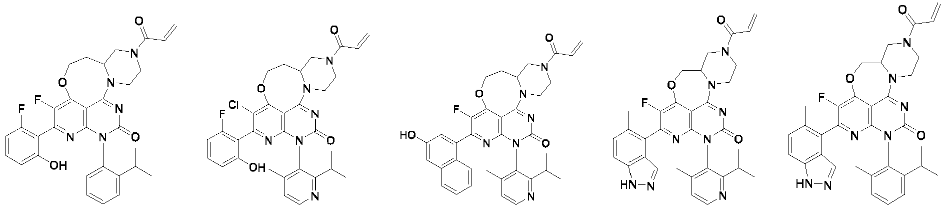

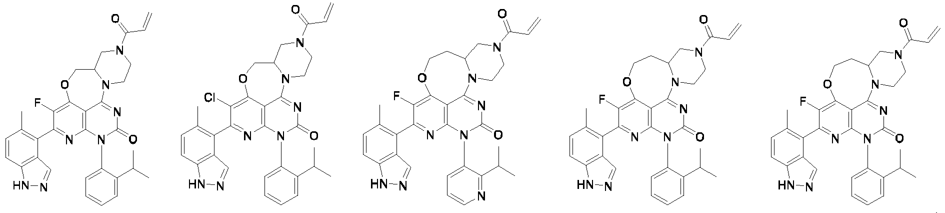

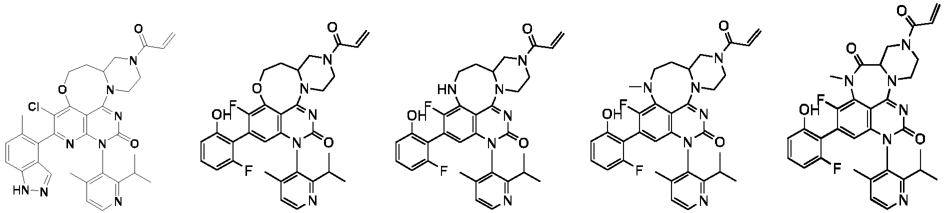

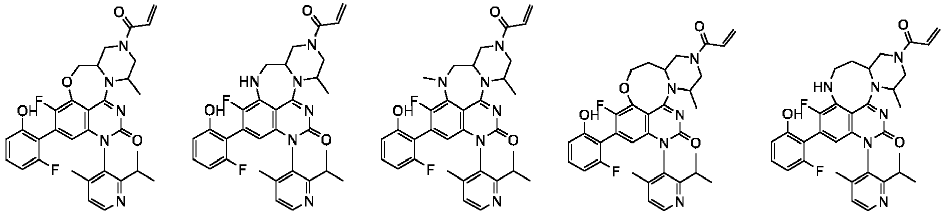

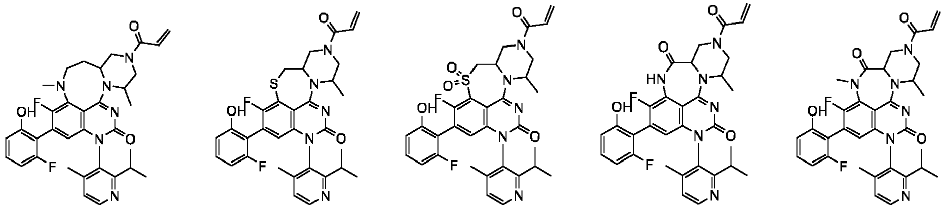

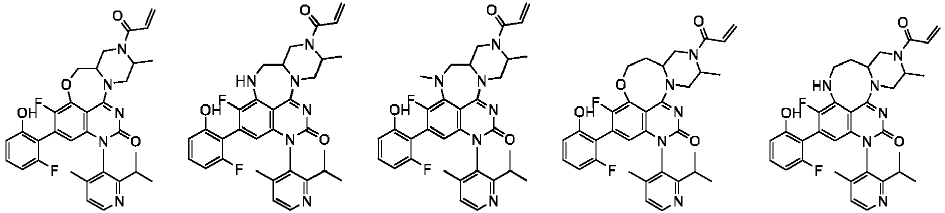

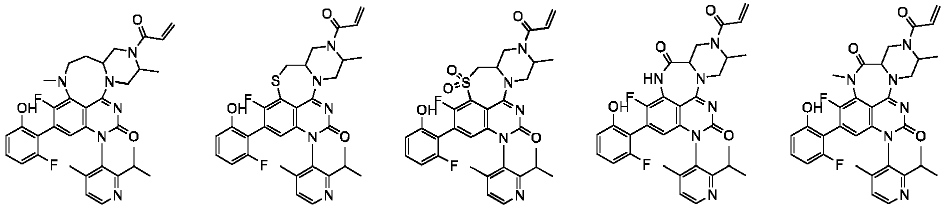

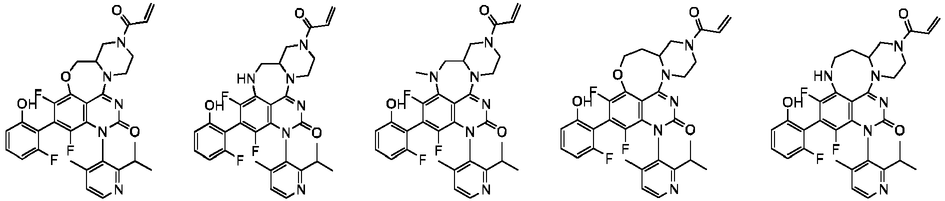

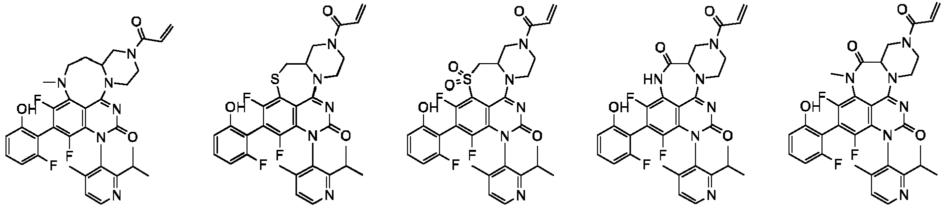

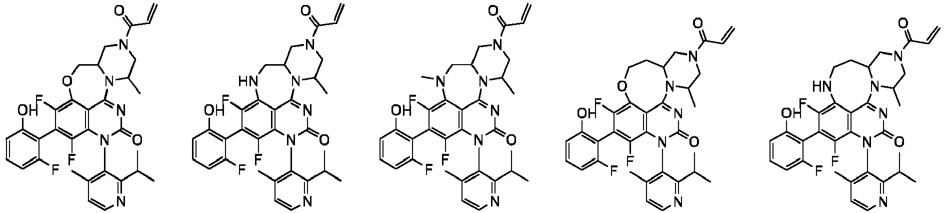

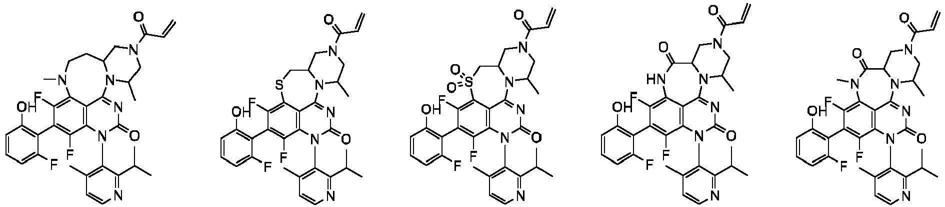

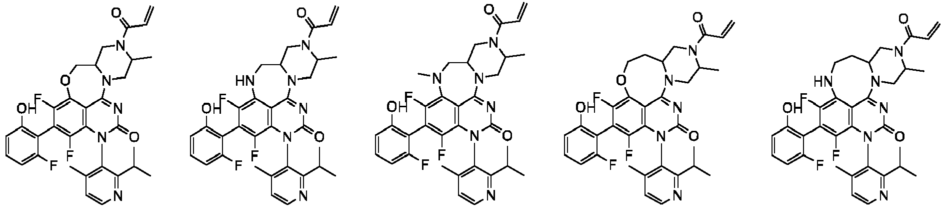

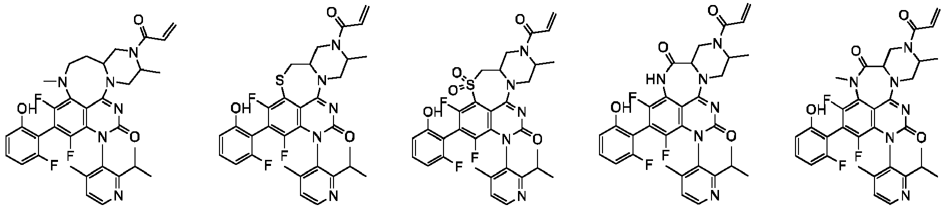

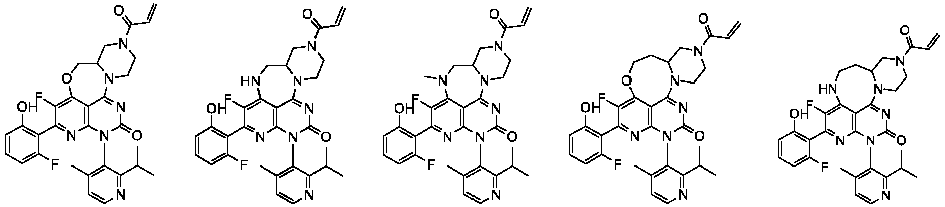

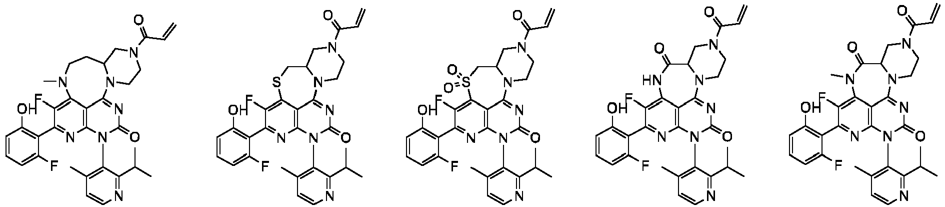

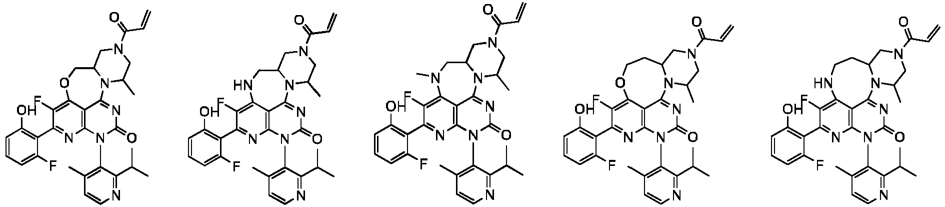

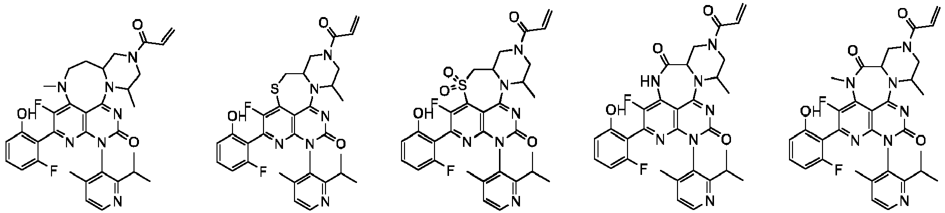

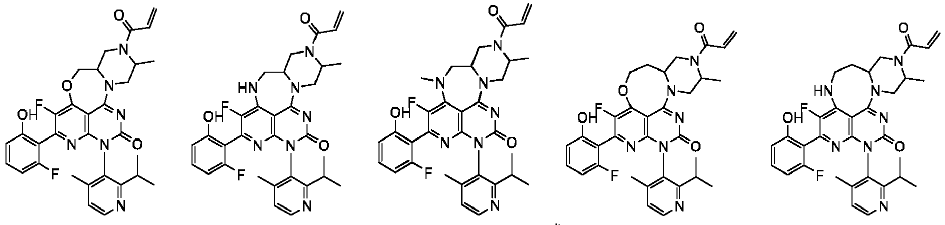

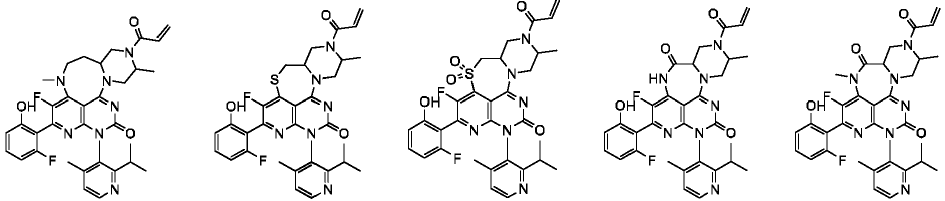

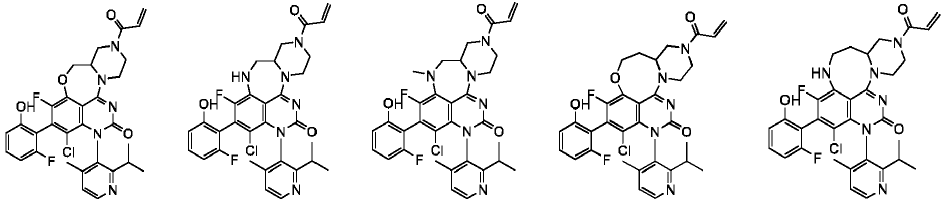

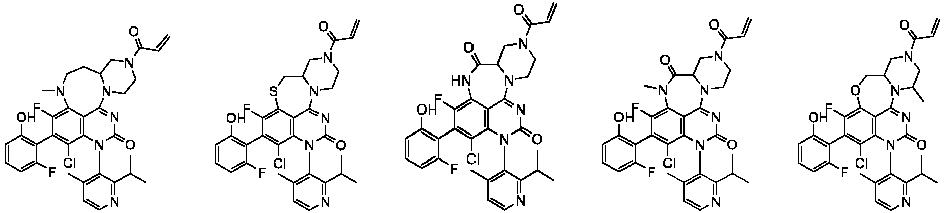

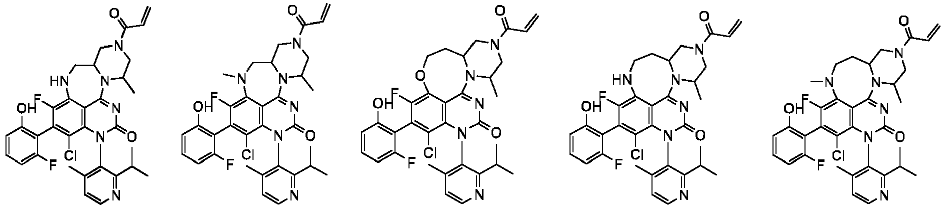

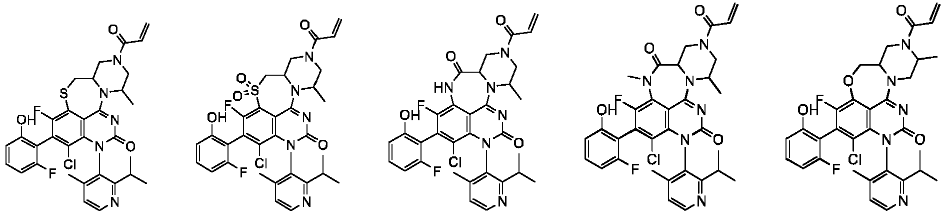

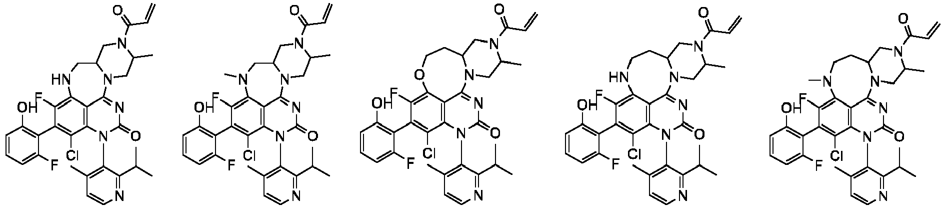

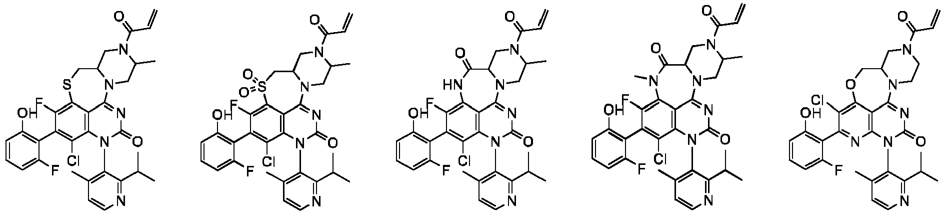

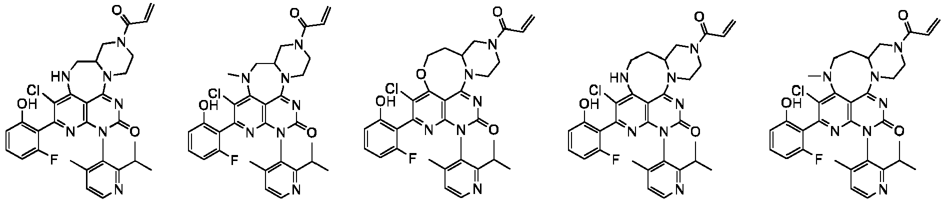

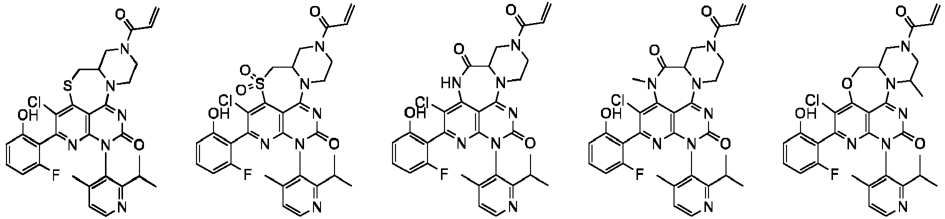

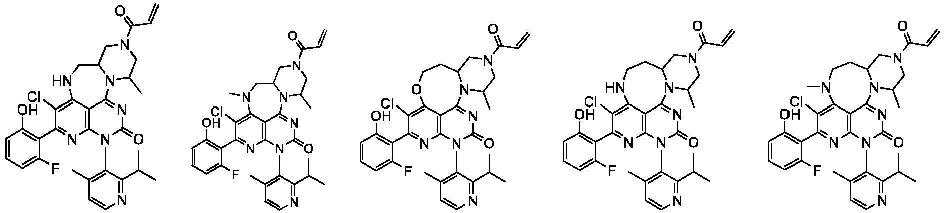

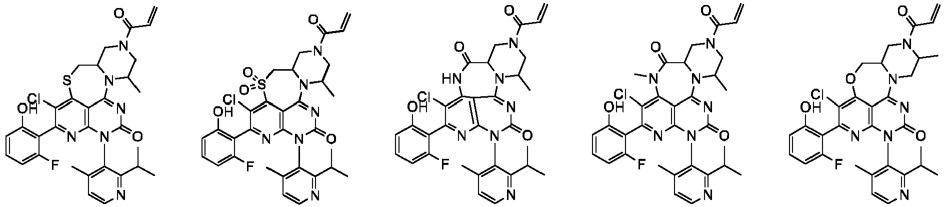

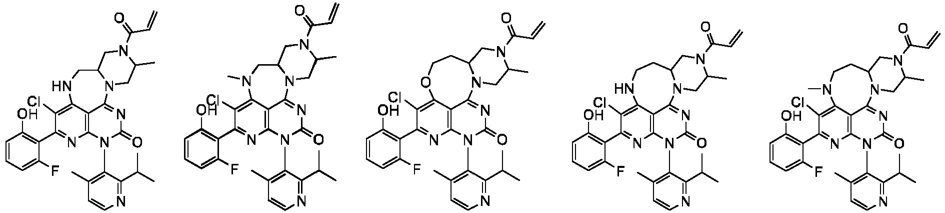

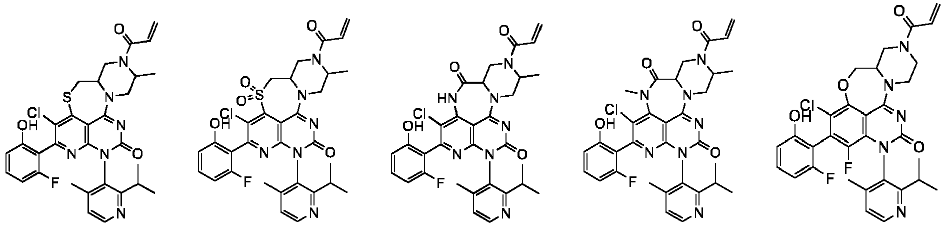

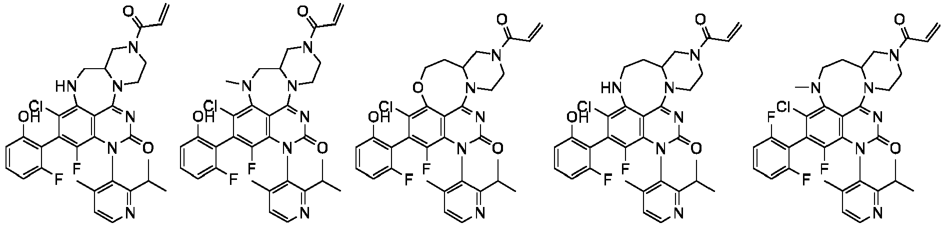

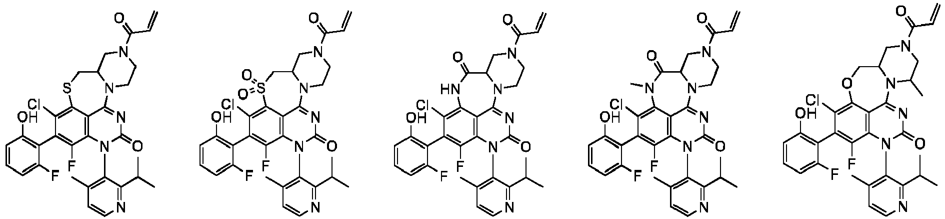

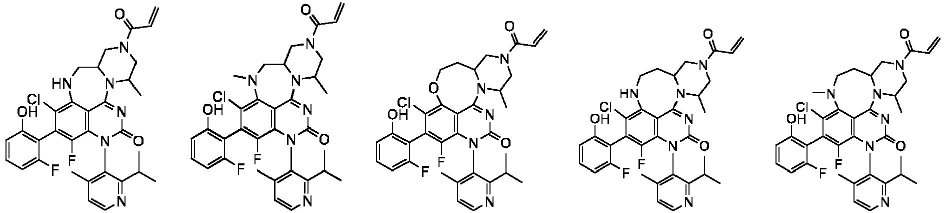

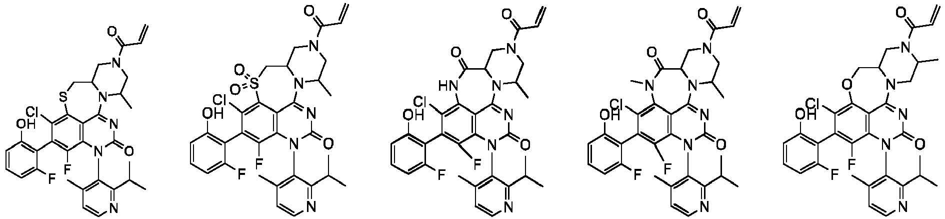

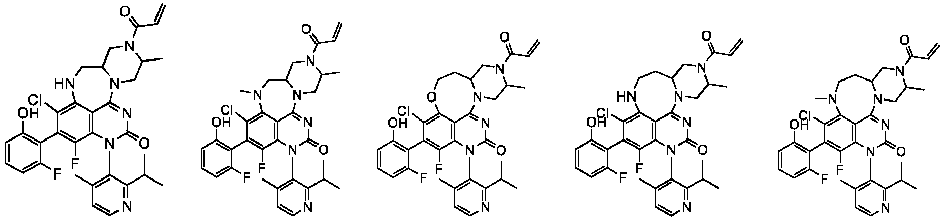

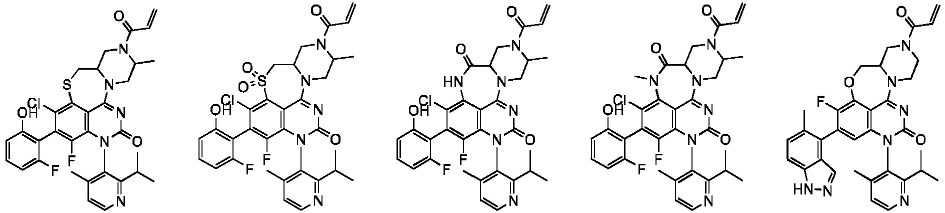

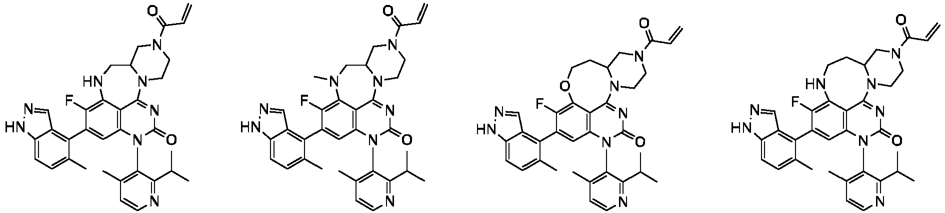

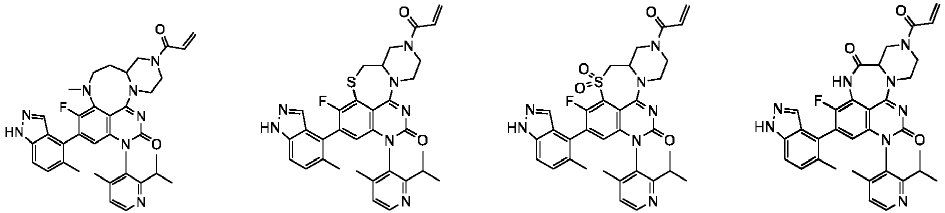

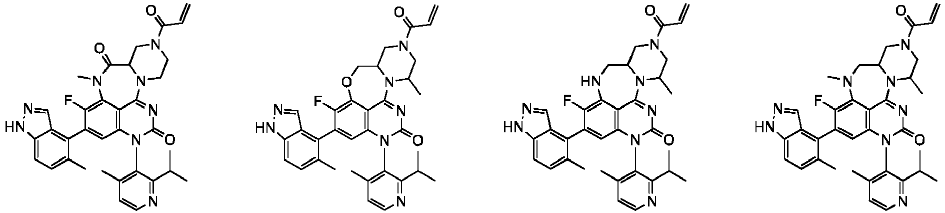

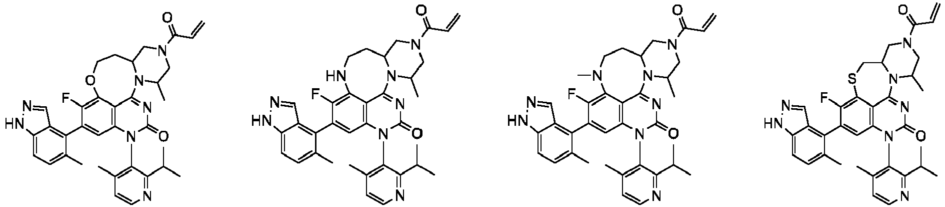

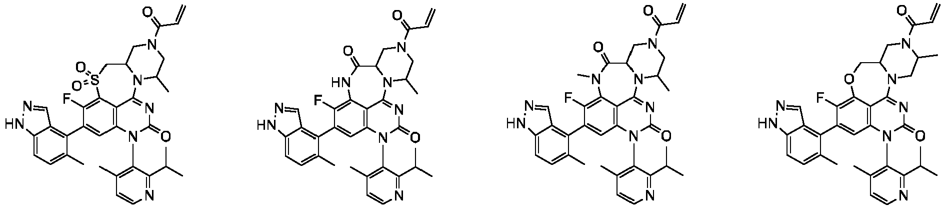

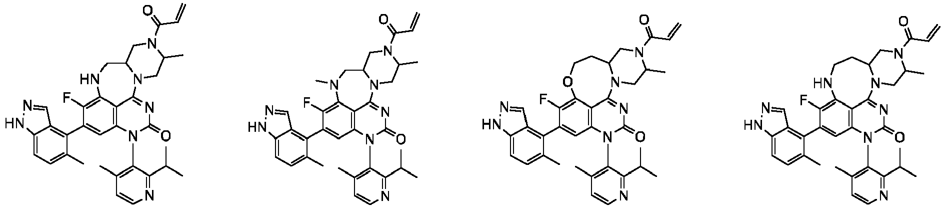

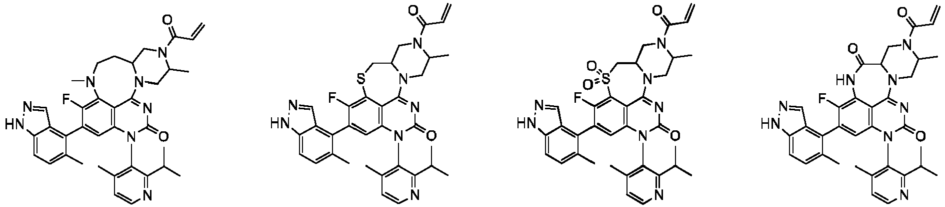

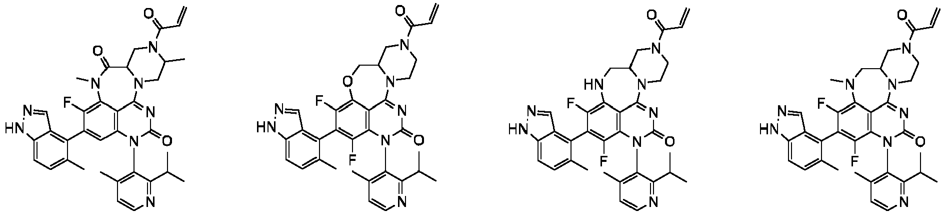

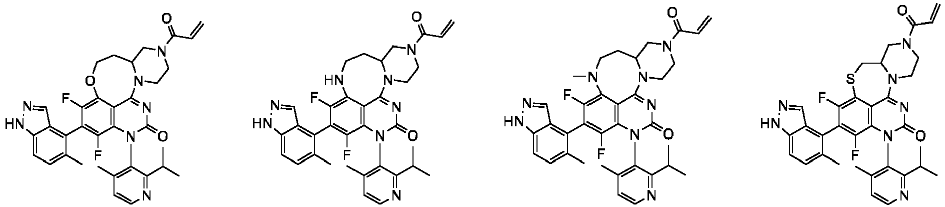

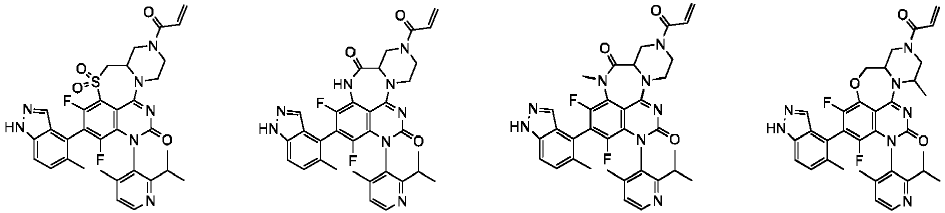

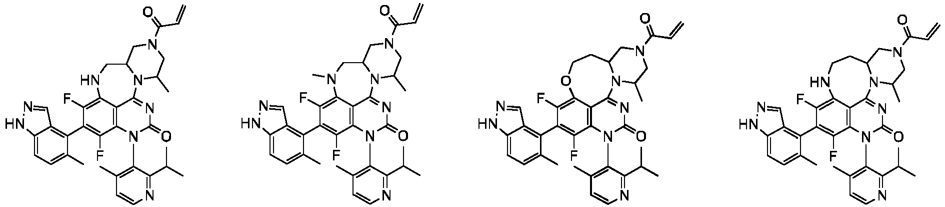

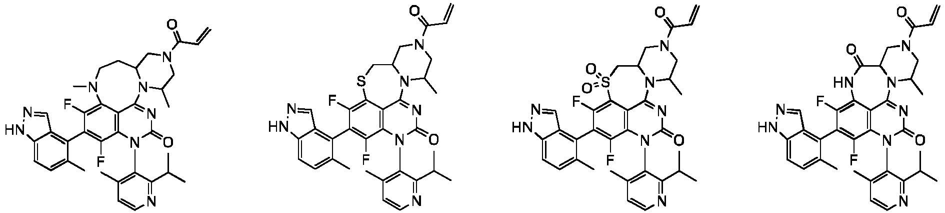

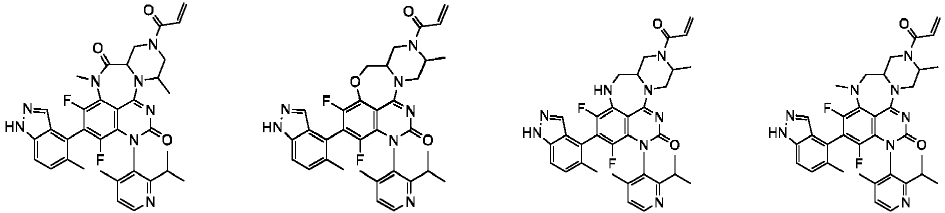

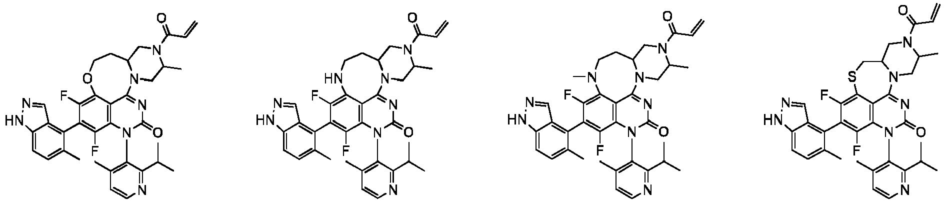

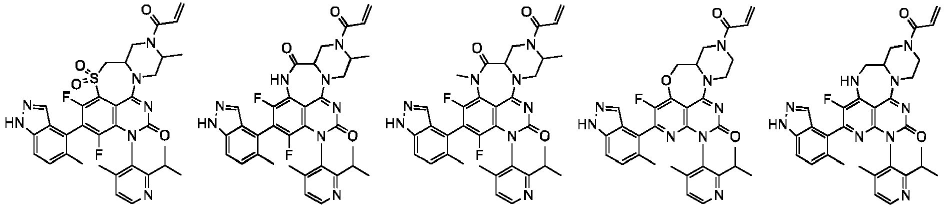

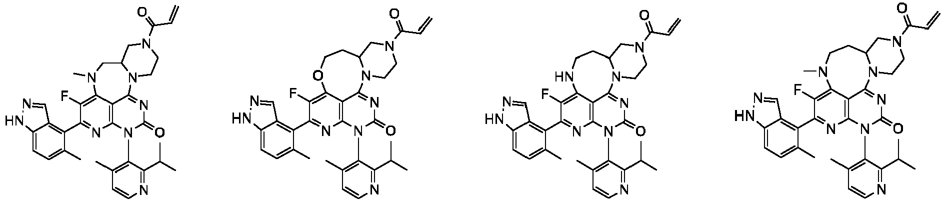

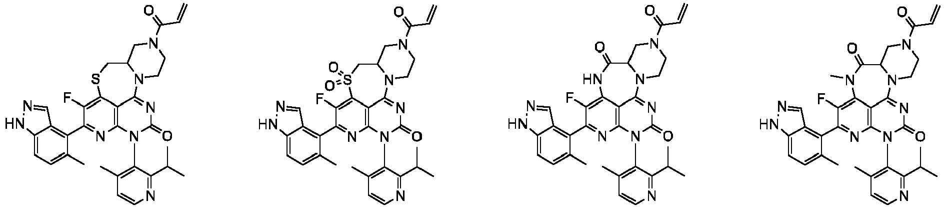

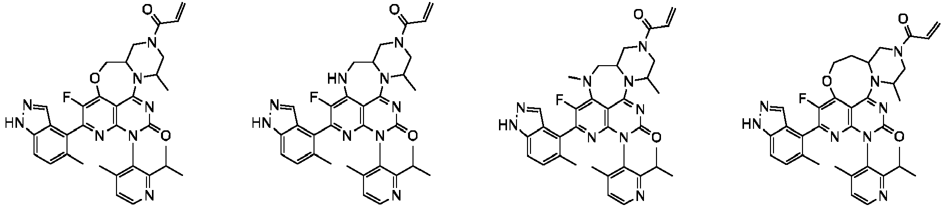

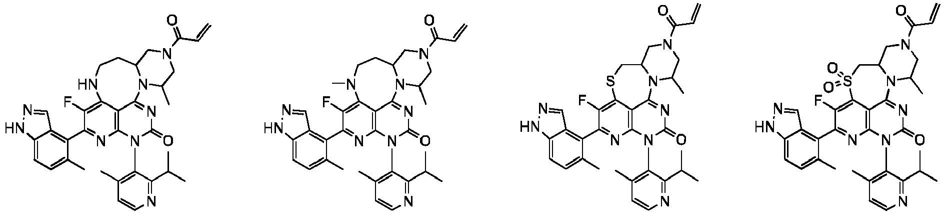

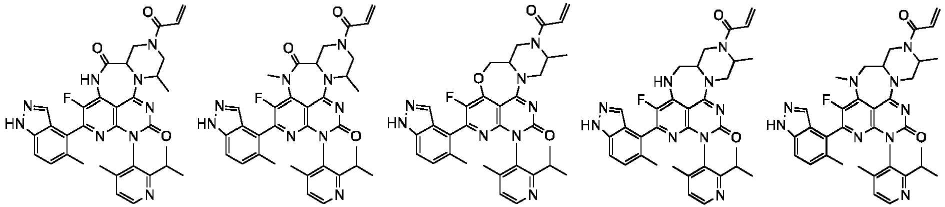

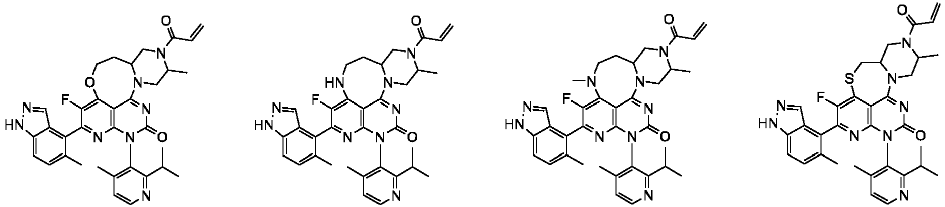

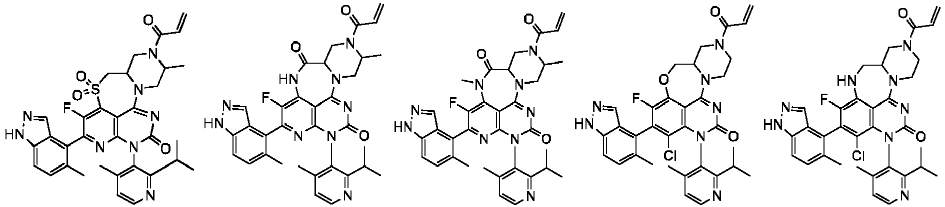

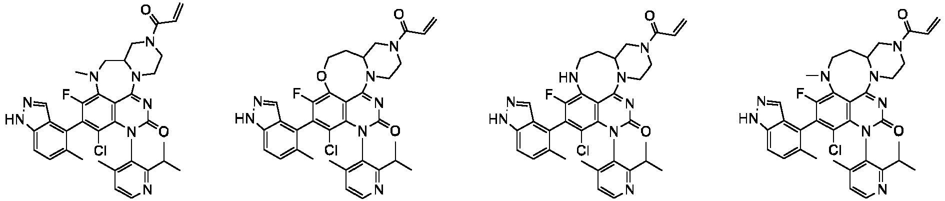

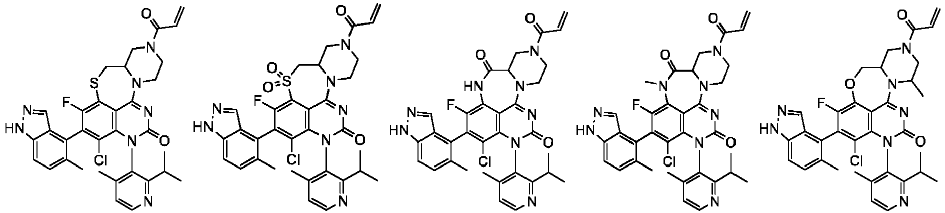

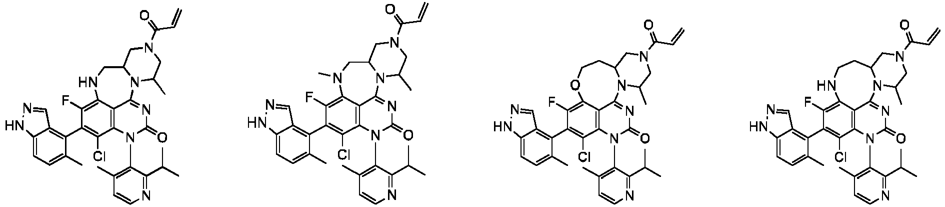

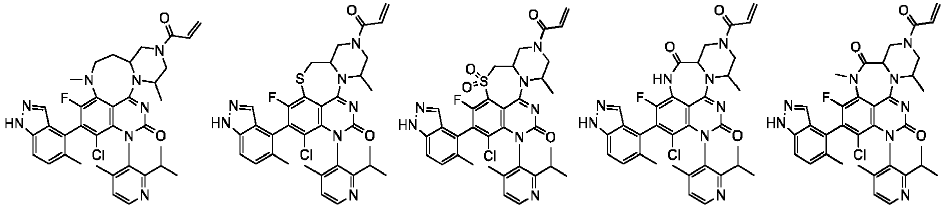

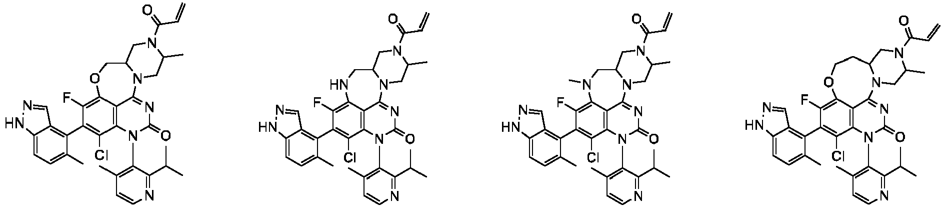

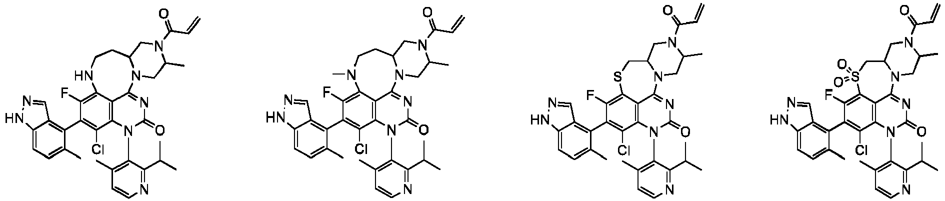

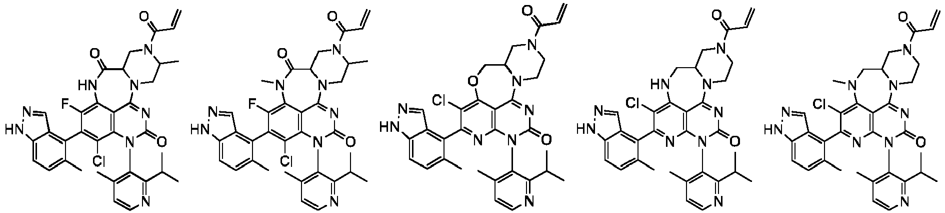

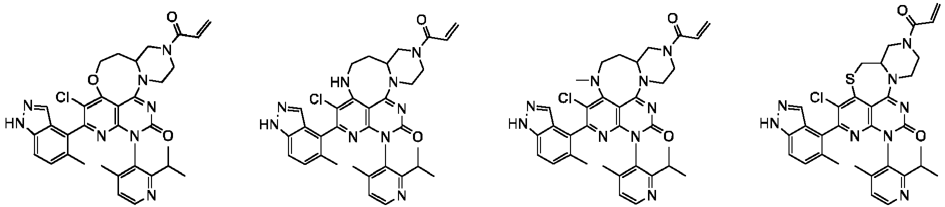

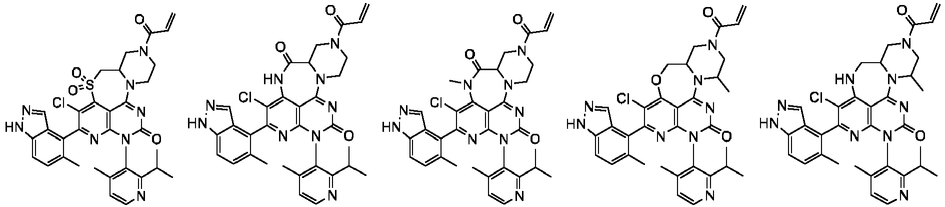

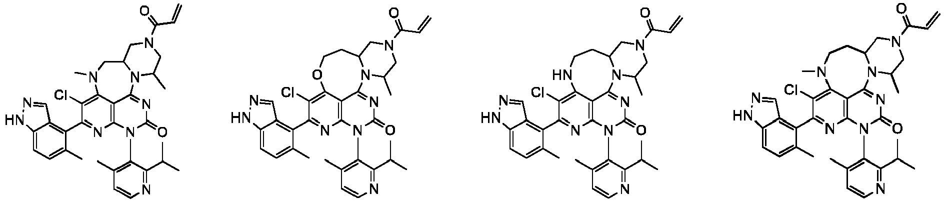

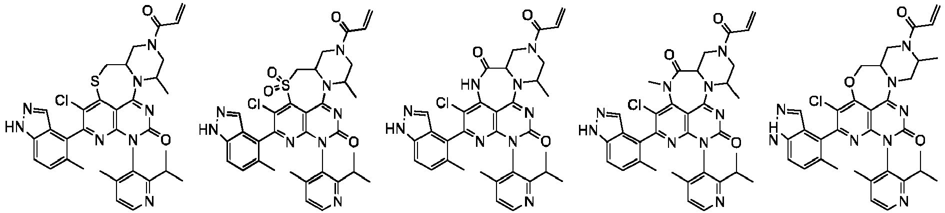

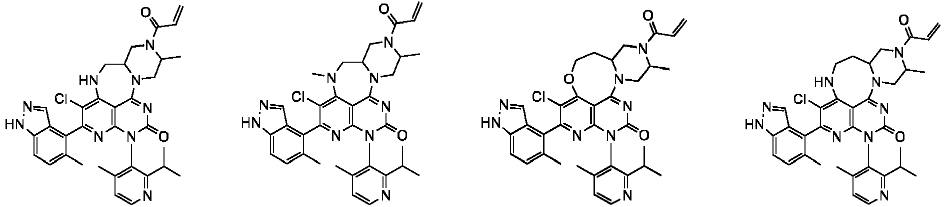

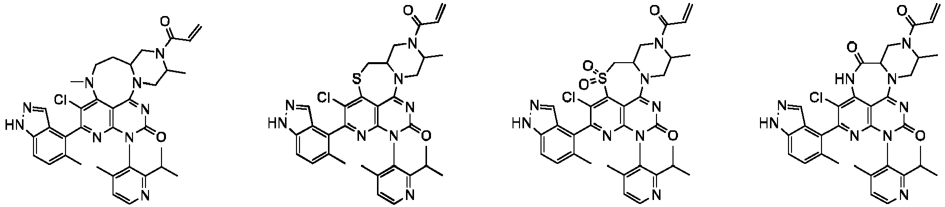

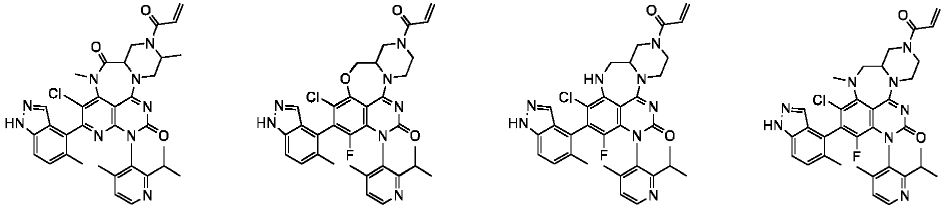

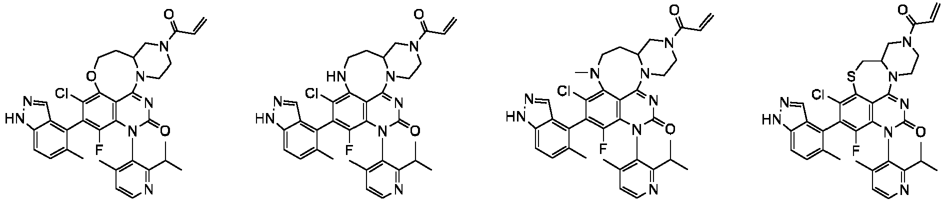

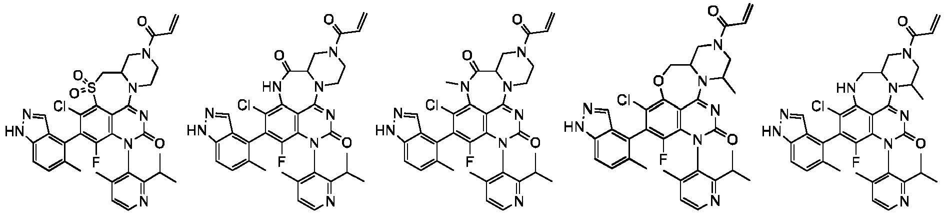

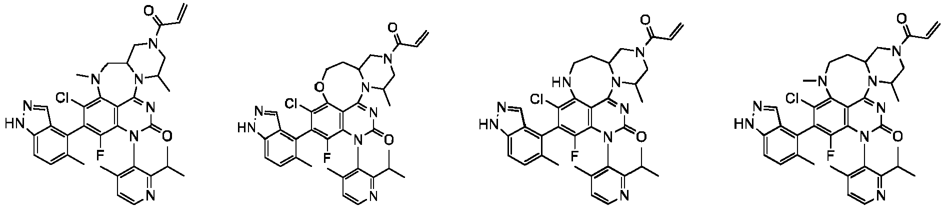

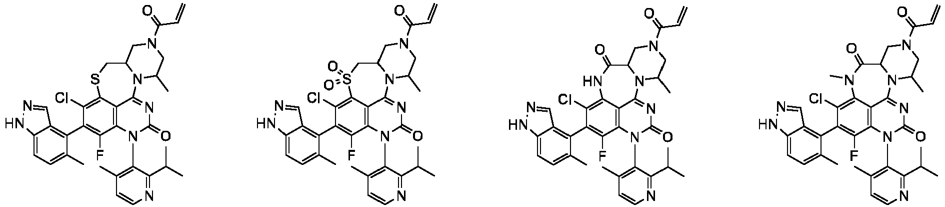

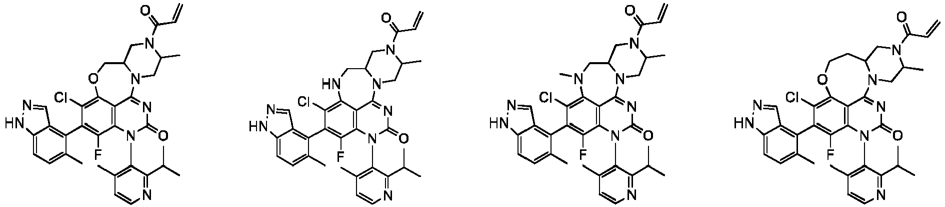

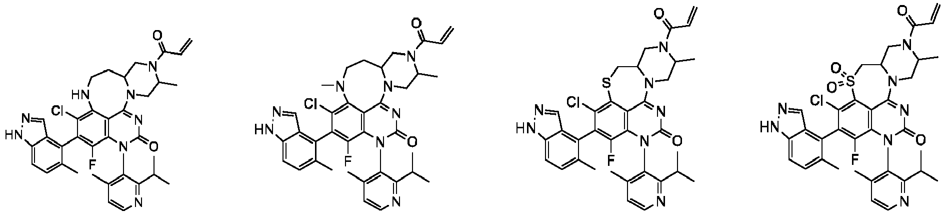

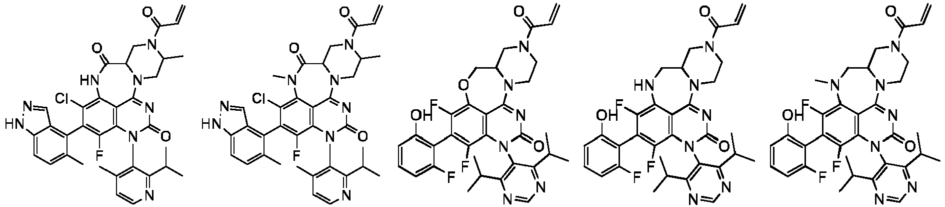

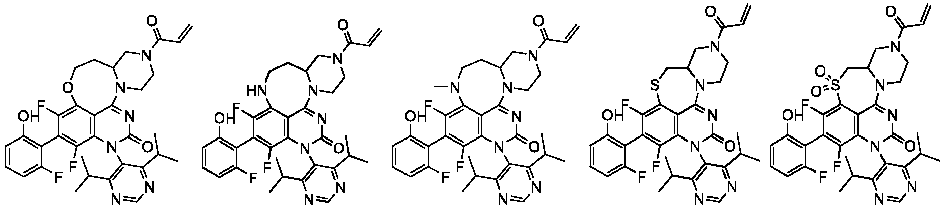

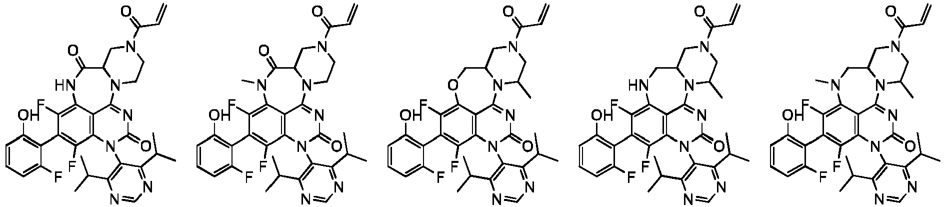

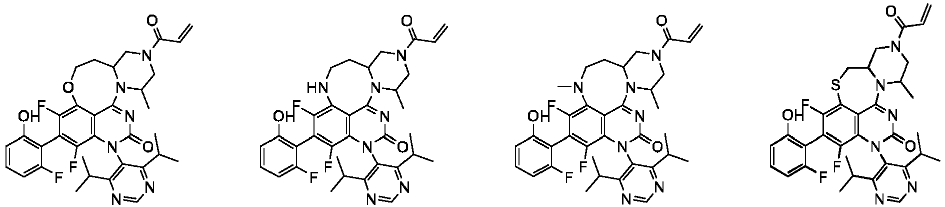

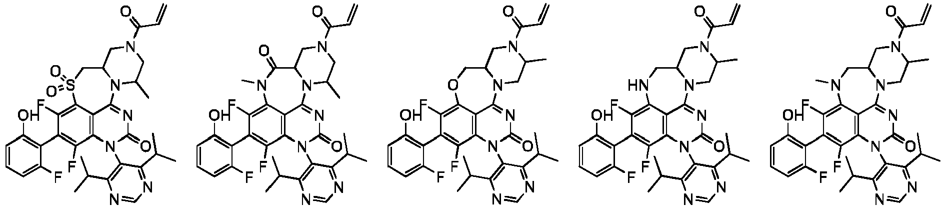

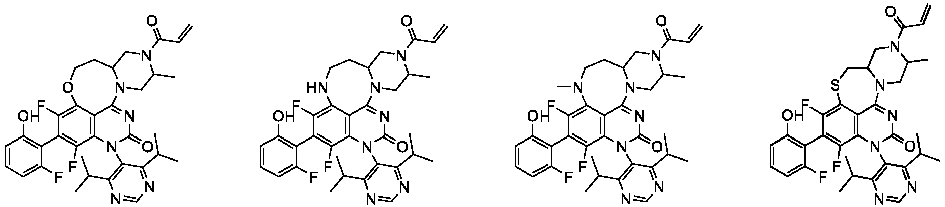

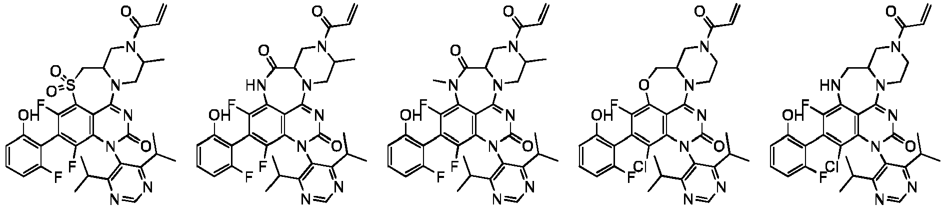

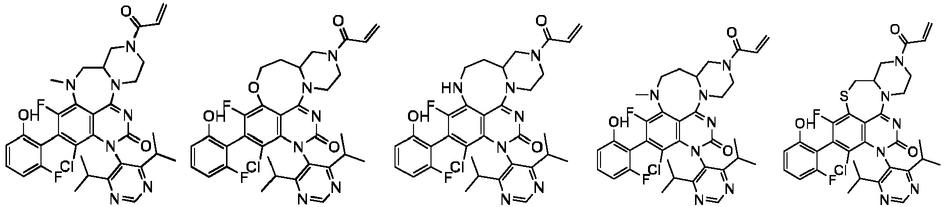

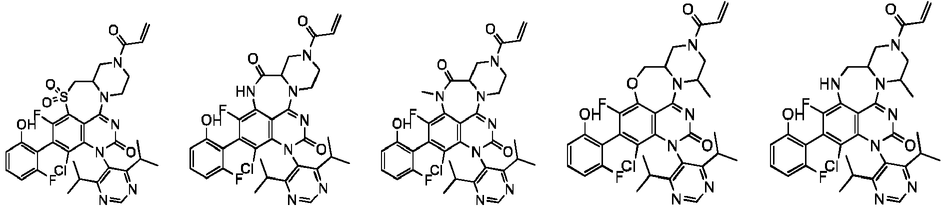

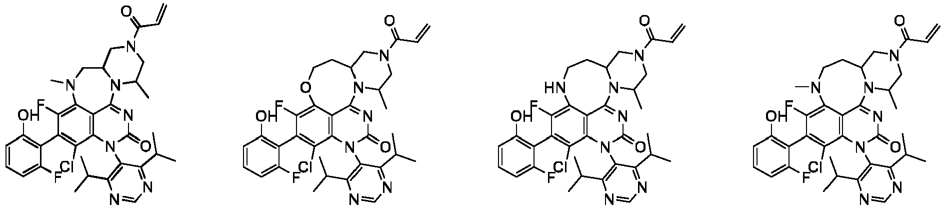

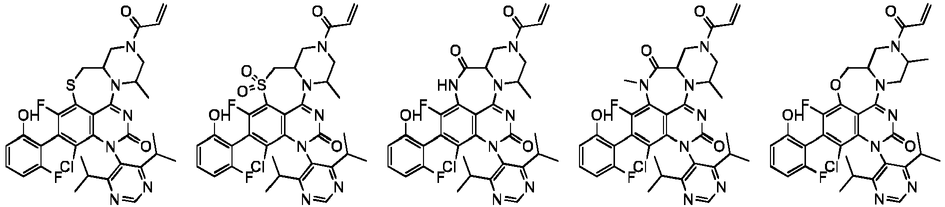

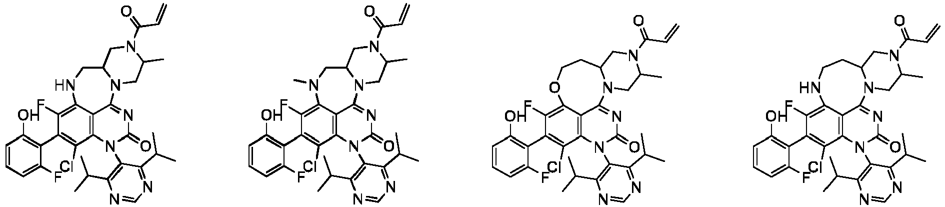

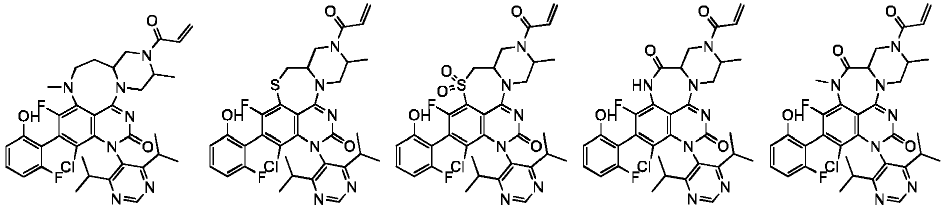

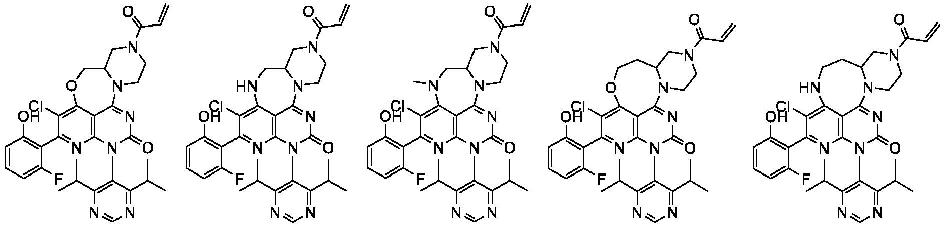

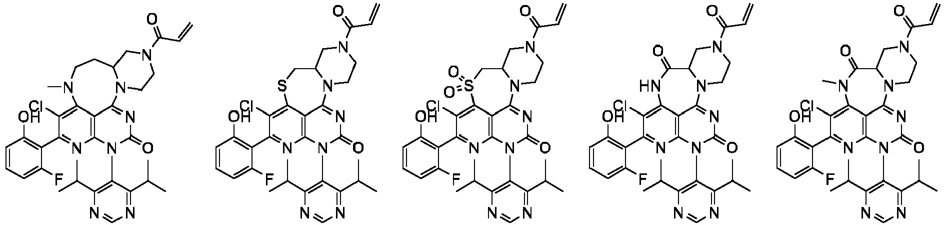

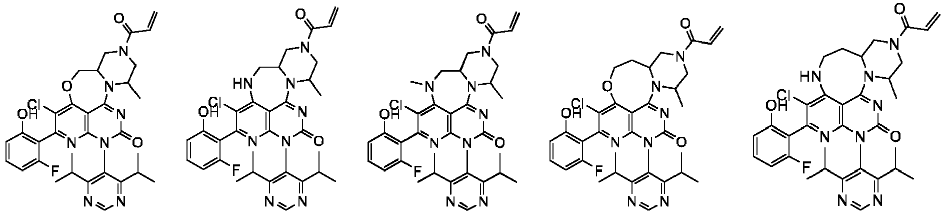

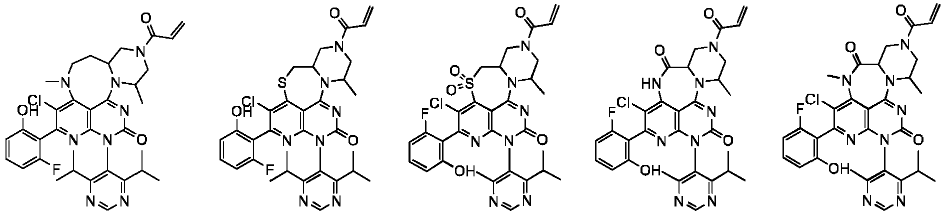

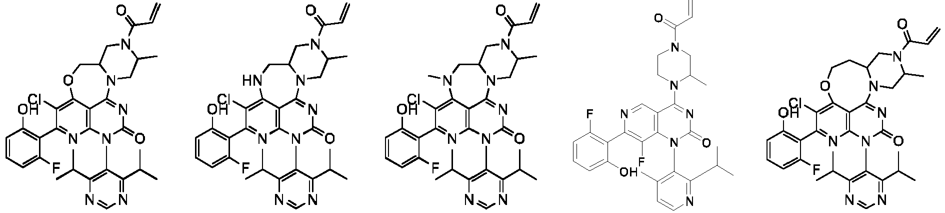

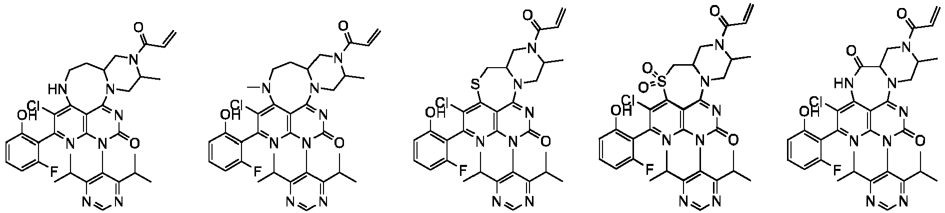

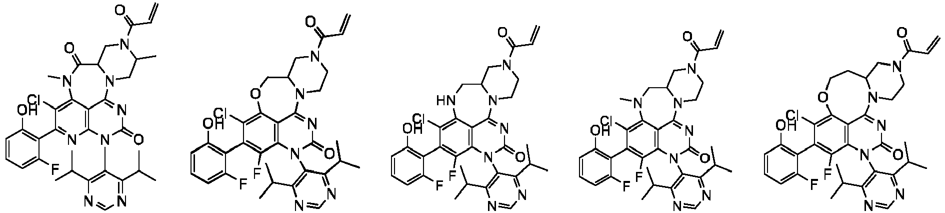

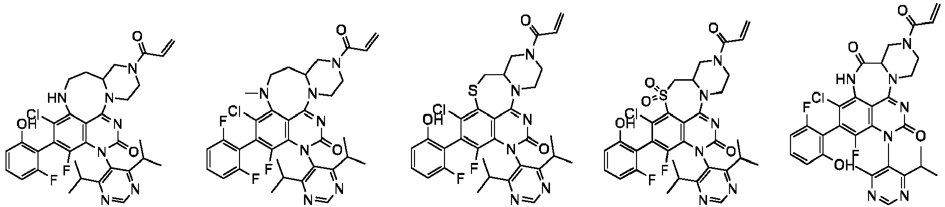

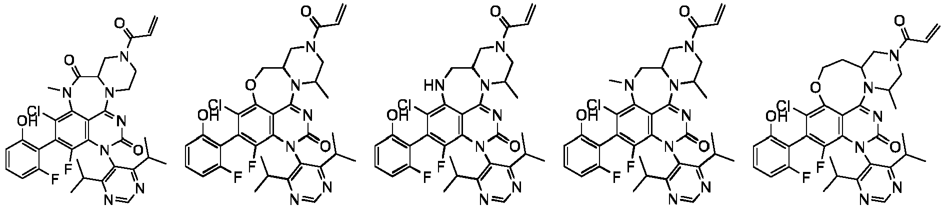

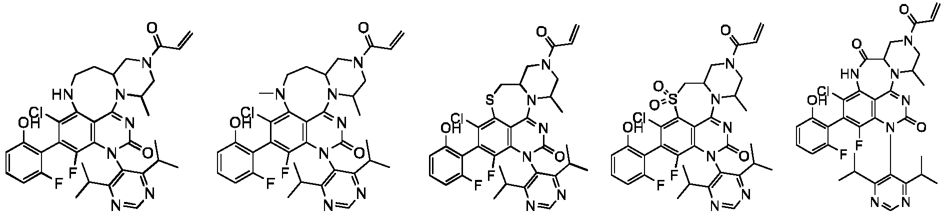

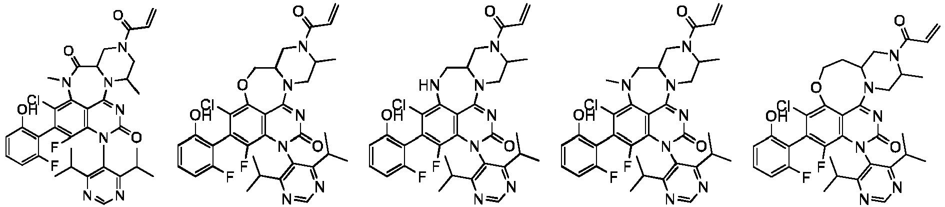

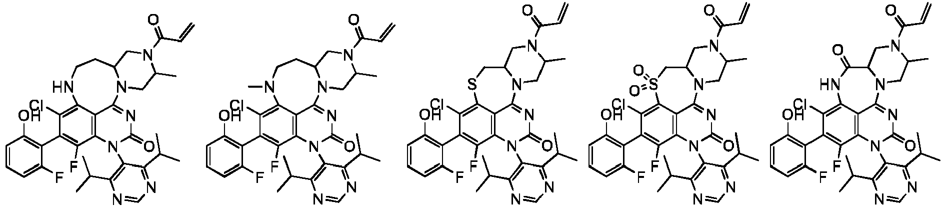

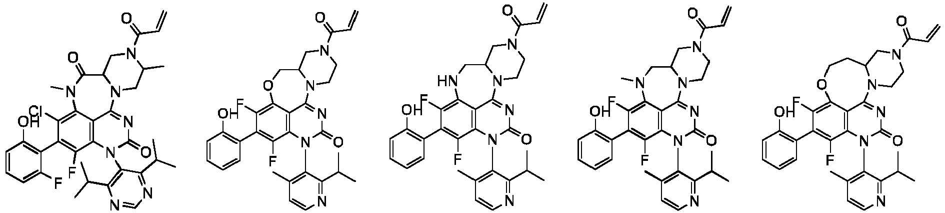

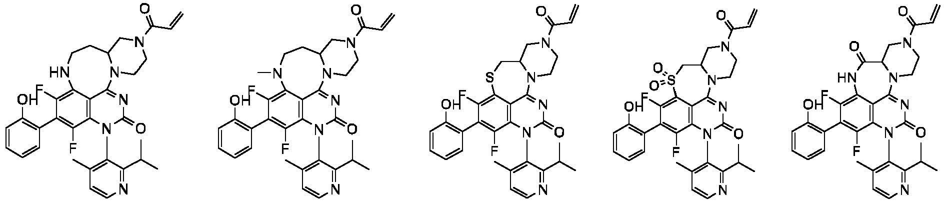

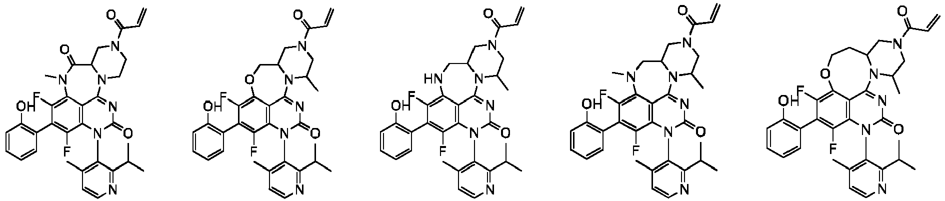

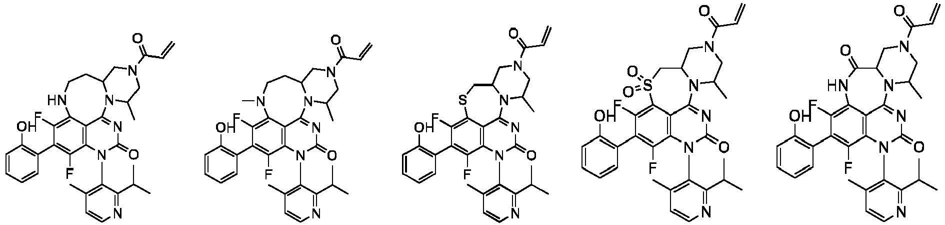

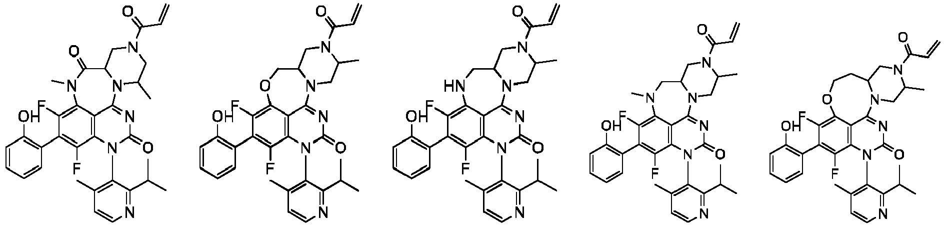

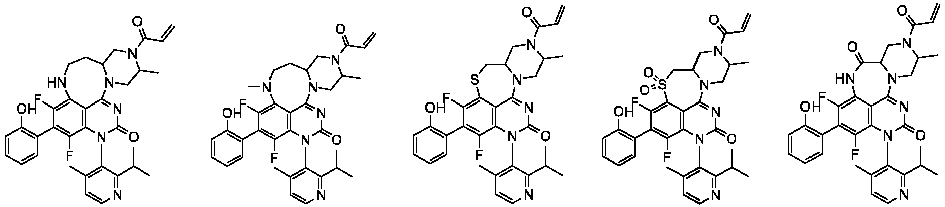

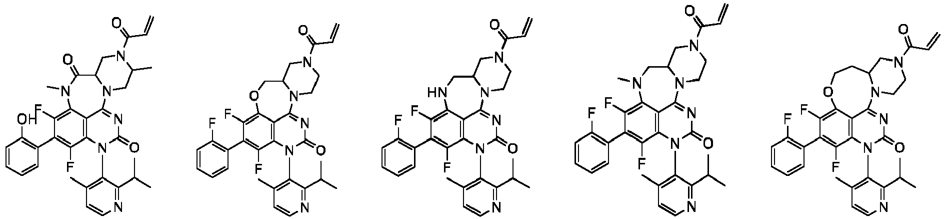

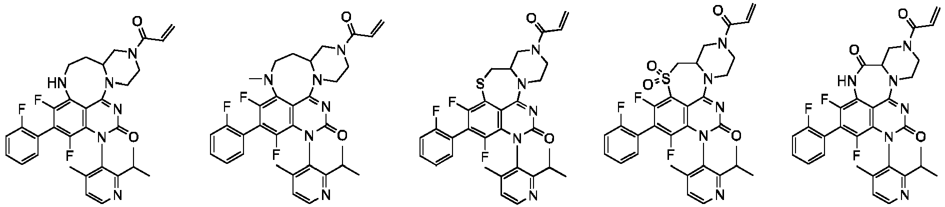

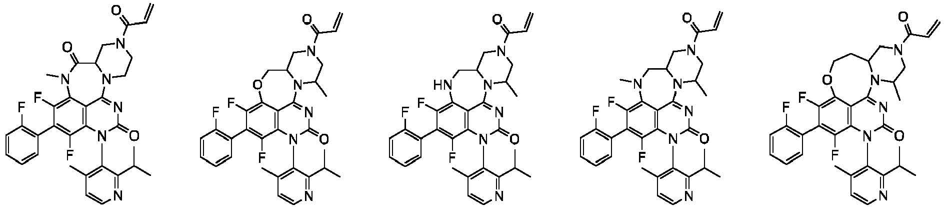

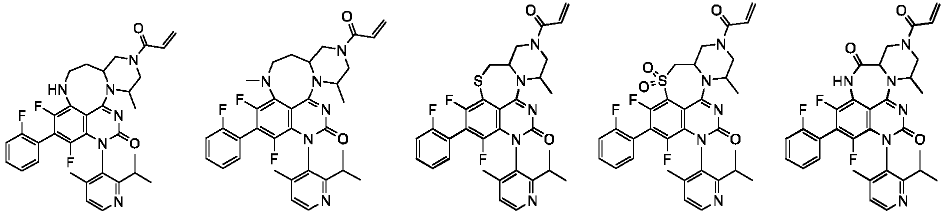

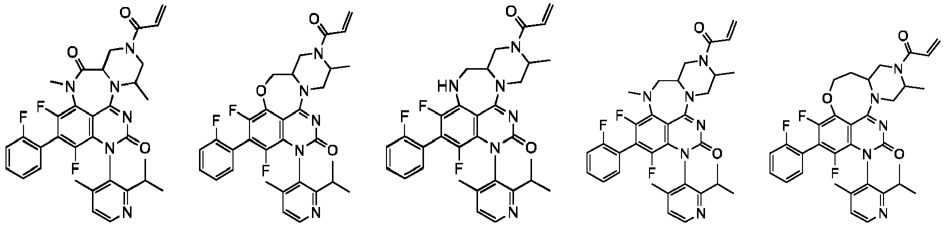

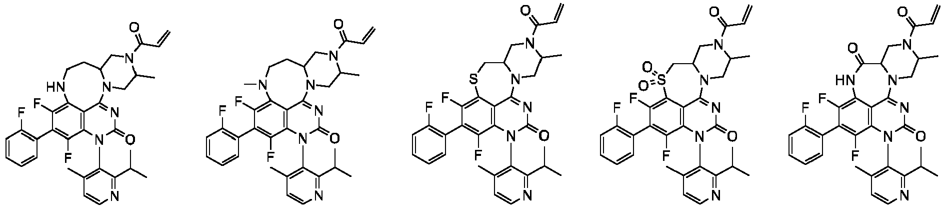

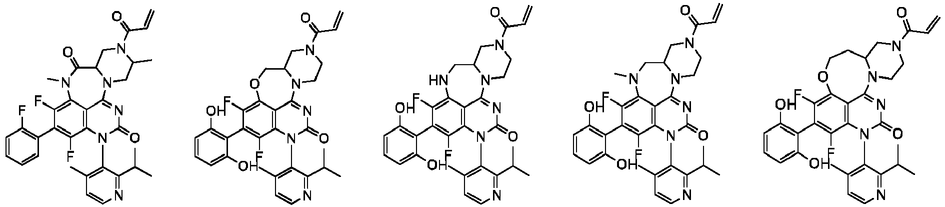

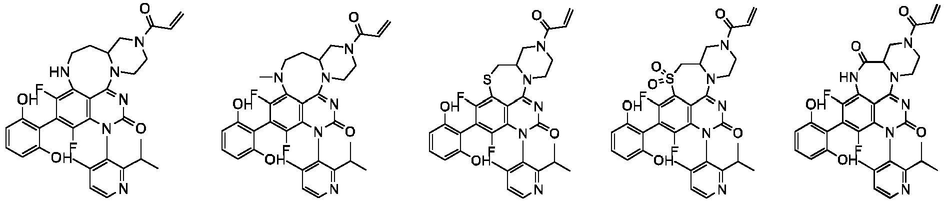

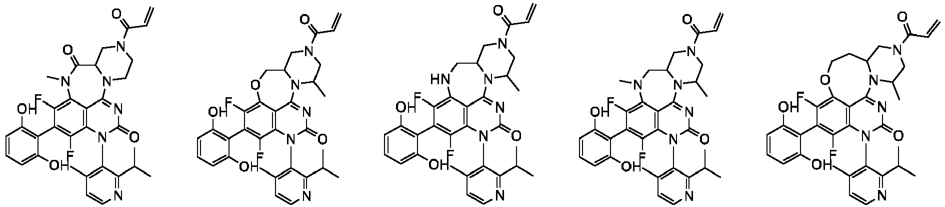

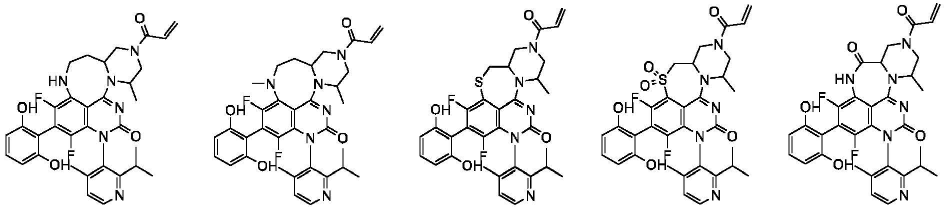

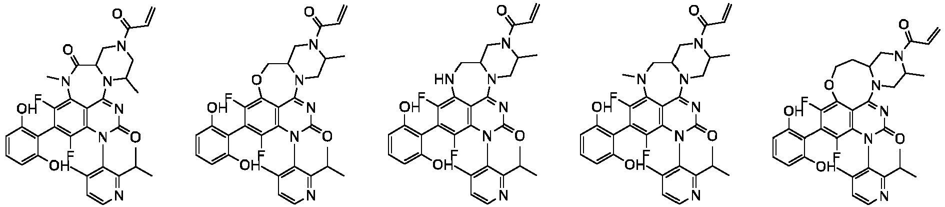

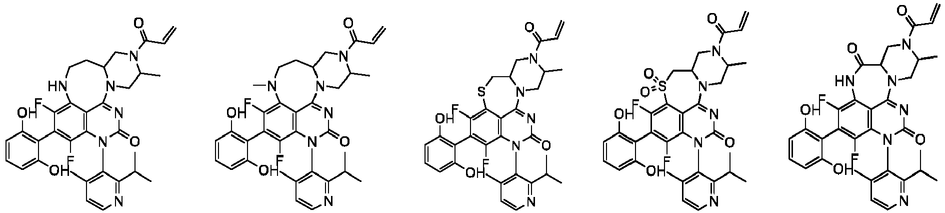

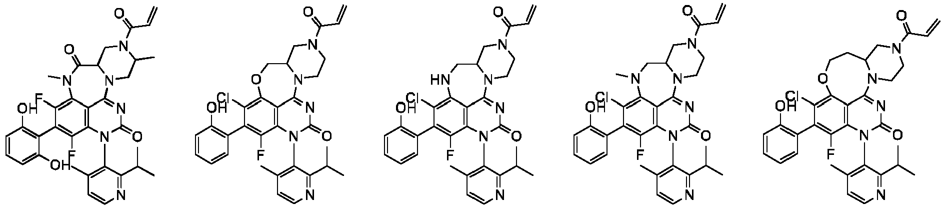

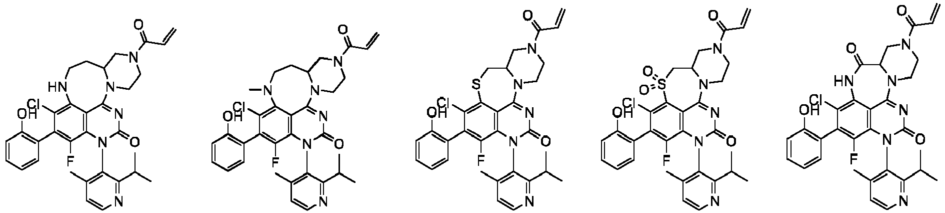

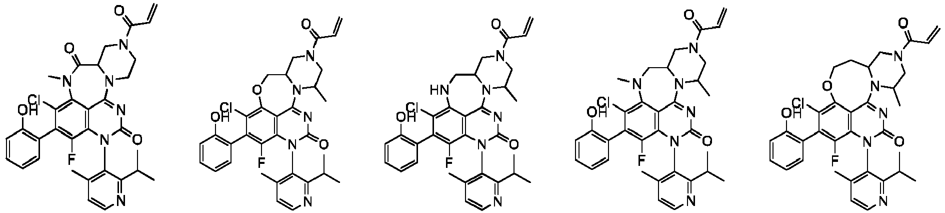

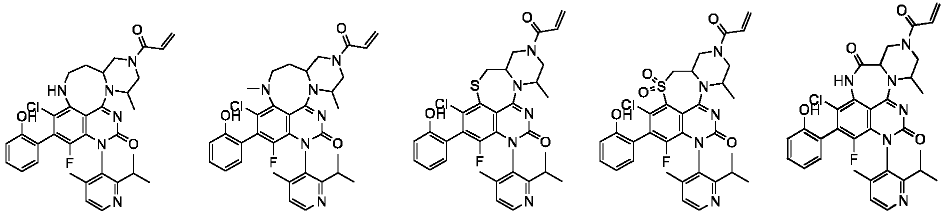

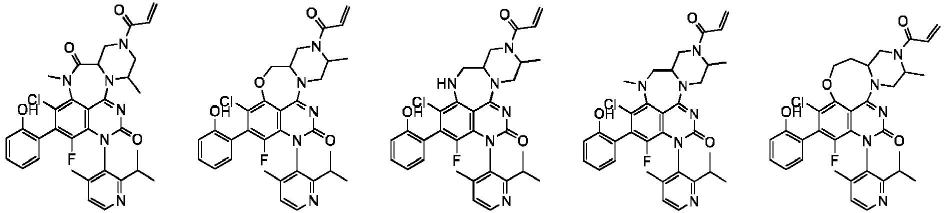

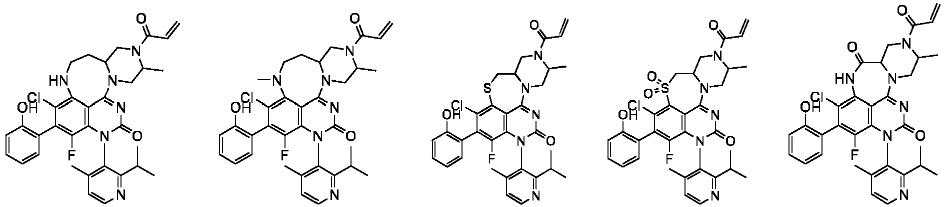

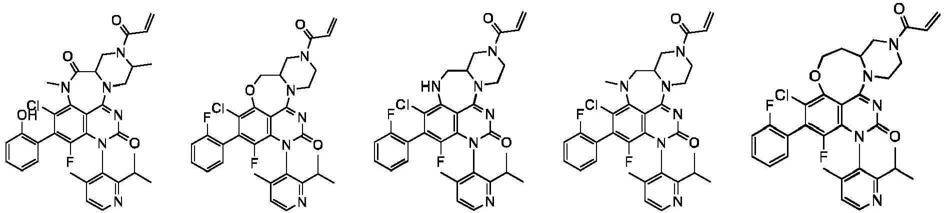

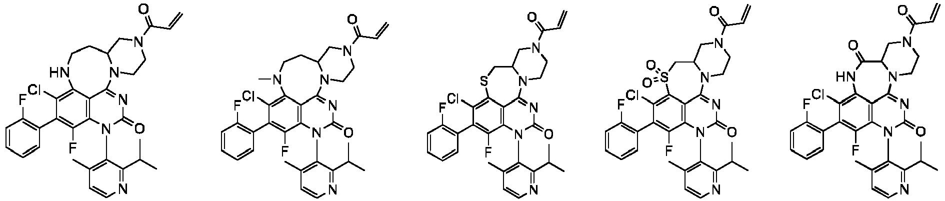

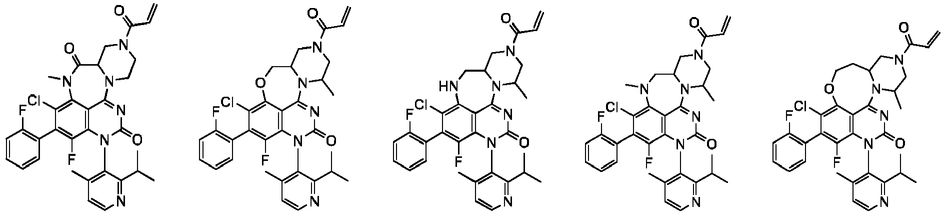

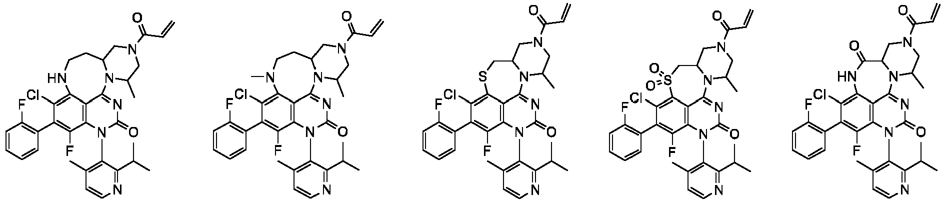

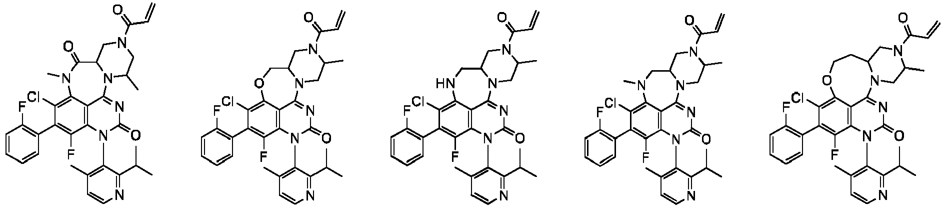

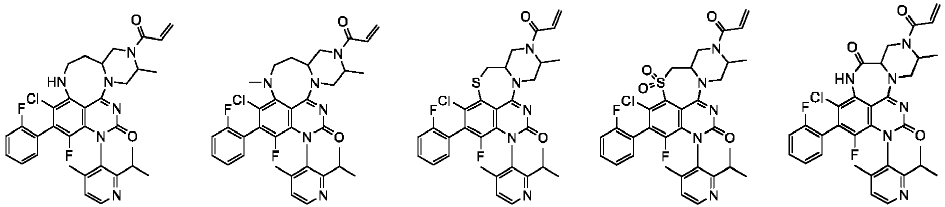

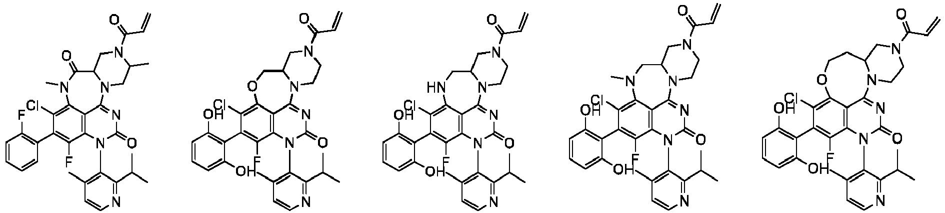

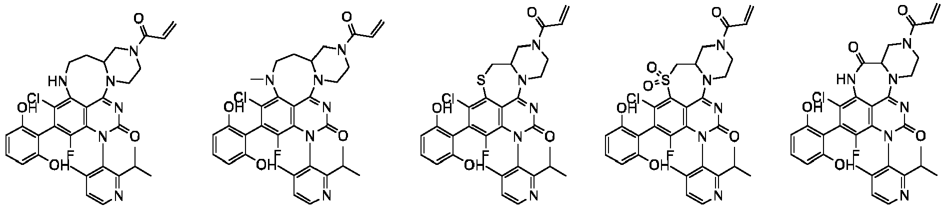

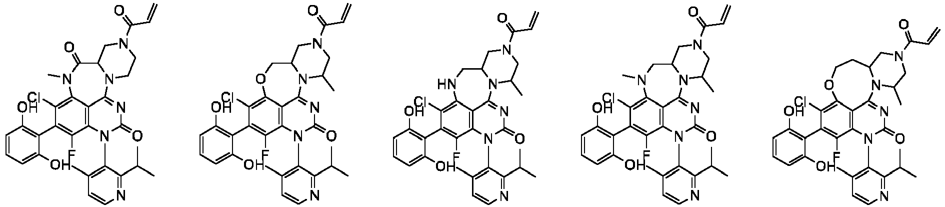

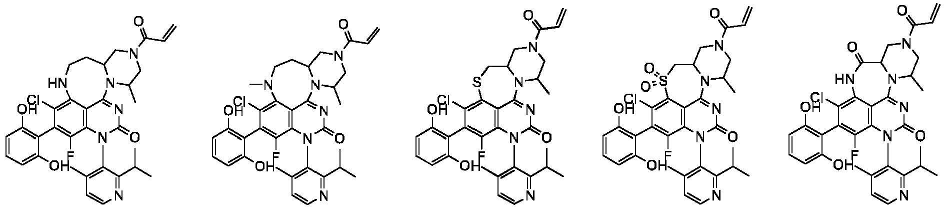

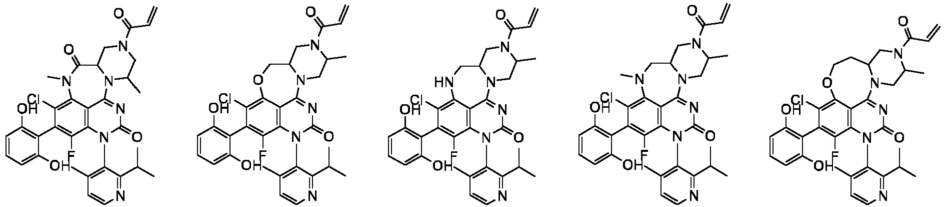

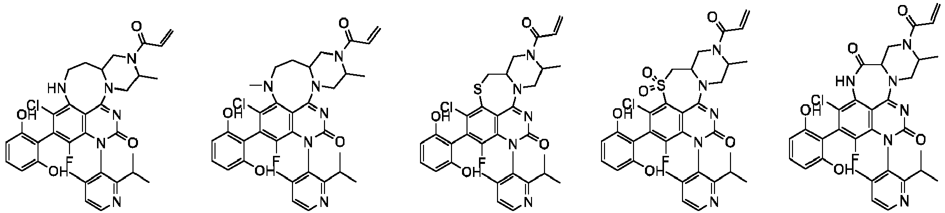

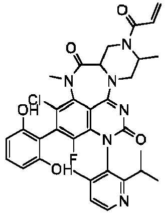

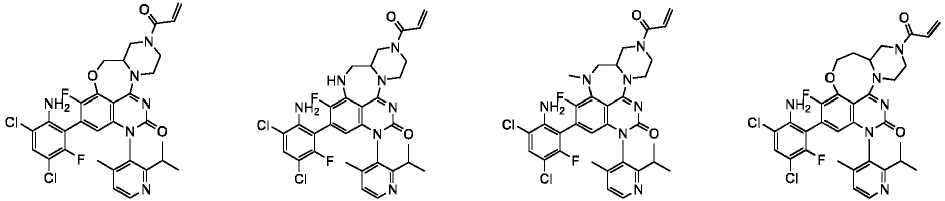

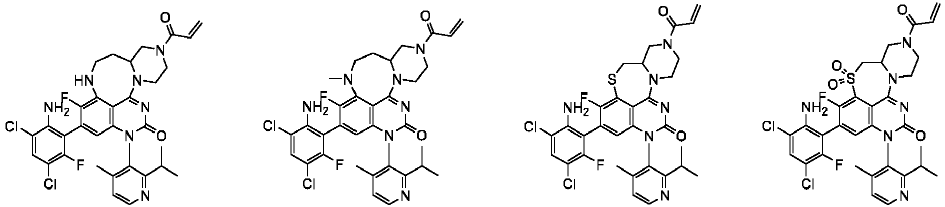

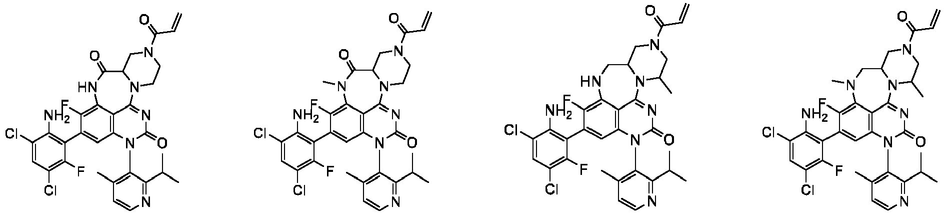

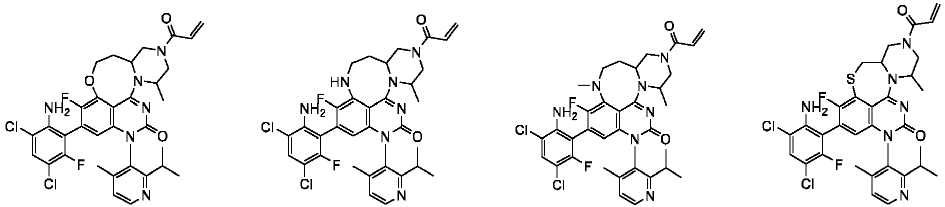

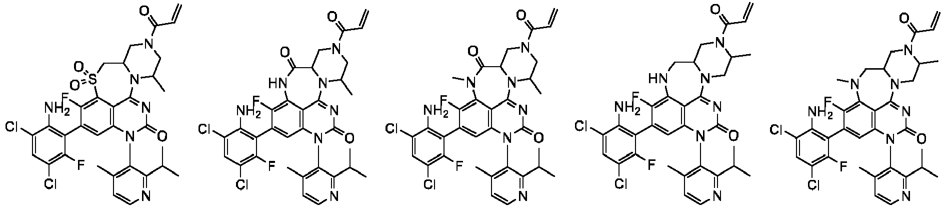

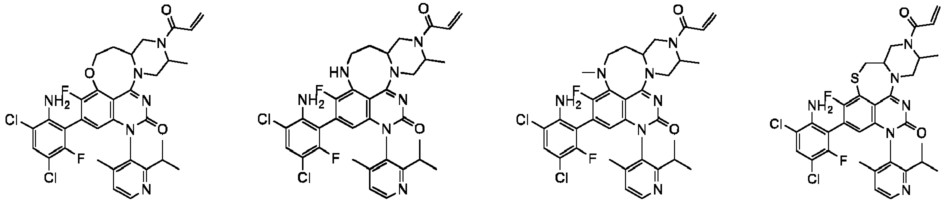

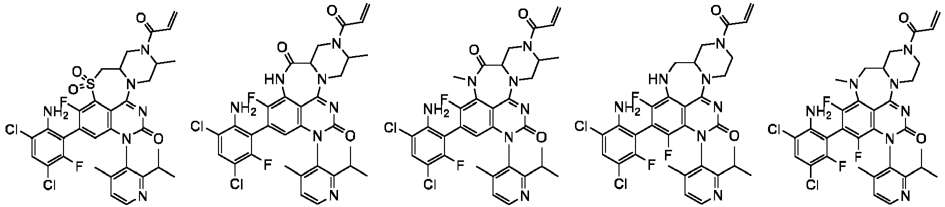

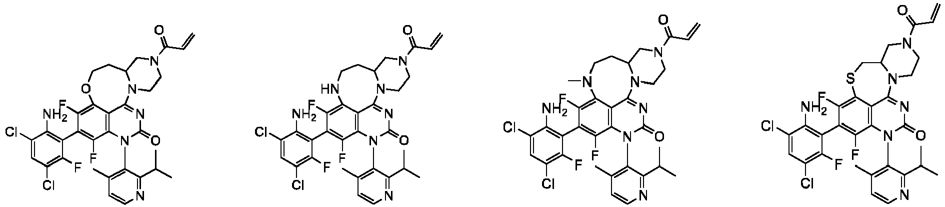

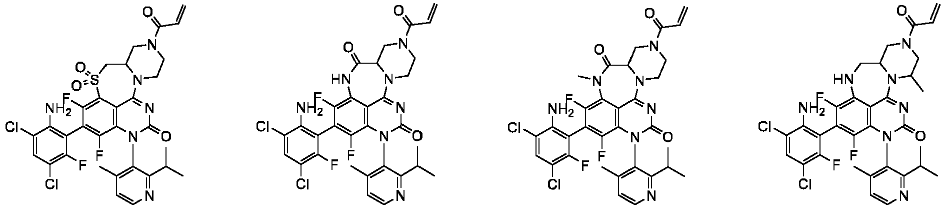

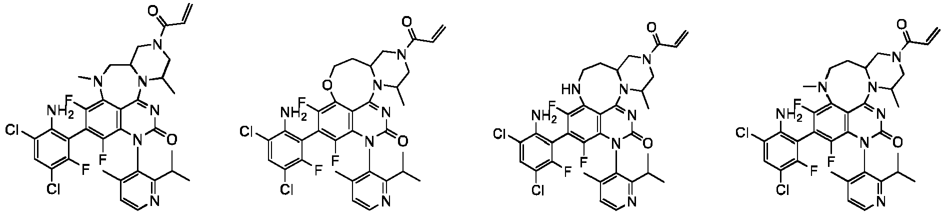

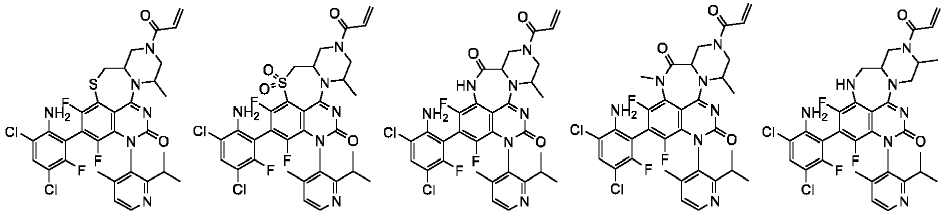

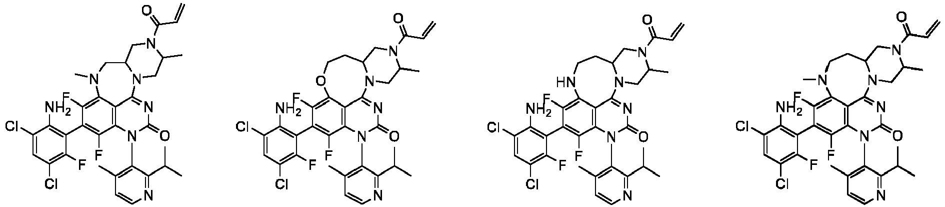

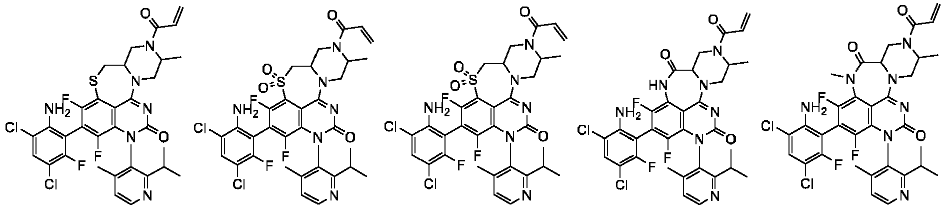

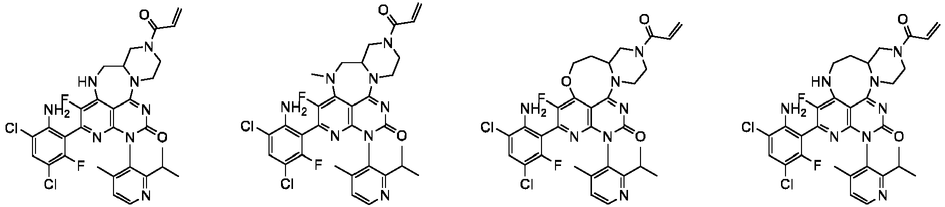

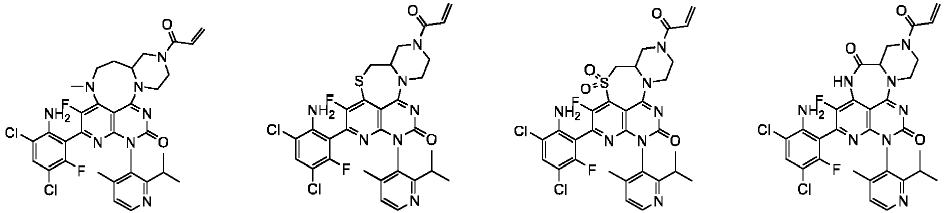

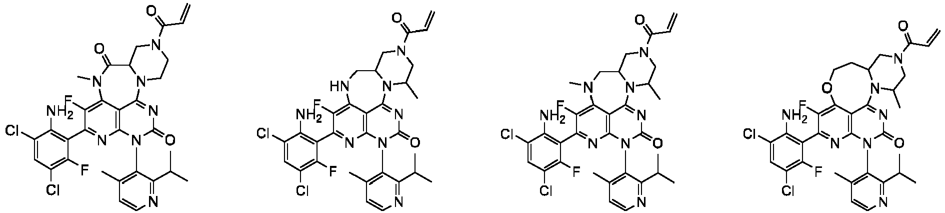

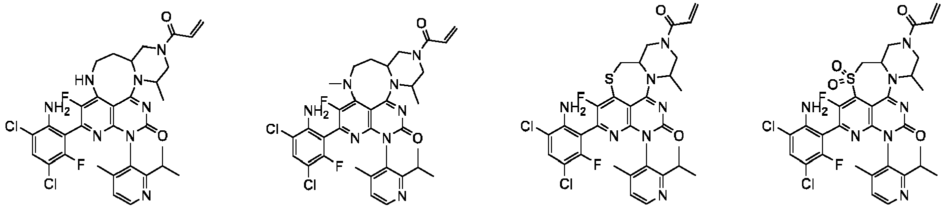

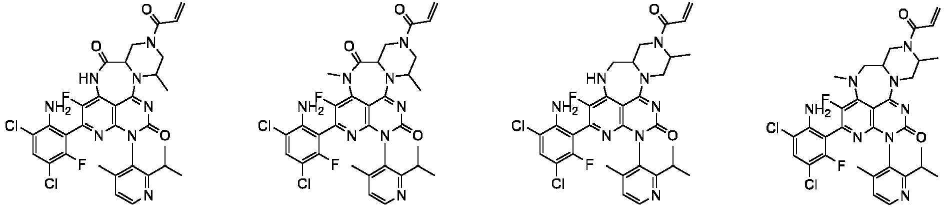

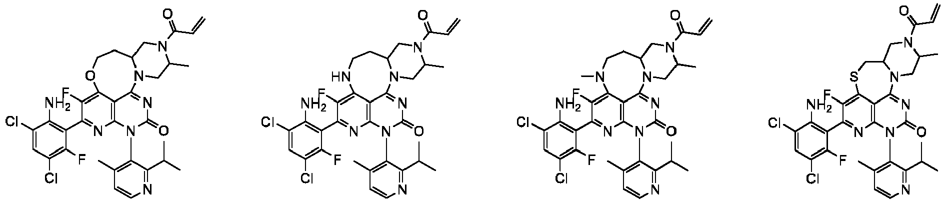

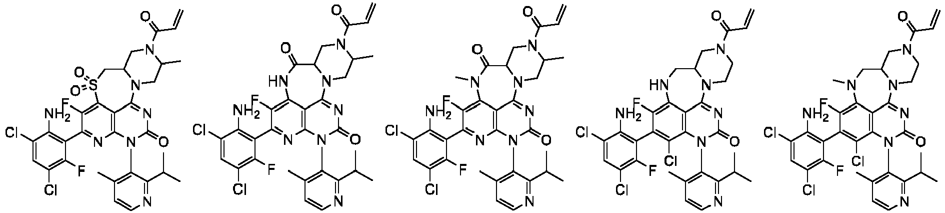

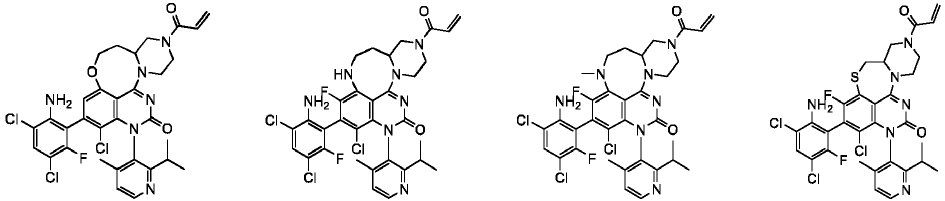

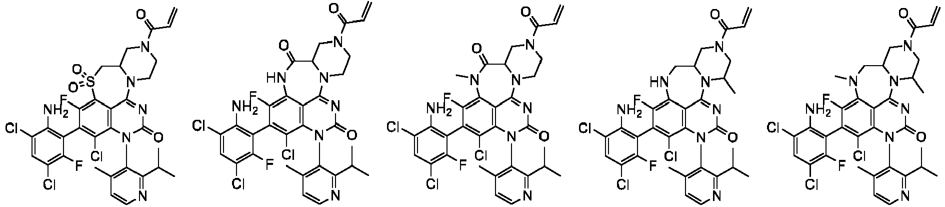

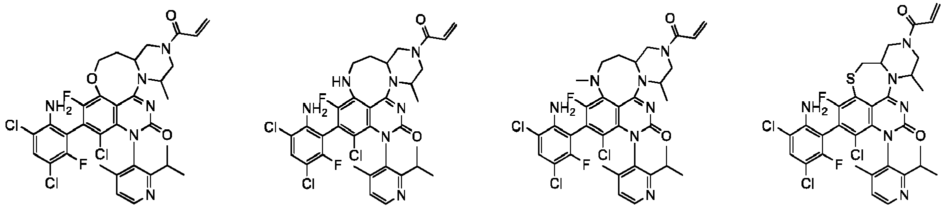

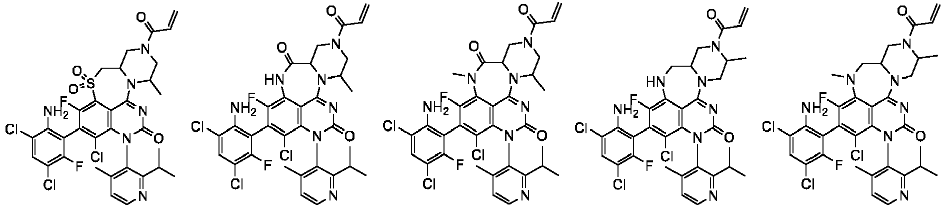

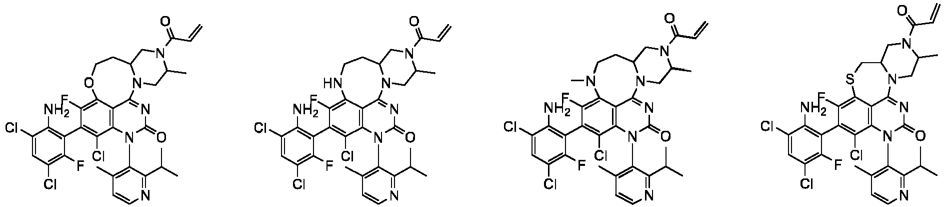

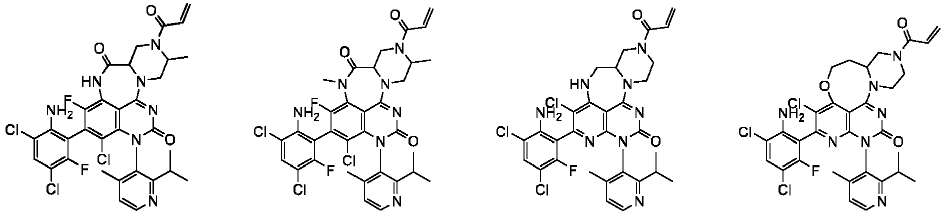

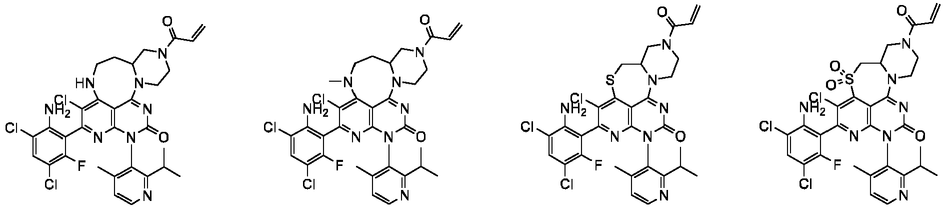

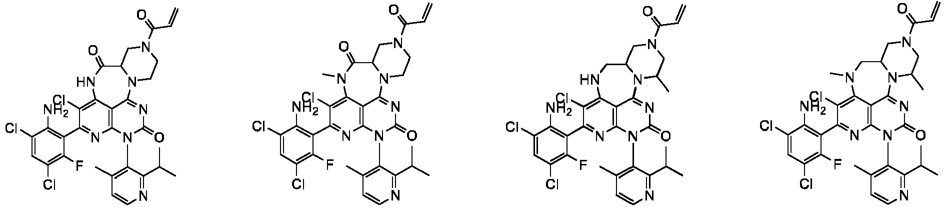

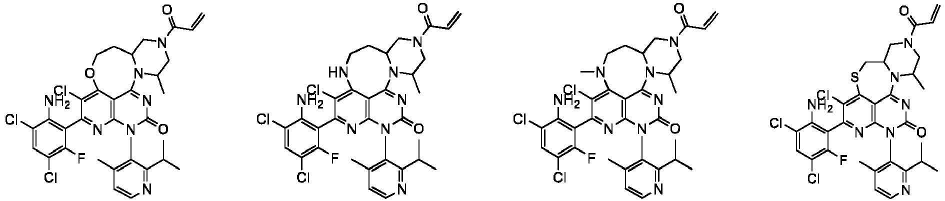

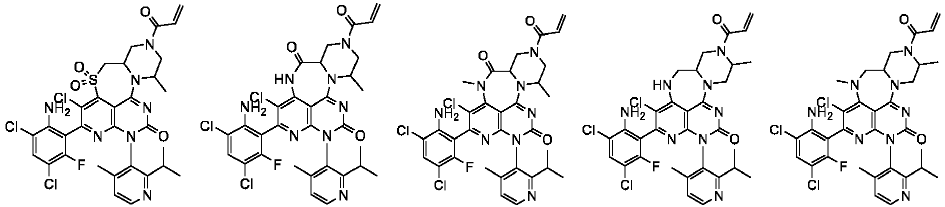

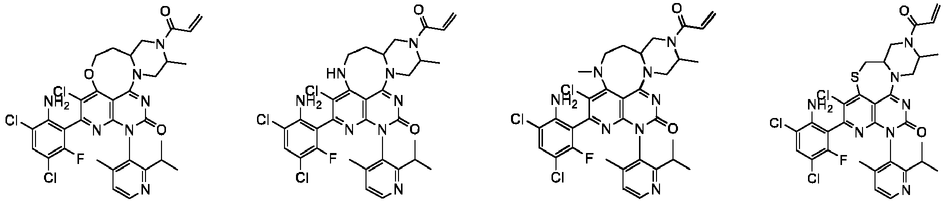

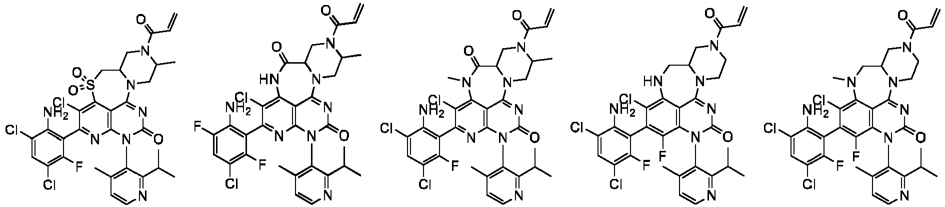

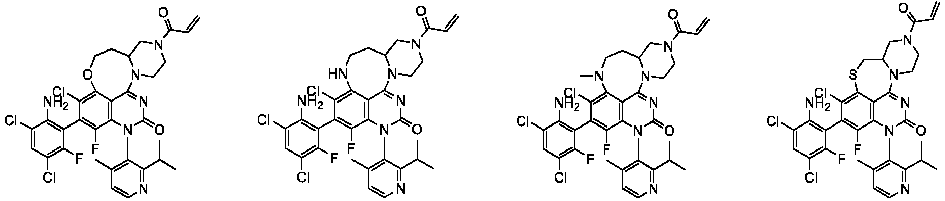

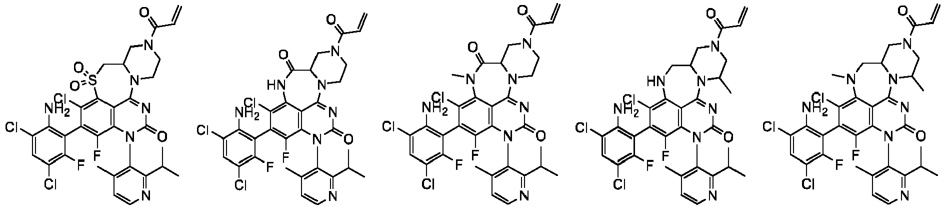

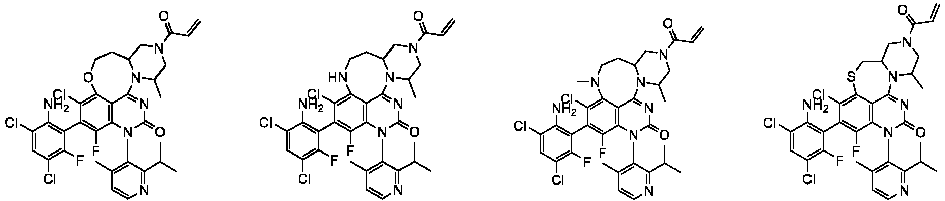

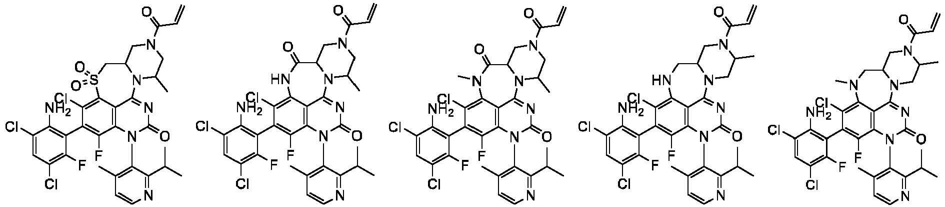

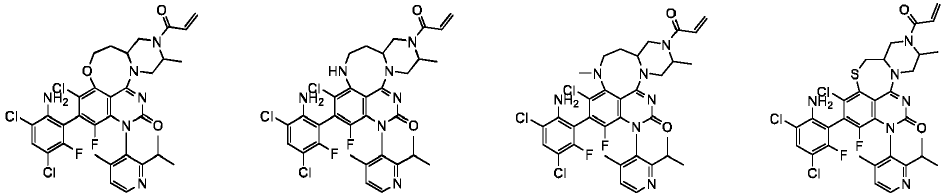

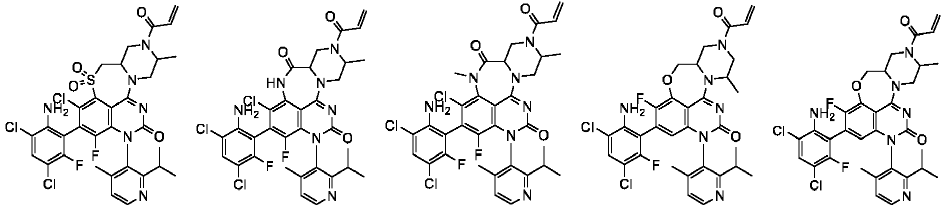

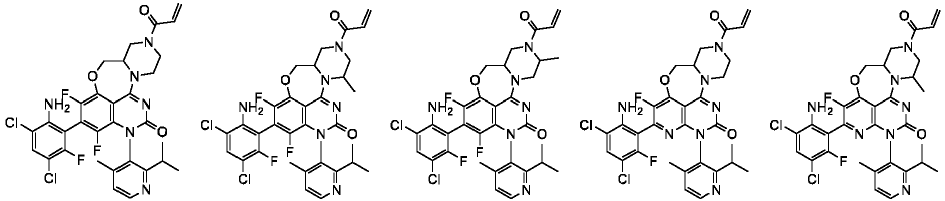

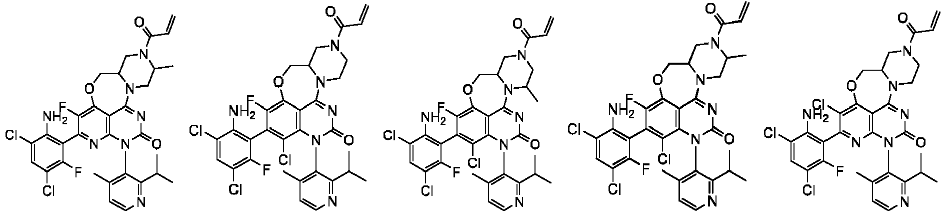

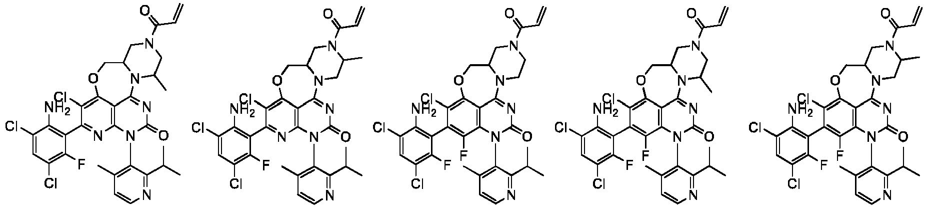

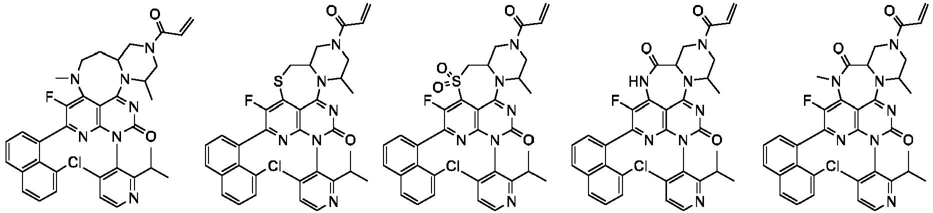

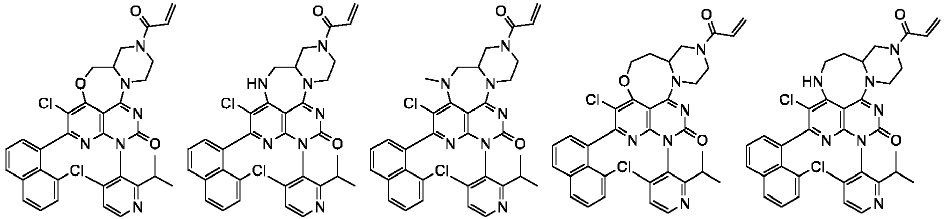

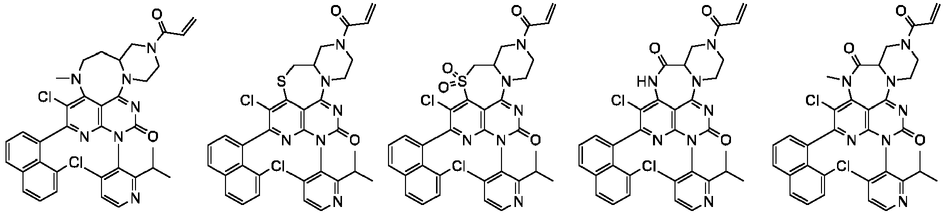

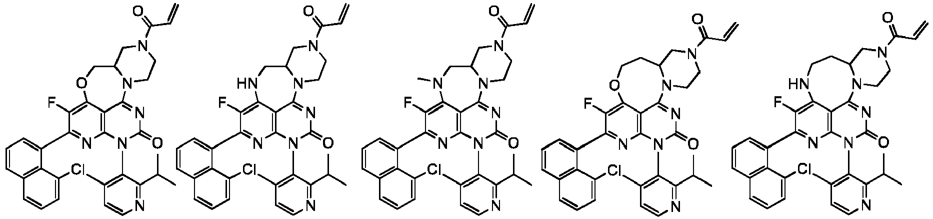

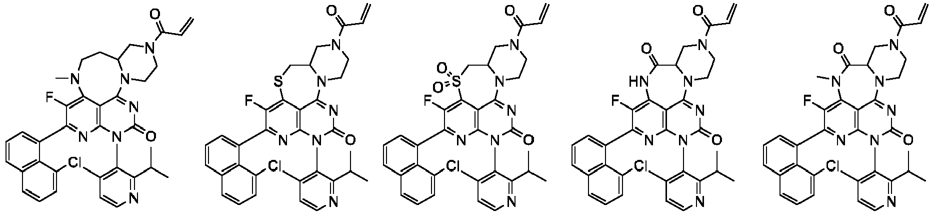

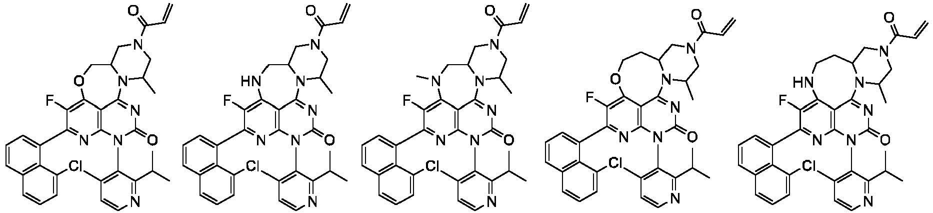

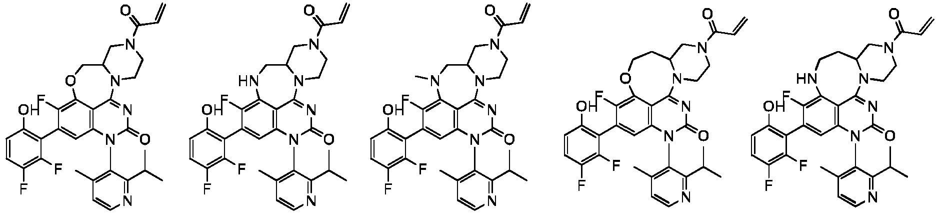

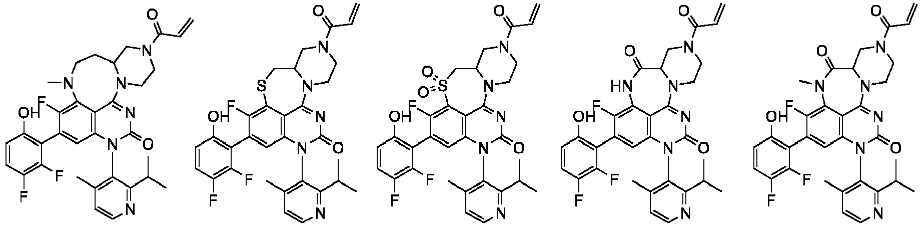

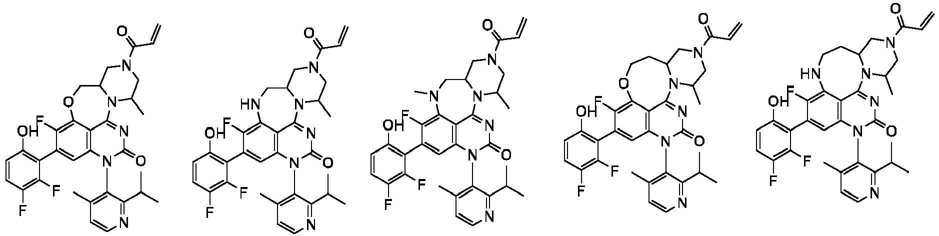

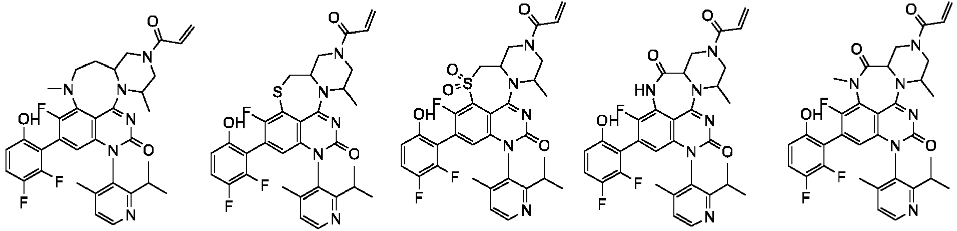

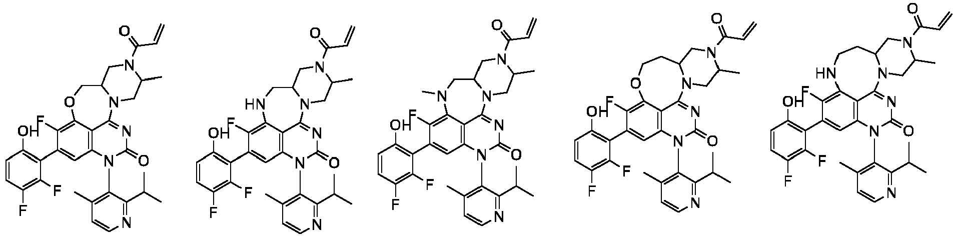

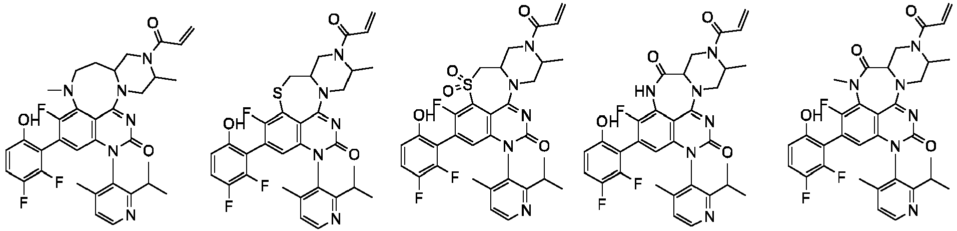

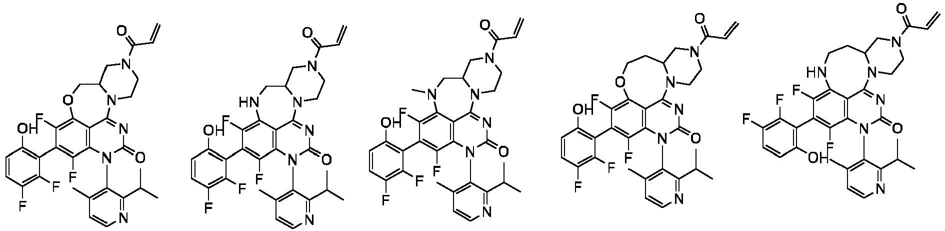

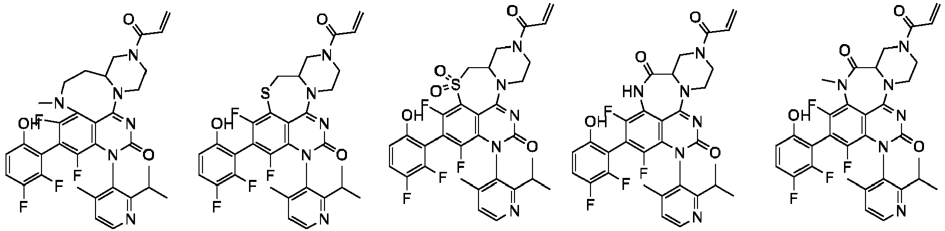

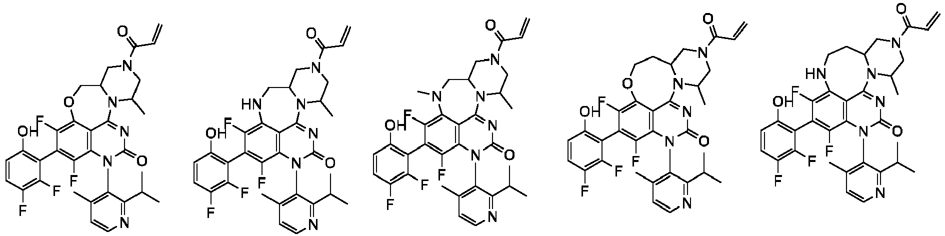

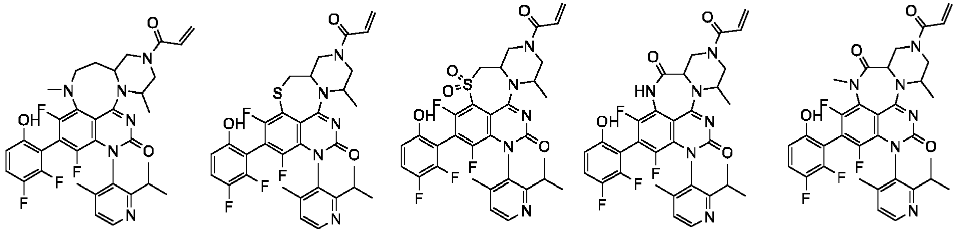

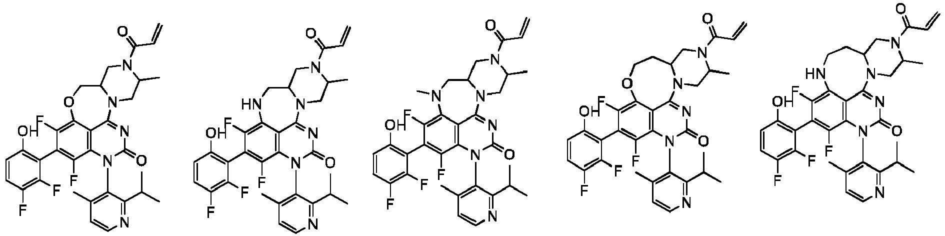

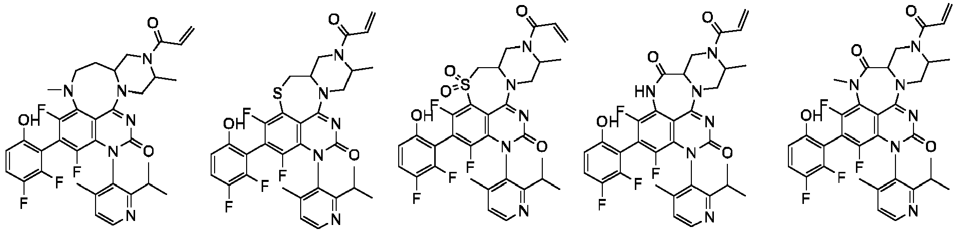

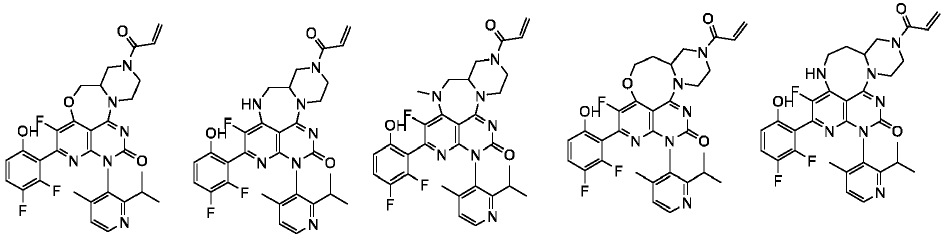

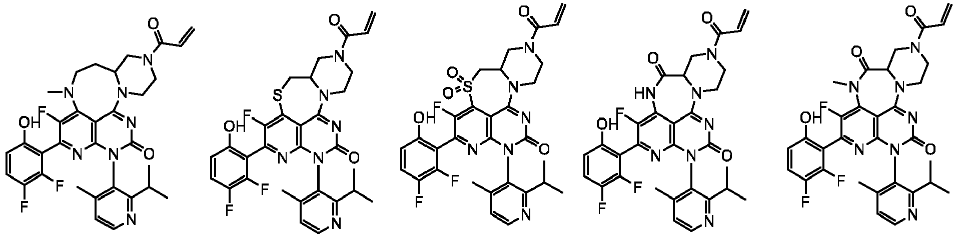

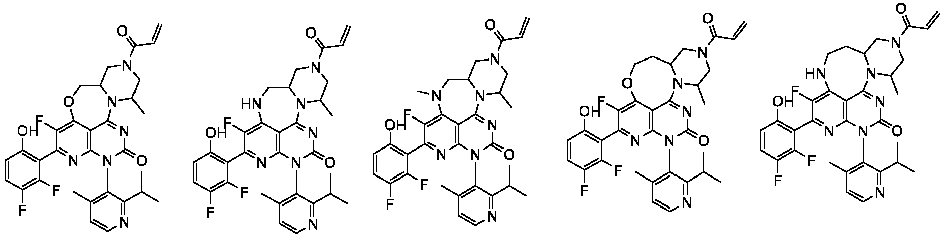

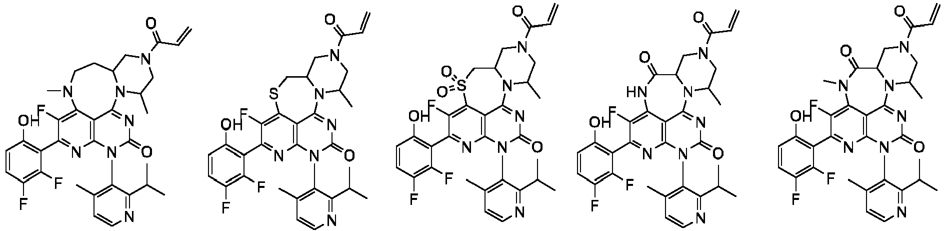

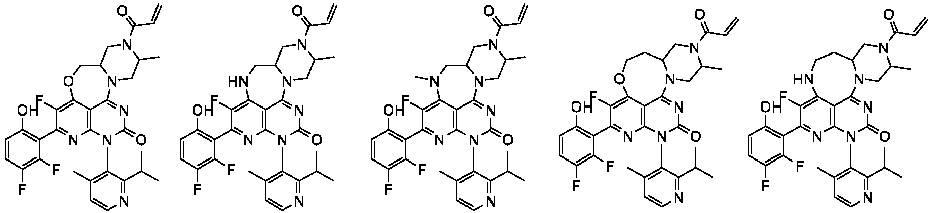

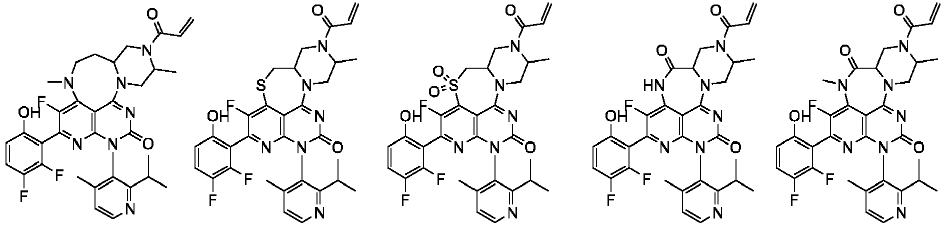

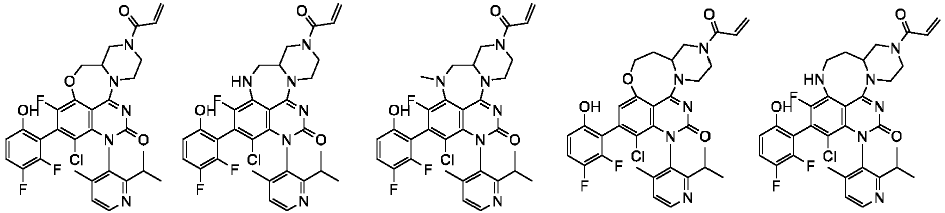

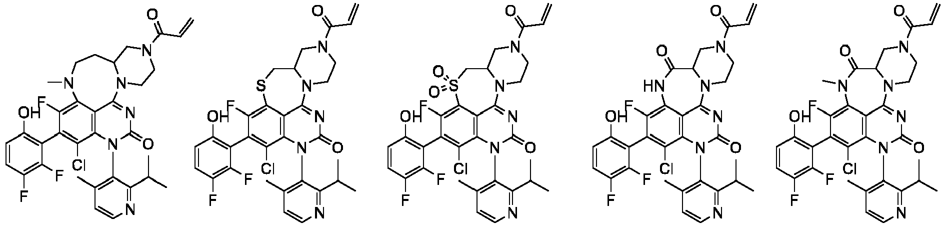

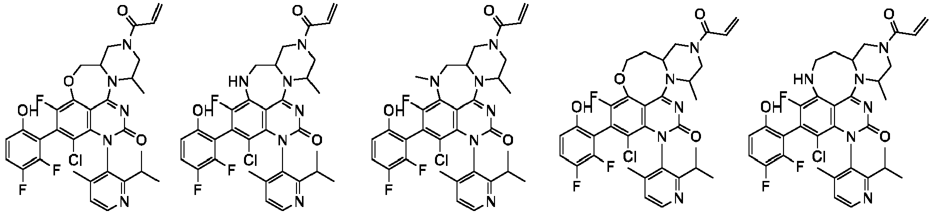

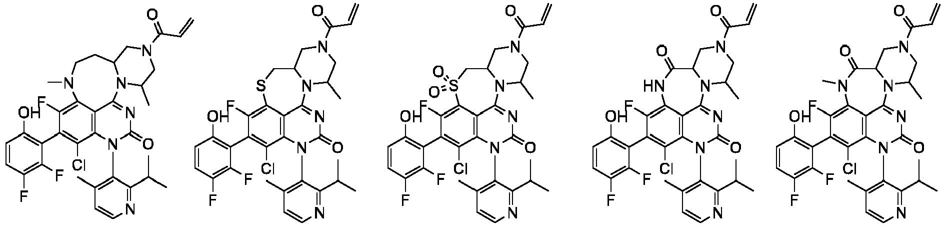

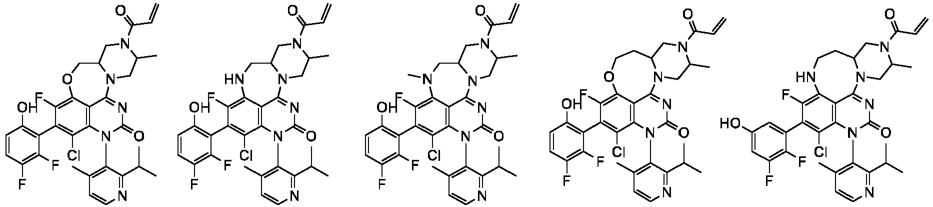

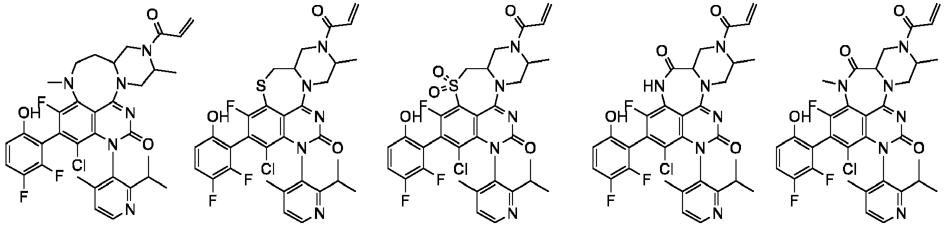

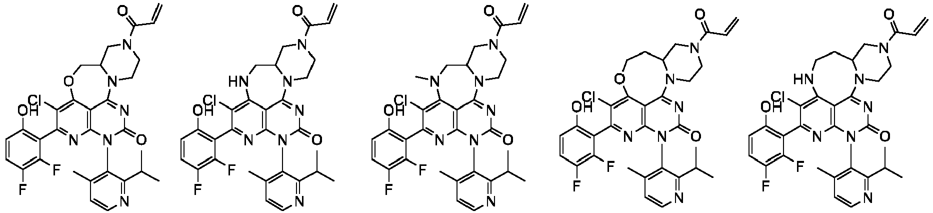

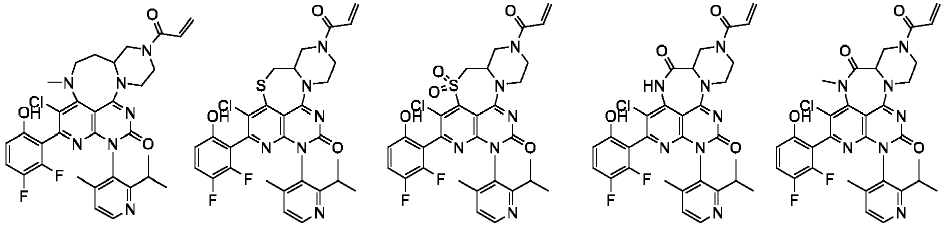

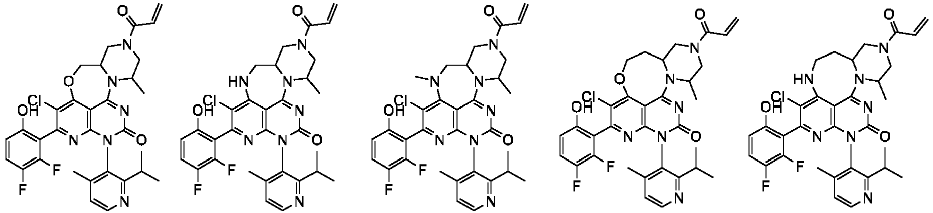

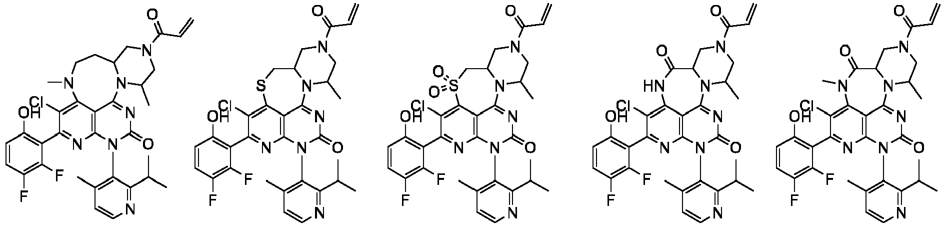

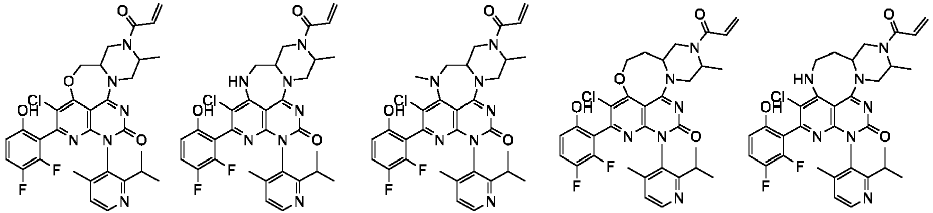

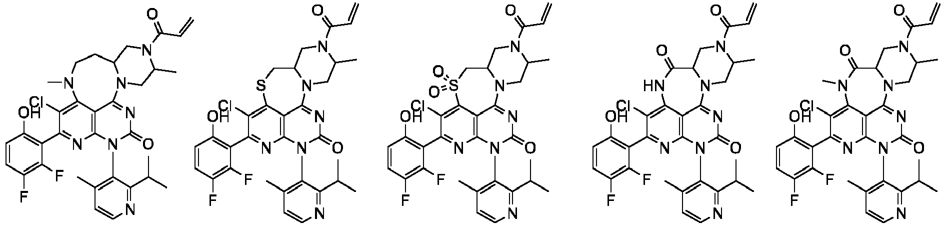

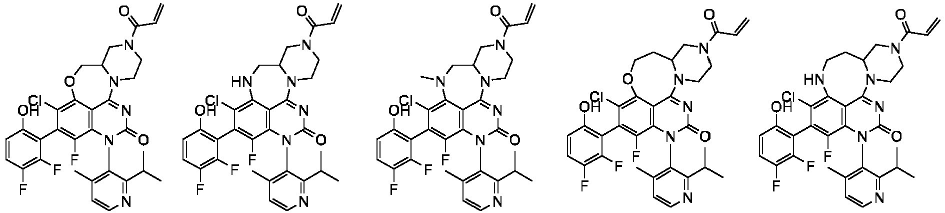

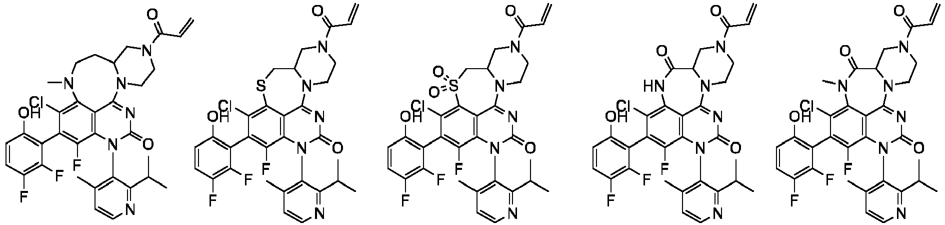

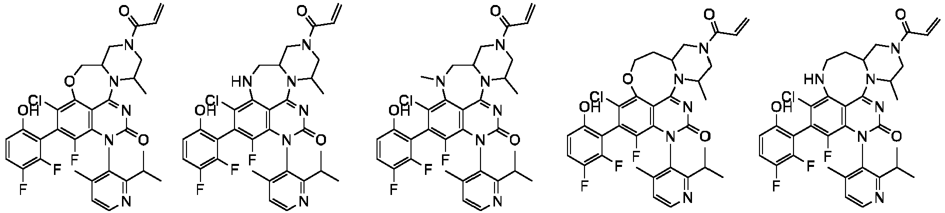

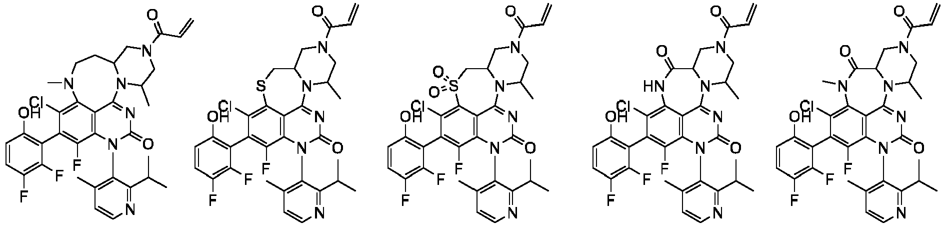

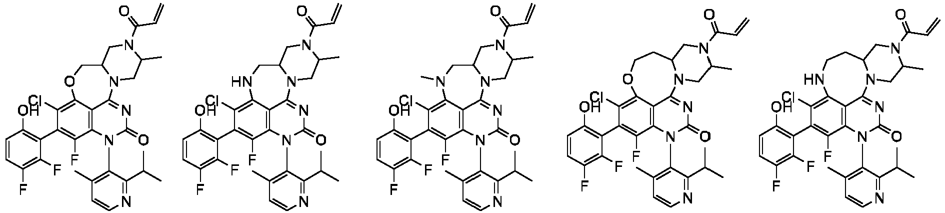

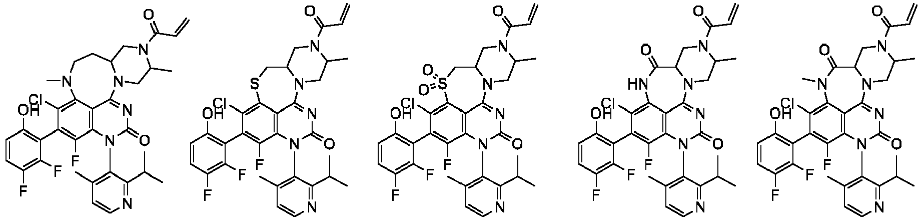

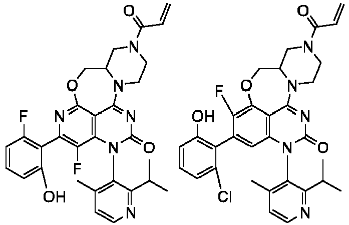

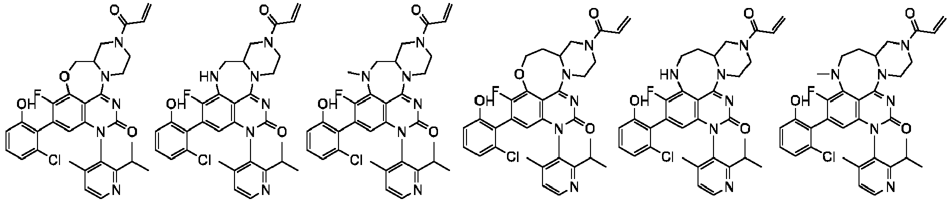

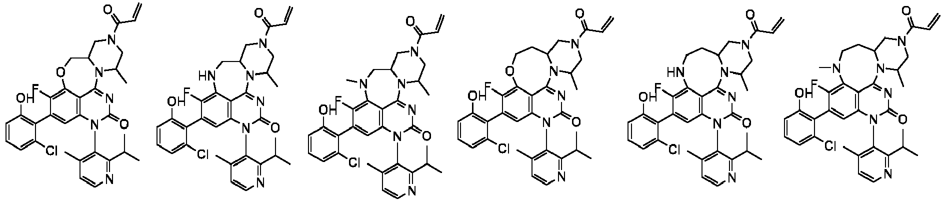

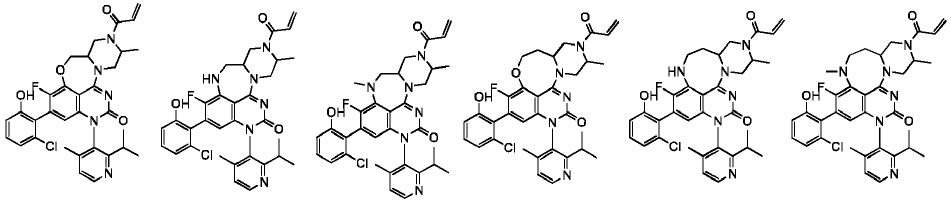

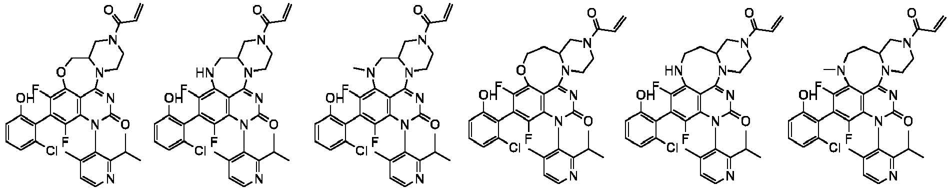

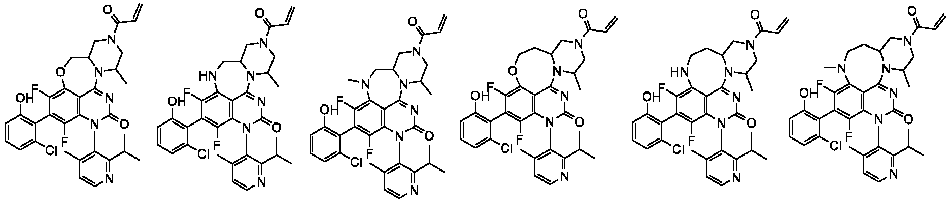

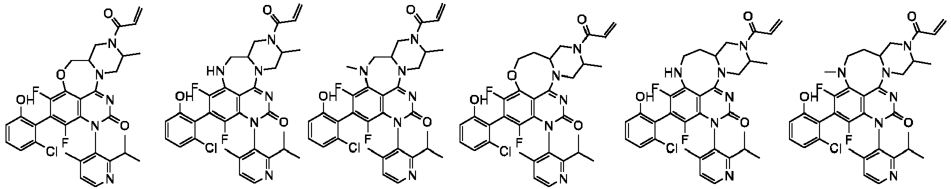

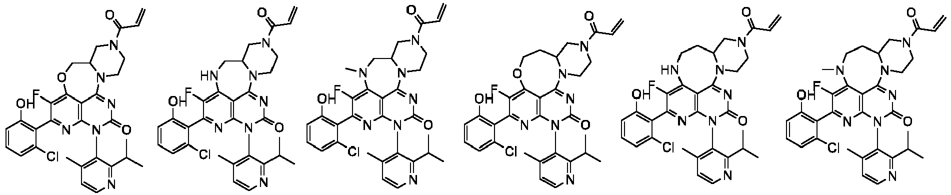

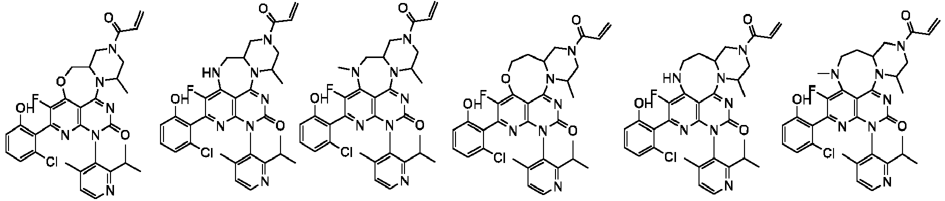

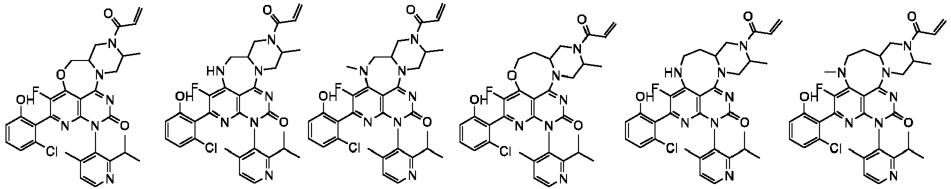

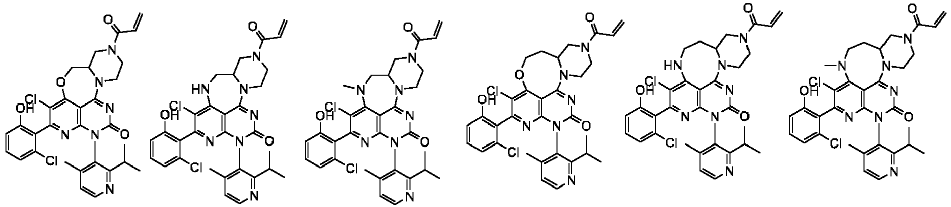

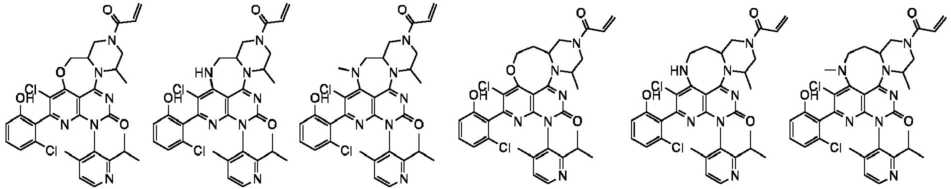

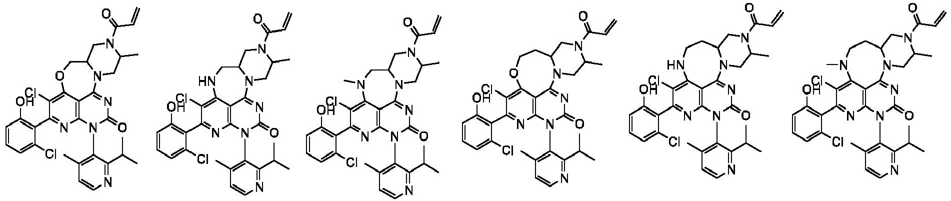

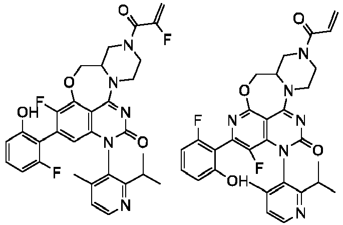

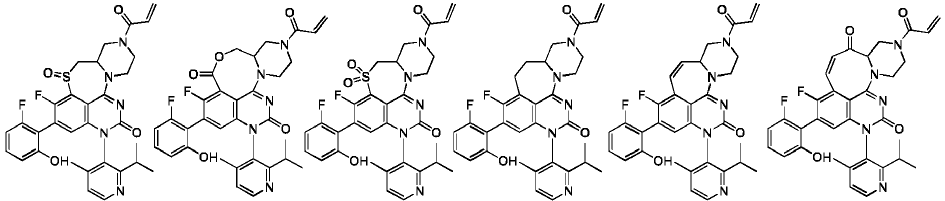

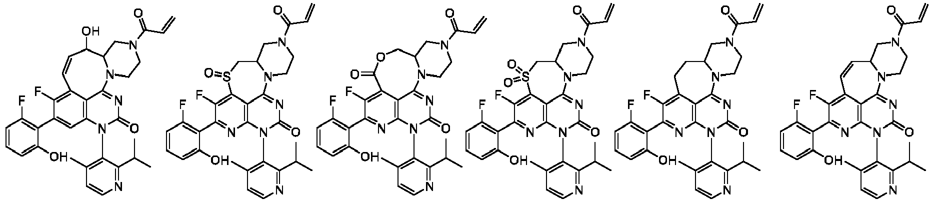

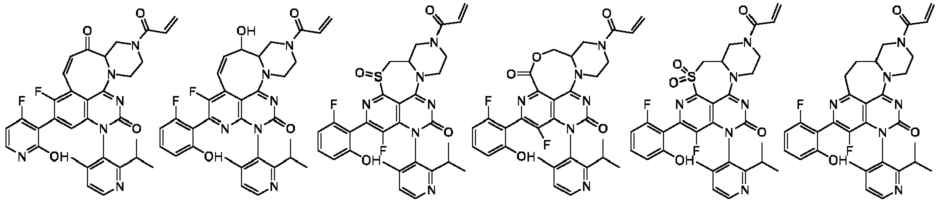

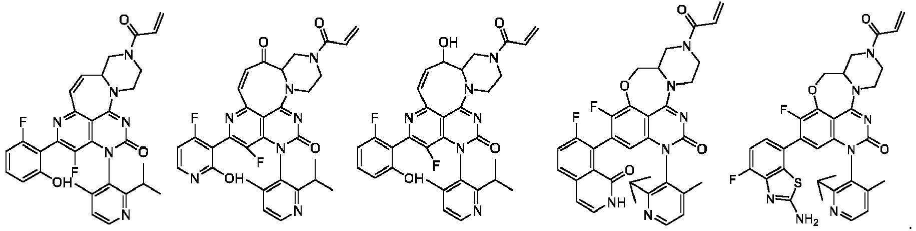

The present disclosure also provides a pharmaceutical composition comprising a therapeutically effective amount of the compound or the pharmaceutically acceptable salt thereof described above, and one or more pharmaceutically acceptable carriers, diluents or excipients.

The present disclosure also provides use of the compound or the pharmaceutically acceptable salt thereof described above or the pharmaceutical composition described above in preparing a medicament for preventing and/or treating a KRAS-G12C-related disease.

In some embodiments of the present disclosure, the KRAS-G12C-related disease is selected from non-small cell lung cancer, colon cancer and pancreatic cancer.

### Definitions and description

Unless otherwise stated, the following terms and phrases used in the present disclosure are intended to have the following meanings. A particular term or phrase, unless otherwise specifically defined, should not be considered as uncertain or unclear, but construed according to its common meaning. When referring to a trade name, it is intended to refer to its corresponding commercial product or its active ingredient.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound of the present disclosure, which is prepared from the compound having particular substituents of the present disclosure and a relatively nontoxic acid or base. When the compound of the present disclosure contains a relatively acidic functional group, a base addition salt can be obtained by contacting the neutral form of such a compound with a sufficient amount of a base in a pure solution or a suitable inert solvent. Pharmaceutically acceptable base addition salts include sodium, potassium, calcium, ammonium, organic amine, or magnesium salts, or similar salts. When the compound of the present disclosure contains a relatively basic functional group, an acid addition salt can be obtained by contacting the neutral form of such a compound with a sufficient amount of an acid in a pure solution or a suitable inert solvent. Examples of pharmaceutically acceptable acid addition salts include salts derived from inorganic acids, such as hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid and phosphorous acid; and salts derived from organic acids, such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid and methanesulfonic acid. Also included are salts of amino acids (e.g., arginine) and salts of organic acids such as glucuronic acid. Certain specific compounds of the present disclosure contain both basic and acidic functional groups that allow the compounds to be converted into either base or acid addition salts.

The pharmaceutically acceptable salts of the present disclosure can be synthesized from a parent compound having an acidic or basic group by conventional chemical methods. In general, such salts are prepared by the following method: performing a reaction of the free acid or base form of the compound and a stoichiometric amount of an appropriate base or acid in water or an organic solvent or a mixture thereof.

The compound of the present disclosure may have a specific geometric or stereoisomeric form. All such compounds are contemplated in the present disclosure, including cis and trans isomers, (-)- and (+)-enantiomers, (*R*)- and (*S*)-enan tiomers, diastereoisomers, (*D*)-isomers, ( )-isomers, and racemic mixtures and other mixtures thereof, such as an enantiomer or diastereomer enriched mixture, all of which are encompassed within the scope of the present disclosure Substituents such as alkyl may have an additional asymmetric carbon atom All these isomers and mixtures thereof are encompassed within the scope of the present disclosure.

Unless otherwise stated, the term "enantiomer" or "optical isomer" refers to stereoisomers that are mirro images of each other.

Unless otherwise stated, the term "*cis-trans* isomer" or "geometric isomer" results from the inability of a single bond of a ring carbon atom or a double bond to rotate freely.

Unless otherwise stated, the term "diastereomer" refers to stereoisomers whose molecules have two or more chiral centers and are not mirror images of each other.

Unless otherwise specified, the absolute configuration of a stereogenic center is shown with a wedged solid bond ( 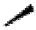 ) and a wedged dashed bond ( 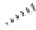 ).

The compounds of the present disclosure may be present in a particular form Unless otherwise stated, the term "tautomer" or "tautomeric form" means that different functional isomers are in dynamic equilibrium at room temperature and can be rapidly converted into each other If tautomers are possible (e.g., in solution), the chemical equilibrium of the tautomers can be achieved For example, a proton tautomer, also known as a prototropic tautomer, includes interconversion by proton migration, such as keto-enol isomerism and imine-enamine isomerism A valence tautomer includes interconversion by recombination of some bonding electrons A specific example of the keto-enol tautomerism is the interconversion between the tautomers pentane-2,4-dione and 4-nydroxypent-3-en-2-one.

The compound of the present disclosure may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen can be substituted by deuterium to form a deuterated drug, and the bond formed by deuterium and carbon is firmer than that formed by common hydrogen and carbon Compared with an un- leuterated drug, the deuterated drug has the advantages of reduced toxic side effect, increased stability, enhanced efficacy, and prolonged biological half-life and the like All isotopic variations of the compounds of the present disclosure, whether radioactive o not, are encompassed within the scope of the present disclosure "Optional" or "optionally" means that the subsequently described event or circumstance may, but does not necessarily, occur, and the description includes instances where the event or circumstance occurs and instances where it does not.

The term "substituted" means that one or more hydrogen atoms on a specific atom are substituted by substituents which may include deuterium and hydrogen variants, as long as the valence of the specific atom is normal and the substituted compound is stable. When the substituent is an oxygen (i.e., =O), it means that two hydrogen atoms are substituted. Substitution with oxygen does not occur on aromatic groups. The term "optionally substituted" means that an atom can be substituted with a substituent or not. Unless otherwise specified, the type and number of the substituent may be arbitrary as long as being chemically achievable.

When any variable (e.g., R) occurs more than once in the constitution or structure of a compound, the variable is independently defined in each case. Thus, for example, if a group is substituted with 0-2 R, the group can be optionally substituted with up to two R, and the definition of R in each case is independent. Furthermore, a combination of a substituent and/or a variant thereof is permissible only if the combination can result in a stable compound.

When one of the variables is selected from a single bond, it means that the two groups are linked directly. For example, when L₃ in 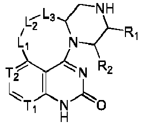 represents a single bond,

it means that the structure is actually

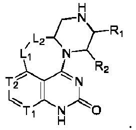

When it is not specified by which atom the listed substituent is linked to the group to be substituted, the substituent can be linked via any atom of the group. For example, pyridyl as a substituent can be linked to the group to be substituted via any carbon atom on the pyridine ring.

When the linking direction of the linking group listed is not specified, the linking direction is arbitrary. For example, when the linking group L in 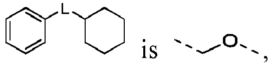 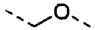 can either link the benzene ring and the cyclohexane in a direction same as left-to-right reading order to form 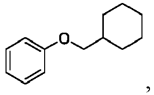 or link the benzene ring and the cyclohexane in a direction opposite to left-to-right reading order to form 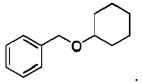 A combination of the linking group, a substituent and/or a variant thereof is permissible only if the combination can result in a stable compound.

Unless otherwise specified, the number of atoms on a ring is generally defined as the member number of the ring. For example, "5-7 membered ring" refers to a "ring" on which 5 to 7 atoms are arranged in a circle.

Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆, and C₅ alkyl, etc., and may be monovalent (e.g., methyl), divalent (e.g., methylene), or polyvalent (e.g., methenyl). Examples of C₁₋₆ alkyl include, but are not limited to, methyl (Me), ethyl (Et), propyl (including *n*-propyl and isopropyl), butyl (including *n*-butyl, isobutyl, s-butyl, and *t*-butyl), pentyl (including *n*-pentyl, isopentyl, and neopentyl), and hexyl and the like.

Unless otherwise specified, the term "C₁₋₅ alkyl" refers to a linear or branched saturated hydrocarbon group consisting of 1 to 5 carbon atoms. The C₁₋₅ alkyl includes C₁₋₄, C₁₋₃, C₁₋₂, C₂₋₅, C₂₋₄ and C₅ alkyl and the like, and may be monovalent (e.g., methyl), divalent (e.g., methylene) or polyvalent (e.g., methenyl). Examples of C₁₋₅ alkyl include, but are not limited to, methyl (Me), ethyl (Et), propyl (including *n*-propyl and isopropyl), butyl (including *n*-butyl, isobutyl, s-butyl, and butyl), and pentyl (including *n*-pentyl, isopentyl, and neopentyl) and the like.

Unless otherwise specified, the term "C₁₋₄ alkyl" refers to a linear or branched saturated hydrocarbon group consisting of 1 to 4 carbon atoms. The C₁₋₄ alkyl includes, but is not limited to, C₁₋₂, C₁₋₃, and C₂₋₃ alkyl and the like, and may be monovalent (e.g., methyl), divalent (e.g., methylene) or polyvalent (e.g., methenyl). Examples of C₁₋₄ alkyl include, but are not limited to, methyl (Me), ethyl (Et), propyl (including *n*-propyl and isopropyl), and butyl (including *n*-butyl, isobutyl, s-butyl and *t*-butyl) and the like.

Unless otherwise specified, the term "C₁₋₃ alkyl" refers to a linear or branched saturated hydrocarbon group consisting of 1 to 3 carbon atoms. The C₁₋₃ alkyl includes, but is not limited to, C₁₋₂ and C₂₋₃ alkyl, etc., and may be monovalent (e.g., methyl), divalent (e.g., methylene), or polyvalent (e.g., methenyl). Examples of C₁₋₃ alkyl include, but are not limited to, methyl (Me), ethyl (Et), and propyl (including *n*-propyl and isopropyl). Unless otherwise specified, "C₂₋₈ alkenyl" is used to denote a linear or branched hydrocarbon group containing 2 to 8 carbon atoms and at least one carbon-carbon double bond, which may be located anywhere in the group. The C₂₋₈ alkenyl includes C₂₋₆, C₂₋₄, C₂₋₃, C₄, C₃, and C₂ alkenyl etc., and may be monovalent, divalent or polyvalent. Examples of C₂₋₈ alkenyl include, but are not limited to, ethenyl, propenyl, butenyl, pentenyl, hexenyl, butadienyl, 1,3-pentadienyl, and 1,3-hexadienyl and the like.

Unless otherwise specified, "C₂₋₄ alkenyl" is used to denote a linear or branched hydrocarbon group containing 2 to 4 carbon atoms and at least one carbon-carbon double bond, which may be located anywhere in the group. The C₂₋₄ alkenyl includes C₂₋₃, C₄, C₃ and C₂ alkenyl and the like, and may be monovalent, divalent or polyvalent. Examples of C₂₋₄ alkenyl include, but are not limited to, ethenyl, propenyl, butenyl, and butadienyl and the like. Unless otherwise specified, "C₂₋₃ alkenyl" is used to denote a linear or branched hydrocarbon group containing 2 to 3 carbon atoms and at least one carbon-carbon double bond, which may be located anywhere in the group. The C₂₋₃ alkenyl includes C₃ and C₂ alkenyl, and may be monovalent, divalent or polyvalent. Examples of C₂₋₃ alkenyl include, but are not limited to, ethenyl, and propenyl and the like.

Unless otherwise specified, "C₂₋₄ alkynyl" is used to denote a linear or branched hydrocarbon group containing 2 to 4 carbon atoms and at least one carbon-carbon triple bond, which may be located anywhere in the group. The C₂₋₄ alkynyl includes C₂₋₃, C₄, C₃ and C₂ alkynyl and the like. It may be monovalent, divalent or polyvalent. Examples of C₂₋₄ alkynyl include, but are not limited to, ethynyl, propynyl, and butynyl and the like.

Unless otherwise specified, "C₂₋₃ alkynyl" is used to denote a linear or branched hydrocarbon group containing 2 to 3 carbon atoms and at least one carbon-carbon triple bond, which may be located anywhere in the group. It may be monovalent, divalent or polyvalent. The C₂₋₃ alkynyl includes C₃ and C₂ alkynyl. Examples of C₂₋₃ alkynyl include, but are not limited to, ethynyl, and propynyl and the like.

The term "heteroalkyl", by itself or in combination with another term, refers to a stable linear or branched alkyl radical or a combination thereof consisting of a specified number of carbon atoms and at least one heteroatom or heteroatom group. In some embodiments, the heteroatom is selected from B, O, N, and S, wherein nitrogen and sulfur atoms are optionally oxidized and the nitrogen heteroatom is optionally quaternized. In other embodiments, the heteroatom group is selected from -C(=O)O-, -C(=O)-, -C(=S)-, -S(=O), -S(=O)₂-, - C(=O)N(H)-, -N(H)-, -C(=NH)-, -S(=O)₂N(H)-, and -S(=O)N(H)-. In some embodiments, the heteroalkyl is C₁₋₆ heteroalkyl. In other embodiments, the heteroalkyl is C₁₋₃ heteroalkyl. The heteroatom or heteroatom group can be located at any interior position of heteroalkyl, including the position where the alkyl is linked to the rest part of the molecule. However, the terms "alkoxy", "alkylamino" and "alkylthio" (or thioalkxoy) are commonly used expressions and refer to those alkyl groups linked to the rest part of the molecule via an oxygen atom, an amino, or a sulfur atom, respectively. Examples of heteroalkyl include, but are not limited to, -OCH₃, -OCH₂CH₃, -OCH₂CH₂CH₃, -OCH₂(CH₃)2, -CH₂-CH₂-O-CH₃, -NHCH₃, -N(CH₃)₂, - NHCH₂CH₃, -N(CH₃)(CH₂CH₃), -CH₂-CH₂-NH-CH₃, -CH₂-CH₂-N(CH₃)-CH₃, -SCH₃, - SCH₂CH₃, -SCH₂CH₂CH₃, -SCH₂(CH₃)₂, -CH₂-S-CH₂-CH₃, -CH₂-CH₂, -S(=O)-CH₃, and - CH₂-CH₂-S(=O)₂-CH₃. At most two heteroatoms can be consecutive, e.g., -CH₂-NH-OCH₃.

Unless otherwise specified, the term "C₁₋₆ alkoxy" refers to those alkyl groups that each contains 1 to 6 carbon atoms and is linked to the rest part of the molecule through an oxygen atom. The C₁₋₆ alkoxy includes C₁₋₄, C₁₋₃, C₁₋₂, C₂₋₆, C₂₋₄, C₆, C₅, C₄ and C₃ alkoxy and the like. Examples of C₁₋₆ alkoxy include, but are not limited to, methoxy, ethoxy, propoxy (including *n*-propoxy and isopropoxy), butoxy (including *n*-butoxy, isobutoxy, s-butoxy and *t*-butoxy), pentyloxy (including *n*-pentyloxy, isopentyloxy and neopentyloxy), and hexyloxy and the like.

Unless otherwise specified, the term "C₁₋₃ alkoxy" refers to those alkyl groups that each contains 1 to 3 carbon atoms and is linked to the rest part of the molecule through an oxygen atom. The C₁₋₃ alkoxy includes C₁₋₂, C₂₋₃, C₃ and C₂ alkoxy and the like. Examples of C₁₋₃ alkoxy include, but are not limited to, methoxy, ethoxy, and propoxy (including n-propoxy and isopropoxy) and the like.

Unless otherwise specified, the term "C₁₋₆ alkylamino" refers to those alkyl groups that each contains 1 to 6 carbon atoms and is linked to the rest part of the molecule through an amino group. The C₁₋₆ alkylamino includes C₁₋₄, C₁₋₃, C₁₋₂, C₂₋₆, C₂₋₄, C₆, C₅, C₄, C₃ and C₂ alkylamino and the like. Examples of C₁₋₆ alkylamino include, but are not limited to, - NHCH₃, -N(CH₃)₂, -NHCH₂CH₃, -N(CH₃)CH₂CH₃, -N(CH₂CH₃)(CH₂CH3), - NHCH₂CH₂CH₃, -NHCH₂(CH₃)₂, and -NHCH₂CH₂CH₂CH₃ and the like.

Unless otherwise specified, the term "C₁₋₃ alkylamino" refers to those alkyl groups that each contains 1 to 3 carbon atoms and is linked to the rest part of the molecule through an amino group. The C₁₋₃ alkylamino includes C₁₋₂, C₃ and C₂ alkylamino and the like. Examples of C₁₋₃ alkylamino include, but are not limited to, -NHCH₃, -N(CH₃)₂, -NHCH₂CH₃, -N(CH₃)CH₂CH₃, and -NHCH₂CH₂CH₃, -NHCH₂(CH₃)₂ and the like.

Unless otherwise specified, the term "C₁₋₆ alkylthio" refers to those alkyl groups that each contains 1 to 6 carbon atoms and is linked to the rest part of the molecule through a sulfur atom. The C₁₋₆ alkylthio includes C₁₋₄, C₁₋₃, C₁₋₂, C₂₋₆, C₂₋₄, C₆, C₅, C₄, C₃ and C₂ alkylthio and the like. Examples of C₁₋₆ alkylthio include, but are not limited to, -SCH₃, -SCH₂CH₃, - SCH₂CH₂CH₃, and -SCH₂(CH₃)₂ and the like.

Unless otherwise specified, the term "C₁₋₃ alkylthio" refers to those alkyl groups that each contains 1 to 3 carbon atoms and is linked to the rest part of the molecule through a sulfur atom. The C₁₋₃ alkylthio includes C₁₋₃, C₁₋₂ and C₃ alkylthio and the like. Examples of C₁₋₃ alkylthio include, but are not limited to, -SCH₃, -SCH₂CH₃, -SCH₂CH₂CH₃, and -SCH₂(CH₃)₂ and the like.

Unless otherwise specified, "C₃₋₆ cycloalkyl" refers to a saturated cyclic hydrocarbon group consisting of 3 to 6 carbon atoms, including monocyclic and bicyclic ring systems. The C₃₋₆ cycloalkyl includes C₃₋₅ cycloalkyl, C₄₋₅ cycloalkyl, C₅₋₆ cycloalkyl and the like, and may be monovalent, divalent or polyvalent. Examples of C₃₋₆ cycloalkyl include, but are not limited to, cyclopropyl, cyclobutyl, cyclopentyl, cyclohexyl, and the like.

Unless otherwise specified, the term "3-6 membered heterocycloalkyl", by itself or in combination with other terms, refers to a saturated cyclic group consisting of 3 to 6 ring atoms, of which 1, 2, 3, or 4 ring atoms are heteroatoms independently selected from the group consisting of O, S and N, with the remaining being carbon atoms. The nitrogen atom is optionally quaternized, and the nitrogen and sulfur heteroatoms can be optionally oxidized (i.e., NO and S(O)ₚ, where p is 1 or 2). This includes monocyclic and bicyclic systems, wherein the bicyclic system includes spirocyclic, fused, and bridged rings. Furthermore, with respect to the "3-6 membered heterocycloalkyl", a heteroatom may occupy the position where the heterocycloalkyl is linked to the rest of the molecule. The 3-6 membered heterocycloalkyl includes 4-6 membered, 5-6 membered, 4 membered, 5 membered, 6 membered heterocycloalkyl, and the like. Examples of 3-6 membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, etc.

Unless otherwise specified, the terms "C₆₋₁₀ aromatic ring" and "C₆₋₁₀ aryl" of the present disclosure are used interchangeably. The term "C₆₋₁₀ aromatic ring" or "C₆₋₁₀ aryl" refers to a cyclic hydrocarbon group consisting of 6 to 10 carbon atoms and having a conjugated π-electron system. The group may be a monocyclic, fused bicyclic or fused tricyclic system, where the rings are aromatic. It may be monovalent, divalent or polyvalent, and the C₆₋₁₀ aryl includes C₆₋₉, C₉, C₁₀ and C₆ aryl groups, etc. Examples of C₆₋₁₀ aryl include, but are not limited to, phenyl, naphthyl (including 1-naphthyl, 2-naphthyl, etc.).

Unless otherwise specified, the terms "5-10 membered heteroaromatic ring" and "5-10 membered heteroaryl" of the present disclosure are used interchangeably. The term " 5-10 membered heteroaryl" refers to a cyclic group consisting of 5 to 10 ring atoms and having a conjugated pi-electron system, in which 1, 2, 3, or 4 ring atoms are heteroatoms independently selected from the group consisting of O, S and N, while the others are carbon atoms. It can be a monocyclic, fused bicyclic or fused tricyclic system, wherein the rings are aromatic. The nitrogen atom is optionally quaternized, and the nitrogen and sulfur heteroatoms are optionally oxidized (i.e., NO and S(O)ₚ, where p is 1 or 2). The 5-10 membered heteroaryl can be linked to the rest of the molecule via a heteroatom or a carbon atom. The 5-10 membered heteroaryl includes 5-8 membered, 5-7 membered, 5-6 membered, 5 membered and 6 membered heteroaryl groups, etc. Examples of the 5-10 membered heteroaryl include, but are not limited to, pyrrolyl (including *N*-pyrrolyl, 2-pyrrolyl, 3-pyrrolyl, etc.), pyrazolyl (including 2-pyrazolyl, 3-pyrazolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (including 1*H*-1,2,3-triazolyl, 2*H*-1,2,3-triazolyl, 1*H*-1,2,4-triazolyl, 4*H*-1,2,4-triazolyl, etc.), tetrazolyl, isoxazolyl (including 3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridyl (including 2-pyridyl, 3-pyridyl, 4-pyridyl, etc.), pyrazinyl, pyrimidinyl (including 2-pyrimidinyl, 4-pyrimidinyl, etc.), benzothiazolyl (including 5-benzothiazolyl, etc.), purinyl, benzimidazolyl (including 2-benzimidazolyl, etc.), benzoxazolyl, indolyl (including 5-indolyl, etc.), isoquinolinyl (including 1-isoquinolinyl, 5-isoquinolinyl, etc.), quinoxalinyl (including 2-quinoxalinyl, 5-quinoxalinyl, etc.) or quinolyl (including 3-quinolyl, 6-quinolyl, etc.).

Unless otherwise specified, the terms "5-6 membered heteroaromatic ring" and "5-6 membered heteroaryl" of the present disclosure are used interchangeably. The term "5-6 membered heteroaryl" refers to a monocyclic group consisting of 5 to 6 ring atoms and having a conjugated π-electron system, in which 1, 2, 3 or 4 ring atoms are heteroatoms independently selected from the group consisting of O, S, and N, while the others are carbon atoms. The nitrogen atom is optionally quaternized, and the nitrogen and sulfur heteroatoms are optionally oxidized (i.e., NO and S(O)ₚ, where p is 1 or 2). The 5-6 membered heteroaryl can be linked to the rest of the molecule via a heteroatom or a carbon atom. The 5-6 membered heteroaryl includes 5 membered and 6 membered heteroaryl. Examples of the 5-6 membered heteroaryl include, but are not limited to, pyrrolyl (including *N*-pyrrolyl, 2-pyrrolyl, 3-pyrrolyl, etc.), pyrazolyl (including 2-pyrazolyl, 3-pyrazolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (including 1*H*-1,2,3-triazolyl, 2*H*-1,2,3-triazolyl, 1*H*-1,2,4-triazolyl, 4*H*-1,2,4-triazolyl, etc.), tetrazolyl, isoxazolyl (including 3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridyl (including 2-pyridyl, 3-pyridyl, 4-pyridyl, etc.), pyrazinyl, or pyrimidinyl (including 2-pyrimidinyl, 4-pyrimidinyl, etc.).

Unless otherwise specified, "benzo 5-6 membered heterocycloalkyl" refers to a double fused ring structure formed by the union of phenyl, heterocycle and 5-6 membered heterocycloalkyl, and the substituent can be linked to other structures through a benzene ring or 5-6 membered heterocycloalkyl ring. Examples of the benzo 5-6 membered heterocycloalkyl include, but are not limited to 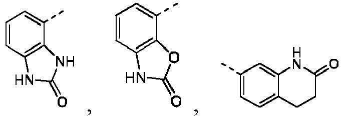 and 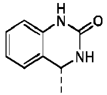 and the like.

Unless otherwise specified, "5-6 membered heteroary-fused 5-6 membered heterocycloalkyl" refers to a double fused ring structure formed by the union of 5-6 membered heteroaryl and heterocycle and 5-6 membered heterocycloalkyl, and the substituent can be linked to other structures through 5-6 membered heteroaryl or 5-6 membered heterocycloalkyl ring. Examples of the benzo 5-6 membered heterocycloalkyl include, but are not limited to, 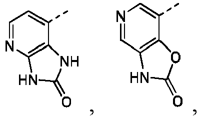 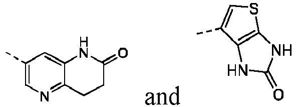 and the like.

Unless otherwise specified, Cₙ₋ₙ₊ₘ or Cₙ-Cₙ₊ₘ includes any one of the specific cases of n to n+m carbon atoms. For example, C₁₋₁₂ includes C₁, C₂, C₃, C₄, C₅, C₆, C₇, C₈, C₉, C₁₀, C₁₁ and C₁₂. Also, any range within n to n+m may be included. For example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂. Similarly, n-n+m membered represents that the number of atoms on the ring is n to n+m. For example, 3-12 membered ring includes 3 membered ring, 4 membered ring, 5 membered ring, 6 membered ring, 7 membered ring, 8 membered ring, 9 membered ring, 10 membered ring, 11 membered ring and 12 membered ring. n-n+m membered also represents any range within n to n+m. For example, 3-12 membered ring includes 3-6 membered ring, 3-9 membered ring, 5-6 membered ring, 5-7 membered ring, 6-7 membered ring, 6-8 membered ring, and 6-10 membered ring.

The compounds of the present disclosure can be prepared by a variety of synthetic methods well known to those skilled in the art, including the specific embodiments listed below, embodiments formed by combinations thereof with other chemical synthetic methods, and equivalents thereof known to those skilled in the art. Preferred embodiments include, but are not limited to, the examples of the present disclosure.

The solvent used in the present disclosure can be commercially available. The following abbreviations are used in the present disclosure: CDCl₃ represents deuterated chloroform; CD₃OD represents deuterated methanol; DMSO-d₆ represents deuterated dimethyl sulfoxide; TBS represents *tert*-butyldimethylsilyl.

Compounds are named according to conventional nomenclature rules in the art or using ChemDraw^{®} software, and supplier's catalog names are given for commercially available compounds.

### Detailed description of the preferred embodiment

The present application is described in detail below by way of examples. However, this is by no means disadvantageously limiting the scope of the present application. Although the present application has been described in detail herein and specific examples have also been disclosed, it will be apparent to those skilled in the art that various changes and modifications can be made to the specific embodiments without departing from the spirit and scope of the present application.

### Example 1: Preparation of Compound 1

### Step 1: Preparation of compound 1-2

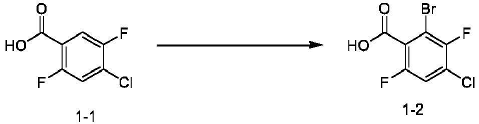

Diisopropylethylamine (1.34 g, 10.33 mmol, 1.80 mL) was dissolved in anhydrous tetrahydrofuran (50 mL). The resulting solution was cooled to -78 °C, and *n*-butyllithium (2.5 M, 47.78 mL) was added dropwise thereto. After the completion of the addition, the system was warmed to -30 °C and stirred for 10 min. The system was cooled to -78 °C, and a solution of compound 1-1 (10 g, 51.94 mmol) in tetrahydrofuran (50 mL) was added thereto. The system was stirred at -78 °C for 4 h. A solution of 1,2-dibromotetrachloroethane (33.82 g, 103.87 mmol) in tetrahydrofuran (50 mL) was added to the system. The system was stirred at -78 °C for 2 h, and then warmed to room temperature (20 °C) and stirred for 16 h. The system was quenched with water (500 mL) and left standing for separation. The aqueous phase was washed with methyl *tert*-butyl ether (3 × 30 mL) and acidified with 2 N aqueous solution of hydrochloric acid to pH = 2 (precipitate appeared). The system was extracted with ethyl acetate (3 × 200 mL), and the organic phases were pooled, dried over anhydrous sodium sulfate, and filtered. The filtrate was concentrated to obtain a crude product. The crude product was purified by medium-pressure column chromatography (ethanol/ethyl acetate (v/v) = 0-10%) to obtain 1-2 in the form of a pale yellow gum.

### Step 2: Preparation of compound 1-3

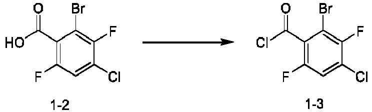

Compound 1-2 (12 g, 44.21 mmol) was dissolved in anhydrous dichloromethane (150 mL), and oxalyl chloride (8.42 g, 66.31 mmol, 5.80 mL) and 1 drop of *N,N*-dimethylformamide were successively added dropwise to the resulting solution at 0 °C. After the completion of the addition, the system was stirred at 0 °C for 15 min, and then warmed to room temperature (20 °C) and stirred for 2 h. The system was concentrated under reduced pressure to obtain crude 1-3, which was used in the next step without further purification.

### Step 3: Preparation of compound 1-4

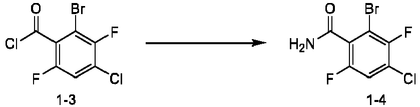

Compound 1-3 (12.8 g, 44.15 mmol) was dissolved in anhydrous dioxane (100 mL), and a solution of ammonia in methanol (7 M, 31.54 mL) was added dropwise to the resulting solution at 0 °C. After the completion of the addition, the system was stirred at room temperature (20 °C) for 2 h. The system was concentrated under reduced pressure, diluted with water (200 mL), and extracted with ethyl acetate (3 × 50 mL). The organic phases were pooled, dried over anhydrous sodium sulfate, and filtered. The filtrate was concentrated to obtain a crude product. The crude product was purified by medium-pressure column chromatography (ethyl acetate/petroleum ether (v/v) = 0-30%) to obtain 1-4 in the form of a pale yellow solid.

MS (ESI) *m*/*z* (M+H)⁺ = 269.9.

¹H NMR (400 MHz, DMSO-d₆) 8.14 (s, 1H), 7.96 (s, 1H), 7.83-7.80 (m, 1H).

### Step 4: Preparation of compound 1-6

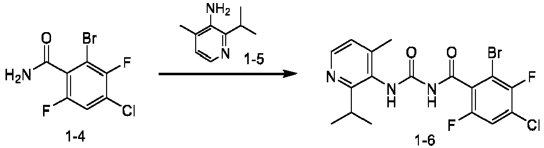

Compound 1-4 (6 g, 22.18 mmol) was dissolved in anhydrous 1,2-dichloroethane (60 mL), and oxalyl chloride (3.94 g, 31.06 mmol) was added dropwise to the resulting solution at room temperature (20 °C). After the completion of the addition, the system was warmed to 80 °C and stirred for 1 h. The reaction system was concentrated to half volume and cooled to 0 °C, and a solution of compound 1-5 (3.50 g, 23.29 mmol) in 1,2-dichloroethane (30 mL) was added dropwise thereto. After the completion of the addition, the system was stirred at room temperature (20 °C) for 1 h. The system was concentrated under reduced pressure. The crude product was dried under vacuum to obtain 1-6 in the form of a white solid, which was used in the next step without further purification.

### Step 5: Preparation of compound 1-7

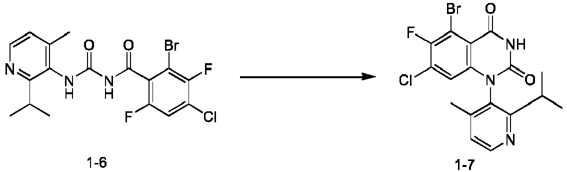

Compound 1-6 (9 g, 20.15 mmol) was dissolved in anhydrous tetrahydrofuran (150 mL), and sodium bis(trimethylsilyl)amide (1 M, 40.30 mL) was added to the resulting solution at 0 °C under a nitrogen atmosphere. After the completion of the addition, the system was warmed to room temperature (20 °C) and stirred for 1 h. The system was poured into water (500 mL), and extracted with ethyl acetate (3 × 150 mL). The organic phases were pooled, dried over anhydrous sodium sulfate, and filtered. The filtrate was concentrated to obtain a crude product. The crude product was purified by medium-pressure column chromatography (ethyl acetate/petroleum ether (v/v) = 0-30%) to obtain 1-7 in the form of a light white solid.

¹H NMR (400 MHz, DMSO-d₆) 8.58 (d, *J* = 5.2 Hz, 1H), 7.34 (d, *J* = 4.8 Hz, 1H), 6.40 (d, *J* = *6* Hz, 1H), 2.86-2.80 (m, 1H), 2.07 (s, 3H), 1.05-0.98 (m, 6 H)

### Step 6: Preparation of compound 1-8

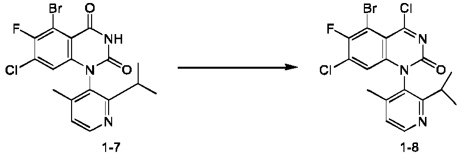

Compound 1-7 (6 g, 14.06 mmol) was dissolved in acetonitrile (150 mL), and diisopropylethylamine (7.27 g, 56.25 mmol) and phosphorus oxychloride (8.62 g, 56.25 mmol) were successively added to the resulting solution. After the completion of the addition, the system was warmed to 80 °C and stirred for 4 h. The system was quenched by pouring into ice water (250 mL), and extracted with ethyl acetate (3 × 100 mL). The organic phases were pooled, dried over anhydrous sodium sulfate, and filtered. The filtrate was concentrated to obtain a brown crude. The crude product was used in the next step without further purification.

### Step 7: Preparation of compound 1-10

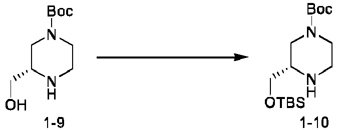

Compound 1-9 (3 g, 13.87 mmol) was dissolved in dichloromethane (40 mL), and imidazole (1.42 g, 20.81 mmol) and *tert*-butyldimethylchlorosilane (2.30 g, 15.26 mmol) were successively added to the resulting solution at 0 °C. After the completion of the addition, the system was warmed to room temperature (20 °C) and stirred for 16 h. The system was quenched with water (10 mL), and extracted with dichloromethane (2 × 10 mL). The organic phases were pooled, dried over anhydrous sodium sulfate, and filtered. The filtrate was concentrated to obtain a crude product. The crude product was purified by silica gel column chromatography (ethyl acetate/petroleum ether (v/v) = 0-100%) to obtain 1-10 in the form of a colorless oil.

### Step 8: Preparation of compound 1-11

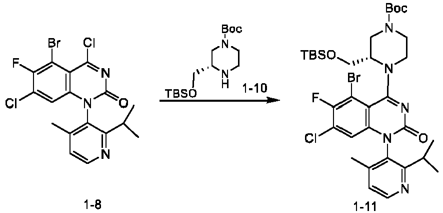

Compound 1-8 (5 g, 11.23 mmol) was dissolved in anhydrous 1,2-dichloroethane (50 mL), and diisopropylethylamine (1.89 g, 14.60 mmol, 2.54 mL) and a solution of compound 1-10 (3.71 g, 11.23 mmol) in 1,2-dichloroethane (5 mL) were successively added to the resulting solution at 0 °C. After the completion of the addition, the system was warmed to room temperature (20 °C) and stirred for 2 h. The reaction system was diluted with water (100 mL), and extracted with dichloromethane (3 × 50 mL). The organic phases were pooled, dried over anhydrous sodium sulfate, and filtered. The filtrate was concentrated to obtain a crude product. The crude product was purified by silica gel column chromatography (petroleum 88/ethyl acetate (v/v) = 0-75%) to obtain 1-11 in the form of a pale yellow gum.

### Step 9: Preparation of compound 1-12

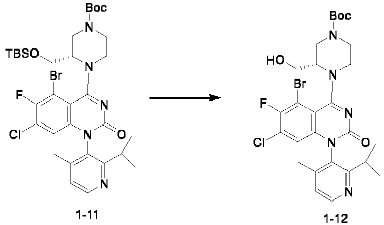

Compounds 1-11 (6.5 g, 8.79 mmol) were dissolved in anhydrous tetrahydrofuran (100 mL), and tetrabutylammonium fluoride (1 M, 10.55 mL) was added to the resulting solution at 0 °C. After the completion of the addition, the system was warmed to room temperature (20 °C) and stirred for 1 h. The system was concentrated to obtain a crude product, which was then purified by silica gel column chromatography (ethyl acetate/petroleum ether (v/v) = 30-90%) to obtain 1-12 in the form of a pale yellow solid.

¹H NMR (400 MHz, DMSO-d₆) 8.84 (s, 1H), 8.57-8.55 (m, 1H), 7.25-7.33 (m, 1H), 6.14-6.10 (m, 1H), 4.24-4.21 (m, 2H), 3.99-3.94 (m, 2H), 2.76-2.71 (m, 5H), 2.26-2.23 (m, 1H), 2.04-2.01 (m, 3H), 1.42 (s, 9 H), 1.11-1.04 (m, 6H)

### Step 10: Preparation of compound 1-13

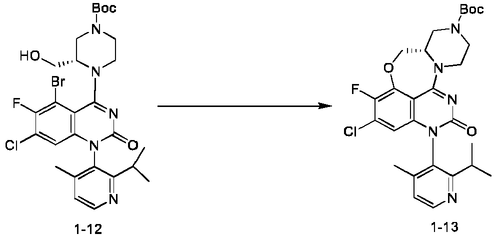

Compound 1-12 (400 mg, 640.07 µmol) was dissolved in toluene (10 mL), and tris(dibenzylideneacetone)dipalladium (36.80 mg, 64.01 µmol), 4,5-bis-diphenylphosphine-9,9-dimethylxanthene (74.07 mg, 128.01 µmol) and potassium phosphate (271.73 mg, 1.28 mmol) were successively added to the resulting solution. After the completion of the addition, the system was warmed to 100 °C and stirred for 16 h under an argon atmosphere. The system was filtered, and the filtrate was concentrated to obtain a crude product. The crude product was purified by silica gel column chromatography (petroleum 88/ethyl acetate (v/v) = 75-100%) and preparative high performance liquid chromatography (separation conditions: chromatographic column: YMC Triart C18 150 × 25 mM × 5 µm; mobile phase: [water (10 mM ammonium bicarbonate solution)-acetonitrile]; acetonitrile%: 52%-82%) to obtain compound 1-13 (HPLC retention time: 9.5 min), which was concentrated and lyophilized to obtain 1-13 in the form of a white solid.

MS (ESI) m/z (M+H)⁺ = 544.3.

### Step 11: Preparation of compound 1-14

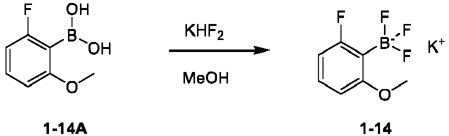

Compound 1-14A (2 g, 11.77 mmol) was dissolved in methanol (3 mL), and potassium bifluoride (4.5 M in water, 8 mL) was added to the resulting solution, with a white precipitate appeared. After the completion of the addition, the turbid system was stirred at room temperature (20 °C) for 2 h. The system was filtered, and the filter cake was washed successively with water (2 × 5 mL) and acetonitrile (5 mL). The resulting crude product was dried under vacuum to obtain 1-14 in the form of a white solid.

### Step 12: Preparation of compound 1-15

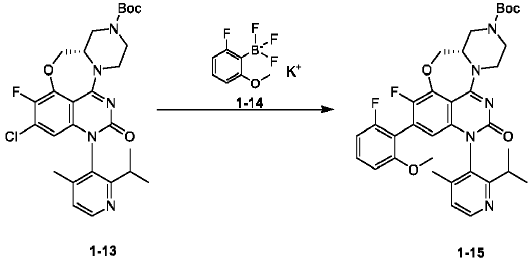

Compound 1-13 (30 mg, 55.15 µmol) was dissolved in dioxane (2 mL) and water (0.4 mL), and compound 1-14 (51.18 mg, 220.58 µmol), 2-dicyclohexylphosphonium-2,4,6-triisopropylbiphenyl (5.26 mg, 11.03 µmol), tris(dibenzylideneacetone)dipalladium (3.17 mg, 5.51 µmol) and potassium carbonate (30.49 mg, 220.58 µmol) were successively added to the resulting solution. After the completion of the addition, the system was warmed to 100 °C and stirred for 16 h under an Ar atmosphere. The system was concentrated to obtain a crude product. The crude product was purified by silica gel column chromatography (ethyl acetate/petroleum ether (v/v) = 0-100%) to obtain 1-15 in the form of a pale yellow solid.

MS (ESI) m/z (M+H)⁺ = 634.3.

### Step 13: Preparation of compound 1-16

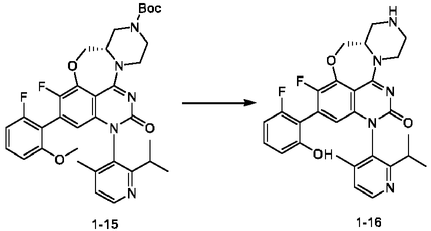

Compound 1-15 (20 mg, 31.56 µmol) was dissolved in anhydrous dichloromethane (1 mL), and boron tribromide (0.5 M in dichloromethane, 378.74 µL) was added to the resulting solution at 0 °C. After the completion of the addition, the system was warmed to room temperature (20 °C) and stirred for 4 h. Methanol (2 mL) was added to the system, and the resulting mixture was stirred for 10 min. The system was concentrated and then lyophilized to obtain 1-16 in the form of a light yellow solid (hydrobromide salt).

MS (ESI) *m*/*z* (M+H)⁺ = 520.3.

### Step 14: Preparation of compound 1

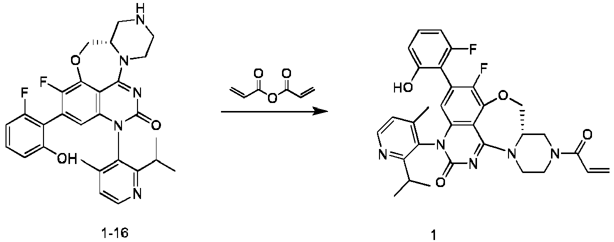

Compound 1-16 (8 mg, 13.32 µmol, hydrobromide salt) was dissolved in tetrahydrofuran (1 mL) and a saturated aqueous solutio of NaHCO (1 mL), and acrylic anhydride (0.2 M, 99.92 µL) was added to the resulting solution at room temperature (20 °C). After the completion of the addition, the system was stirred at room temperature (20 °C) for 1 h. Methanol (1 mL) and lithium hydroxide (0.2 M, 0.1 mL) were added to the system, which was then stirred at room temperature (20 °C) for another 2 h. The system was concentrated, diluted with methanol (2 mL), and filtered. The filtrate was concentrated to obtain a crude product. The crude product was purified by preparative high performance liquid chromatography (separation conditions: chromatographic column: YMC Triart C18 150 × 25 mm × 5 µm; mobile phase: [water (10 mM ammonium bicarbonate solution)-acetonitrile]; acetonitrile%: 29%-59%) to obtain compound 1 (HPLC retention time: 9.5 min).

MS (ESI) *m*/*z* (M+H)⁺ = 574.4.

¹H NMR (400 MHz, DMSO-d₆) 8.51 (d, *J=* 5.2 Hz, 1H), 7.34 (d, J = 4.8 Hz, 1H), 7.24-7.20 (m, 1H), 6.88-6.80 (m, 1H), 6.68-6.60 (m, 2H), 6.35-6.30 (m, 1H), 6.06-6.01 (m, 1H), 5.87-5.83 (m, 1H), 5.08-5.02 (m, 1H), 4.73-4.61 (m, 3H), 4.49-4.35 (m, 1H), 4.25-4.15 (m, 2H), 3.68-3.46 (m, 2H), 2.88-2.78 (m, 1H), 2.11-2.07 (m, 3H), 1.23-1.21 (m, 3 H), 1.21-1.07 (m, 3 H).

¹⁹F NMR (376 MHz, DMSO-d₆) -115.93, -139.68.

### Step 15: Resolution of compound 1 isomers

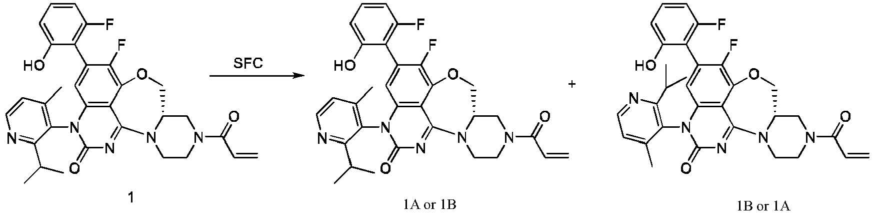

Diastereomer compound 1 (16 mg, 27.89 µmol) was purified by SFC (separation conditions: chromatographic column: DAICEL CHIRALCEL OJ-H (250 mm × 30 mm, 5 µm); mobile phase: [Neu-ethanol]; ethanol%: 30%-30%). After concentration, compound 1A (peak 1) and compound 1B (peak 2) were obtained.

### Compound 1A

¹H NMR (400 MHz, DMSO-d₆) 8.51 (d, *J* = 5.2 Hz, 1H), 7.34 (d, J = 4.8 Hz, 1H), 7.24-7.20 (m, 1H), 6.88-6.80 (m, 1H), 6.68-6.60 (m, 2H), 6.35-6.30 (m, 1H), 6.06-6.01 (m, 1H), 5.87-5.83 (m, 1H), 5.08-5.02 (m, 1H), 4.73-4.61 (m, 3H), 4.49-4.35 (m, 1H), 4.25-4.15 (m, 2H), 3.68-3.46 (m, 2H), 2.88-2.78 (m, 1H), 2.11-2.07 (m, 3H), 1.23-1.21 (m, 3 H), 1.21-1.07 (m, 3 H)

¹⁹F NMR (376 MHz, DMSO-d₆) -115.93, -139.68.

### HPLC retention time: 6.443 min

Separation conditions: chromatographic column: Waters Xbridge C18 3.5 µm, 150 × 4.6 mm; column temperature: 40 °C; mobile phase: water (0.05% aqueous ammonia)-acetonitrile; acetonitrile: 0%-95% 10 min, 95% 5 min; flow rate: 1.0 mL/min.

### SFC retention time: 3.436 min

Separation conditions: chromatographic column: Chiralcel OJ-3 100 × 4.6 mm I.D., 3 µm; column temperature: 35 °C; mobile phase: [Neu-isopropanol (0.05% DEA)], isopropanol%: 5%-40% in 4 min, 40% 2.5 min, 5% 1.5 min; flow rate: 2.8 mL/min. MS (ESI) *m*/*z* (M+H)⁺ = 574.4.

### Compound 1B

¹H NMR (400 MHz, DMSO-d₆) 8.51 (d, *J* = 5.2 Hz, 1H), 7.34 (d, J = 4.8 Hz, 1H), 7.24-7.20 (m, 1H), 6.88-6.80 (m, 1H), 6.68-6.60 (m, 2H), 6.35-6.30 (m, 1H), 6.06-6.01 (m, 1H), 5.87-5.83 (m, 1H), 5.08-5.02 (m, 1H), 4.73-4.61 (m, 3H), 4.49-4.35 (m, 1H), 4.25-4.15 (m, 2H), 3.68-3.46 (m, 2H), 2.88-2.78 (m, 1H), 2.11-2.07 (m, 3H), 1.23-1.21 (m, 3 H), 1.21-1.07 (m, 3 H)

### HPLC retention time: 6.498 min

Separation conditions: chromatographic column: Waters Xbridge C18 3.5 µm, 150 × 4.6 mm; column temperature: 40 °C; mobile phase: water (0.05% aqueous ammonia)-acetonitrile; acetonitrile: 0%-95% 10 min, 95% 5 min; flow rate: 1.0 mL/min.

### SFC retention time: 3.780 min

Separation conditions: chromatographic column: Chiralcel OJ-3 100 × 4.6 mm I.D., 3 µm; column temperature: 35 °C; mobile phase: [Neu-isopropanol (0.05% DEA)], isopropanol%: 5%-40% in 4 min, 40% 2.5 min, 5% 1.5 min; flow rate: 2.8 mL/min. MS (ESI) *m*/*z* (M+H)⁺ = 574.4.

### Example 2: Preparation of Compound 2

### Step 1: Preparation of compound 2-2

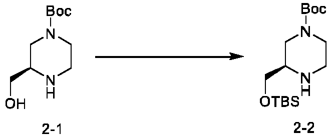

Compound 2-1 (2.4 g, 11.1 mmol) was dissolved in dichloromethane (30 mL), and imidazole (1.13 g, 16.65 mmol) and *tert*-butyldimethylchlorosilane (2.17 g, 14.43 mmol) were successively added to the resulting solution at 0 °C. After the completion of the addition, the system was warmed to room temperature (20 °C) and stirred for 16 h. The system was quenched with water (10 mL), and extracted with dichloromethane (2 × 10 mL). The organic phases were pooled, dried over anhydrous sodium sulfate, and filtered. The filtrate was concentrated to obtain a crude product. The crude product was purified by silica gel column chromatography (ethyl acetate/petroleum ether (v/v) = 0-100%) to obtain compound 2-2 in the form of a colorless oil.

¹H NMR (400 MHz, CDC13) 4.02-3.85 (m, 2H), 3.65-3.55 (m, 1H), 3.49-3.42 (m, 1H), 3.03-2.97 (m, 1H), 2.82-2.75 (m, 1H), 2.75-2.68 (m, 2H), 2.55-2.45 (m, 1H), 1.46 (s, 9H), 0.90 (s, 9H), 0.06 (s, 6 H).

### Step 2: Preparation of compound 2-3

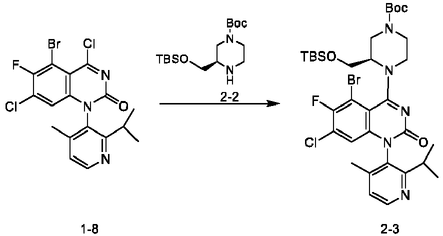

Compound 1-8 (2 g, 4.49 mmol) was dissolved in anhydrous acetonitrile (20 mL), and diisopropylethylamine (4.65 g, 35.92 mmol, 6.26 mL) and a solution of compound 2-2 (1.48 g, 4.49 mmol) in acetonitrile (10 mL) were successively added to the resulting solution at 0 °C. After the completion of the addition, the system was warmed to room temperature (20 °C) and stirred for 2 h. The reaction system was diluted with water (100 mL), and extracted with ethyl acetate (3 × 50 mL). The organic phases were pooled, dried over anhydrous sodium sulfate, and filtered. The filtrate was concentrated to obtain a crude product. The crude product was purified by silica gel column chromatography (petroleum 88/ethyl acetate (v/v) = 0-75%) to obtain compound 2-3 in the form of a pale yellow gum.

### Step 3: Preparation of compound 2-4

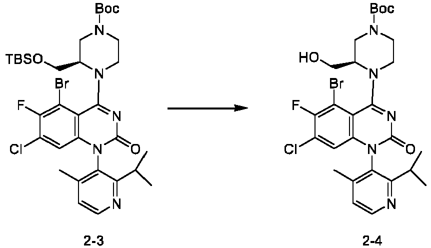

Compounds 2-3 (3.00 g, 4.06 mmol) were dissolved in anhydrous tetrahydrofuran (100 mL), and tetrabutylammonium fluoride (1.17 g, 4.46 mmol) was added to the resulting solution at 0 °C. After the completion of the addition, the system was warmed to room temperature (20 °C) and stirred for 1 h. The system was concentrated to obtain a crude product, which was then purified by silica gel column chromatography (ethyl acetate/petroleum ether (v/v) = 30-90%) to obtain compound 2-4 in the form of a pale yellow solid.

¹H NMR (400 MHz, DMSO-d₆) 8.84 (s, 1H), 8.57-8.55 (m, 1H), 7.25-7.33 (m, 1H), 6.14-6.10 (m, 1H), 4.24-4.21 (m, 2H), 3.99-3.94 (m, 2H), 2.76-2.71 (m, 4H), 2.26-2.23 (m, 1H), 2.04-2.01 (m, 3H), 1.42 (s, 9H), 1.11-1.04 (m, 6H)

### Step 4: Preparation of compound 2-5

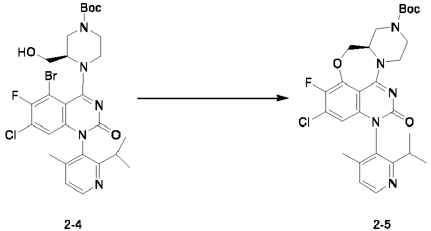

Compound 2-4 (400 mg, 640.07 µmol) was dissolved in toluene (10 mL), and tris(dibenzylideneacetone)dipalladium (36.80 mg, 64.01 µmol), 4,5-bis-diphenylphosphine-9,9-dimethylxanthene (74.07 mg, 128.01 µmol) and potassium phosphate (271.73 mg, 1.28 mmol) were successively added to the resulting solution. After the completion of the addition, the system was warmed to 100 °C and stirred for 16 h under an argon atmosphere. The system was filtered, and the filtrate was concentrated to obtain a crude product. The crude product was purified by silica gel column chromatography (petroleum ether/ethyl acetate (v/v) = 75-100%) and preparative high performance liquid chromatography (separation conditions: chromatographic column: YMC Triart C18 150 × 25 mM × 5 µm; mobile phase: [water (10 mM ammonium bicarbonate solution)-acetonitrile]; acetonitrile%: 52%-82%) to obtain compound 1-13 (HPLC retention time: 9.5 min), which was concentrated and lyophilized to obtain compound 2-5 in the form of a white solid.

MS (ESI) *m*/*z* (M+H)⁺ = 544.3.

### Step 5: Preparation of compound 2-6

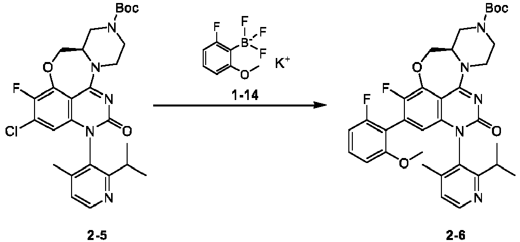

Compound 2-5 (15 mg, 27.57 µmol) was dissolved in dioxane (0.5 mL) and water (0.1 mL), and compound 1-14 (19.19 mg, 82.72 µmol), 2-dicyclohexylphosphonium-2,4,6-triisopropylbiphenyl (2.63 mg, 5.51 µmol), tris(dibenzylideneacetone)dipalladium (1.59 mg, 2.76 µmol) and potassium carbonate (11.43 mg, 82.73 µmol) were successively added to the resulting solution. After the completion of the addition, the system was warmed to 100 °C and stirred for 16 h under an Ar atmosphere. The system was concentrated to obtain a crude product. The crude product was purified by silica gel column chromatography (ethyl acetate/petroleum ether (v/v) = 0-100%) to obtain compound 2-6 in the form of a pale yellow solid.

### Step 6: Preparation of compound 2-7

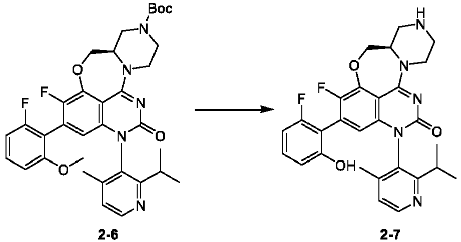

Compound 2-6 (15 mg, 23.67 µmol) was dissolved in anhydrous dichloromethane (1 mL), and boron tribromide (0.5 M in dichloromethane, 236.71 µL) was added to the resulting solution at 0 °C. After the completion of the addition, the system was warmed to room temperature (20 °C) and stirred for 4 h. Methanol (2 mL) was added to the system, and the resulting mixture was stirred for 10 min. The system was concentrated and then lyophilized to obtain compound 2-7 in the form of a light yellow solid (hydrobromide salt).

### Step 7: Preparation of compound 2

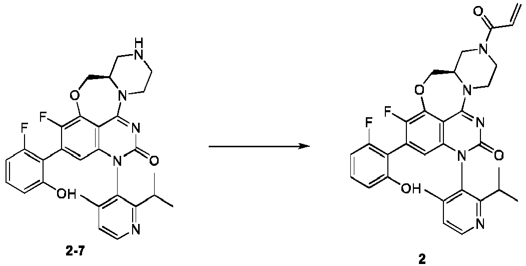

Compound 2-7 (14 mg, 26.95 µmol, hydrobromid salt) was dissolved in tetrahydrofuran (1 mL) and a saturated aqueous solution of sodium bicarbonate (1 mL), and acrylic anhydride (0.2 M, 161.68 µL) was added to the resulting solution at room temperature (20 °C). After the completion of the addition, the system was stirred at room temperature (20 °C) for 1 h. Methanol (1 mL) and lithium hydroxide (0.2 M, 0.1 mL) were added to the system, which was then stirred at room temperature (20 °C) for another 2 h. The system was concentrated, diluted with meth nol (2 mL), and filtered. The filtrate was concentrated to obtain a crude product. The crude product was purified by preparative high performance liquid chromatography (separation conditions: chromatographic column: YMC Triart C18 150 × 25 mm × 5 µm; mobile phase: [water (10 mM ammonium bicarbonate solution)-acetonitrile]; acetonitrile%: 29%-59% 9.5 min) to obtain compound 2.

¹H NMR (400 MHz, DMSO-d₆) 8.51 (d, *J=* 5.2 Hz, 1H), 7.34 (d, J = 4.8 Hz, 1H), 7.24-7.20 (m, 1H), 6.88-6.80 (m, 1H), 6.68-6.60 (m, 2H), 6.35-6.30 (m, 1H), 6.06-6.01 (m, 1H), 5.87-5.83 (m, 1H), 5.08-5.02 (m, 1H), 4.73-4.61 (m, 3H), 4.49-4.35 (m, 1H), 4.25-4.15 (m, 2H), 3.68-3.46 (m, 2H), 2.88-2.78 (m, 1H), 2.11-2.07 (m, 3H), 1.23-1.21 (m, 3 H), 1.21-1.07 (m, 3 H)

MS (ESI) *m*/*z* (M+H)⁺ = 574.4.

### Step 8: Preparation of compounds 2A and 2B

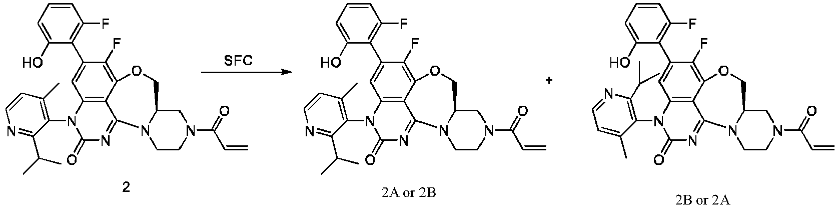

Diastereomer compound 2 was purified by SFC (separation conditions: chromatographic column: DAICEL CHIRALPAK AD-H (250 mm × 30 mm, 5 µm);mobile phase: [Neu-isopropanol (0.1% aqueous ammonia)]; isopropanol%: 35%). After concentration, compound 2A (peak 1) and compound 2B (peak 2) were obtained.

### Compound 2A:

**¹H NMR** (400 MHz, DMSO-d₆) 8.51 (d, *J=* 5.2 Hz, 1H), 7.34 (d, *J=* 5.2 Hz, 1H), 7.24-7.20 (m, 1H), 6.88-6.80 (m, 1H), 6.68-6.60 (m, 2H), 6.35-6.30 (m, 1H), 6.06-6.01 (m, 1H), 5.87-5.83 (m, 1H), 5.08-5.02 (m, 1H), 4.73-4.31 (m, 4H), 4.25-4.15 (m, 2H), 3.68-3.46 (m, 2H), 2.88-2.78 (m, 1H), 2.11-2.07 (m, 3H), 1.23-1.05 (m, 6 H)

**¹⁹F NMR** (376 MHz, METHANOL-d₄) δ = -115.6 (s, IF), -139.6 (s, IF)

**MS (ESI)** *m*/*z* (M+H)⁺ = 574.4.

HPLC retention time: 6.91 min

Separation conditions: chromatographic column: WELCH Ultimate LP-C18 150 × 4.6 mm 5 µm; column temperature: 40 °C; mobile phase: water (0.0688% trifluoroacetic acid solution)-acetonitrile (0.0625% trifluoroacetic acid solution); acetonitrile: 10%-80% 10 min, 80% 5 min; flow rate: 1.5 mL/min.

**SFC** retention time: 1.657 min.

Separation conditions: chromatographic column: ChiralpakAD-3 150 × 4.6 mm I.D., 3 µm; column temperature: 35 °C; mobile phase: [Neu-isopropanol (0.05% DEA)]; isopropanol%: 5%-40% in 2 min, 40% 1.2 min, 5% 0.8 min; flow rate: 4 mL/min.

### Compound 2B:

**¹H NMR** (400 MHz, DMSO-d₆) 8.40 (d, *J* = *5.2* Hz, 1H), 7.22 (d, J = 5.2 Hz, 1H), 7.14-7.07 (m, 1H), 6.80-6.75 (m, 1H), 6.58-6.45 (m, 2H), 6.25-6.15 (m, 1H), 6.01-5.91 (m, 1H), 5.75-5.70 (m, 1H), 4.98-4.92 (m, 1H), 4.63-4.21 (m, 4H), 4.15-4.05 (m, 2H), 3.60-3.37 (m, 2H), 2.78-2.68 (m, 1H), 2.01-1.95 (m, 3H), 1.13-0.95 (m, 6 H)

**¹⁹F NMR** (376 MHz, METHANOL-d₄) δ = -115.7 (s, IF), -139.6 (s, IF)

**MS (ESI)** *m*/*z* (M+H)⁺ = 574.4.

HPLC retention time: 6.91 min

Separation conditions: chromatographic column: WELCH Ultimate LP-C18 150 × 4.6 mm 5 µm; column temperature: 40 °C; mobile phase: water (0.0688% trifluoroacetic acid solution)-acetonitrile (0.0625% trifluoroacetic acid solution); acetonitrile: 10%-80% 10 min, 80% 5 min; flow rate: 1.5 mL/min.

**SFC** retention time: 1.844 min.

Separation conditions: chromatographic column: ChiralpakAD-3 150 × 4.6 mm I.D., 3 µm; column temperature: 35 °C; mobile phase: [Neu-isopropanol (0.05% DEA)]; isopropanol%: 5%-40% in 2 min, 40% 1.2 min, 5% 0.8 min; flow rate: 4 mL/min.

### Example 3: Preparation of Compound 3

### Step 1: Preparation of compound 3-2

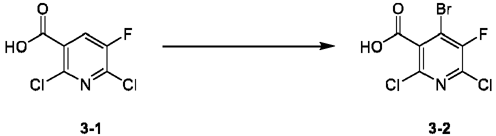

Diisopropylethylamine (861.66 mg, 6.67 mmol, 1.16 mL) was dissolved in anhydrous tetrahydrofuran (40 mL). The resulting solution was cooled to -78 °C, and *n*-butyllithium (2.5 M, 26.67 mL) was added dropwise thereto. After the completion of the addition, the system was warmed to -30 °C and stirred for 10 min. The system was cooled to -78 °C, and a solution of compound 3-1 (7 g, 33.33 mmol) in tetrahydrofuran (40 mL) was added thereto. The system was stirred at -78 °C for 4 h. A solution of 1,2-dibromotetrachloroethane (21.71 g, 66.67 mmol) in tetrahydrofuran (40 mL) was added to the system. The system was stirred at -78 °C for 2 h, and then warmed to room temperature (20 °C) and stirred for 16 h. The system was quenched with water (200 mL) and left standing for separation. The aqueous phase was washed with methyl *tert*-butyl ether (3 × 30 mL) and acidified with 2 N aqueous solution of hydrochloric acid to pH = 2 (precipitation occurred). The system was extracted with ethyl acetate (3 × 50 mL), and the organic phases were pooled, dried over anhydrous sodium sulfate, and filtered. The filtrate was concentrated to obtain a crude product. The crude product was slurried with petroleum ether (20 mL) to obtain compound 3-2.

### Step 2: Preparation of compound 3-3

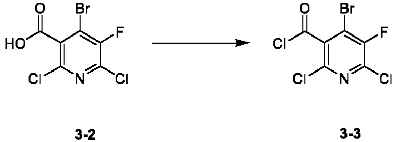

Compound 3-2 (9 g, 31.15 mmol) was dissolved in anhydrous dichloromethane (80 mL), and oxalyl chloride (5.93 g, 46.73 mmol, 4.09 mL) and 1 drop of *N,N*-dimethylformamide were successively added dropwise to the resulting solution at 0 °C. After the completion of the addition, the system was stirred at 0 °C for 15 min, and then warmed to room temperature (20 °C) and stirred for 2 h. The system was concentrated under reduced pressure to obtain compound 3-3, which was used in the next step without further purification.

### Step 3: Preparation of compound 3-4

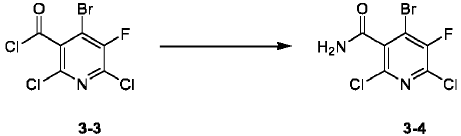

Compound 3-3 (9 g, 29.28 mmol) was dissolved in anhydrous dioxane (80 mL), and a solution of ammonia in methanol (7 M, 20 mL) was added dropwise to the resulting solution at 0 °C. After the completion of the addition, the system was stirred at room temperature (20 °C) for 2 h. The system was concentrated, diluted with water (200 mL), and filtered. The filter cake was washed with water (2 × 10 mL), and dried under vacuum to obtain compound 3-4.

**¹H NMR** (400 MHz, DMSO-d₆) δ = 8.20 (s, 1H), 8.14 (s, 1H)

### Step 4: Preparation of compound 3-5

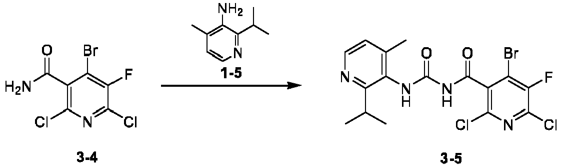

Compound 3-4 (7.5 g, 26.05 mmol) was dissolved in anhydrous 1,2-dichloroethane (80 mL), and oxalyl chloride (4.63 g, 36.47 mmol) was added dropwise to the resulting solution at room temperature (20 °C). After the completion of the addition, the system was warmed to 80 °C and stirred for 1 h. The reaction system was concentrated to half volume and cooled to 0 °C, and a solution of compound 1-5 (4.11 g, 27.35 mmol) in 1,2-dichloroethane (20 mL) was added dropwise thereto. After the completion of the addition, the system was stirred at room temperature (20 °C) for 1 h. The system was concentrated under reduced pressure. The crude product was dried under vacuum to obtain compound 3-5, which was used in the next step without further purification.

**MS (ESI)** *m*/*z* (M+H)⁺ = 465.0.

### Step 5: Preparation of compound 3-6

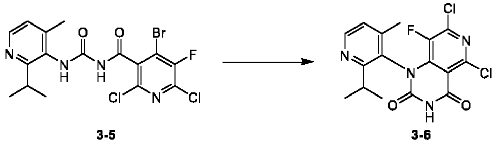

Compound 3-5 (12 g, 20.68 mmol) was dissolved in anhydrous tetrahydrofuran (100 mL), and potassium bis(trimethylsilyl)amide (1 M, 45.51 mL) was added to the resulting solution at 0 °C under a nitrogen atmosphere. After the completion of the addition, the system was warmed to room temperature (20 °C) and stirred for 1 h. The system was poured into water (300 mL), and extracted with ethyl acetate (3 × 150 mL). The organic phases were pooled, dried over anhydrous sodium sulfate, and filtered. The filtrate was concentrated to obtain a crude product. The crude product was purified by medium-pressure column chromatography (ethyl acetate/petroleum ether (v/v) = 0-70%) to obtain compound 3-6.

**¹H NMR** (400 MHz, DMSO-d₆) δ = 12.42 (br, 1H), 8.53 (d, *J* = 4.8, 1H), 7.27 (d, *J* = 4.8, 1H), 3.11-3.04 (m, 1H), 2.13 (s,3H), 1.09-1.03 (m, 6H)

**MS (ESI)** *m*/*z* (M+H)⁺ = 383.0.

### Step 6: Preparation of compound 3-7

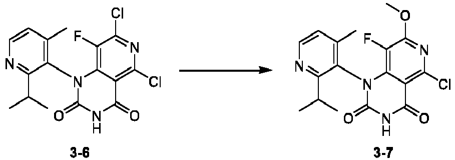

Compound 3-6 (1.5 g, 3.91 mmol) was dissolved in methanol (35 mL), and sodium methoxide (465.23 mg, 8.61 mmol) was added dropwise to the resulting solution at room temperature (20 °C). After the completion of the addition, the system was warmed to 60 °C and stirred for 16 h. The system was concentrated under reduced pressure to obtain crude 3-7, which was used in the next step without further purification.

**MS (ESI)** *m*/*z* (M+H)⁺ = 379.0.

### Step 7: Preparation of compound 3-9

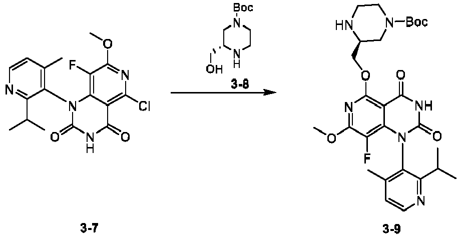

Compounds 3-7 (2.33 g, 10.77 mmol) were dissolved in tetrahydrofuran (80 mL), and sodium hydride (718.02 mg, 17.95 mmol, 60% purity) was added to the resulting solution at 0 °C. After the completion of the addition, the system was warmed to room temperature (20 °C) and stirred for 20 min, and compound 3-8 (3.4 g, 8.98 mmol) was added thereto. After the completion of the addition, the system was warmed to 60 °C and stirred for 2 h. The system was quenched with a saturated aqueous solution of ammonium chloride (2 mL) and filtered. The filtrate was concentrated to obtain a crude product. The crude product was purified by medium-pressure column chromatography (methanol/dichloromethane (v/v) = 0-10%) to obtain compound 3-9.

**MS (ESI)** *m*/*z* (M+H)⁺ = 559.2.

### Step 8: Preparation of compound 3-10

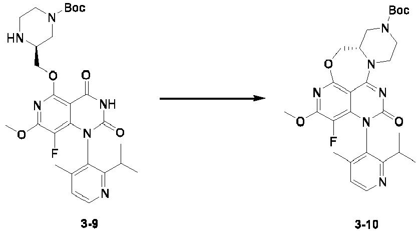

Compound 3-9 (0.9 g, 1.61 mmol) and 1*H*-benzotriazol-1-yloxytripyrrolidinyl hexafluorophosphate (1.68 g, 3.22 mmol) were dissolved in acetonitrile (10 mL), and 1,8-diazabicyclo[5.4.0]undec-7-ene (1.23 g, 8.06 mmol, 1.21 mL) was added dropwise to the resulting solution at 0 °C. After the completion of the addition, the system was warmed to room temperature (20 °C) and stirred for 16 h under a nitrogen atmosphere. The reaction system was diluted with water (20 mL), and extracted with ethyl acetate (3 × 20 mL). The organic phases were pooled, dried over anhydrous sodium sulfate, and filtered. The filtrate was concentrated to obtain a crude product. The crude product was purified by silica gel column chromatography (petroleum ether/ethyl acetate (v/v) = 0-100%) to obtain compound 3-10.

**¹H NMR** (400 MHz, DMSO-*d*₆) δ = 8.44 (d, *J=* 4.8 Hz, 1H), 7.28-7.13 (m, 1H), 4.72-4.53 (m, 3H), 4.04 (br s, 2H), 3.88 (s, 4H), 3.18 (br s, 3H), 2.92-2.81 (m, 1H), 2.02 (d, *J=* 4.9 Hz, 3H), 1.45 (s, 9H), 1.16-0.99 (m, 6H)

**MS (ESI)** *m*/*z* (M+H)⁺ = 541.3.

### Step 9: Preparation of compound 3-11

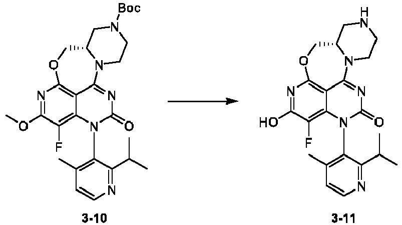

Compound 3-10 (350 mg, 647.45 µmol) was dissolved in anhydrous dichloromethane (2 mL), and boron tribromide (1.62 g, 6.47 mmol) was added to the resulting solution at 0 °C. After the completion of the addition, the system was warmed to room temperature (20 °C) and stirred for 16 h. Methanol (10 mL) was added to the system, and the resulting mixture was stirred for 10 min. The system was concentrated and lyophilized to obtain compound 3-11.

### Step 10: Preparation of compound 3-12

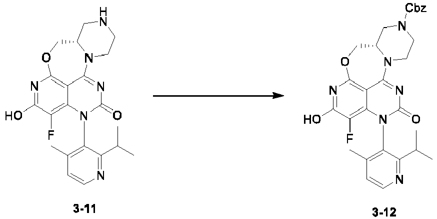

Compound 3-11 (270 mg, 506.52 µmol) was dissolved in a mixed solvent of tetrahydrofuran (1 mL) and saturated sodium bicarbonate (2 mL), and CbzCl (0.5 M, 1.52 mL) was added to the resulting solution at room temperature (20 °C). After the completion of the addition, the system was stirred at room temperature (20 °C) for 4 h. Methanol (2 mL) and an aqueous solution of lithium hydroxide (2 N, 0.2 mL) were added to the system. After the completion of the addition, the system was stirred at room temperature for 1 h. The system was concentrated to obtain a crude product. The crude product was purified by silica gel column chromatography (methanol/dichloromethane (v/v) = 0-10%) to obtain compound 3-12.

**¹H NMR** (400 MHz, DMSO-d₆) δ = 8.38 (d, *J* = 4.6 Hz, 1H), 7.44-7.27 (m, 5H), 7.20-7.12 (m, 1H), 5.21-5.09 (m, 2H), 4.70-4.46 (m, 3H), 4.09-3.85 (m, 3H), 3.57-3.54 (m, 3H), 2.91-2.81 (m, 1H), 2.02 (d, *J* = 6.4 Hz, 3H), 1.11-1.01 (m, 6H).

### Step 11: Preparation of compound 3-13

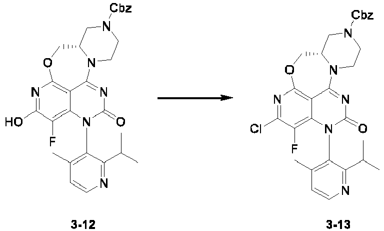

Compound 3-12 (250 mg, 445.97 µmol) was dissolved in acetonitrile (5 mL), and diisopropylethylamine (230.55 mg, 1.78 mmol) and phosphorus oxychloride (2.05 g, 13.38 mmol) were successively added to the resulting solution. After the completion of the addition, the system was warmed to 80 °C and stirred for 2 h. The system was cooled to room temperature and concentrated. The residue was quenched by pouring into ice water (10 mL). The resulting mixture was adjusted to neutral pH with saturated sodium bicarbonate, and extracted with ethyl acetate (3 × 10 mL). The organic phases were pooled, dried over anhydrous sodium sulfate, and filtered. The filtrate was concentrated to obtain a crude product. The crude product was purified by silica gel column chromatography (methanol/dichloromethane (v/v) = 0-10%) to obtain compound 3-13.

**MS (ESI)** *m*/*z* (M+H)⁺ = 579.3.

### Step 12: Preparation of compound 3-14

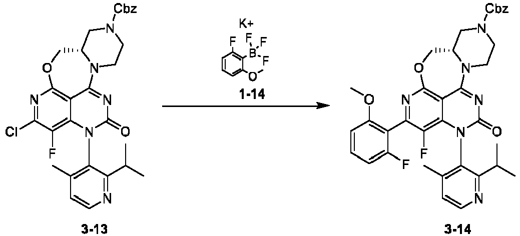

Compound 3-13 (60 mg, 103.62 µmol) was dissolved in dioxane (0.8 mL) and water (0.2 mL), and compound 1-14 (72.13 mg, 310.87 µmol), 2-dicyclohexylphosphonium-2,4,6-triisopropylbiphenyl (9.88 mg, 20.72 µmol), tris(dibenzylideneacetone)dipalladium (9.49 mg, 10.36 µmol) and potassium carbonate (42.97 mg, 310.87 µmol) were successively added to the resulting solution. After the completion of the addition, the system was warmed to 100 °C and stirred for 16 h under an Ar atmosphere. The system was concentrated to obtain a crude product. The crude product was purified by silica gel column chromatography (methanol/dichloromethane (v/v) = 0-10%) to obtain compound 3-14.

**MS (ESI)** *m*/*z* (M+H)⁺ = 669.2.

### Step 13: Preparation of compound 3-15

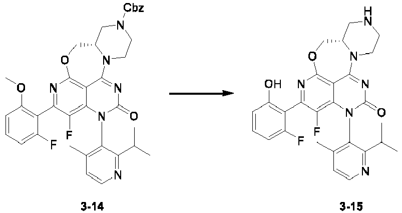

Compound 3-14 (18 mg, 26.92 µmol) was dissolved in anhydrous dichloromethane (1 mL), and boron tribromide (1 M in dichloromethane, 269.18 µL) was added to the resulting solution at 0 °C. After the completion of the addition, the system was warmed to room temperature (20 °C) and stirred for 4 h. Methanol (2 mL) was added to the system, and the resulting mixture was stirred for 10 min. The system was concentrated and lyophilized to obtain compound 3-15 (HBr salt).

**MS (ESI)** *m*/*z* (M+H)⁺ = 521.1.

### Step 14: Preparation of compound 3

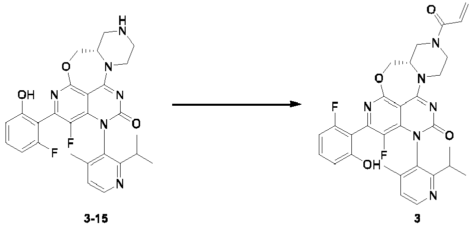

Compound 1-16 (13.85 mg, 21.28 µmol, HBr salt) was dissolved in tetrahydrofuran (1 mL) and a saturated aqueous solution of sodium bicarbonate (1 mL), and acrylic anhydride (0.2 M, 161.68 µL) was added to the resulting solution at room temperature (20 °C). After the completion of the addition, the system was stirred at room temperature (20 °C) for 1 h. Methanol (1 mL) and lithium hydroxide (0.2 M, 0.1 mL) were added to the system, which was then stirred at room temperature (20 °C) for another 2 h. The system was concentrated, diluted with methanol (2 mL), and filtered. The filtrate was concentrated to obtain a crude product. The crude product was purified by preparative high performance liquid chromatography (separation conditions: chromatographic column: Phenomenex Gemini-NX 80 × 30 mm × 3 µm; mobile phase: [water (10 mM ammonium bicarbonate solution)-acetonitrile]; acetonitrile%: 10%-80%, 9.5 min) to obtain compound 3 in the form of a white solid.

**¹H NMR** (400 MHz, METHANOL-d₄) δ = 8.44 (d, *J* = 5.0 Hz, 1H), 7.32-7.21 (m, 2H), 6.95-6.79 (m, 1H), 6.73-6.61 (m, 2H), 6.32 (dd, *J=* 1.7, 16.7 Hz, 1H), 5.85 (dd, *J* = 1.7, 10.7 Hz, 1H), 4.86-4.66 (m, 3H), 4.60-4.14 (m, 3H), 3.79-3.37 (m, 3H), 2.99 (qd, *J=* 6.5, 13.1 Hz, 1H), 2.19 (s, 3H), 1.26-1.13 (m, 6H)

**¹⁹F NMR** (376 MHz, METHANOL-*d*₄) δ = -116.81 (s, IF), -137.44 --140.32 (m, IF)

**MS (ESI)** *m*/*z* (M+H)⁺ = 575.5.

### Example 4: Preparation of Compound 4

### Step 1: Preparation of compound 4-1

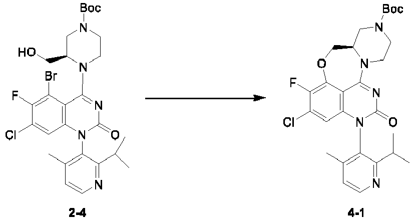

Compound 2-4 (750 mg, 1.20 mmol) was dissolved in acetonitrile (20 mL), and (2-di-*tert*-butylphosphino-3, 6-dimethoxy-2',4',6'-triisopropyl-1,1'-biphenyl) (2-amino-1,1'-biphenyl-2-yl)palladium methanesulfonate (150 mg, 175.56 µmol), 2-di-*tert*-butylphosphine-2',4',6'-triisopropyl-3,6-dimethoxy-1,1'-biphenyl (75.00 mg, 154.74 µmol) and cesium carbonate (825 mg, 2.53 mmol) were successively added to the resulting solution. After the completion of the addition, the system was warmed to 80 °C and stirred for 16 h under an argon atmosphere. The system was filtered, and the filtrate was concentrated to obtain a crude product. The crude product was purified by silica gel column chromatography (petroleum ether/ethyl acetate (v/v) = 70% to methanol/dichloromethane (v/v) = 30%) to obtain compound 4-1.

**¹H NMR** (400 MHz, Methanol-*d*₄) δ 8.57 (d, *J* = 4.9 Hz, 1H), 7.38 (d, *J* = 5.1 Hz, 1H), 6.12 (d, *J=* 6.0 Hz, 1H), 4.97 (br d, *J=* 10.8 Hz, 1H), 4.78-4.55 (m, 2H), 4.22-3.96 (m, 3H), 3.48-3.35 (m, 3H), 2.88-2.64 (m, 1H), 2.08 (d, *J* = 4.4Hz, 3H), 1.51 (s, 9H), 1.18 (dd, *J* = 4.1, 6.7 Hz, 3H), 1.10 (d, *J* = 6.8 Hz, 3H).

**MS (ESI)** *m*/*z* (M+H)⁺ = 544.2.

### Step 2: Preparation of compound 4-3

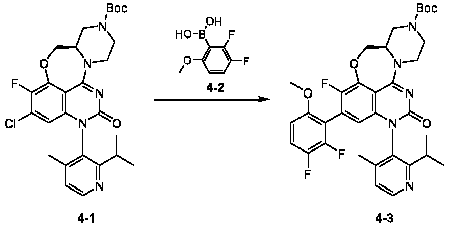

Compound 4-1 (60 mg, 110.29 µmol) was dissolved in dioxane (2.5 mL) and water (0.5 mL), and compound 4-2 (60.00 mg, 319.26 µmol), 2-dicyclohexylphosphine-2,4,6-triisopropylbiphenyl (12 mg, 25.17 µmol), (2-dicyclohexylphosphino-2',4',6'-tri-isopropyl-1,1'-biphenyl)(2'-amino-1,1'-biphenyl-2-yl)palladium methanesulfonate (12 mg, 14.18 µmol) and potassium carbonate (60 mg, 434.12 µmol) were successively added to the resulting solution. After the completion of the addition, the system was warmed to 100 °C and stirred for 16 h under an argon atmosphere. The system was concentrated to obtain a crude product. The crude product was purified by silica gel column chromatography (methanol/dichloromethane (v/v) = 0-7%) to obtain compound 4-3.

**¹H NMR** (400 MHz, Methanol-*d*₄) δ 8.59-8.46 (m, 1H), 7.44-7.20 (m, 2H), 6.83 (d, *J* = 8.6 Hz, 1H), 6.10-5.90 (m, 1H), 5.03 (br d, *J=* 12.8 Hz, 1H), 4.75-4.58 (m, 2H), 4.25-4.02 (m, 3H), 3.78-3.61 (m, 3H), 3.54-3.33 (m, 3H), 2.91-2.72 (m, 1H), 2.11-2.02 (m, 3H), 1.52 (s, 9H), 1.25-1.18 (m, 3H), 1.13-1.00 (m, 3H).

**MS (ESI)** *m*/*z* (M+H)⁺ = 652.4.

### Step 3: Preparation of compound 4-4

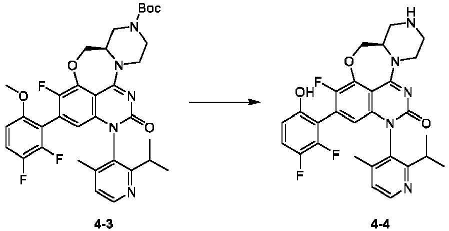

Compound 4-3 (65 mg, 99.74 µmol) was dissolved in anhydrous dichloromethane (1.5 mL), and boron tribromide (1 M, 598.46 µL) was added to the resulting solution at 0 °C. After the completion of the addition, the system was warmed to room temperature (20 °C) and stirred for 2 h. Methanol (2 mL) was added to the system, and the resulting mixture was stirred for 10 min. The system was concentrated and lyophilized to obtain compound 4-4 (hydrobromide salt).

**MS (ESI)** *m*/*z* (M+H)⁺ = 538.3.

### Step 4: Preparation of compound 4

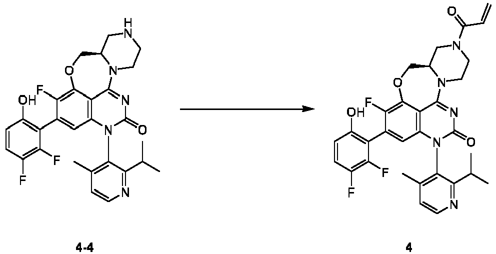

Compound 4-4 (80 mg, 129.36 µmol, hydrobromide salt) was dissolved in tetrahydrofuran (5 mL) and a saturated aqueous solution of sodium bicarbonate (3 mL), and acrylic anhydride (20 mg, 158.59 µmol) was added to the resulting solution at room temperature (20 °C). After the completion of the addition, the system was stirred at room temperature (20 °C) for 2 h. Methanol (3 mL) and lithium hydroxide (20 mg, 476.60 µmol) were added to the system, which was then stirred at room temperature (20 °C) for another 2 h. The system was adjusted to neutral pH with 1 N HCl and extracted with ethyl acetate (10 mL × 2). The organic phase was dried over anhydrous sodium sulfate and filtered. The filtrate was concentrated to obtain a crude product. The crude product was purified by preparative high performance liquid chromatography (separation conditions: chromatographic column: Phenomenex Gemini-NX 80 × 30 mm × 3 µm; mobile phase: [water (10 mM ammonium bicarbonate solution)-acetonitrile]; acetonitrile%: 10%-80% 9.5 min; flow rate: 30 mL/min) to obtain compound 4.

**¹H NMR** (400 MHz, Methanol-*d*₄) δ 8.51 (d, *J=* 5.0 Hz, 1H), 7.33 (d, *J* = 5.3 Hz, 1H), 7.16-7.09 (m, 1H), 6.94-6.82 (br s, 1H), 6.62 (br s, 1H), 6.31 (d, *J* = 15.3 Hz, 1H), 6.09-6.03 (br s, 1H), 5.84 (d, *J=* 12.3 Hz, 1H), 5.08-4.99 (m, 1H), 4.76-4.68 (m, 2H), 4.61 (s, 1H), 4.51-4.31 (m, 1H), 4.27-4.18 (m, 1H), 3.80-3.40 (m, 3H), 2.90 -2.80 (m, 1H), 2.14-1.99 (m, 3H), 1.27-1.00 (m, 6H).

**MS (ESI)** *n*/*z* (M+H)⁺ = 592.3.

### Step 5: Preparation of compounds 4A and 4B

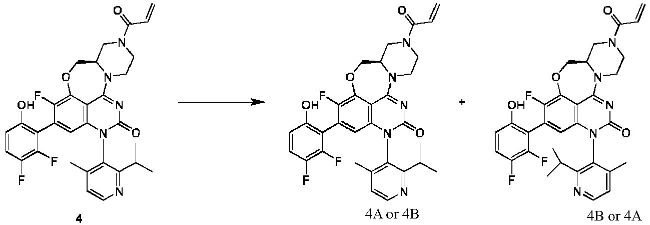

Diastereomer compound 4 was purified by SFC (separation conditions: chromatographic column: DAICEL CHIRALPAK AD (250 mm × 30 mm, 10 µm); mobile phase: [Neu-isopropanol (0.1% aqueous ammonia)]; isopropanol%: 30%-30%; flow rate: 70 mL/min) After concentration, compound 4A (peak 1) and compound 4B (peak 2) were obtained.

### Compound 4A

**¹H NMR** 400 MHz, Methanol- *d*4) δ 8.48 (d, *J*=4.9 Hz, 1H), 7.30 (d, *J* = 4.9 Hz, 1H), 7.13-7.16 (m, 1H), 6.94-6.77 (m, 1H), 6.59 (br s, 1H), 6.28 (dd, *J=* 1.8, 16.8 Hz, 1H), 6.03 (br s, 1H), 5.81 (dd, *J* = 1.8, 10.6 Hz, 1H), 4.99 (br s, 1H), 4.74-4.66 (m, 2H), 4.58 (s, 1H), 4.45-4.28 (m, 1H), 4.23-4.15 (m, 1H), 3.74-3.37 (m, 3H), 2.88-2.78 (m, 1H), 2.05 (br *d,J* = 8.8 Hz, 3H), 1.18-1.01 (m, 6H).

**MS (ESI)** *n*/*z* (M+H) = 592.2.

**HPLC** retention time: 3.26 min

Separation conditions: chromatographic column: Ultimate C18 3.0 × 50 mm, 3 µm; column temperature: 40 °C; mobile phase: water (0.0688% trifluoroacetic acid solution)-acetonitrile (0.0625% trifluoroacetic acid solution); acetonitrile: 10%-80% 6 min, 80% 2 min; flow rate: 1.2 mL/min.

**SFC** retention time: 1.577 min.

Separation conditions: chromatographic column: Chiralpak AD-3 50 × 4.6 mm I.D., 3 µm; column temperature: 35 °C; mobile phase: [Neu-isopropanol (0.05% DEA)]; isopropanol%: 5%-40% 2 min, 40% 1.2 min, 5% 0.8 min; flow rate: 4 mL/min.

### Compound 4B

**¹H NMR** (400 MHz, Methanol-*d*₄) δ 8.50 (d, *J* = 4.9 Hz, 1H), 7.45-7.26 (m, 1H), 7.21-7.04 (m, 1H), 6.91-6.81 (m, 1H), 6.60 (br s, 1H), 6.30 (dd, *J=* 1.9, 16.6 Hz, 1H), 6.05 (br s, 1H), 5.91-5.74 (m, 1H), 5.07-4.99 (m, 1H), 4.80-4.66 (m, 2H), 4.60 (s, 1H), 4.51-4.31 (m, 1H), 4.23 (br s, 1H), 3.70-3.62 (m, 1H), 3.58-3.44 (m, 1H), 3.42 -3.35 (m, 1H), 2.87-2.77 (m, 1H), 2.19-2.01 (m, 3H), 1.25-1.00 (m, 6H).

**MS (ESI)** *m*/*z* (M+H)⁺ = 592.3.

**HPLC** retention time: 3.26 min

Separation conditions: chromatographic column: Ultimate C18 3.0 × 50 mm, 3 µm; column temperature: 40 °C; mobile phase: water (0.0688% trifluoroacetic acid solution)-acetonitrile (0.0625% trifluoroacetic acid solution); acetonitrile: 10%-80% 6 min, 80% 2 min; flow rate: 1.2 mL/min.

**SFC** retention time: 1.761 min

Separation conditions: chromatographic column: ChiralpakAD-3 50 × 4.6 mm I.D., 3 µm; column temperature: 35 °C; mobile phase: [Neu-isopropanol (0.05% DEA)]; isopropanol%: 5%-40% 2 min, 40% 1.2 min, 5% 0.8 min; flow rate: 4 mL/min.

### Example 5: Preparation of Compound 5

### Step 1: Preparation of compound 5

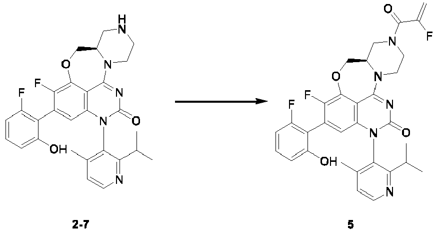

Compound 2-7 (100 mg, 157.84 µmol) and 2-fluoroacrylic acid (50 mg, 555.23 µmol) were dissolved in dimethylformamide (4 mL), and *O*-(7-azabenzotriazol-1-yl)-*N,N,N*'*,N*'-tetramethyluronium hexafluorophosphate (240 mg, 631.20 µmol) and *N,N-*diisopropylethylamine (222.60 mg, 1.72 mmol, 0.3 mL) were added to the resulting solution at room temperature (20 °C). After the completion of the addition, the system was stirred at room temperature (20 °C) for 1 h. Water (30 mL) was added to the system, which was then extracted with ethyl acetate (30 mL × 2). The organic phase was washed with saturated brine (30 mL), dried over anhydrous sodium sulfate, and filtered. The filtrate was concentrated to obtain a crude product. The crude product was dissolved in methanol (5 mL), and potassium carbonate (2 M, 1 mL) was added to the resulting solution. After the completion of the addition, the system was stirred at room temperature (20 °C) for 30 min Water (30 mL) was added to the system, which was then adjusted to neutrality with 1 N HCl and extracted with ethyl acetate (30 mL × 2). The organic phase was washed with saturated brine (30 mL), dried over anhydrous sodium sulfate, and filtered. The filtrate was concentrated to obtain a crude product. The crude product was purified by preparative high performance liquid chromatography (separation conditions: chromatographic column: Phenomenex Gemini-NX 80 × 30 mm × 3 µm; mobile phase: [water (10 mM ammonium bicarbonate solution)-acetonitrile]; acetonitrile%: 38%-68% 9.5 min; flow rate: 30 mL/min) to obtain compound 5.

**¹H NMR** (400 MHz, Methanol-*d*₄) δ 8.50 (d, *J* = 5.1 Hz, 1H), 7.33 (br d, *J=* 3.5 Hz, 1H), 7.20 (br d, *J* = 7.5 Hz, 1H), 6.70-6.56 (m, 2H), 6.04 (br s, 1H), 5.42-5.26 (m, 2H), 5.06 (br d, *J* = 13.5 Hz, 1H), 4.80-4.61 (m, 2H), 4.58 -4.35 (m, 1H), 4.31-4.13 (m, 2H), 3.87-3.60 (m, 1H), 3.60-3.42 (m, 2H), 2.83 (br s, 1H), 2.09 (br d, *J* = 11.9 Hz,3H), 1.28-1.00 (m, 6H).

**MS (ESI)** *n*/*z* (M+H) = 592.4.

### Step 2: Preparation of compounds 5A and 5B

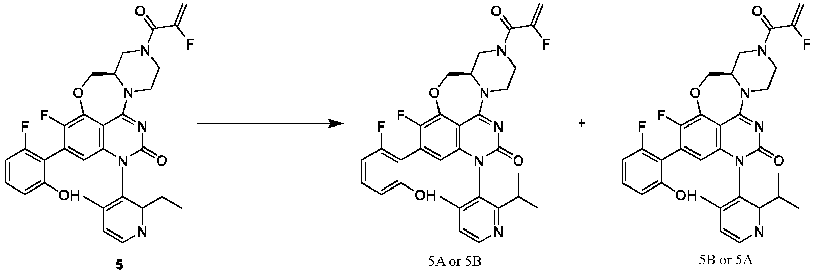

Diastereomer compound 4 was purified by SFC (separation conditions: chromatographic column: Phenomenex Lux Cellulose-4 250 × 30 mm × 5 µm; mobile phase: [Neu-ethanol (0.1% aqueous ammonia)]; ethanol%: 40%-40%, flow rate: 60 mL/min). After concentration, compound 5A (peak 1) and compound 5B (peak 2) were obtained.

### Compound 5A

**¹H NMR** (400 MHz, Methanol-*d*₄) δ 8.48 (d, *J* = 4.9 Hz, 1H), 7.31 (d, *J* = 4.9 Hz, 1H), 7.21-7.15 (m, 1H), 6.71-6.52 (m, 2H), 6.11-5.92 (m, 1H), 5.44-5.22 (m, 2H), 5.05 (br d, *J=* 12.1 Hz, 1H), 4.75-4.62 (m, 2H), 4.58-4.11 (m, 3H), 3.71-3.37 (m, 3H), 3.03-2.70 (m, 1H), 2.07 (br *d, J* = 13.7 Hz, 3H), 1.24-0.96 (m, 6H).

**MS (ESI)** *n*/*z* (M+H) = 592.2.

**HPLC** retention time: 3.26 min

Separation conditions: chromatographic column: Ultimate C18 3.0 × 50 mm, 3 µm; column temperature: 40 °C; mobile phase: water (0.0688% trifluoroacetic acid solution)-acetonitrile (0.0625% trifluoroacetic acid solution); acetonitrile: 10%-80% 6 min, 80% 2 min; flow rate: 1.2 mL/min.

**SFC** retention time: 1.449 min

Separation conditions: chromatographic column: Cellulose-4 100 × 4.6 mm I.D., 3 µm; column temperature: 35 °C; mobile phase: [Neu-ethanol (0.05% DEA)]; ethanol%: 40%; flow rate: 28 mL/min.

### Compound 5B

**¹H NMR** (400 MHz, Methanol-*d*₄) δ 8.50 (d, *J* = 5.1 Hz, 1H), 7.32 (d, *J* = 4.9 Hz, 1H), 7.26-7.13 (m, 1H), 6.75-6.55 (m, 2H), 6.12-5.95 (m, 1H), 5.51-5.21 (m, 2H), 5.06 (br d, *J=* 11.5 Hz, 1H), 4.78-4.65 (m, 2H), 4.55-4.13 (m, 3H), 3.78-3.39 (m, 3H), 2.92-2.73 (m, 1H), 2.09 (br *d,J* = 12.8 Hz, 3H), 1.24-1.01 (m, 6H).

**MS (ESI)** *m*/*z* (M+H)⁺ = 592.2.

HPLC 96.11% purity; retention time 3.25 min

Separation conditions: chromatographic: Ultimate C18 3.0 × 50 mm, 3 µm; column temperature: 40 °C; mobile phase: water (0.0688% trifluoroacetic acid solution)-acetonitrile (0.0625% trifluoroacetic acid solution); acetonitrile: 10%-80% 6 min, 80% 2 min; flow rate: 1.2 mL/min.

**SFC** 99.10% ee. Retention time 1.912 min

Separation conditions: chromatographic column: Cellulose-4 100 × 4.6 mm I.D., 3 µm; column temperature: 35 °C; mobile phase: [Neu-ethanol (0.05% DEA)]; ethanol%: 40%; flow rate: 28 mL/min.

### Example 6: Preparation of Compound 6

### Step 1: Preparation of compound 6-2

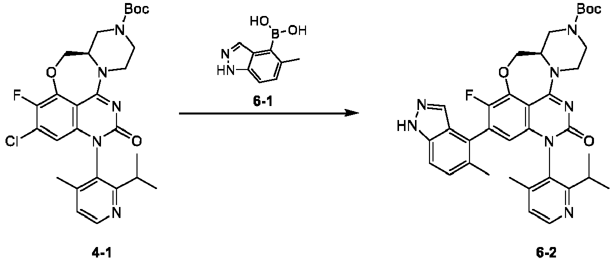

Compound 4-1 (80 mg, 147.05 µmol) was dissolved in dioxane (4 mL) and water (0.8 mL), and compound 6-1 (74.79 mg, 424.99 µmol), 2-dicyclohexylphosphine-2,4,6-triisopropylbiphenyl (15.98 mg, 33.53 µmol), (2-dicyclohexylphosphino-2',4',6'-tri-isopropyl-1,1'-biphenyl)(2'-amino-1,1'-biphenyl-2-yl)palladium methanesulfonate (16.06 mg, 18.97 µmol) and potassium carbonate (80.08 mg, 579.39 µmol) were successively added to the resulting solution. After the completion of the addition, the system was warmed to 100 °C and stirred for 16 h under an Ar atmosphere. The system was concentrated to obtain a crude product. The crude product was purified by silica gel column chromatography (methanol/dichloromethane (v/v) = 0-7%) to obtain compound 6-2.

**¹H NMR** (400 MHz, Methanol-*d*₄) δ 8.42 (d, *J=* 5.0 Hz, 1H), 7.49-7.41 (m, 2H), 7.32-7.26 (m, 2H), 5.94 (d, *J* = 5.5 Hz, 1H), 5.06 (br d, *J* = 9.8 Hz, 1H), 4.79-4.64 (m, 2H), 4.29-4.01 (m, 3H), 3.53-3.32 (m, 3H), 2.98-2.75 (m, 1H), 2.20-2.10 (m, 6H), 1.52 (m, 9H), 1.29-1.13 (m, 6H). **MS (ESI)** *m*/*z* (M+ H)⁺ = 640.1.

### Step 2: Preparation of compound 6-3

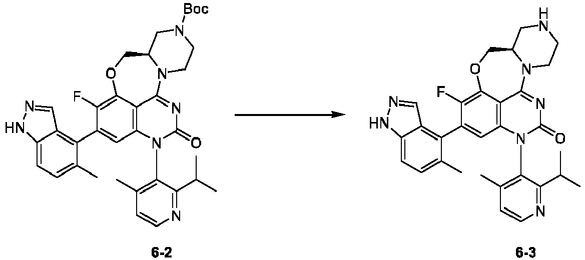

Compound 6-2 (70 mg, 109.42 µmol) was dissolved in anhydrous dichloromethane (2.5 mL), and trifluoroacetic acid (770 mg, 6.75 mmol, 0.5 mL) was added to the resulting solution at 0 °C. After the completion of the addition, the system was warmed to room temperature (20 °C) and stirred for 3 h. The system was concentrated to obtain compound 6-3 (trifluoroacetate salt), which was used in the next step without further purification.

**MS (ESI)** *m*/*z* (M+H)⁺ = 5403.

### Step 3: Preparation of compound 6

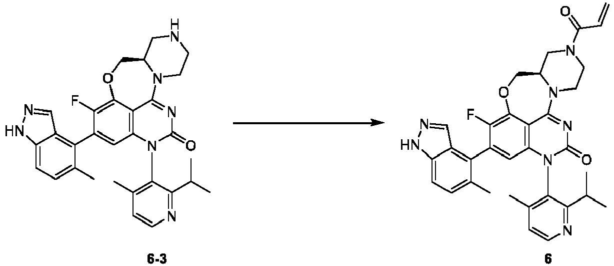

Compound 6-3 (71 mg, 108.62 µmol, trifluoroacetate salt) was dissolved in a mixed solvent of tetrahydrofuran (2 mL) and a saturated aqueous solution of sodium bicarbonate (2 mL), and acrylic anhydride (13.70 mg, 108.62 µmol) was added to the resulting solution at room temperature (20 °C). After the completion of the addition, the system was stirred at room temperature (20 °C) for 30 min. The system was diluted with water (10 mL) and extracted with ethyl acetate (10 mL × 2). The organic phase was dried over anhydrous sodium sulfate, and filtered. The filtrate was concentrated to obtain a crude product. The crude product was purified by p eparative high performance liquid chromatography (separation conditions: chromatographic column: Phenomenex Gemini-NX 80 × 30 mm × 3 µm; mobile phase: [water (10 mM ammonium bicarbonate solution)-acetonitrile]; acetonitrile%: 21%-51% 9.5 min; flow rate: 60 mL/min) to obtain compound 6.

**¹H NMR** (400 MHz, Methanol-*d*₄) δ 8.43 (d, *J* = 5.1 Hz, 1H), 7.54-7.41 (m, 2H), 7.37-7.26 (m, 2H), 6.95-6.79 (m, 1H), 6.31 (br d *J* = 16.5 Hz, 1H), 5.95 (d, *J=* 5.7 Hz, 1H), 5.84 (br d *J* = 10.8 Hz, 1H), 5.05 (br s, 1H), 4.77 ( r s, 1H), 4.60 (br s, 2H), 4.51-4.15 (m, 2H), 3.85-3.42 (m, 3H), 3.00-2.77 (m, 1H), 2.20-2.05 (m, 6H), 1.23-1.16 (m, 3H), 1.12-0.97 (m, 3H).

**MS (ESI)** *n*/*z* (M+H)⁺ = 594.4.

### Step 4: Preparation of compounds 6A and 6B

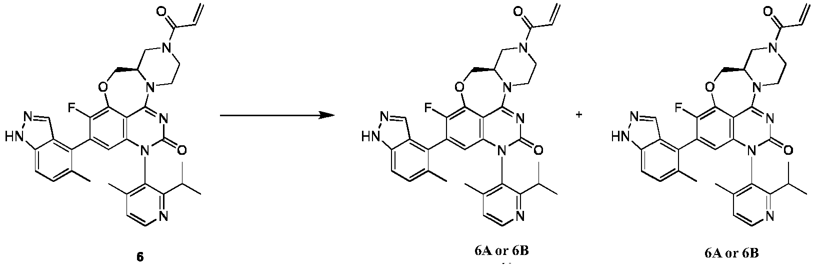

Diastereomer compound 6 was purified by SFC (separation conditions: chromatographic column: REGIS (s,s) WHELK- O1 (250 mm × 30 mm, 5 µm);mobile phase: [Neu-isopropanol (0.1% aqueous ammonia)]; isopropanol%: 50%-50%; flow rate: 80 mL/min). After concentrat on, compound 6A (peak 1) and compound 6B (peak 2) were obtained.

### Compound 6A

**¹H NMR** (400 MHz, Methanol- *d*₄)δ 8.42 (d, *J* = 4.9 Hz, 1H), 7.53-7.39 (m, 2H), 7.35-7.22 (m, 2H), 6.85 (dd, *J* = 10.1, 16.8 Hz, 1H), 6.31 (br d, *J* = 15.2 Hz, 1H), 5.95 (br d, *J* = 5.3 Hz, 1H), 5.84 (br d, *J* = 11.2 Hz, 1H), 5.03 (br d, *J* = 10.4 Hz, 1H), 4.77 (br s, 1H), 4.65-4.56 (m, 1H), 4.50-4.16 (m, 3H), 3.81-3.45 (m, 3H), 3.00-2.82 (m, 1H), 2.35-1.95 (m, 6H), 1.19 (br t, *J* = 7.4 Hz, 3H), 1.14-0.97 (m, 3H).

**MS (ESI)** n/z (M+H) = 594.3.

**HPLC** retention time: 6.73 min

Separation conditions: chromatographic column: Ultimate LP-C18 150 × 4.6 mm 5 µm; column temperature: 40 °C; mobile phase: water (0.0688% trifluoroacetic acid solution)-acetonitrile (0.0625% trifluoroacetic acid solution); acetonitrile: 10%-80% 10 min, 80% 5 min; flow rate: 1.5 mL/min.

**SFC** retention time: 5.039 min

Separation conditions: chromatographic column: (S,S)-Whelk-O1 100 × 4.6 mm I.D., 3 µm; column temperature: 35 °C; mobile phase: [Neu-isopropanol (0.05% DEA)]; isopropanol%: 40%-40%; flow rate: 2.8 mL/min.

### Compound 6B

**1H NMR** (400 MHz, Methanol-*d*₄)δ 8.42 (br d, *J=* 2.2 Hz, 1H), 7.47 (br dd, *J=* 8.9, 16.2 Hz, 2H), 7.36-7.23 (m, 2H), 6.95-6.80 (m, 1H), 6.31 (br d, *J=* 16.3 Hz, 1H), 5.94 (br d, *J* = 5.3 Hz, 1H), 5.84 (br d,*J* = 10.1 Hz, 1H), 5.06 (br d, *J* = 12.6 Hz, 1H), 4.77 (br s, 1H), 4.61 (br s, 1H), 4.55 -4.16(m, 3H), 3.76-3.37 (m, 3H), 2.97-2.75 (m, 1H), 2.25-2.04 (m, 6H), 1.20 (t, *J* = 7.3 Hz, 3H), 1.12-0.97 (m, 3H).

**MS (ESI)** *m*/*z* (M+H)⁺ = 594.3.

**HPLC** retention time: 6.75 min

Separation conditions: chromatographic column: Ultimate LP-C18 150 × 4.6 mm 5 µm; column temperature: 40 °C; mobile phase: water (0.0688% trifluoroacetic acid solution)-acetonitrile (0.0625% trifluoroacetic acid solution); acetonitrile: 10%-80% 10 min, 80% 5 min; flow rate: 1.5 mL/min.

**SFC** retention time: 7.271 min

Separation conditions: chromatographic column: (S,S)-Whelk-O1 100 × 4.6 mm I.D., 3 µm; column temperature: 35 °C; mobile phase: [Neu-isopropanol (0.05% DEA)]; isopropanol%: 40%-40%; flow rate: 2.8 mL/min.

### Example 7: Preparation of Compound 7

### Step 1: Preparation of compound 7-1

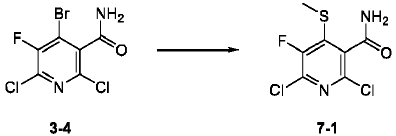

Compound 3-4 (1.60 g, 5.55 mmol) was dissolved in dioxane (1.20 mL), and a solution of sodium thiomethoxide in *N,N*-dimethylformamide (20%, 2.30 g) was added dropwise to the resulting solution at room temperature (20 °C). After the completion of the addition, the system was stirred at room temperature (20 °C) for 2 h. The system was quenched with water (8 mL) and extracted with ethyl acetate (6 mL × 2). The organic phases were pooled, dried over anhydrous sodium sulfate, and filtered. The filtrate was concentrated to obtain compound 7-1, which was used in the next step without further purification.

MS (ESI) *m*/*z* (M+H)⁺ = 255.0.

### Step 2: Preparation of compound 7-2

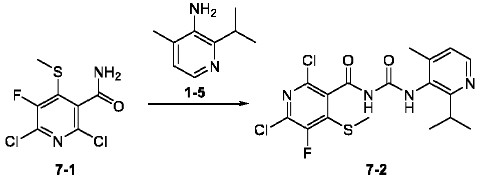

Compound 7-1 (1.00 g, 0.39 mmol) was dissolved in 1,2-dichloroethane (10 mL), and oxalyl chloride (1.00 g, 0.78 mmol) was added dropwise to the resulting solution at room temperature (20 °C). After the completion of the addition, the system was warmed to 80 °C and stirred for 1 h. The system was concentrated. The residue was dissolved in tetrahydrofuran (10 mL), and compound 1-5 (0.75 g, 5 mmol) was added dropwise to the resulting solution at 0 °C. After the completion of the addition, the system was stirred at room temperature (20 °C) for 1 h. The system was filtered. The filter cake was washed with ethyl acetate, and the filtrate was concentrated to obtain a crude product. The crude product was purified by medium-pressure column chromatography (ethyl acetate/petroleum ether (v/v) = 0-30%) to obtain compound 7-2.

MS (ESI) *m*/*z* (M+H)⁺ = 431.0.

### Step 3: Preparation of compound 7-3

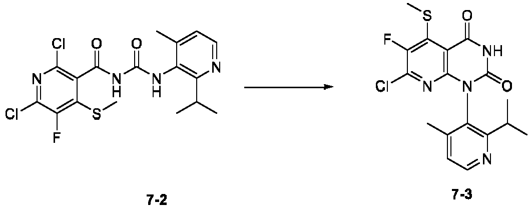

Compound 7-2 (500 mg, 1.16 mmol) was dissolved in anhydrous tetrahydrofuran (5 mL) under a nitrogen atmosphere, and a solution of sodium bis(trimethylsilyl)amide in tetrahydrofuran (2 N, 1.2 mL) was added dropwise to the resulting solution at 0 °C. After the completion of the addition, the system was cooled to 0 °C and stirred for 1 h. The system was quenched with water (10 mL), adjusted to pH 5.0 with dilute hydrochloric acid (3 N), and extracted with ethyl acetate (5 mL × 2). The organic phases were pooled, dried over anhydrous sodium sulfate, and filtered. The filtrate was concentrated to obtain a crude product. The crude product was purified by medium-pressure column chromatography (ethyl acetate/petroleum ether (v/v) = 0-30%) to obtain compound 7-3.

MS (ESI) *m*/*z* (M+H)⁺ = 395.0.

### Step 4: Preparation of compound 7-4

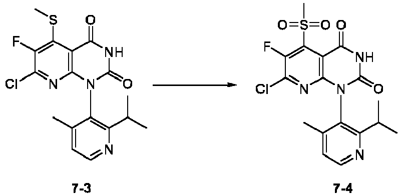

Compound 7-3 (260 mg, 0.66 mmol), *m*-chloroperoxybenzoic acid (300 mg, 1.74 mmol) were dissolved in dichloromethane (4 mL). The system was stirred at room temperature (20 °C) for 15 min, and then warmed to 50 °C and stirred for 15 min. The system was cooled to room temperature and concentrated to obtain a crude product. The crude product was purified by medium-pressure column chromatography (dichloromethane/methanol (v/v) = 0-10%) to obtain compound 7-4.

MS (ESI) *m*/*z* (M+H)⁺ = 427.0.

### Step 5: Preparation of compound 7-5

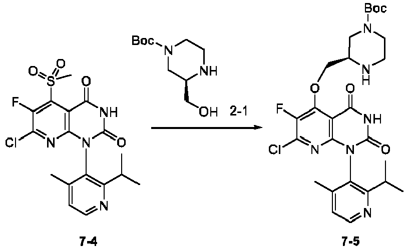

Compound 2-1 (137 mg, 0.64 mmol) and sodium hydride (51.2 mg, 60%, 1.28 mmol) were dissolved in tetrahydrofuran (3.0 mL) under a nitrogen atmosphere. The system was stirred at room temperature (20 °C) for 5 min. Compound 7-4 (210 mg, 0.49 mmol) was added to the system, which was then stirred at room temperature (20 °C) for 1 h. The system was quenched with water (5 mL) and extracted with ethyl acetate (5 mL × 2). The organic phases were pooled, dried over anhydrous sodium sulfate, and filtered. The filtrate was concentrated to obtain a crude product. The crude product was purified by medium-pressure column chromatography (methanol/dichloromethane (v/v) = 0-10%) to obtain compound 7-5.

MS (ESI) *m*/*z* (M+H)⁺ = 563.0.

### Step 6: Preparation of compound 7-6

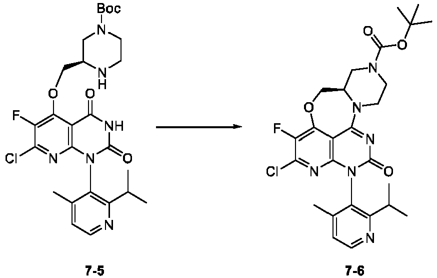

Compound 7-5 (75 mg, 0.133 mmol) and bromotripyrrolidinophosphonium hexafluorophosphate (124 mg, 0.267 mmol) were dissolved in acetonitrile (2 mL), and 1,8-diazabicycloundec-7-ene (81.0 mg, 0.534 mmol) was added dropwise to the resulting solution at room temperature (20 °C). The system was stirred at room temperature (20 °C) for 18 h. The system was quenched with water (2 mL) and extracted with ethyl acetate (2 mL × 2). The organic phases were pooled, dried over anhydrous sodium sulfate, and filtered. The filtrate was concentrated to obtain a crude product. The crude product was purified by medium-pressure column chromatography (methanol/dichloromethane (v/v) = 0-10%) to obtain compound 7-6.

MS (ESI) *m*/*z* (M+H)⁺ = 545.0.

### Step 7: Preparation of compound 7-8

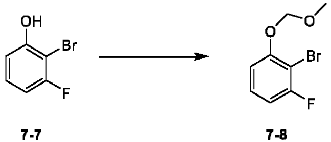

Compound 7-7 (2.87 g, 15 mmol) was dissolved in anhydrous *N*,*N*-dimethylacetamide (10 mL), and sodium hydride (60%, 660 mg, 16.5 mol) was added to the resulting solution in batches at 0 °C. After the completion of the addition, the system was warmed to room temperature and stirred for 10 min. Chloromethyl methyl ether (2.4 g, 30 mmol) was added dropwise to the system. After the completion of the addition, the system was stirred at room temperature for 10 min. The system was quenched by pouring into ice water (50 mL) and extracted with methyl tert-butyl ether (3 × 50 mL). The organic phases were pooled, washed once with a saturated aqueous solution of sodium chloride, dried over anhydrous sodium sulfate, and filtered. The filtrate was concentrated to obtain a crude product. The crude product was purified by medium-pressure column chromatography (ethyl acetate/petroleum ether (v/v) = 0-10%) to obtain compound 7-8.

¹HNMR (400 MHz, CDCl₃-*d*₁) 7.24-7.18 (m, 1H), 6.95-6.93 (m, 1H), 6.83-6.79 (m, 1H), 5.26(s, 2H), 3.52 (s, 3H).

### Step 8: Preparation of compound 7-9

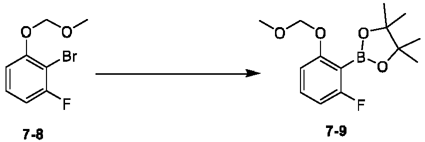

Compound 7-8 (650 mg, 2.77 mmol) was dissolved in anhydrous tetrahydrofuran (5 mL), and *n*-butyllithium (2.5 N, 1.22 mL, 3.05 mmol) was added dropwise to the system at-78 °C. The system was stirred at -78 °C for 30 min, followed by dropwise addition of isopropyl pinacol boronate (567 mg, 3.05 mmol). The system was stirred at -78 °C for 30 min. The system was warmed to room temperature, quenched with water, and extracted with ethyl acetate (10 mL). The organic phases were pooled, dried over anhydrous sodium sulfate, and filtered. The filtrate was concentrated to obtain a crude product. The crude product was purified by medium-pressure column chromatography (ethyl acetate/petroleum ether (v/v) = 0-20%) to obtain compound 7-9.

### Step 9: Preparation of compound 7-10

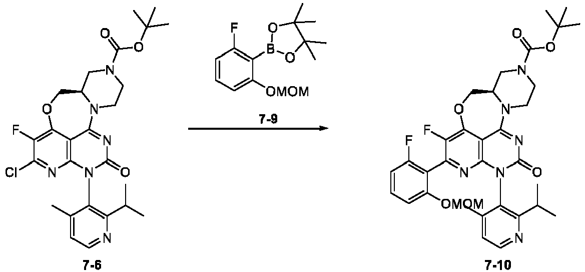

Compound 7-6 (35 mg, 0.064 mmol), compound 7-9 (36.3 mg, 0.128 mmol), [1,1'-bis(diphenylphosphino)ferrocene]palladium dichloride (4.7 mg, 0.0064 mmol) and potassium carbonate (26.0 mg, 0.192 mmol) were dissolved in a mixed solvent of tetrahydrofuran/water (2 mL, v/v 10:1) under a nitrogen atmosphere. The system was heated to 80 °C and stirred for 2 h. The system was cooled to room temperature, quenched with water (1 mL), and extracted with ethyl acetate (1 mL × 2). The organic phases were pooled, dried over anhydrous sodium sulfate, and filtered. The filtrate was concentrated to obtain a crude product. The crude product was purified by medium-pressure column chromatography (methanol/dichloromethane (v/v) = 0-10%) to obtain compound 7-10.

MS (ESI) *m*/*z* (M+H)⁺ = 665.0.

### Step 10: Preparation of compound 7-11

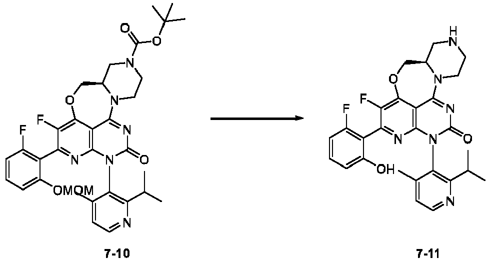

Compound 7-10 (20 mg, 0.03 mmol) was dissolved in a mixed solvent of methanol and hydrochloric acid (6 N) (2 mL, v/v = 1:1). The system was warmed to 55 °C and stirred for 15 min. The system was cooled to room temperature and concentrated to obtain crude compound 7-11, which was used in the next step without further purification.

MS (ESI) *m*/*z* (M+H)⁺ = 521.0.

### Step 9: Preparation of compound 7

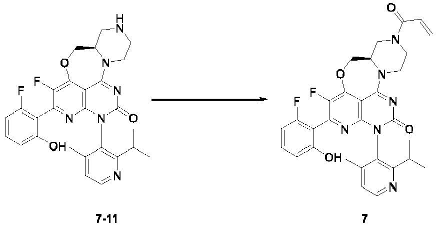

Compound 7-11 was dissolved in dichloromethane (1 mL), and triethylamine (9.0 mg) and acryloyl chloride (4.0 mg) were successively added to the resulting solution at 0 °C. The reaction system was stirred at 0 °C for 30 min. The system was quenched with water (1 mL) and extracted with dichloromethane (1 mL × 2). The organic phases were pooled, dried over anhydrous sodium sulfate, and filtered. The filtrate was concentrated to obtain a crude product. The crude product was purified by preparative high performance liquid chromatography (separation conditions: chromatographic column: Phenomenex Gemini-NX 80 × 30 mm × 3 µm; mobile phase: [water (10 mM ammonium bicarbonate solution)-acetonitrile]; acetonitrile%: 21%-51% 9.5 min; flow rate: 60 mL/min) to obtain compound 7.

MS (ESI) *m*/*z* (M+H)⁺ = 575.0.

### Step 10: Preparation of compounds 7A and 7B

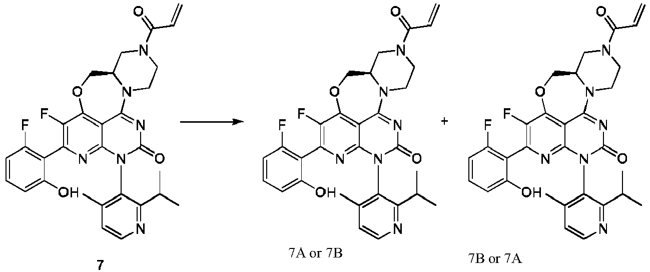

Diastereomer compound 7 was purified by SFC (separation conditions: chromatographic column: DAICEL CHIRALCEL OJ-H (250 mm × 30 mm, 5 µm); mobile phase: [Neu- sopropanol (0.1% aqueous ammonia)]; isopropanol%: 40%-40%). After concentration, compound 7A (peak 1) and compound 7B (peak B) were obtained.

### Compound 7A:

**¹H NMR** (400 MHz, METHANOL-*d*₄) δ = 8.44 (d, *J* = 5.0 Hz, 1H), 7.34-7.20 (m, 2H), 6.88-6.82 (m, 1H), 6.72-6.60 (m, 2H), 6.32(dd, *J* = 1.9, 16.7 Hz, 1H), 5.85 (dd, *J* = 1.9, 10.7 Hz, 1H), 4.86-4.64 (m, 3H), 4.62-4.11 (m, 3H), 3.74-3.37 (m, 3H), 3.02-2.95 (m, 1H), 2.19 (s, 3H), 1.29-1.14 (m, 6H)

**¹⁹F NMR** (376 MHz, METHANOL-*d*₄) δ = -116.80 (s, IF), -137.87--139.98 (m, IF)

**MS (ESI)** *m*/*z* (M+H) = 575.3.

**HPLC** retention time: 6.30 min

Separation conditions: chromatographic column: WELCH Ultimate LP-C18 150 × 4.6 mm 5 µm; column temperature: 40 °C; mobile phase: water (0.06875 % trifluoroacetic acid)-acetonitrile (0.0625 % trifluoroacetic acid); acetonitrile: 10%-80% 10 min, 80% 5 min; flow rate: 1.5 mL/min.

**SFC** retention time: 5.488 min

Separation conditions: chromatographic column: Chiralcel OJ-3 150 × 4.6 mm I.D., 3 µm; column temperature: 35 °C; mobile phase: [Neu-isopropanol (0.05% DEA)], isopropanol%: 5%-40% 5 min, 40% 2.5 min, 5% 2.5 min; flow rate: 2.5 mL/min.

### Compound 7B:

**¹H NMR** (400 MHz, METHANOL-*d*₄) δ = 8.44 (d, *J* = 5.0 Hz, 1H), 7.31-7.21 (m, 2H), 6.96-6.80 (m, 1H), 6.73-6.60 (m, 2H), 6.32 (dd, *J* = 1.8, 16.8 Hz, 1H), 5.85 (dd, *J* = 1.8, 10.5 Hz, 1H), 4.86-4.64 (m, 3H), 4.56-4.14 (m, 3H), 3.87-3.42 (m, 3H), 3.04-2.93 (m, 1H), 2.19 (s, 3H), 1.24-1.15 (m, 6H)

**¹⁹F NMR** (376 MHz, METHANOL-d₄) δ = -116.81 (s, IF), -139.04--139.11 (m, IF)

**MS (ESI)** *m*/*z* (M+H) = 575.3.

**HPLC** retention time: 6.32 min

Separation conditions: chromatographic column: WELCH Ultimate LP-C18 150 × 4.6 mm 5 µm; column temperature: 40 °C; mobile phase: water (0.06875 % trifluoroacetic acid)-acetonitrile (0.0625 % trifluoroacetic acid); acetonitrile: 10%-80% 10 min, 80% 5 min; flow rate: 1.5 mL/min.

**SFC** retention time: 5.970 min

Separation conditions: chromatographic column: Chiralcel OJ-3 150 × 4.6 mm I.D., 3 µm; column temperature: 35 °C; mobile phase: [Neu-isopropanol (0.05% DEA)], isopropanol%: 5%-40% 5 min, 40% 2.5 min, 5% 2.5 min; flow rate: 2.5 mL/min.

### Example 8: Preparation of Compound 8

### Step 1: Preparation of compound 8-2

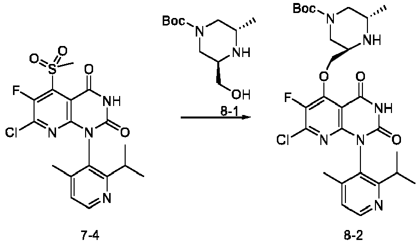

Compound 8-1 (410 mg, 1.774 mmol) and sodium hydride (142 mg, 60%, 3.55 mmol) were dissolved in tetrahydrofuran (3.0 mL) under a nitrogen atmosphere. The system was stirred at room temperature (20 °C) for 5 min. Compound 7-4 (750 mg, 1.774 mmol) was added to the system, which was then stirred at room temperature (20 °C) for 1 h. The system was quenched with water (5 mL) and extracted with ethyl acetate (5 mL × 2). The organic phases were pooled, dried over anhydrous sodium sulfate, and filtered. The filtrate was concentrated to obtain a crude product. The crude product was purified by medium-pressure column chromatography (methanol/dichloromethane (v/v) = 0-10%) to obtain compound 8-2.

**MS (ESI)** *m*/*z* (M+H)⁺ = 577.2.

### Step 2: Preparation of compound 8-3

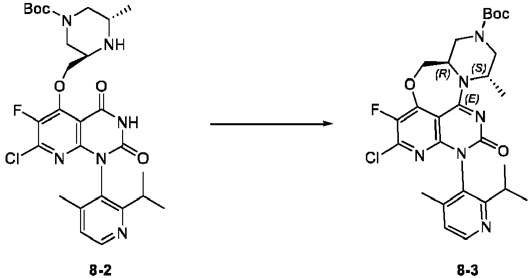

Compound 8-2 (300 mg, 0.52 mmol) and bromotripyrrolidinophosphonium hexafluorophosphate (485 mg, 1.04 mmol) were dissolved in *N*,*N* dimethylformamide (4 mL), and 1,8-diazabicycloundec-7-ene (316 mg, 2.08 mmol) was added dropwise to the resulting solution at room temperature (20 °C). The system was stirred at room temperature (20 °C) for 8 h. The system was quenched with water (10 mL) and extracted with ethyl acetate (4 mL × 2). The organic phases were pooled, dried over anhydrous sodium sulfate, and filtered. The filtrate was concentrated to obtain a crude product. The crude product was purified by medium-pressure column chromatography (methanol/dichloromethane (v/v) = 0-10%) to obtain compound 8-3.

**MS (ESI)** *m*/*z* (M+H)⁺ = 559.2.

### Step 3: Preparation of compound 8-4

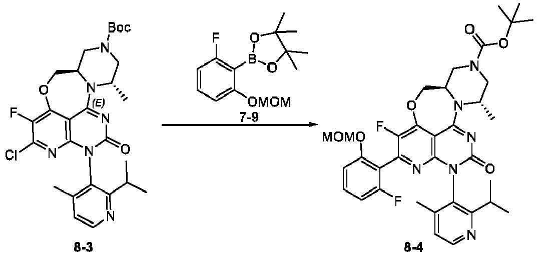

Compound 8-3 (100 mg, 0.179 mmol), compound 7-9 (100 mg, 0.358 mmol), [1,1'-bis(diphenylphosphino)ferrocene]palladium dichloride (13 mg, 0.0179 mmol) and potassium carbonate (50.0 mg, 0.358 mmol) were dissolved in a mixed solvent of tetrahydrofuran/water (2 mL, v/v 10:1) under a nitrogen atmosphere. The system was heated to 80 °C and stirred for 2 h. The system was cooled to room temperature, quenched with water (1 mL), and extracted with ethyl acetate (1 mL × 2). The organic phases were pooled, dried over anhydrous sodium sulfate, and filtered. The filtrate was concentrated to obtain a crude product. The crude product was purified by medium-pressure column chromatography (methanol/dichloromethane (v/v) = 0-5%) to obtain compound 8-4.

**MS (ESI)** *m*/*z* (M+H)⁺ = 679.2.

### Step 4: Preparation of compound 8-5

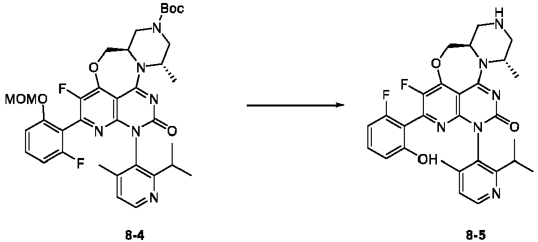

Compound 8-4 (18 mg, 0.0265 mmol) was dissolved in a mixed solvent of methanol and hydrochloric acid (6 N) (2 mL, v/v = 1:1). The system was warmed to 55 °C and stirred for 15 min. The system was cooled to room temperature and concentrated to obtain compound 8-5.

**MS (ESI)** *m*/*z* (M+H)⁺ = 535.2.

### Step 5: Preparation of compound 8

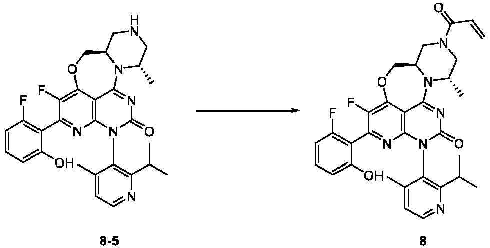

Compound 8-5 was dissolved in dichloromethane (1 mL), and triethylamine (8.1 mg) and acryloyl chloride (2.1 mg) were successively added to the resulting solution at 0 °C. The reaction system was stirred at 0 °C for 30 min. The system was quenched with water (1 mL) and extracted with dichloromethane (1 mL × 2). The organic phases were pooled, dried over anhydrous sodium sulfate, and filtered. The filtrate was concentrated to obtain a crude product. The crude product was purified by preparative high performance liquid chromatography (separation conditions: chromatographic column: Welch Xtimate C18 21.2 × 250 mm, 10 µm; column temperature: 25 °C, mobile phase: water (10 mM ammonium bicarbonate solution)-acetonitrile; mobile phase acetonitrile proportion 25%-45% in 12 min; flow rate: 30 mL/min) to obtain compound 8.

**MS (ESI)** *m*/*z* (M+H)⁺ = 589.3.

### Step 6: Preparation of compounds 8A and 8B

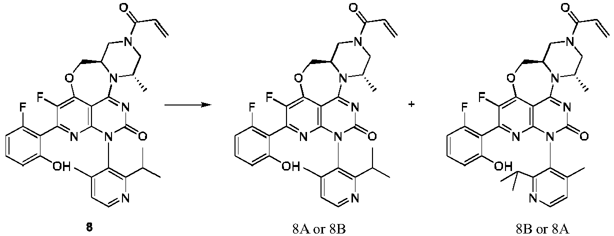

Diastereomer compound 8 was purified by SFC (separation conditions: chromatographic column: ChiralPak AD, 300 × 50 mm I.D., 10 µm; column temperature: 38 °C; mobile phase [Neu-isopropanol (0.1% aqueous ammonia)]; isopropanol%: 35%; flow rate: 80 mL/min). After concentration, compound 8A (peak 1) and compound 8B (peak 2) were obtained.

### Compound 8A:

**¹H NMR** (400 MHz, DMSO-*d*₆): 10.10 (s, 1H), 8.38-8.32 (m, 1H), 7.25-7.05 (m, 2H), 6.95-6.75 (m, 1H), 6.67-6.55 (m, 2H), 6.19-6.10 (m, 1H), 5.75-5.65 (m, 1H), 5.00-4.83 (m, 1H), 4.73-4.57 (m, 2H), 4.45-4.13 (m, 2H), 4.13-4.00 (m, 1H), 3.93-3.80 (m, 1H), 2.90-2.78 (m, 1H), 2.05-1.85 (m, 4H), 1.20-1.12 (s, 3H), 1.08-0.93 (m, 6 H).

**SFC** retention time: 5.339 min.

Separation conditions: chromatographic column: Chiralpak AD-3 150 × 4.6 mm I.D., 3 µm; column temperature: 35 °C; mobile phase: [Neu-isopropanol (0.05% DEA)]; isopropanol%: 5%-40% in 5 min, 40% 2.5 min; flow rate: 2.5 mL/min.

### Compound 8B:

**¹H NMR** (400 MHz, DMSO-*d*₆) 10.10 (s, 1H), 8.40-8.28 (d, *J* = 8.0Hz, 1H), 7.25-7.05 (m, 2H), 6.95-6.75 (m, 1H), 6.70-6.55 (m, 2H), 6.20-6.10 (m, 1H), 5.75-5.68 (m, 1H), 5.30-5.20 (m, 1H), 5.05-4.80 (m, 1H), 4.45-4.00 (m, 4H), 3.95-3.83 (m, 1H),, 2.85-2.75(m, 1H), 2.03-1.93 (m, 4H), 1.28-1.21 (s, 3H), 1.10-0.90 (m, 6H).

**SFC** retention time: 5.681 min

Separation conditions: chromatographic column: Chiralpak AD-3 150 × 4.6 mm I.D., 3 µm; column temperature: 35 °C; mobile phase: [Neu-isopropanol (0.05% DEA)]; isopropanol%: 5%-40% in 5 min, 40% 2.5 min; flow rate: 2.5 mL/min.

**MS (ESI)** *m*/*z* (M+H) = 589.3.

### Example 9: Preparation of Compound 9

### Step 1: Preparation of compound 9-2

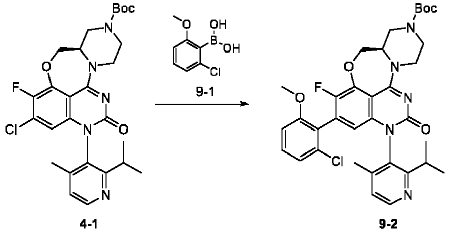

Compound 4-1 (120 mg, 220.58 µmol) was dissolved in dioxane (5 mL) and water (1 mL), and compound 9-1 (118.83 mg, 637.48 µmol), 2-dicyclohexylphosphine-2,4,6-triisopropylbiphenyl (23.98 mg, 50.29 µmol), (2-dicyclohexylphosphino-2',4',6'-tri-isopropyl-1,1'-biphenyl)(2'-amino-1,1'-biphenyl-2-yl)palladium methanesulfonate (24.09 mg, 28.46 µmol) and potassium carbonate (120.11 mg, 869.09 µmol) were successively added to the resulting solution. After the completion of the addition, the system was warmed to 100 °C and stirred for 16 h under an argon atmosphere. The system was concentrated to obtain a crude product. The crude product was purified by silica gel column chromatography (methanol/dichloromethane (v/v) = 0-7%) to obtain compound 9-2.

**MS (ESI)** *m*/*z* (M+H)⁺ = 650.3.

### Step 2: Preparation of compound 9-3

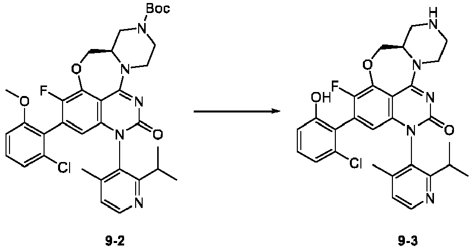

Compound 9-2 (100 mg, 153.81 µmol) was dissolved in anhydrous dichloromethane (1 mL), and boron tribromide (260.00 mg, 1.04 mmol, 0.1 mL) was added to the resulting solution at 0 °C. After the completion of the addition, the system was warmed to room temperature (20 °C) and stirred for 2 h. Methanol (2 mL) was added to the system, and the resulting mixture was stirred for 30 min. The system was concentrated and lyophilized to obtain compound 9-3 (hydrobromide salt).

**MS (ESI)** *m*/*z* (M+H)⁺ = 536.2.

### Step 3: Preparation of compound 9

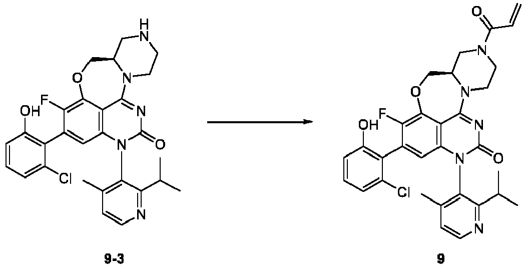

Compound 9-3 (100 mg, 162.10 µmol, hydrobromide salt) was dissolved in tetrahydrofuran (3 mL) and a saturated aqueous solution of sodium bicarbonate (1.7 mL), and acrylic anhydride (20.44 mg, 162.10 µmol) was added to the resulting solution at room temperature (20 °C). After the completion of the addition, the system was stirred at room temperature (20 °C) for 30 min. Isopropanol (5 mL) and an aqueous solution of potassium carbonate (2 M, 2 mL) were added to the system, which was then stirred at room temperature (20 °C) for another 3 h. The system was adjusted to neutral pH with 1 N HCl and extracted with ethyl acetate (10 mL × 2). The organic phase was dried over anhydrous sodium sulfate and filtered. The filtrate was concentrated to obtain a crude product. The crude product was purified by preparative high performance liquid chromatography (separation conditions: chromatographic column: Phenomenex Gemini-NX 80 × 30 mm × 3 µm; mobile phase: [water (10 mM ammonium bicarbonate solution)-acetonitrile]; acetonitrile%: 36%-66% 9.5 min; flow rate: 30 mL/min) to obtain compound 9.

**¹H NMR** (400 MHz, Acetonitrile-*d*₃) δ 8.47 (d, *J* = 4.9 Hz, 1H), 7.24-7.17 (m, 2H), 7.00-6.93 (m, 1H), 6.88-6.82 (m, 1H), 6.74 (br s, 1H), 6.25 (dd, *J* = 2.0, 16.8 Hz, 1H), 5.93-5.86 (m, 1H), 5.75 (dd, *J* = 2.2, 10.6 Hz, 1H), 4.79 (br s, 1H), 4.67-4.33 (m, 3H), 4.27-3.99 (m, 2H), 3.66-3.11 (m, 3H), 2.92-2.77 (m, 1H), 2.04-1.99 (m, 3H), 1.13-1.03 (m, 6H).

**MS (ESI)** *m*/*z* (M+H)⁺ = 590.1.

### Example 10: Preparation of Compound 10

### Step 1: Preparation of compound 10-2

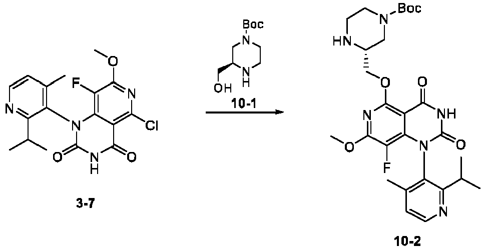

Compounds 10-1 (2.06 g, 9.50 mmol) were dissolved in tetrahydrofuran (70 mL), and sodium hydride (763.54 mg, 15.84 mmol, 60% purity) was added to the resulting solution at 0 °C. After the completion of the addition, the system was warmed to room temperature (20 °C) and stirred for 20 min, and compound 3-7 (3 g, 7.92 mmol) was added thereto. After the completion of the addition, the system was warmed to 20 °C and stirred for 20 min. The system was quenched with a saturated aqueous solution of ammonium chloride (2 mL) and filtered. The filtrate was concentrated to obtain a crude product. The crude product was purified by medium-pressure column chromatography (methanol/dichloromethane (v/v) = 0-10%) to obtain compound 10-2.

**MS (ESI)** *m*/*z* (M+H)⁺ = 559.2.

### Step 2: Preparation of compound 10-3

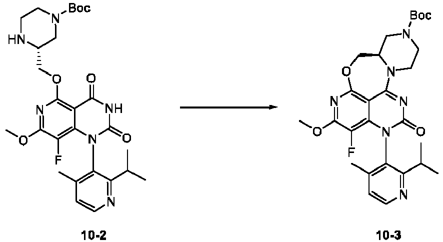

Compound 10-2 (1.6 g, 2.86 mmol) and PyBOP (2.98 g, 5.73 mmol) were dissolved in acetonitrile (22 mL), and 1,8-diazabicycloundec-7-ene (2.18 g, 14.32 mmol, 2.16 mL) was added dropwise to the resulting solution at 0 °C. After the completion of the addition, the system was warmed to room temperature (20 °C) and stirred for 16 h under a nitrogen atmosphere. The reaction system was diluted with water (40 mL), and extracted with ethyl acetate (3 × 20 mL). The organic phases were pooled, dried over anhydrous sodium sulfate, and filtered. The filtrate was concentrated to obtain a crude product. The crude product was purified by silica gel column chromatography (petroleum 88/ethyl acetate (v/v) = 0-100%) to obtain compound 10-3.

**MS (ESI)** *m*/*z* (M+H)⁺ = 541.3.

### Step 3: Preparation of compound 10-4

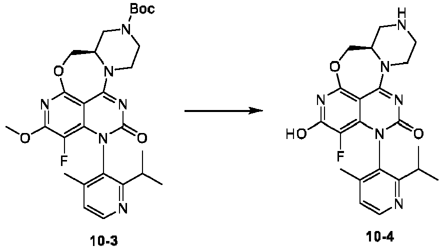

Compound 10-3 (754 mg, 1.39 mmol) was dissolved in anhydrous dichloromethane (4 mL), and boron tribromide (3.49 g, 13.95 mmol, 1.34 mL) was added to the resulting solution at 0 °C. After the completion of the addition, the system was warmed to room temperature (20 °C) and stirred for 16 h. Methanol (10 mL) was added to the system, and the resulting mixture was stirred for 10 min. The system was concentrated and lyophilized to obtain compound 10-4.

### Step 4: Preparation of compound 10-5

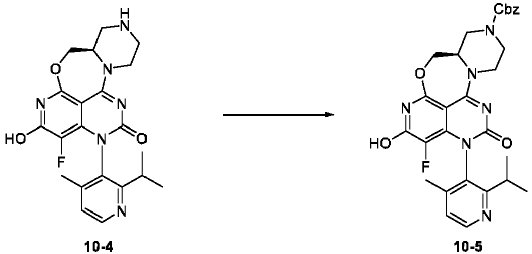

Compound 10-4 (594 mg, 1.11 mmol) was dissolved in a mixed solvent of tetrahydrofuran (10 mL) and saturated sodium bicarbonate (5.5 mL), and benzyl chloroformate (380.20 mg, 2.23 mmol, 316.83 µL) was added dropwise to the resulting solution at room temperature (20 °C). After the completion of the addition, the system was stirred at room temperature (20 °C) for 4 h. Methanol (2 mL) and an aqueous solution of lithium hydroxide (2 N, 0.2 mL) were added to the system. After the completion of the addition, the system was stirred at room temperature for 1 h. The system was concentrated to obtain a crude product. The crude product was purified by silica gel column chromatography (methanol/dichloromethane (v/v) = 0-10%) to obtain compound 10-5.

**MS (ESI)** *m*/*z* (M+H)⁺ = 561.2.

### Step 5: Preparation of compound 10-6

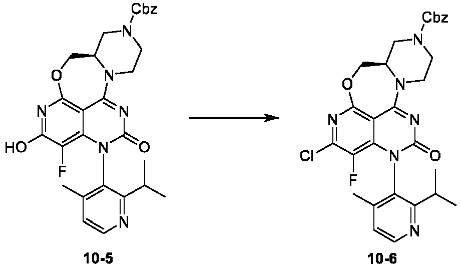

Compound 10-5 (750 mg, 1.34 mmol) was dissolved in acetonitrile (10 mL), and phosphorus oxychloride (8.21 g, 53.52 mmol, 4.97 mL) was added to the resulting solution. After the completion of the addition, the system was warmed to 80 °C and stirred for 2 h. The system was cooled to room temperature and concentrated. The residue was quenched by pouring into ice water (10 mL). The resulting mixture was adjusted to neutral pH with saturated sodium bicarbonate, and extracted with ethyl acetate (3 × 10 mL). The organic phases were pooled, dried over anhydrous sodium sulfate, and filtered. The filtrate was concentrated to obtain a crude product. The crude product was purified by silica gel column chromatography (methanol/dichloromethane (v/v) = 0-10%) to obtain compound 10-6.

**MS (ESI)** *m*/*z* (M+H)⁺ = 579.2.

### Step 6: Preparation of compound 10-7

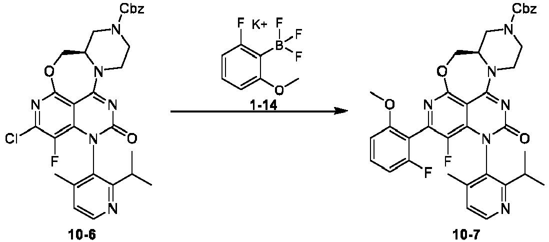

Compound 10-6 (350 mg, 604.47 µmol) was dissolved in dioxane (8 mL) and water (1 mL), and compound 1-14 (280.50 mg, 1.21 mmol), 2-dicyclohexylphosphonium-2,4,6-triisopropylbiphenyl (57.63 mg, 120.89 µmol), tris(dibenzylideneacetone)dipalladium (34.76 mg, 60.45 µmol) and potassium carbonate (250.63 mg, 1.81 mmol) were successively added to the resulting solution. After the completion of the addition, the system was warmed to 100 °C and stirred for 16 h under an argon atmosphere. The system was concentrated to obtain a crude product. The crude product was purified by silica gel column chromatography (methanol/dichloromethane (v/v) = 0-5%) to obtain compound 10-7.

**MS (ESI)** *m*/*z* (M+H)⁺ = 669.3.

### Step 7: Preparation of compound 10-8

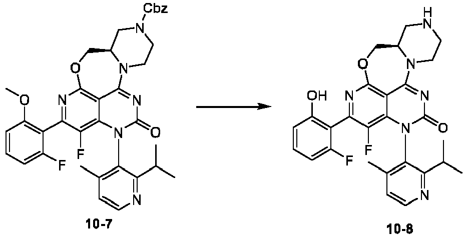

Compound 10-7 (90 mg, 134.59 µmol) was dissolved in anhydrous dichloromethane (0.5 mL), and boron tribromide (1 M in dichloromethane, 807.55 µL) was added to the resulting solution at 0 °C. After the completion of the addition, the system was warmed to room temperature (20 °C) and stirred for 6 h. Methanol (5 mL) was added to the system, and the resulting mixture was stirred for 10 min. The system was concentrated and lyophilized to obtain compound 10-8 (hydrobromide salt).

**MS (ESI)** *m*/*z* (M+H)⁺ = 521.2.

### Step 8: Preparation of compound 10

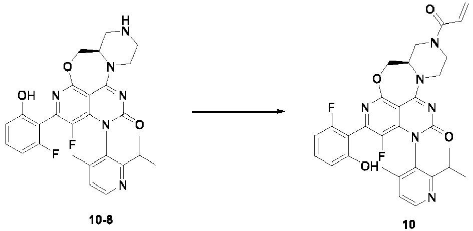

Compound 10-8 (81 mg, 107.74 µmol, hydrobromide salt) was dissolved in tetrahydrofuran (5 mL) and a saturated aqueous solution of NaHCO₃ (5 mL), and acrylic anhydride (0.2 M, 646.45 µL) was added to the resulting solution at room temperature (20 °C). After the completion of the addition, the system was stirred at room temperature (20 °C) for 1 h. Methanol (1 mL) and lithium hydroxide (0.2 M, 0.1 mL) were added to the system, which was then stirred at room temperature (20 °C) for another 2 h. The system was concentrated, diluted with methanol (5 mL), and filtered. The filtrate was concentrated to obtain a crude product. The crude product was purified by preparative high performance liquid chromatography (separation conditions: chromatographic column: Phenomenex Gemini-NX 80 × 30 mm × 3 µm; mobile phase: [water (10 mM ammonium bicarbonate solution)-acetonitrile]; acetonitrile%: 24%-54% 9.5 min) to obtain compound 10.

### Step 9: Preparation of compounds 10A and 10B

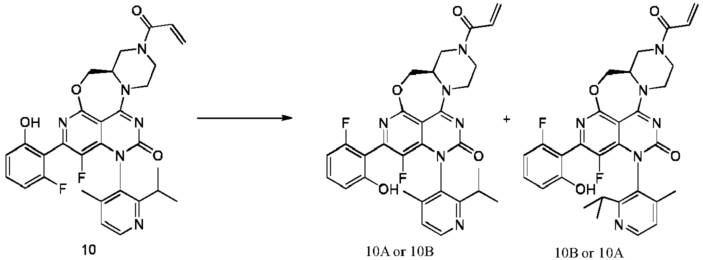

Diastereomer compound 10 was purified by SFC (separation conditions: chromatographic column: DAICEL CHIRALPAK AD (250 mm × 30 mm, 10 µm); mobile phase: [Neu-isopropanol (0.1% aqueous ammonia)]; isopropanol%: 35%-35%; flow rate: 80 mL/min) After concentration, compound 10A (peak 1) and compound 10B (peak 2) were obtained.

### Compound 10A:

**¹H NMR** (400 MHz, METHANOL-*d*₄) δ = 8.32 (d, *J* = 5.0 Hz, 1H), 7.20-7.07 (m, 2H), 6.80-6.67 (m, 1H), 6.61-6.49 (m, 2H), 6.20 (d, *J* = 16.8 Hz, 1H), 5.73 (dd, *J* = 1.4, 10.7 Hz, 1H), 4.72-4.49 (m, 3H), 4.48-4.02 (m, 3H), 3.74-3.27 (m, 3H), 2.87 (m, 1H), 2.07 (s, 3H), 1.14-1.00 (m, 6H)

**¹⁹F NMR** (376 MHz, METHANOL-*d*₄) δ = -116.83 (s, IF), -139.10 (s, IF)

**MS (ESI)** *m*/*z* (M+H) = 575.3.

**HPLC** retention time: 6.31 min

Separation conditions: chromatographic column: WELCH Ultimate LP-C18 150 × 4.6 mm 5 µm; column temperature: 40 °C; mobile phase: water (0.0688% trifluoroacetic acid solution)-acetonitrile (0.0625% trifluoroace ic acid solution); acetonitrile: 10%-80% 10 min, 80% 5 min; flow rate: 1.5 mL/min.

**SFC** retention time: 1.853 min

Separation conditions: chromatographic column: Chiralpak AD-3 50 × 4.6 mm I.D., 3 µm; column temperature: 35 °C; mobile phase: [Neu-isopropanol (0.05% DEA)]; isopropanol%: 5%-40% in 2 min, 40% 1.2 min, 5% 0.8 min; flow rate: 4 mL/min.

### Compound 10B:

**¹H NMR** (400 MHz, METHANOL-*d*₄) δ = 8.31 (d, *J* = 5.0 Hz, 1H), 7.19-7.10 (m, 2H), 6.83-6.67 (m, 1H), 6.61-6.48 (m, 2H), 6.20 (dd, J = 1.5, 16.8 Hz, 1H), 5.73 (dd, J = 1.5, 10.6 Hz, 1H), 4.75-4.50 (m, 3H), 4.50-3.98 (m, 3H), 3.60-3.23 (m, 3H), 2.86 (m, 1H), 1.15-1.01 (m, 6H)

**¹⁹F NMR** (376 MHz, METHANOL-*d*₄) δ = -116.81 (s, IF), -138.96 (s, IF)

**MS (ESI)** *m*/*z* (M+H)⁺ = 575.3.

**HPLC** retention time: 6.31 min

Separation conditions: chromatographic column: WELCH Ultimate LP-C18 150 × 4.6 mm 5 µm; column temperature: 40 °C; mobile phase: water (0.0688% trifluoroacetic acid solution)-acetonitrile (0.0625% trifluoroacetic acid solution); acetonitrile: 10%-80% 10 min, 80% 5 min; flow rate: 1.5 mL/min.

**SFC** retention time: 2.071 min

Separation conditions: chromatographic column: Chiralpak AD-3 50 × 4.6 mm I.D., 3 µm; column temperature: 35 °C; mobile phase: [Neu-isopropanol (0.05% DEA)]; isopropanol%: 5%-40% in 2 min, 40% 1.2 min, 5% 0.8 min; flow rate: 4 mL/min.

### Example 11: Preparation of Compound 11

### Step 1: Preparation of compound 11-1

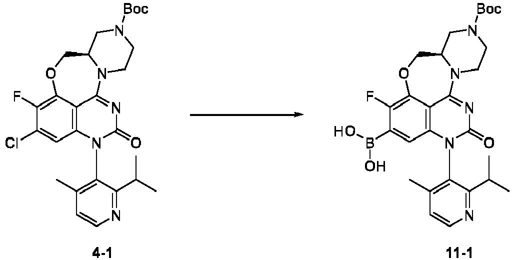

Compound 4-1 (270 mg, 496.31 µmol) was dissolved in dioxane (10 mL), and the compounds bis(pinacolato)diboron (270.00 mg, 1.06 mmol), tricyclohexylphosphine tetrafluoroborate (54.00 mg, 146.64 µmol), tris(dibenzylideneacetone)dipalladium (54.00 mg, 58.97 µmol) and potassium acetate (270 mg, 2.75 mmol) were successively added to the resulting solution. After the completion of the addition, the system was warmed to 100 °C and stirred for 3 h under an Ar atmosphere. The system was concentrated to obtain a crude product. The crude product was purified by silica gel column chromatography (ethyl acetate/petroleum ether (v/v) = 0-50%) to obtain 11-1.

**¹H NMR** (400 MHz, MeOD) δ 8.52 (d, *J* = 5.1 Hz, 1H), 7.35 (d, *J* = 4.9 Hz, 1H), 6.09 (br s, 1H), 4.99 (br d,*J* = 14.3 Hz, 1H), 4.73-4.46 (m, 2H), 4.29-3.88 (m, 3H), 3.43-3.31 (m, 3H), 2.87-2.58 (m, 1H), 2.05 (d, *J* = 3.5 Hz, 3H), 1.50 (s, 9H), 1.33-0.76 (m, 6H).

**MS (ESI)** *m*/*z* (M+H)⁺ = 554.3.

### Step 2: Preparation of compound 11-3

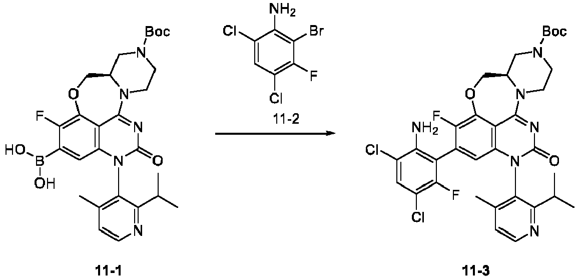

Compound 11-1 (100.00 mg, 126.49 µmol) was dissolved in dioxane (3 mL) and water (0.3 mL), and compound 11-2 (70.08 mg, 270.70 µmol), 2-dicyclohexylphosphine-2,4,6-triisopropylbiphenyl (5 mg, 10.49 µmol), (2-dicyclohexylphosphino-2',4',6'-tri-isopropyl-1,1'-biphenyl)(2'-amino-1,1'-biphenyl-2-yl)palladium methanesulfonate (5 mg, 5.91 µmol) and potassium carbonate (70 mg, 506.49 µmol) were successively added to the resulting solution. After the completion of the addition, the system was warmed to 100 °C and stirred for 2 h under an argon atmosphere. The system was concentrated to obtain a crude product. The crude product was purified by silica gel column chromatography (methanol/dichloromethane (v/v) = 0-7%) to obtain compound 113. **MS (ESI)** *m*/*z* (M+H)⁺ = 687.0.

### Step 3: Preparation of compound 11-4

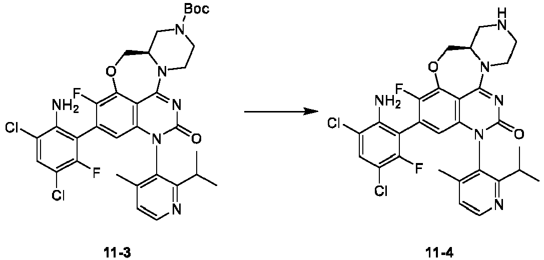

Compound 11-3 (100 mg, 145.44 µmol) was dissolved in dichloromethane (2.5 mL), and trifluoroacetic acid (154.00 mg, 1.35 mmol, 100 µL) was added to the resulting solution. The system was reacted at room temperature (20 °C) for 3 h. The system was concentrated to obtain compound 11-4 (trifluoroacetate salt).

**MS (ESI)** *m*/*z* (M+H)⁺ = 587.2.

### Step 4: Preparation of compound 11

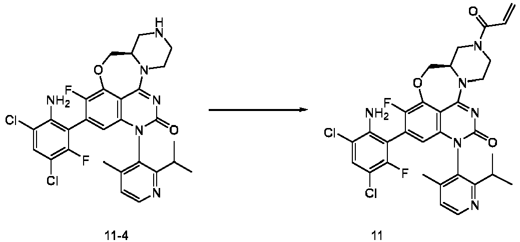

Compound 11-4 (100 mg, 142.56 µmol, trifluoroacetate salt) was dissolved in tetrahydrofuran (3 mL) and a saturated aqueous solution of sodium bicarbonate (2 mL), and acrylic anhydride (17.98 mg, 142.56 µmol) was added to the resulting solution at room temperature (20 °C). After the completion of the addition, the system was stirred at room temperature (20 °C) for 30 min. Water (10 mL) was added to the system, which was then extracted with ethyl acetate (10 mL × 2). The organic phase was dried over anhydrous sodium sulfate and filtered. The filtrate was concentrated to obtain a crude product. The crude product was purified by preparative high performance liquid chromatography (separation conditions: chromatographic column: Phenomenex Gemini-NX 80 × 30 mm × 3 µm; mobile phase: [water (10 mM ammonium bicarbonate solution)-acetonitrile]; acetonitrile%: 42%-72%, 9.5 min) to obtain compound 11.

**¹H NMR** (400 Hz, Acetonitrile-*d*₃) δ 8.56-8.42 (m, 1H), 7.40 (br d, *J* = 7.3 Hz, 1H), 7.21 (br d, *J* = 4.0 Hz, 1H), 6.75 (br s, 1H), 6.32-6.18 (m, 1H), 5.99 (br t, *J* = 5.2 Hz, 1H), 5.75 (br dd, *J* = 2.1, 10.7 Hz, 1H), 4.89-4.36 (m, 5H), 4.17-4.05 (m, 1H), 3.63-3.38 (m, 2H), 2.92-2.74 (m, 1H), 2.73-2.72 (m, 1H), 2.02 (br dd, *J* = 4.7, 18.4 Hz,3H), 1.42-0.78 (m, 6H).

**MS (ESI)** *m*/*z* (M+H)⁺ = 641.1.

### Step 5: Preparation of compounds 11A, 11B, 11C and 11D

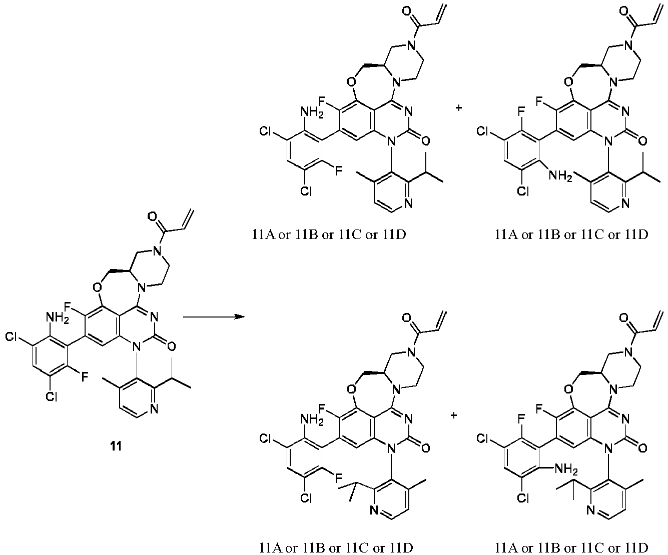

Diastereomer compound 11 was purified by SFC (separation conditions: chromatographic column: DAICEL CHIRALCEL OD (250 mm × 30 mm, 10 µm); mobile phase: [Neu-ethanol (0.1% aqueous ammonia)]; ethanol%: 45%-45%) After concentration, compound 11A, compound 11B, compound 11C and compound 11D were obtained.

### Compound 11A:

**¹H NMR** (400 MHz, MeOD) δ 8.50 (br d, *J* = 5.1 Hz, 1H), 7.39 (br d, *J* = 7.5 Hz, 1H), 7.32 (br d, *J* = 3.3 Hz, 1H), 6.84 (br s, 1H), 6.31 (br d, *J* = 18.7 Hz, 1H), 5.97 (br dd, *J* = 5.4, 8.5 Hz, 1H), 5.84 (br d, *J* = 12.3 Hz, 1H), 5.00-4.94 (m, 1H), 4.74 (br d, *J* = 10.6 Hz, 2H), 4.59-4.23 (m, 3H), 3.63-3.40 (m, 3H), 2.90-2.79 (m, 1H), 2.18 -1.98 (m,3H), 1.19 (br dd, *J* = 2.5, 6.7 Hz, 3H), 1.14-1.01 (m, 3H).

**MS (ESI)** *m*/*z* (M+H) = 641.1.

**HPLC** retention time: 4.142 min

Separation conditions: chromatographic column: Xbridge Shield RP-18, 5 µm, 2.1 × 50 mm; column temperature: 50 °C; mobile phase: water (0.02% aqueous ammonia)-acetonitrile; acetonitrile: 10%-80% 6 min, 80% 2 min; flow rate: 0.8 mL/min.

**SFC** retention time: 2.975 min + 7.090 min

Separation conditions: chromatographic column: Chiralcel OD-3 150 × 4.6 mm I.D., 3 µm; column temperature: 35 °C; mobile phase: [Neu-;thanol (0.05% DEA)]; ethanol%: 40%-40%; flow rate: 2.5 mL/min.

### Compound 11B:

**¹H NMR** (400 MHz, MeOD) δ 8.58-8.40 (m, 1H), 7.39 (br d, *J* = 7.3 Hz, 1H), 7.32 (br d,*J* = 4.6 Hz, 1H), 6.85 (br dd, *J* = 9.0, 16.3 Hz, 1H), 6.31 (br d, *J* = 15.9 Hz, 1H), 5.96 (br dd, *J* = 5.5, 8.6 Hz, 1H), 5.84 (br d, *J* = 11.9 Hz, 1H), 5.00 (br d, *J* = 14.8 Hz, 1H), 4.84-4.66 (m, 2H), 4.66-4.55 (m, 1H), 4.50-4.33 (m, 1H), 4.23 (br s, 1H), 3.71 -3.45 (m, 3H), 2.91-2.74 (m, 1H), 2.23-1.97 (m,3H), 1.20 (br dd, *J* = 3.1, 6.4 Hz, 3H), 1.19-1.00 (m, 3H).

**MS (ESI)** *m*/*z* (M+H)⁺ = 641.1.

**HPLC** retention time: 4.133 min

Separation conditions: chromatographic column: Xbridge Shield RP-18, 5 µm, 2.1 × 50 mm; column temperature: 50 °C; mobile phase: water (0.02% aqueous ammonia)-acetonitrile; acetonitrile: 10%-80% 6 min, 80% 2 min; flow rate: 0.8 mL/min.

**SFC** retention time: 2.999 min + 4.228 min + 7.114 min + 9.409 min

Separation conditions: chromatographic column: Chiralcel OD-3 150 × 4.6 mm I.D., 3 µm; column temperature: 35 °C; mobile phase: [Neu-ethanol (0.05% DEA)]; ethanol%: 40%-40%; flow rate: 2.5 mL/min.

### Compound 11C:

**¹H NMR** (400 MHz, MeOD) δ 8.50 (d, *J* = 5.1 Hz, 1H), 7.39 (br d, *J* = 7.5 Hz, 1H), 7.32 (br d, *J* = 4.2 Hz, 1H), 6.85 (br dd, *J* = 8.6, 16.5 Hz, 1H), 6.31 (br d, *J* = 16.8 Hz, 1H), 5.97 (br dd, *J* = 5.4, 8.3 Hz, 1H), 5.84 (br dd, *J* = 1.8, 10.6 Hz, 1H), 5.00 (br d, *J* = 13.2 Hz, 1H), 4.80-4.68 (m, 2H), 4.60-4.23 (m, 3H), 3.63-3.39 (m, 3H), 2.91-2.77 (m, 1H), 2.17-2.00 (m, 3H), 1.25-0.96 (m, 6H).

**MS (ESI)** m/z (M+H)⁺ = 641.1.

**HPLC** retention time: 4.136 min

Separation conditions: chromatographic column: Xbridge Shield RP-18, 5 µm, 2.1 × 50 mm; column temperature: 50 °C; mobile phase: water (0.02% aqueous ammonia)-acetonitrile; acetonitrile: 10%-80% 6 min, 80% 2 min; flow rate: 0.8 mL/min.

**SFC** retention time: 2.999 min + 4.234 min + 7.118 min + 9.426 min

Separation conditions: chromatographic column: Chiralcel OD-3 150 × 4.6 mm I.D., 3 µm; column temperature: 35 °C; mobile phase: [Neu-ethanol (0.05% DEA)]; ethanol%: 40%-40%; flow rate: 2.5 mL/min.

### Compound 11D:

**¹H NMR** (400 MHz, MeOD) δ 8.49 (br d, *J* = 4.9 Hz, 1H), 7.39 (br d, *J* = 7.5 Hz, 1H), 7.32 (br d, *J* = 4.9 Hz, 1H), 6.91-6.79 (m, 1H), 6.31 (br d, *J* = 16.8 Hz, 1H), 5.96 (br dd, *J* = 5.4, 8.7 Hz, 1H), 5.84 (br d, *J* = 10.6 Hz, 1H), 5.02-4.68 (m, 3H), 4.60-4.23 (m, 3H), 3.65-3.48 (m, 3H), 2.87-2.74 (m, 1H), 2.15-2.01 (m, 3H), 1.26-0.99 (m, 6H).

**MS (ESI)** m/z (M+H)⁺ = 641.1.

**HPLC** retention time: 4.140 min

Separation conditions: chromatographic column: Xbridge Shield RP-18, 5 µm, 2.1 × 50 mm; column temperature: 50 °C; mobile phase: water (0.02% aqueous ammonia)-acetonitrile; acetonitrile: 10%-80% 6 min, 80% 2 min; flow rate: 0.8 mL/min.

**SFC** retention time: 4.210 min + 9.403 min

Separation conditions: chromatographic column: Chiralcel OD-3 150 × 4.6 mm I.D., 3 µm; column temperature: 35 °C; mobile phase: [Neu-ethanol (0.05% DEA)]; ethanol%: 40%-40%; flow rate: 2.5 mL/min.

### Example 12: Preparation of Compound 12

### Step 1: Preparation of compound 12-2

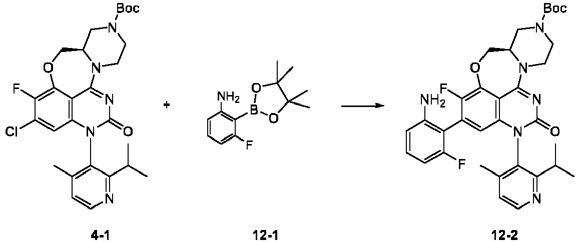

Under a nitrogen atmosphere, compound 4-1 (100 mg, 183.82 µmol), compound 12-1 (80 mg, 292.46 µmol) were dissolved in dioxane (2 mL) and water (0.4 mL), and (2-dicyclohexylphosphino-2',4',6'-tri-isopropyl-1,1'-biphenyl)(2'-amino-1,1'-biphenyl-2-yl)palladium methanesulfonate (20 mg, 23.63 µmol), 2-dicyclohexylphosphonium-2,4,6-triisopropylbiphenyl (20 mg, 41.95 µmol) and potassium carbonate (100 mg, 723.54 µmol) were successively added. The reaction system was stirred at 100 °C for 16 h. The reaction system was concentrated under reduced pressure. The crude product was purified by preparative thin-layer chromatography (methanol/dichloromethane (v/v) = 0-7%) to separate compound 12-2.

**MS (ESI)** m/z (M+H)⁺ = 619.3.

### Step 2: Preparation of compound 12-3

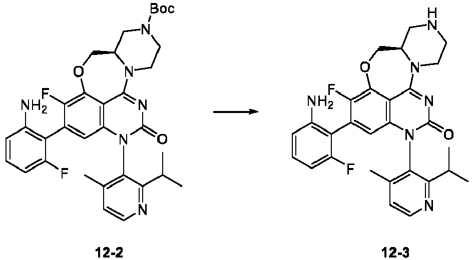

Compound 12-2 (0.12 g, 193.96 µmol) was dissolved in dichloromethane (5 mL) at 25 °C, and trifluoroacetic acid (770 mg, 6.75 mmol, 0.5 mL) was added. The reaction system was stirred for 3 h. The reaction system was concentrated under reduced pressure to obtain compound 12-3.

**MS (ESI)** m/z (M+H)⁺ = 519.3.

### Step 3: Preparation of compound 12

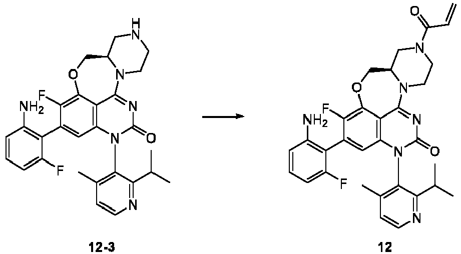

Compound 12-3 (0.1 g, 192.84 µmol) was dissolved in tetrahydrofuran (4 mL), and saturated sodium bicarbonate solution (6.05 g, 71.99 mmol, 2.8 mL), acrylic anhydride (21.89 mg, 173.56 µmol) were successively added. The reaction system was stirred at 25 °C for 2 h. Methanol (3 mL) and lithium hydroxide (32.37 mg, 771.37 µmol) were added, and the reaction system was stirred for another 2 h. The reaction system was adjusted to pH 7.0 with 1 M hydrochloric acid solution and extracted with ethyl acetate (10 mL × 2). The organic phase was dried over anhydrous sodium sulfate and filtered. The filtrate was concentrated. The crude product was subjected to purification by preparative high performance liquid chromatography (separation conditions: chromatographic column: Phenomenex Gemini-NX 80 × 30 mM × 3 µm; mobile phase: [water (10 mM ammonium bicarbonate)-acetonitrile]; acetonitrile%: 29%-59%, 9.5 min) to obtain compound 12.

**MS (ESI)** m/z (M+H)⁺ = 573.3.

### Step 4: Preparation of compounds 12A and 12B

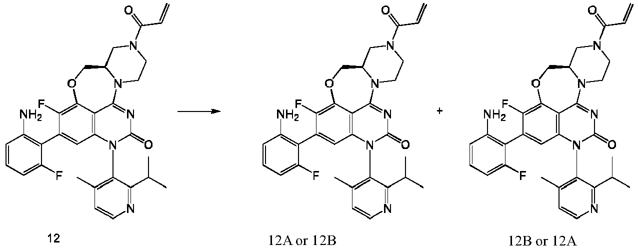

Diastereomer compound 12 was purified by SFC (separation conditions: chromatographic column: Phenomenex-Cellulose-2 (250 mm × 50 mm, 10 µm); mobile phase: [Neu-methanol (0.1% aqueous ammonia)]; methanol%: 50%-50%) After concentration, compound 12A (peak 1) and compou d 12B (peak 2) were obtained.

### Compound 12A

**¹H NMR** (400 MHz, Methanol-*d*₄) δ 8.49 (d, *J* = 5.1 Hz, 1H), 7.31 (d, *J* =4.9 Hz, 1H), 7.14-7.03 (m, 1H), 6.92-6.80 (m, *J* = 10.9, 16.4 Hz, 1H), 6.53 (t, *J* = 3.5 Hz, 1H), 6.42-6.26 (m, 2H), 5.97 (dd, *J* = 5.4, 9.8 Hz, 1H), 5.83 (dd, *J* = 1.8, 10.6 Hz, 1H), 5.02 (s, 1H), 4.74-4.32 (m, 5H), 4.22 (s, 1H), 3.81-3.38 (m, 2H), 2.97-2.77 (m, 1H), 2.14-2.01 (m,3H), 1.19 (dd, *J* = 3.5, 6.6 Hz, 3H), 1.14-1.03 (m, 3H).

**MS (ESI)** m/z (M+H) = 573.3.

**HPLC** retention time: 7.15 min

Separation conditions: chromatographic column: WELCH Ultimate LP-C18 150 × 4.6 mm 5 µm; column temperature: 40 °C; mobile phase: water (0.06875 % trifluoroacetic acid)-acetonitrile (0.0625 % trifluoro acetic acid); acetonitrile: 10%-80% 10 min, 80% 5 min; flow rate: 1.5 mL/min.

**SFC** retention time: 8.176 min

Separation conditions: chromatographic column: Cellulose 2 100 × 4.6 mm I.D., 3 µm; column temperature: 35 °C; mobile phase: [Neu-methanol (0.05% DEA)]; methanol%: 40%-40%; flow rate: 2.8 mL/min.

### Compound 12B

**¹H NMR** (400 MHz, Methanol-*d*₄) 8.49 (d, *J* = 5.0 Hz, 1H), 7.32 (d, *J* = 4.8 Hz, 1H), 7.13-7.03 (m, 1H), 6.94-6.80 (m, 1H), 6.57-6.49 (m, 1H), 6.41 -6.26 (m, 2H), 5.96 (dd, *J* = 5.5, 9.3 Hz, 1H), 5.83 (dd, *J* = 1.6, 10.7 Hz, 1H), 5.05 (br d, *J* = 3.0 Hz, 1H), 4.80-4.16 (m, 5H), 3.76-3.35 (m, 3H), 2.90-2.72 (m, 1H), 2.14-2.04 (m, 3H), 1.19 (dd, *J* = 4.4, 6.9 Hz, 3H), 1.14-1.02 (m, 3H).

**MS (ESI)** n/z (M+H) = 573.3.

**HPLC** retention time: 7.12 min

Separation conditions: chromatographic column: WELCH Ultimate LP-C18 150 × 4.6 mm 5 µm; column temperature: 40 °C; mobile phase: water (0.06875 % trifluoroacetic acid)-acetonitrile (0.0625 % trifluoroacetic acid); acetonitrile: 10%-80% 10 min, 80% 5 min; flow rate: 1.5 mL/min.

**SFC** retention time: 10.832 min

Separation conditions: chromatographic column: Cellulose 2 100 × 4.6 mm I.D., 3 µm; column temperature: 35 °C; mobile phase: [Neu-methanol (0.05% DEA)]; methanol%: 40%-40%; flow rate: 2.8 mL/min.

### Example 13: Preparation of Compound 13

### Step 1: Preparation of compound 13-1

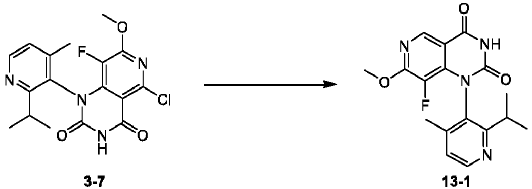

Compound 3-7 (2 g, 5.28 mmol), triethylamine (801.42 mg, 7.92 mmol) and palladium on carbon (666 mg, 10% in carbon) were dissolved in methanol (100 mL). The system was warmed to 30 °C under a hydrogen atmosphere (50 psi) for 20 h. The system was filtered, and the filtrate was concentrated to obtain compound 13-1.

**MS (ESI)** *m*/*z* (M+H)⁺ = 344.9.

### Step 2: Preparation of compound 13-2

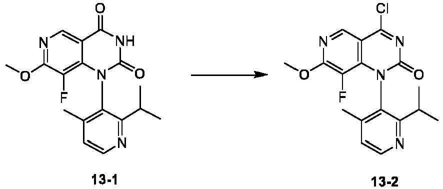

Compound 13-1 (1.8 g, 5.23 mmol) and *N*,*N*-diisopropylethylamine (2.03 g, 15.68 mmol, 2.73 mL) were dissolved in acetonitrile (30 mL), and phosphorus oxychloride (1.60 g, 10.45 mmol) was added to the resulting solution at room temperature (20 °C). After the completion of the addition, the system was warmed to 70 °C and stirred for 1 h. The system was concentrated to obtain compound 13-2.

### Step 3: Preparation of compound 13-4

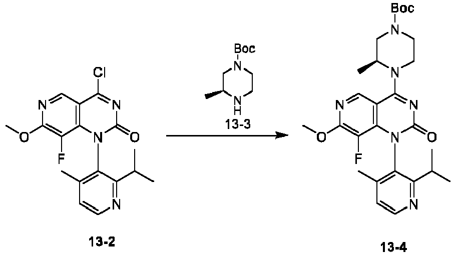

Compound 13-2 (2.6 g, 4.30 mmol), 13-3 (1.03 g, 5.16 mmol) and *N*,*N-*diisopropylethylamine (2.22 g, 17.20 mmol) were dissolved in tetrahydrofuran (20 mL). The system was stirred at room temperature (20 °C) under a nitrogen atmosphere for 1 h. The system was concentrated to obtain a crude product. The crude product was purified by medium-pressure column chromatography (ethyl acetate/petroleum ether (v/v) = 0-70%) to obtain compound 13-4.

**MS (ESI)** *m*/*z* (M+H)⁺ = 527.1.

### Step 4: Preparation of compound 13-5

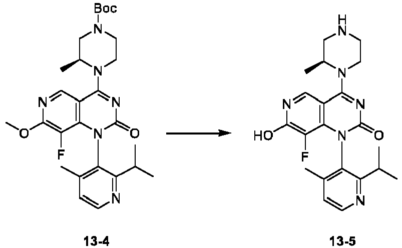

Compound 13-4 (1.8 g, 3.42 mmol) was dissolved in dichloromethane (5 mL), and boron tribromide (8.56 g, 34.18 mmol, 3.29 mL) was added. The reaction system was stirred at 20 °C for 16 h. The reaction system was quenched with methanol (20 mL), stirred for 10 min, and concentrated under reduced pressure to obtain compound 13-5.

### Step 5: Preparation of compound 13-6

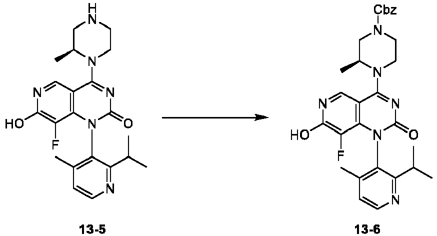

Compound 13-5 (1.2 g, 2.43 mmol) was dissolved in tetrahydrofuran (10 mL), and an aqueous solution of sodium bicarbonate (1.02 g, 12.16 mmol in 5 mL H₂O 5 mL) and benzyl chloroformate (622.39 mg, 3.65 mmol, 518.65 µL) were added to the resulting solution. After the completion of the addition, the system was allowed to react at room temperature (20 °C) for 16 h. Ethyl acetate (50 mL) was added to the system for liquid separation and extraction. The organic phases were pooled, dried over anhydrous sodium sulfate, and filtered. The filtrate was concentrated to obtain a crude product, which was then purified by medium-pressure column chromatography (methanol/dichloromethane (v/v) = 0-10%) to obtain compound 13-6.

**MS (ESI)** *m*/*z* (M+H)⁺ = 547.1.

### Step 6: Preparation of compound 13-7

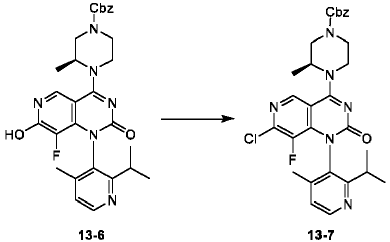

Compound 13-6 (80 mg, 146.36 µmol) was dissolved in acetonitrile (5 mL), and phosphorus oxychloride (448.83 mg, 2.93 mmol) was added to the resulting solution at room temperature (20 °C). After the completion of the addition, the system was warmed to 80 °C and stirred for 4 h. The system was concentrated to obtain a crude product, which was then purified by medium-pressure column chromatography (methanol/dichloromethane (v/v) = 0-10%) to obtain compound 13-7.

**MS (ESI)** *m*/*z* (M+H)⁺ = 565.1.

### Step 7: Preparation of compound 13-8

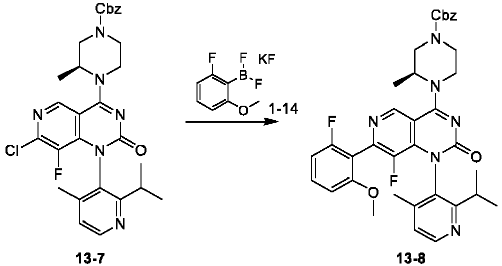

Compound 13-7 (70 mg, 123.89 µmol) was dissolved in dioxane (1 mL) and water (0.1 mL), and compound 1-14 (43.12 mg, 185.83 µmol), 2-dicyclohexylphosphonium-2,4,6-triisopropylbiphenyl (11.81 mg, 24.78 µmol), tris(dibenzylideneacetone)dipalladium (11.34 mg, 12.39 µmol) and sodium carbonate (26.26 mg, 247.77 µmol) were successively added to the resulting solution. After the completion of the addition, the system was warmed to 100 °C and stirred for 16 h under an Ar atmosphere. The system was concentrated to obtain a crude product. The crude product was purified by silica gel column chromatography (methanol/dichloromethane (v/v) = 0-5%) to obtain compound 13-8.

**MS (ESI)** *m*/*z* (M+H)⁺ = 655.2.

### Step 8: Preparation of compound 13-9

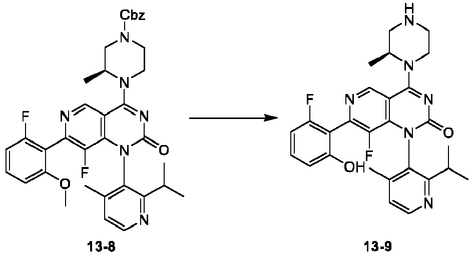

Compound 13-8 (16 mg, 24.44 µmol) was dissolved in anhydrous dichloromethane (1 mL), and boron tribromide (1 M in dichloromethane, 807.55 µL) was added to the resulting solution at 0 °C. After the completion of the addition, the system was warmed to room temperature (20 °C) and stirred for 2 h. Methanol (5 mL) was added to the system, and the resulting mixture was stirred for 10 min. The system was concentrated and lyophilized to obtain compound 13-9 (hydrobromide salt).

**MS (ESI)** *m*/*z* (M+H)⁺ = 507.2.

### Step 9: Preparation of compound 13

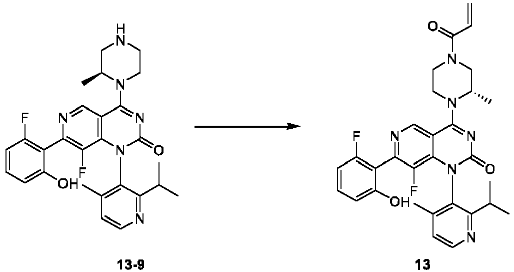

Compound 13-9 (14 mg, 23.83 µmol, hydrobromide salt) was dissolved in tetrahydrofuran (2 mL) and a saturated aqueous solution of sodium bicarbonate (2 mL), and acrylic anhydride (0.2 M, 178.74 µL) was added to the resulting solution at room temperature (20 °C). After the completion of the addition, the system was stirred at room temperature (20 °C) for 1 h. The system was quenched with methanol (1 mL) and extracted with ethyl acetate (5 mL). The system separated into layers. The organic phases were pooled, dried over anhydrous sodium sulfate, and filtered. The filtrate was concentrated to obtain a crude product. The crude product was purified by preparative high performance liquid chromatography (separation conditions: chromatographic column: Phenomenex Gemini-NX 80 × 30 mm × 3 µm; mobile phase: [water (10 mM ammonium bicarbonate solution)-acetonitrile]; acetonitrile%: 18%-48%, 9 min) to obtain compound 13.

**¹H NMR** (400 MHz, METHANOL-*d*₄) δ = 9.01 (s, 1H), 8.45 (d, *J* = 5.0 Hz, 1H), 7.34-7.24 (m, 2H), 6.96-6.78 (m, 1H), 6.74-6.61 (m, 2H), 6.33 (br d, *J* = 16.3 Hz, 1H), 5.89-5.82 (m, 1H), 5.16-5.04 (m, 1H), 4.62-4.41 (m, 2H), 4.29-4.07 (m, 1H), 3.97-3.51 (m, 2H), 3.41-3.34 (m, 1H), 3.05-2.88 (m, 1H), 2.18-2.15 (m, 3H), 1.56-1.45 (m,3H), 1.24-1.13 (m, 6H)

**¹⁹F NMR** (376 MHz, METHANOL-*d*₄) 5 = -116.75 (s, IF)

**MS (ESI)** *m*/*z* (M+H)⁺ = 583.0.

### Example 14: Preparation of Compound 14

### Step 1: Preparation of compound 14-3

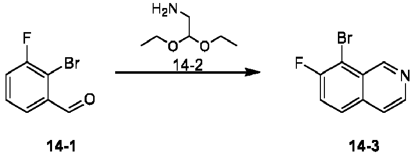

Compound 14-1 (8.5 g, 41.87 mmol) was dissolved in toluene (20 mL), and compound 14-2 (5.56 g, 41.75 mmol, 6.07 mL) was added to the resulting solution with stirring. After the completion of the addition, the system was warmed to 100 °C and stirred for 18 h under a nitrogen atmosphere. The system was cooled to room temperature and concentrated. The residue was dissolved in dichloromethane (30 mL). The system was cooled to 0 °C, and aluminum chloride (18.38 g, 137.84 mmol, 7.53 mL) was added thereto in batches. After the completion of the addition, the system was stirred at 0 °C for 30 min. The system was slowly warmed to room temperature (25 °C), and then allowed to react at room temperature (25 °C) for 16 h. The system was poured into ice water, and dichloromethane (100 mL) was added to the resulting mixture. The system was alkalified with 2 M aqueous solution of sodium hydroxide and then separated into layers. The organic phase was dried over anhydrous sodium sulfate and filtered. The filtrate was concentrated to obtain a crude product, which was then purified by silica gel column chromatography (ethyl acetate/petroleum ether (v/v) = 0-100%) to obtain compound 14-3.

**¹H NMR** (400 MHz, Chloroform-*d*) δ 9.63 (s, 1H), 8.63 (d, *J* = 5.7 Hz, 1H), 7.83 (dd, *J* = 5.0, 8.9 Hz, 1H), 7.65 (d, *J* = 5.5 Hz, 1H), 7.53 (t, *J* = 8.6 Hz, 1H).

### Step 2: Preparation of compound 14-4

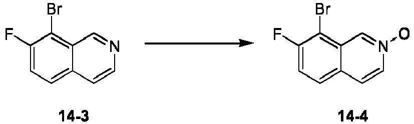

Compound 14-3 (2 g, 8.85 mmol) was dissolved in dichloromethane (30 mL), and m-chloroperoxybenzoic acid (2.80 g, 13.78 mmol, 85% purity) was added to the resulting solution. After the completion of the addition, the system was allowed to react at room temperature (25 °C) for 2 h. Dichloromethane (100 mL) was added to the system. After washing with a saturated aqueous solution of sodium bicarbonate (100 mL × 2), the organic phase was dried over anhydrous sodium sulfate and filtered. The filtrate was concentrated to obtain compound 14-4, which was used in the next step without further purification.

**¹H NMR** (400 MHz, Chloroform-*d*) 5 9.15 (s, 1H), 8.17 (dd, *J* = 1.3, 7.1 Hz, 1H), 7.77 (dd, *J* = 5.0, 8.9 Hz, 1H), 7.68 (d, *J* = 7.1 Hz, 1H), 7.44-7.37 (m, 1H).

**MS (ESI)** m/z (M+H)⁺ = 243.8.

### Step 3: Preparation of compound 14-5

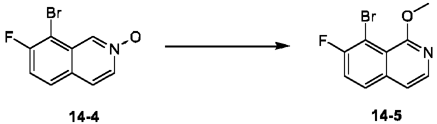

Compound 14-4 (500 mg, 2.07 mmol) and methyl chloroformate (253.77 mg, 2.69 mmol, 208.01 µL) were dissolved in methanol (10 mL) at 0 °C, and triethylamine (420.15 mg, 4.15 mmol, 577.92 µL) was added to the resulting solution at 0 °C. After the completion of the addition, the system was warmed to room temperature (25 °C) and stirred for 16 h. The system was concentrated, and the residue was dissolved in dichloromethane (10 mL). The resulting solution was washed successively with water (20 mL) and saturated brine (20 mL). The organic phase was dried over anhydrous sodium sulfate and filtered. The filtrate was concentrated to obtain a crude product, which was then purified by silica gel column chromatography (ethyl acetate/petroleum ether (v/v) = 0-20%) to obtain compound 14-5.

**¹H NMR** (400 MHz, Chloroform-*d*) δ 8.00 (d, *J* = 5.7 Hz, 1H), 7.69 (dd, *J* = 5.3, 9.0 Hz, 1H), 7.44 (t, *J* = 8.5 Hz, 1H), 7.22 (d, *J* = 5.7 Hz, 1H), 4.12 (s, 3H).

**MS (ESI)** m/z (M+H)⁺ = 257.7.

### Step 4: Preparation of compound 14-6

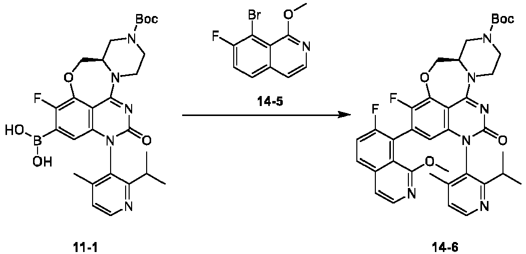

Compound 11-1 (130 mg, 234.92 µmol) and compound 14-5 (120.31 mg, 469.83 µmol) were dissolved in dioxane (2 mL) and water (0.4 mL), and 2-dicyclohexylphosphine-2,4,6-triisopropylbiphenyl (13.00 mg, 27.27 µmol), (2-dicyclohexylphosphino-2',4',6'-triisopropyl-1,1'-biphenyl)(2'-amino-1,1'-biphenyl-2-yl)palladium methanesulfonate (19.50 mg, 23.04 µmol) and potassium carbonate (97.40 mg, 704.75 µmol) were added to the resulting solution. After the completion of the addition, the system was warmed to 100 °C and stirred for 16 h under an Ar atmosphere. The system was concentrated to obtain a crude product. The crude product was purified by silica gel column chromatography (methanol/dichloromethane (v/v) = 0-7%) to obtain compound 14-6.

**MS (ESI)** *m*/*z* (M+H)⁺ = 685.3.

### Step 5: Preparation of compound 14-7

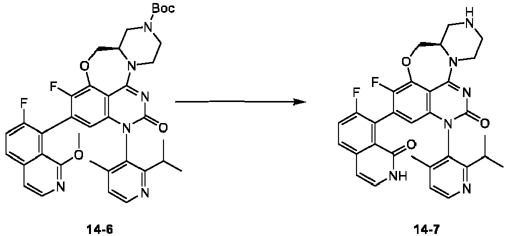

Compound 14-6 (50 mg, 73.02 µmol), lithium chloride (12.38 mg, 292.09 µmol, 5.98 µL), *p*-toluenesulfonic acid (55.56 mg, 292.09 µmol) were dissolved in *N,N-*dimethylformamide (2 mL). The system was warmed to 120 °C and allowed to react under microwave conditions for 2 h. The system was concentrated to obtain compound 14-7, which was used in the next step without further purification.

**MS (ESI)** *m*/*z* (M+H)⁺ = 571.1.

### Step 6: Preparation of compounds 14A and 14B

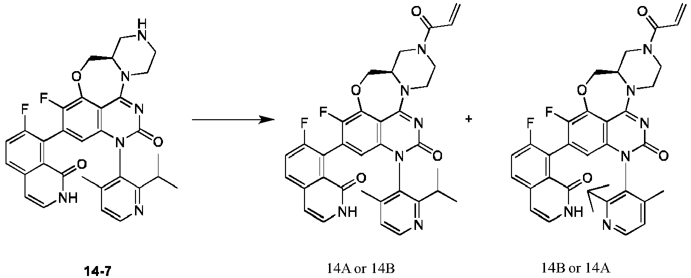

Compound 14-7 (50 mg, 87.63 µmol) was dissolved in tetrahydrofuran (2 mL) and a saturated aqueous solution of sodium bicarbonate (2.16 g, 25.71 mmol, 1 mL), and acrylic anhydride (11.05 mg, 87.63 µmol) was added to the resulting solution at room temperature (25 °C). After the completion of the addition, the system was stirred at room temperature (25 °C) for 30 min Methanol (2 mL) and an aqueous solution of potassium carbonate (2 mL) were added to the system, which was the stirred at room temperature (25 °C) for another hour The system was diluted with water (10 mL) and extracted with ethyl acetate (10 mL × 2) The organic phase was dried over anhydrous sodium sulfate and filtered. The filtrate was concentrated to obtain a crude product The crude product was purified by preparative high performance liquid chromatography (separation conditions: chromatographic column: Phenomenex Gemini-NX 80 × 30 mm × 3 µm; mobile phase: [water (10 mM ammonium bicarbonate solution)-acetonitrile]; acetonitrile%: 29%-59% 9.5 min) to obtain compounds 14A and 14B.

### Compound 14A:

**¹H NMR** (400 MHz, Methanol-*d*₄) δ 8.53-8.38 (m, 1H), 7.89-7.75 (m, 1H), 7.56 (br t, *J* = 9.2 Hz, 1H), 7.36 -7.21 (m, 1H), 7.19-7.07 (m, 1H), 7.03-6.88 (m, 1H), 6.73-6.61 (m, 1H), 6.44-6.29 (m, 1H), 5.93-5.83 (m, 1H), 5.81 (br d, *J* = 4.4 Hz, 1H), 4.78 (br s, 1H), 4.77-4.72 (m, 1H), 4.63 (br s, 1H), 4.58 (br s, 1H), 4.42 (br d, *J* = 10.8 Hz, 1H), 3.88 (br s, 1H), 3.61 (br s, 1H), 3.49 (br s, 1H), 3.01-2.88 (m, 1H), 2.77 (td, *J* = 6.9, 13.6 Hz, 1H), 2.26-1.98 (m, 3H), 1.88-1.60 (m, 3H), 1.38-0.96 (m, 6H).

**MS (ESI)** *m*/*z* (M+H) = 625.2.

**LCMS** retention time: 2.341 & 2.427 & 2.558 min

Separation condi ions: chromatographic column: WELCH Ultimate LP-C18 150 × 4.6 mm 5 µm; column temperature: 40 °C; mobile phase: water (0.0688% trifluoroacetic acid solution)-acetonitrile (0.0625% trifluoroacetic acid solution); acetonitrile: 5%-95% 0.7 min, 95% 0.4 min; flow rate: 1.5 mL/min.

### Compound 14B:

**¹H NMR** (400 MHz, Methanol-*d*₄)δ 8.54-8.39 (m, 1H), 7.85-7.74 (m, 1H), 7.64-7.48 (m, 1H), 7.33-7.22 (m, 1H), 7.18-7.08 (m, 1H), 7.01-6.88 (m, 1H), 6.73-6.61 (m, 1H), 6.41-6.30 (m, 1H), 5.97-5.75 (m, 2H), 5.06-4.95 (m, 1H), 4.88-4.41 (m, 5H), 3.70-3.34 (m, 3H), 2.99-2.75 (m, 1H), 2.14-2.04 (m, 3H), 1.22-1.00 (m, 6H).

**MS (ESI)** *m*/*z* (M+H)⁺ = 625.3.

**LCMS** retention time: 3.026 min

Separation conditions: chromatographic column: WELCH Ultimate LP-C18 150 × 4.6 mm 5 µm; column temperature: 40 °C; mobile phase: water (0.0375% trifluoroacetic acid solution)-acetonitrile (0.01875% trifluoroacetic acid solution); acetonitrile: 5%-95% 0.7 min, 95% 0.4 min; flow rate: 1.5 mL/min.

### Example 15: Preparation of Compound 15

### Step 1: Preparation of compound 15-2

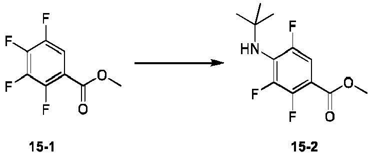

Compound 15-1 (50 g, 240.26 mmol) was dissolved in acetonitrile (100 mL), and *tert-*butylamine (98.40 g, 1.35 mol, 141.38 mL) was added to the resulting solution. After the completion of the addition, the system was warmed to 40 °C and stirred for 16 h. The system was concentrated. The residue was diluted with water (50 mL) and extracted with dichloromethane (50 mL × 3). The organic phases were pooled and washed with saturated brine (50 mL × 3), dried over anhydrous sodium sulfate, and filtered. The filtrate was concentrated to obtain compound 15-2, which was used in the next step without further purification.

### Step 2: Preparation of compound 15-3

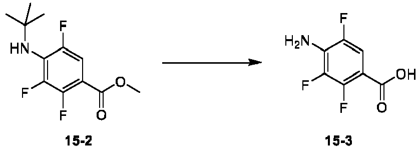

Compound 15-2 (57.5 g, 220.10 mmol) was dissolved in concentrated hydrochloric acid (12 M, 24.64 mL). After the completion of the addition, the system was warmed to 80 °C and stirred for 6 h. The system was concentrated. The residue was diluted with water (50 mL) and extracted with dichloromethane (50 mL × 3). The organic phases were pooled and washed with saturated brine (50 mL × 3), dried over anhydrous sodium sulfate, and filtered. The filtrate was concentrated to obtain a crude product. The crude product was dissolved in a mixed solvent of tetrahydrofuran (50 mL) and water (50 mL). The resulting solution was adjusted to about pH 10 with 1 N sodium hydroxide. After liquid separation and extraction, the aqueous phase was adjusted to about pH 3 with 6 M hydrochloric acid and extracted with ethyl acetate (50 mL × 3). The organic phases were pooled, washed with saturated brine (50 mL × 3), dried over anhydrous sodium sulfate, and filtered. The filtrate was concentrated to obtain compound 15-3.

**¹H NMR** (400 MHz, DMSO-*d*₆) δ 7.02 (br s, 2 H), 7.90(ddd, *J* = 11.58, 6.28, 1.76 Hz, 1 H).

**MS (ESI)** *m*/*z* (M+H)⁺ = 192.1.

### Step 3: Preparation of compound 15-4

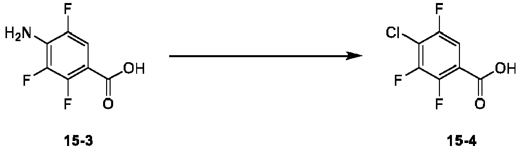

Cuprous chloride (13.52 g, 136.57 mmol, 3.27 mL) and tert-butyl nitrite (12.80 g, 124.13 mmol, 14.76 mL) were dissolved in acetonitrile (150 mL), and compound 15-3 (16 g, 83.72 mmol) was added to the resulting solution at 60 °C. The system was stirred at 60 °C for 20 min under a nitrogen atmosphere. The system was cooled to room temperature (25 °C), and hydrochloric acid (4.32 mol, 273 mL, 15% purity) was added thereto. The system was stirred at room temperature (25 °C) for 1 h. The system was quenched with sodium thiosulfate (10%, 5 mL) and extracted with ethyl acetate (50 mL × 3). The organic phases were pooled, washed with saturated brine (50 mL × 3), dried over anhydrous sodium sulfate, and filtered. The filtrate was concentrated to obtain a crude product, which was then purified by medium-pressure column chromatography (ethyl acetate/petroleum ether (v/v) = 0-20%) followed by slurrying with petroleum ether (30 mL) at room temperature (25 °C) for 1 h to obtain compound 15-4.

**¹H NMR** (400 MHz, Methanol-*d*₄) δ 7.60-7.81 (m, 1 H).

### Step 4: Preparation of compound 15-5

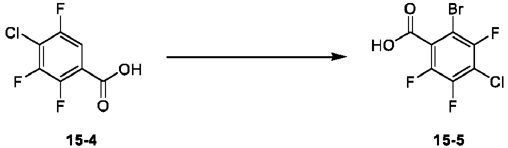

Diisopropylethylamine (1.23 g, 9.50 mmol, 1.65 mL) was dissolved in anhydrous tetrahydrofuran (60 mL), and *n*-butyllithium (2.5 M, 57.00 mL) was added dropwise to the resulting solution at -78 °C. After the completion of the addition, the system was stirred at-30 °C for 10 min. The system was cooled to -78 °C, and a solution of compound 15-4 (10 g, 47.50 mmol) in tetrahydrofuran (60 mL) was added thereto. After the completion of the addition, the system was stirred at -78 °C for 4 h. A solution of 1,2-dibromotetrachloroethane (30.93 g, 94.99 mmol, 11.41 mL) in tetrahydrofuran (60 mL) was added to the system. After the completion of the addition, the system was stirred at -78 °C for 2 h, and then warmed to room temperature (25 °C) and stirred for 16 h. The system was quenched with water (5 mL) and washed with ethyl acetate (50 mL × 3). The aqueous phase was adjusted to about pH 2 with 2 N hydrochloric acid and extracted with ethyl acetate (50 mL × 3). The organic phases were pooled, dried over anhydrous sodium sulfate, and filtered. The filtrate was concentrated to obtain a crude product. The crude product was purified by preparative high performance liquid chromatography (separation conditions: chromatographic column: Xtimate C18 150 × 40 mm × 5 µm; mobile phase: water (10 mM ammonium bicarbonate)-acetonitrile; acetonitrile: 30%-50% 8 min) to obtain compound 15-5.

### Step 5: Preparation of compound 15-6

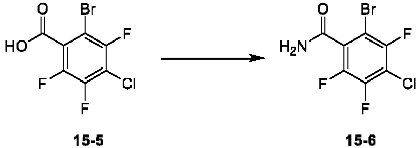

Compound 15-5 (3 g, 10.37 mmol) was dissolved in anhydrous dichloromethane (10 mL), and oxalyl chloride (1.97 g, 15.55 mmol, 1.36 mL) was added dropwise to the resulting solution at 0 °C, followed by 1 drop of *N*,*N*-dimethylformamide after the completion of the addition. The system was stirred at 0 °C for 15 min, and then warmed to 25 °C and stirred for 1 h. The system was concentrated to obtain a crude product, which was then dissolved in dioxane (5 mL), and aqueous ammonia (885.05 mg, 51.97 mmol, 867.69 µL) was added dropwise to the resulting solution at 0 °C. After the completion of the addition, the system was stirred at room temperature (25 °C) for 1 h. The system was concentrated, and the residue was subjected to liquid separation and extraction with water (20 mL) and ethyl acetate (50 mL × 3). The organic phases were pooled, dried over anhydrous sodium sulfate, and filtered. The filtrate was concentrated to obtain compound 15-6.

**MS (ESI)** *m*/*z* (M+H)⁺ = 289.7.

### Step 6: Preparation of compound 15-7

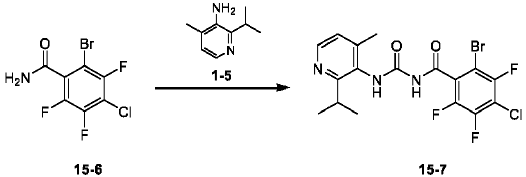

Compound 15-6 (2.8 g, 9.71 mmol) was dissolved in anhydrous 1,2-dichloroethane (20 mL), and oxalyl chloride (1.72 g, 13.59 mmol, 1.19 mL) was added dropwise to the resulting solution at room temperature (20 °C). After the completion of the addition, the system was warmed to 80 °C and stirred for 1 h. The reaction system was concentrated to half volume and cooled to 0 °C, and a solution of compound 1-5 (1.46 g, 9.71 mmol) in 1,2-dichloroethane (20 mL) was added dropwise thereto. After the completion of the addition, the system was stirred at room temperature (20 °C) for 1 h. The system was concentrated under reduced pressure. The crude product was dried under vacuum to obtain 15-7 in the form of a white solid, which was used in the next step without further purification.

**MS (ESI)** *m*/*z* (M+H)⁺ = 465.9.

### Step 7: Preparation of compound 15-8

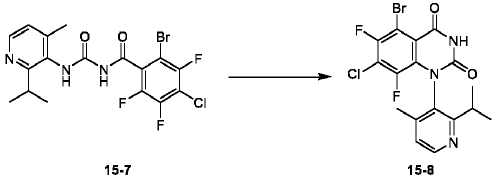

Compound 15-7 (2.2 g, 4.73 mmol) was dissolved in anhydrous tetrahydrofuran (20 mL), and sodium bis(trimethylsilyl)amide (1 M, 10.42 mL) was added to the resulting solution at 0 °C under a nitrogen atmosphere. After the completion of the addition, the system was warmed to room temperature (20 °C) and stirred for 0.5 h. The system was poured into water (50 mL), and extracted with ethyl acetate (3 × 150 mL). The organic phases were pooled, dried over anhydrous sodium sulfate, and filtered. The filtrate was concentrated to obtain a crude product. The crude product was slurried with a mixed solvent of methyl *tert*-butyl ether and petroleum ether (methyl tert-butyl ether : petroleum ether = 1:5) to obtain compound 15-8.

**MS (ESI)** *m*/*z* (M+H)⁺ = 445.9.

### Step 8: Preparation of compound 15-9

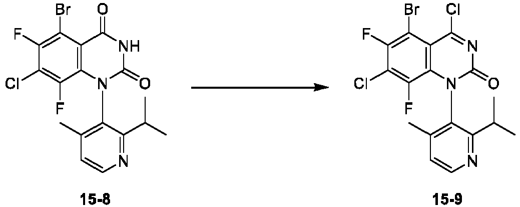

Compound 15-8 (1.4 g, 3.15 mmol) was dissolved in acetonitrile (20 mL), and *N*,*N-*diisopropylethylamine (2.03 g, 15.74 mmol, 2.74 mL) and phosphorus oxychloride (2.41 g, 15.74 mmol, 1.46 mL) were successively added to the resulting solution. After the completion of the addition, the system was warmed to 80 °C and stirred for 0.5 h. The system was quenched by pouring into ice water (50 mL). The resulting mixture was adjusted to about pH 7 with 1 M aqueous solution of sodium hydroxide and extracted with ethyl acetate (3 × 10 mL). The organic phases were pooled, dried over anhydrous sodium sulfate, and filtered. The filtrate was concentrated to obtain compound 15-9, which was used in the next step without further purification.

**(ESI)** *m*/*z* (M+H)⁺ = 459.9.

### Step 9: Preparation of compound 15-10

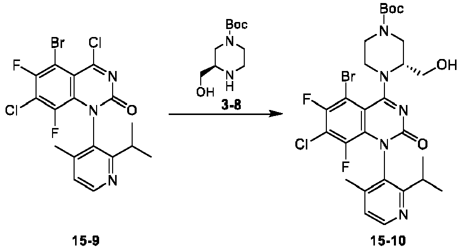

Compound 15-9 (1.45 g, 3.13 mmol) was dissolved in anhydrous acetonitrile (10 mL), and diisopropylethylamine (2.02 g, 15.66 mmol, 2.73 mL) and compound 3-8 (812.61 mg, 3.76 mmol) were successively added to the resulting solution. After the completion of the addition, the system was warmed to room temperature (20 °C) and stirred for 16 h. The reaction system was diluted with water (10 mL) and extracted with dichloromethane (3 × 50 mL). The organic phases were pooled, dried over anhydrous sodium sulfate, and filtered. The filtrate was concentrated to obtain a crude product. The crude product was purified by silica gel column chromatography (ethyl acetate/petroleum ether (v/v) = 60-80%) to obtain compound 15-10.

**(ESI)** *m*/*z* (M+H)⁺ = 644.0.

### Step 10: Preparation of compound 15-11

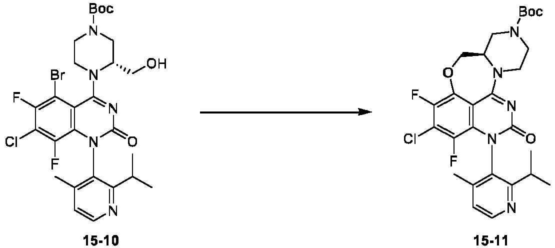

Compound 15-10 (400 mg, 622.16 µmol) was dissolved in acetonitrile (10 mL), and (2-di-*tert*-butylphosphino-3, 6-dimethoxy-2',4',6'-triisopropyl-1,1'-biphenyl) (2-amino-1,1'-biphenyl-2-yl)palladium methanesulfonate (100 mg, 117.04 µmol), 2-(di-*tert-*butylphosphino)-3,6-dimethoxy-2',4',6'-triisopropyl-1,1'-biphenyl (60 mg, 123.79 µmol) and cesium carbonate (405.42 mg, 1.24 mmol) were successively added to the resulting solution. After the completion of the addition, the system was warmed to 80 °C and stirred for 16 h under an Ar atmosphere. The system was filtered, and the filtrate was concentrated to obtain a crude product. The crude product was purified by silica gel column chromatography (ethyl acetate/dichloromethane (v/v) = 40-40%) to obtain compound 15-11.

**(ESI)** *m*/*z* (M+H)⁺ = 562.2.

### Step 11: Preparation of compound 15-12

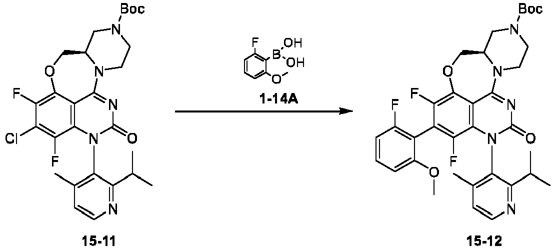

Compounds 15-11 (100 mg, 177.93 µmol) and 1-14A (90.72 mg, 533.80 µmol) were dissolved in dioxane (2.5 mL) and water (0.5 mL), and (2-dicyclohexylphosphino-2',4',6'-tri-isopropyl-1,1'-biphenyl)(2'-amino-1,1'-biphenyl-2-yl)palladium methanesulfonate (19.43 mg, 22.95 µmol), potassium carbonate (72.55 mg, 524.90 µmol) and 2-dicyclohexylphosphorus-2,4,6-triisopropylbiphenyl (19.34 mg, 40.57 µmol) were successively added to the resulting solution. After the completion of the addition, the system was warmed to 100 °C and stirred for 12 h under an Ar atmosphere. The system was concentrated to obtain a crude product. The crude product was purified by silica gel column chromatography (methanol/dichloromethane (v/v) = 0-5%) to obtain compound 15-12.

**(ESI)** *m*/*z* (M+H)⁺ = 652.3.

### Step 12: Preparation of compound 15-13

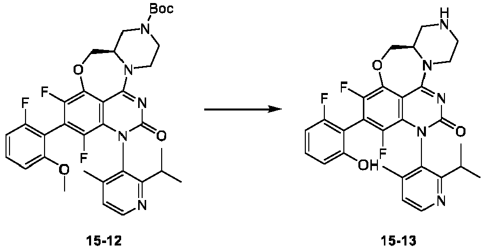

Compound 15-12 (40 mg, 61.38 µmol) was dissolved in anhydrous dichloromethane (0.5 mL), and boron tribromide (76.89 mg, 306.90 µmol, 29.57 µL) was added to the resulting solution at 0 °C. After the completion of the addition, the system was warmed to room temperature (20 °C) and stirred for 1 h. Methanol (1 mL) was added to the system, which was then stirred for 10 min. The system was concentrated and lyophilized to obtain compound 15-13, which was used in the next step without further purification.

**(ESI)** *m*/*z* (M+H)⁺ = 538.1.

### Step 13: Preparation of compounds 15A, 15B, 15C and 15D

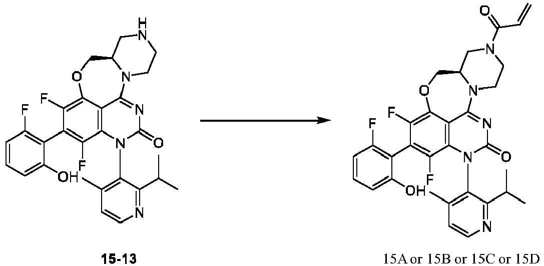

Compound 15-13 (37 mg, 59.83 µmol, hydrobromide salt) was dissolved in tetrahydrofuran (1 mL) and a saturated aqueous solution of sodium bicarbonate (6.15 g, 73.18 mmol, 2.85 mL), and acrylic anhydride (9.05 mg, 71.79 µmol) was added to the resulting solution at room temperature (20 °C). After the completion of the addition, the system was stirred at room temperature (20 °C) for 10 min. Methanol (1 mL) and lithium hydroxide (7 mg, 476.60 µmol) were added to the system, which was then stirred at room temperature (20 °C) for another 2 h. The system was adjusted to about pH 7 with 1 *N* hydrochloric acid and extracted with ethyl acetate (10 mL × 2). The organic phases were pooled, dried over anhydrous sodium sulfate, and filtered. The filtrate was concentrated to obtain a crude product. The crude product was purified by preparative high performance liquid chromatography (separation conditions: chromatographic column: Phenomenex Gemini-NX 80 × 30 mM × 3 µm; mobile phase: [water (10 mM ammonium bicarbonate solution)-acetonitrile]; acetonitrile%: 24%-54%) followed by two SFC purifications (separation conditions: chromatographic column: DAICEL CHIRALPAK AD (250 mM × 30 mM × 10 µm); mobile phase: [CO₂-isopropanol (0.1% aqueous ammonia)]; isopropanol%: 35%-35%) (separation conditions: chromatographic column: DAICEL CHIRALCEL OJ (250 mM × 30 mM × 10 µm); mobile phase: [CO₂-ethanol (0.1% aqueous ammonia)]; ethanol%: 25%-25%) to obtain compounds 15A, 15B, 15C and 15D.

### Compound 15A:

**¹H NMR** (400 MHz, Methanol-*d*₄) δ 8.39 (br d, *J* = 4.9 Hz, 1H), 7.34-7.13 (m, 2H), 6.84 (br dd, *J* = 10.9, 16.3 Hz, 1H), 6.67 (td, *J* = 8.3, 16.9 Hz, 2H), 6.31 (br d, *J* = 16.1 Hz, 1H), 5.84 (br d, *J* = 10.0 Hz, 1H), 4.79-4.54 (m, 3H), 4.46 (br d, *J* = 11.0 Hz, 1H), 4.22 (br s, 2H), 3.81 -3.67(m, 1H), 3.66 -3.43 (m, 2H), 3.01-2.88 (m, 1H), 2.14 (s, 3H), 1.46-0.97 (m, 6H).

**MS (ESI)** *m*/*z* (M+H)⁺ = 592.3.

**SFC** retention time: 4.229 min

Separation conditions: chromatographic column: Chiralcel OD-3 100 × 4.6 mm I.D., 3 µm; column temperature: 35 °C; mobile phase: CO₂-ethanol (0.05% DEA), ethanol%: 5%-40% 4 min, 40% 2.5 min, 5% 1.5 min; flow rate: 2.8 mL/min.

### Compound 15B:

**¹H NMR** (400 MHz, METHANOL-*d*₄) δ 8.37 (br d, *J* = 4.9 Hz, 1H), 7.33 -7.14 (m, 2H), 6.83 (br dd, *J* = 11.1, 16.0 Hz, 1H), 6.72-6.54 (m, 2H), 6.30 (br d, *J* = 17.4 Hz, 1H), 5.83 (br d, *J* = 9.3 Hz, 1H), 4.71-4.54 (m, 4H), 4.31-4.15 (m, 2H), 3.67(br d, *J* = 12.7 Hz, 1H), 3.48 (br d, *J* = 1.7 Hz, 2H), 2.89 (br d, *J* =7.1 Hz, 1H), 2.14 (s, 3H), 1.37-1.04 (m, 6H).

**MS (ESI)** *m*/*z* (M+H)⁺ = 592.3.

**SFC** retention time: 4.375 min

Separation conditions: chromatographic column: Chiralcel OD-3 100 × 4.6 mm I.D., 3 µm; column temperature: 35 °C; mobile phase: CO₂-ethanol (0.05% DEA), ethanol%: 5%-40% 4 min, 40% 2.5 min, 5% 1.5 min; flow rate: 2.8 mL/min.

### Compound 15C:

**¹H NMR** (400 MHz, METHANOL-*d*₄) δ 8.40 (d, *J* = 4.9 Hz, 1H), 7.29-7.19 (m, 2H), 6.85 (br dd, *J* = 10.8, 16.4 Hz, 1H), 6.73-6.61 (m, 2H), 6.32 (br d, *J* = 16.9 Hz, 1H), 5.85 (br d, *J* = 10.8 Hz, 1H), 4.62 (br s, 3H), 4.45 (br d, *J* = 13.0 Hz, 1H), 4.22 (br s, 2H), 3.82-3.68 (m, 1H), 3.65-3.49 (m, 2H), 3.04-2.92 (m, 1H), 2.15 (s, 3H), 1.23-1.04 (m, 6H).

**MS (ESI)** *m*/*z* (M+H)⁺ = 592.3.

**SFC** retention time: 4.688 min

Separation conditions: chromatographic column: Chiralcel OD-3 100 × 4.6 mm I.D., 3 µm; column temperature: 35 °C; mobile phase: CO₂-ethanol (0.05% DEA), ethanol%: 5%-40% 4 min, 40% 2.5 min, 5% 1.5 min; flow rate: 2.8 mL/min.

### Compound 15D:

**¹H NMR** (400 MHz, METHANOL-*d*₄) δ 8.39 (d, *J* = 4.8 Hz, 1H), 7.30-7.20 (m, 2H), 6.85 (br dd, *J* = 10.3, 16.6 Hz, 1H), 6.71-6.61 (m, 2H), 6.31 (br d, *J* = 16.8 Hz, 1H), 5.84 (br d, *J* = 10.5 Hz, 1H), 4.71-4.59 (m, 3H), 4.58-4.43 (m, 1H), 4.23 (br s, 2H), 3.75-3.64 (m, 1H), 3.64-3.37 (m, 2H), 2.96-2.86 (m, 1H), 2.15 (s, 3H), 1.21 (d, *J* = 6.8 Hz, 3H), 1.14(d, *J* = 6.8 Hz, 3H).

**MS (ESI)** *m*/*z* (M+H)⁺ = 592.3.

**SFC** retention time: 4.891 min

Separation conditions: chromatographic column: Chiralcel OD-3 100 × 4.6 mm I.D., 3 µm; column temperature: 35 °C; mobile phase: CO₂-ethanol (0.05% DEA), ethanol%: 5%-40% 4 min, 40% 2.5 min, 5% 1.5 min; flow rate: 2.8 mL/min.

### Experimental Example 1: Inhibition of RAS-Mediated Signal Transduction

The ability of the compounds of the present disclosure to inhibit RAS-mediated signaling was assessed and demonstrated as follows. NCI-H358 cells (ATCC accession No. CRL-5807) expressing mutant RAS (G12C) were cultured in an RPMI medium containing 10% fetal bovine serum, penicillin/streptomycin double antibody. The cells were plated on to a 96-well plate (Coming Cat. No. 3699) at 40,000 cells per well and left standing overnight to adhere to the bottom of the plate. The cells were treated with or without the compounds of the present disclosure (dimethylsulfoxide, DMSO), with a final DMSO concentration of 0.5% ensured. After 2 hours of treatment, the medium was removed, and 4% paraformaldehyde (Beyotime Cat. No. E672002-0100) was added. The resulting mixture was left standing for 20 min. After being immobilized, the cells were washed with PBS and incubated with precooled methanol for 10 min to permeabilize the cell membrane. Non-specific antibody binding was blocked by incubation for 1 h with 1× blocking buffer (Thermo Cat. No. 37520).

The ERK phosphorylation level was detected by enzyme-linked immunosorbent assay (ELISA). A phospho-ERK antibody (Cell Signal Technology Cat. No. 4370) was 1:400 diluted with 1× blocking solution containing 0.05% tween 20 and added to the 96-well plate for overnight incubation at 4 °C. The plate was washed 5 times with PBS containing 0.05% tween 20. An HRP-conjugated secondary antibody (Thermo Cat. No. 31460) was 1:10,000 diluted with 1× blocking buffer containing 0.05% tween 20 and added to the 96-well plate for incubation for 2 h at room temperature. The plate was washed 5 times with PBS containing 0.05% Tween and incubated with TMB (Thermo Cat. No. 4816) at room temperature for 15 min. The reaction was terminated with 1 mol/L H₂SO₄, and the OD values were read at a wavelength of 450 nm using an EnVision (PerkinElmer).

The total number of cells per well was measured using the Janus green staining method. The 96-well plate after the detection of the ERK phosphorylation level was washed to colorlessness with PBS and incubated with 0.1% Janus green (Abcam Cat. No. ab111622) for 10 min. After washing with double distilled water, the plate was incubated with 0.1 mol/L HCl with shaking for 10 min. The OD values were read at a wavelength of 595 nm using an EnVision (PerkinElmer).

The signal values of pERK (Thr202/Tyr204) were normalized using the signal values of Janus green, and the inhibition percentages relative to the DMSO reference were calculated after treatment with the drugs. The percentage values were subjected to four-parameter dose response curve fitting to generate IC50 values. The results are shown in Table 1.

**Table 1**

| Compound No. | p-ERK IC50 (NCI H358, µM) |
|---|---|
| ARS-1620 | 0.325 |
| Compound 1 | 0.288 |
| Compound 1A | 0.610 |
| Compound 1B | 0.044 |
| Compound 2 | 0.100 |
| Compound 2A | 0.053 |
| Compound 2B | 0.618 |
| Compound 3 | 0.647 |
| Compound 4A | 0.038 |
| Compound 4B | 0.564 |
| Compound 5A | 0.637 |
| Compound 5B | 3.81 |
| Compound 6A | 1.774 |
| Compound 6B | 0.088 |
| Compound 7A | 0.196 |
| Compound 7B | 0.073 |
| Compound 8A | 0.260 |
| Compound 8B | 0.919 |
| Compound 10A | 0.065 |
| Compound 10B | 0.105 |
| Compound 11A | 0.052 |
| Compound 11D | 0.772 |
| Compound 12A | 0.032 |
| Compound 12B | 0.327 |
| Compound 13 | 0.159 |
| Compound 14A | > 10 |
| Compound 14B | > 10 |
| Compound 15A | 6.362 |
| Compound 15B | > 10 |
| Compound 15C | 0.063 |
| Compound 15D | 0.114 |

The compounds of the present disclosure exhibited excellent ability to inhibit RAS-mediated signaling.

### Experimental Example 2: Cell Proliferation Assay

The ability of the compounds of the present disclosure to inhibit the growth of KRAS-G12C-expressing tumor cell lines was assessed and demonstrated as follows.

The ability of the compounds of the present disclosure to inhibit the growth of KRAS-G12C-expressing cells was assessed by measuring cell viability and calculating GI50 values.

Tumor cell line NCI-H358 (ATCC accession No. CRL-5807) expressing KRAS-G12C was cultured in an RPMI medium supplemented with 10% fetal bovine serum and penicillin/streptomycin double antibody; tumor cell line MIA PaCa2 (ATCC CRL-1420) expressing KRAS-G12C was cultured in a DMEM medium supplemented with 10% fetal bovine serum, 2.5% horse serum and penicillin/streptomycin double antibody.

NCI-H358 and MIA-Paca2 cells were seeded on a black clear-bottom 384-well plate (PerkinElmer Cat. No. 6007460) at densities of 1000 and 800 cells, respectively, and allowed to adhere to the walls overnight (8-12 h). After the cells adhered to the walls, the diluted compounds of the present disclosure with a concentration that was 5 times that of the working solution (containing 0.1% dimethyl sulfoxide, namely DMSO, at the final concentration) was added to the experimental groups; the same dilution (containing 0.1% DMSO at the final concentration) as in the experimental groups was added to the control group. After 72 h, the amount of cell proliferation was measured by measuring ATP content using a Cell Titer Glo reagent (Promega Cat. No. G7572) according to the method described in the manuals. Steps are briefly as follows: the cell plate was taken out and equilibrated at normal temperature for 30 min; the Cell Titer Glo reagent with an equal volume to that of the culture was added; the culture plate was placed on a shaker for lysis for 2 min; the culture plate was left standing at normal temperature for 10 min; the optical signal values were read using a microplate reader EnVision (PerkinElmer).

The inhibition percentages were calculated from the data of the experimental groups relative to the DMSO group, and GI50 was calculated using the data processing software GraphPad by analyzing the inhibitory rates yielded by 9 dosing concentrations produced by 1:3 dilution of the compounds, with the results shown in Table 2.

**Table 2**

| Compound No. | GI₅₀(NCI-H358, µM) | GI₅₀(MIA-Paca2, µM) |
|---|---|---|
| ARS-1620 | 0.510 | 1.209 |
| Compound 1B | 0.084 | 0.296 |
| Compound 2A | 0.029 | 0.048 |
| Compound 2B | 1.116 | 15.993 |
| Compound 4A | 0.034 | 0.324 |
| Compound 6B | 0.066 | 0.297 |
| Compound 7A | 0.133 | 0.629 |
| Compound 7B | 0.080 | 0.200 |
| Compound 8A | 0.350 | 0.304 |
| Compound 10A | 0.111 | 0.333 |
| Compound 10B | 0.155 | 0.403 |
| Compound 11A | 0.070 | 0.685 |
| Compound 12A | 0.010 | 0.054 |
| Compound 13 | 0.320 | 1.273 |
| Compound 15A | 3.428 | 4.522 |
| Compound 15C | 0.060 | 0.087 |
| Compound 15D | 0.959 | 1.099 |

The compounds of the present disclosure have excellent inhibitory activity against the proliferation of NCI-H358 and MIA-Paca2 cells.

## Claims

1. A compound of formula (I), an optical isomer thereof and a pharmaceutically acceptable salt thereof, 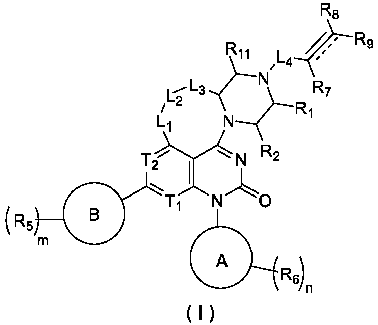 wherein,
R₁, R₂, R₁₁ are each independently selected from H, halogen, OH, NH₂, CN, C₁₋₆ alkyl, C₁₋₆ alkoxy and C₁₋₆ alkylamino, wherein the C₁₋₆ alkyl, C₁₋₆ alkoxy or C₁₋₆ alkylamino is optionally substituted with 1, 2 or 3 R;
T₁ is selected from N and C(R₃);
T₂ is selected from N and C(R₄);
R₃ and R₄ are each independently selected from H, halogen, OH, NH₂, CN, C₂₋₄ alkenyl, C₂₋₄ alkynyl, C₁₋₆ alkyl, C₁₋₆ alkoxy, C₁₋₆ alkylamino, C₃₋₆ cycloalkyl and 3-6 membered heterocycloalkyl, wherein the C₂₋₄ alkenyl, C₂₋₄ alkynyl, C₁₋₆ alkyl, C₁₋₆ alkoxy, C₁₋₆ alkylamino, C cycloalkyl or 3-6 membered heterocycloalkyl is optionally substituted with 1, 2 or 3 R;
R₅ is independently selected from H, halogen, OH, NH₂, CN, C₁₋₆ alkyl, C₁₋₆ alkoxy, C₁₋₆ alkylamino, C₁₋₆ alkyl-OC(=O)-, C₃₋₆ cycloalkyl, 3-6 membered heterocycloalkyl, C₆₋₁₀ aryl and 5-10 membered heteroaryl, wherein the C₁₋₆ alkyl, C₁₋₆ alkoxy, C₁₋₆ alkylamino, C₁₋₆ alkyl-OC(=O)-, C₃₋₆ cycloalkyl, 3-6 membered heterocycloalkyl, C₆₋₁₀ aryl or 5-10 membered heteroaryl is optionally substituted with 1, 2 or 3 R;
R₆ is independently selected from H, halogen, OH, NH₂, CN, C₁₋₆ alkyl, C₁₋₆ alkoxy, C₁₋₆ alkylamino, C₁₋₆ alkyl-OC(=O)-, C₃₋₆ cycloalkyl and 3-6 membered heterocycloalkyl, the C₁₋₆ alkyl, C₁₋₆ alkoxy, C₁₋₆ alkylamino, C₁₋₆ alkyl-OC(=O)-, C₃₋₆ cycloalkyl or 3-6 membered heterocycloalkyl is optionally substituted with 1, 2 or 3 R;
ring A is selected from C₃₋₆ cycloalkyl, 3-6 membered heterocycloalkyl, C₆₋₁₀ aryl and 5-10 membered heteroaryl;
ring B is selected from C₆₋₁₀ aryl, 5-10 membered heteroaryl, benzo 5-6 membered heterocycloalkyl and 5-6 membered heteroary-fused 5-6 membered heterocycloalkyl;
R₇ is selected from H, halogen, CN, C₁₋₆ alkyl, C₁₋₆ alkoxy and C₁₋₆ alkylamino, wherein the C₁₋₆ alkyl, C₁₋₆ alkoxy or C₁₋₆ alkylamino is optionally substituted with 1, 2 or 3 R;
R₈ and R₉ are each independently selected from H, halogen, CN, C₁₋₆ alkyl, C₃₋₆ cycloalkyl and 3-6 membered heterocycloalkyl, wherein the C₁₋₆ alkyl, C₁₋₆ heteroalkyl, C₁₋₆ alkylamino, C₃₋₆ cycloalkyl or 3-6 membered heterocycloalkyl is optionally substituted with 1, 2 or 3 R; 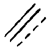 represents 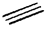 or 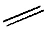 , and when 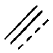 is 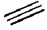 , R₇ and R₉ are absent;
L₁ is selected from a single bond, CH₂, 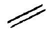 , O, S, S(=O), C(=O), S(=O)₂ and N(R₁₀);
L₂ is selected from CH₂, O, S and C(=O);
L₃ is selected from a single bond, C(R₁₂R₁₂) and C(=O);
L₄ is selected from S(=O), S(=O)₂ and C(=O);
m is selected from 1, 2, 3 and 4;
n is selected from 1, 2, 3 and 4;
R₁₀ is selected from H, C₁₋₆ alkyl, C₃₋₆ cycloalkyl and 3-6 membered heterocycloalkyl, wherein the C₁₋₆ alkyl, C₃₋₆ cycloalkyl or 3-6 membered heterocycloalkyl is optionally substituted with 1, 2 or 3 R;
R₁₂ is independently selected from H, F, Cl, Br, I, OH, NH₂, CN, Me and CF₃;
R is independently selected from H, halogen, OH, NH₂, CN, 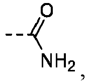 C₁₋₆ alkyl, C₁₋₆ alkyl-OC(=O)-, C₁₋₆ alkoxy, C₁₋₆ alkylthio, C₁₋₆ alkylamino and 5-6 membered heterocycloalkyl, wherein the C₁₋₆ alkyl, C₁₋₆ alkyl-OC(=O)-, C₁₋₆ alkoxy, C₁₋₆ alkylthio, C₁₋₆ alkylamino or 5-6 membered heterocycloalkyl is optionally substituted with 1, 2 or 3 R';
R' is selected from F, Cl, Br, I, OH, NH₂ and CH₃;
the 3-6 membered heterocycloalkyl, 5-6 membered heterocycloalkyl, 5-10 membered heteroaryl or C₁₋₆ heterocycloalkyl contains 1, 2 or 3 heteroatoms or heteroatom groups independently selected from O, NH, S, C(=O)O, S(=O), S(=O)₂ and N.

2. The compound, the optical isomer thereof and the pharmaceutically acceptable salt thereof according to claim 1, wherein R is independently selected from H, halogen, OH, NH₂, CN, 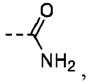 C₁₋₃ alkyl, C₁₋₃ alkoxy, C₁₋₃ alkylthio, C₁₋₃ alkylamino, and 5-6 membered heterocycloalkyl, wherein the C₁₋₃ alkyl, C₁₋₃ alkoxy, C₁₋₃ alkylthio, C₁₋₃ alkylamino, or 5-6 membered heterocycloalkyl is optionally substituted with 1, 2, or 3 R'.

3. The compound, the optical isomer thereof and the pharmaceutically acceptable salt thereof according to claim 2, wherein R is independently selected from H, F, Cl, Br, I, OH, NH2, CN, Me, 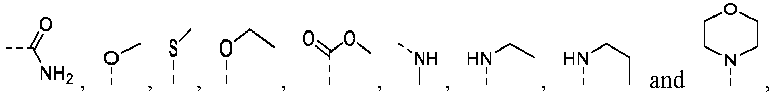 wherein Me, 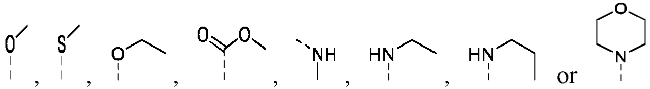 is optionally substituted by 1, 2 or 3 R'.

4. The compound, the optical isomer thereof and the pharmaceutically acceptable salt thereof according to claim 3, wherein R is independently selected from H, F, Cl, Br, I, OH, NH₂, CN, Me,

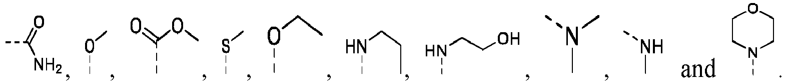

5. The compound, the optical isomer thereof and the pharmaceutically acceptable salt thereof according to any one of claims 1-4, wherein R₁, R₂, R₁₁ are each independently selected from H, halogen, OH, NH₂, CN, C₁₋₃ alkyl, C₁₋₃ alkoxy and C₁₋₃ alkylamino, wherein the C₁₋₃ alkyl, C₁₋₃ alkoxy or C₁₋₃ alkylamino is optionally substituted with 1, 2 or 3 R.

6. The compound, the optical isomer thereof and the pharmaceutically acceptable salt thereof according to claim 5, wherein R₁, R₂ and R₁₁ are each independently selected from H, F, Cl, Br, I, OH, NH₂, CN, Me, CF₃,

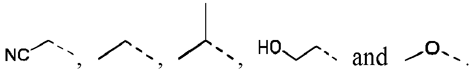

7. The compound, the optical isomer thereof and the pharmaceutically acceptable salt thereof according to claim 1, wherein R₃ and R₄ are each independently selected from H, F, Cl, Br, I, OH, NH₂, CN, Me, CF₃,

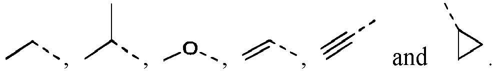

8. The compound, the optical isomer thereof and the pharmaceutically acceptable salt thereof according to claim 1, wherein R₅ is selected from H, F, Cl, Br, I, OH, NH₂, CN, Me, CF₃,

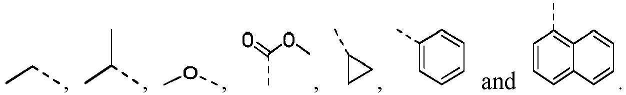

9. The compound, the optical isomer thereof and the pharmaceutically acceptable salt thereof according to claim 1, wherein R₆ is selected from H, F, Cl, Br, I, OH, NH₂, CN, Me, CF₃, N(CH₃)₂,

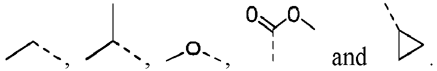

10. The compound, the optical isomer thereof and the pharmaceutically acceptable salt thereof according to claim 1, wherein ring A is selected from C₃₋₆ cycloalkyl, 3-6 membered heterocycloalkyl, phenyl, naphthyl, thienyl, pyrazolyl, thiazolyl, imidazolyl, pyridyl, pyrimidinyl, indazolyl, and indolyl.

11. The compound, the optical isomer thereof and the pharmaceutically acceptable salt thereof according to claim 9 or 10, wherein the moiety 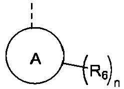 is selected from 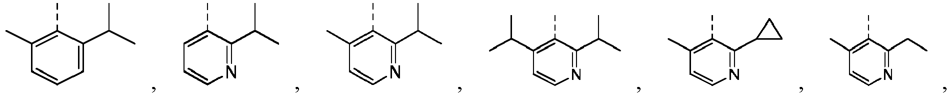 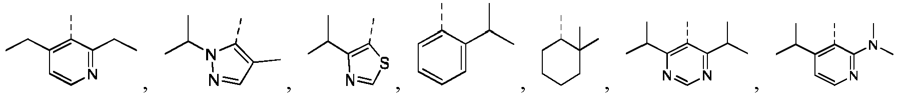 and

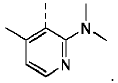

12. The compound, the optical isomer thereof and the pharmaceutically acceptable salt thereof according to claim 1, wherein ring B is selected from phenyl, naphthyl, thienyl, pyrazolyl, pyrimidinyl, indazolyl, indolyl, 1*H*-benzo[*d*][1,2,3]triazolyl, 1,3-dihydro-2*H-*benzo[d]imidazol-2-onyl, benzo[d]oxazol-2(3*H*)-onyl, 1*H*-pyrazolo[3,4-b]pyridyl, isoquinolin-1(2*H*)-onyl and 1*H*-benzo[d]imidazolyl.

13. The compound, the optical isomer thereof and the pharmaceutically acceptable salt thereof according to claim 8 or 12, wherein the moiety 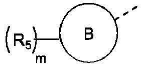 is selected from

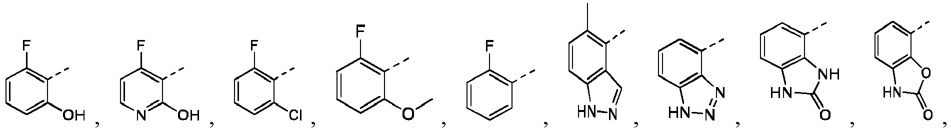

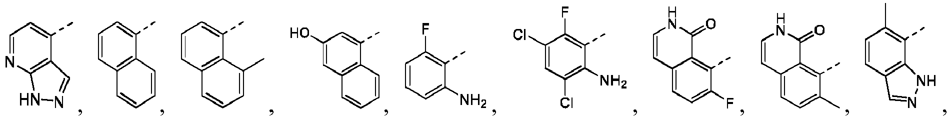

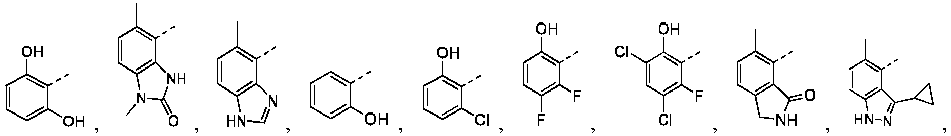

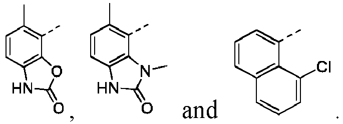

14. The compound, the optical isomer thereof and the pharmaceutically acceptable salt thereof according to any one of claim 1, 6 and 7, wherein the moiety 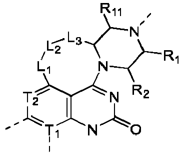 is selected from

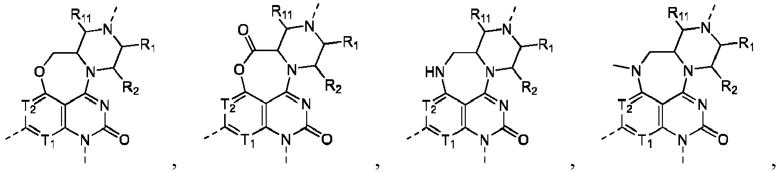

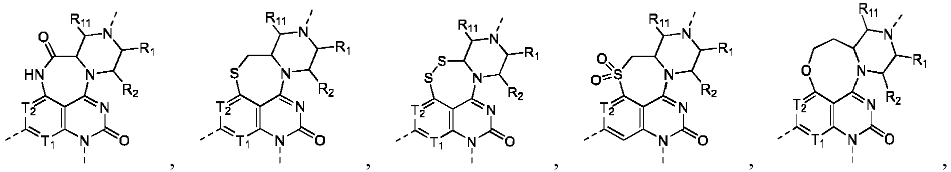

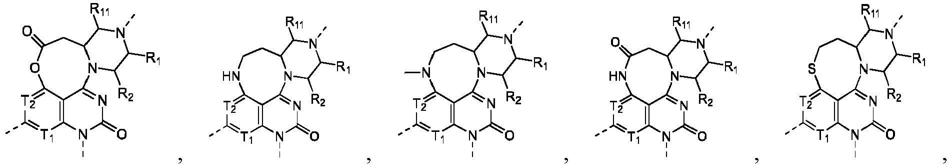

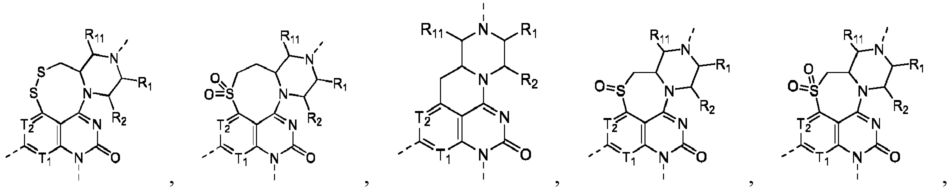

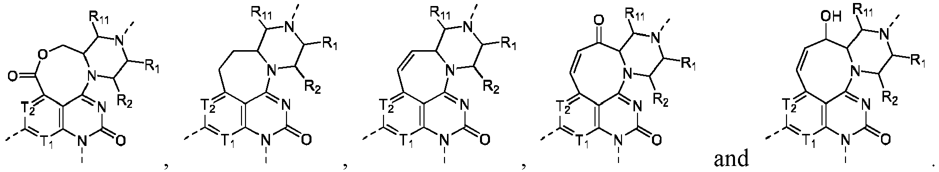

15. The compound, the optical isomer thereof and the pharmaceutically acceptable salt thereof according to claim 14, wherein the moiety 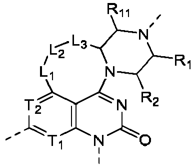 is selected from

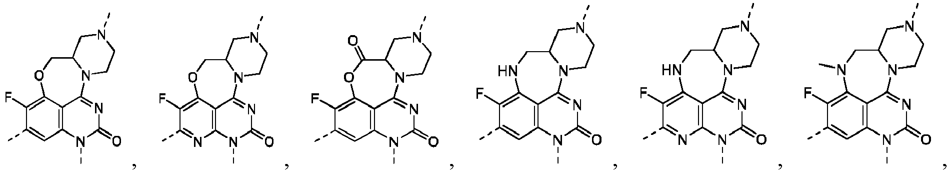

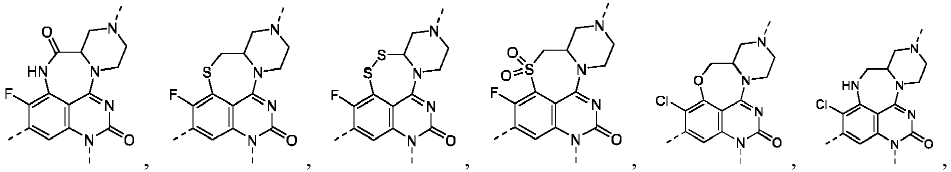

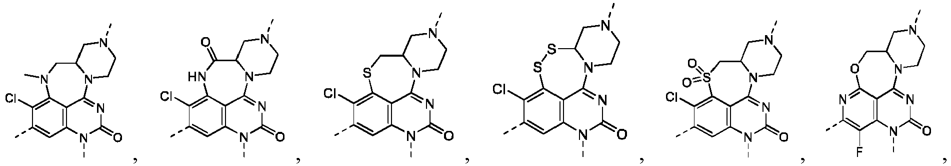

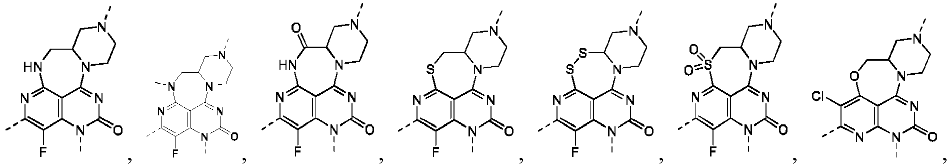

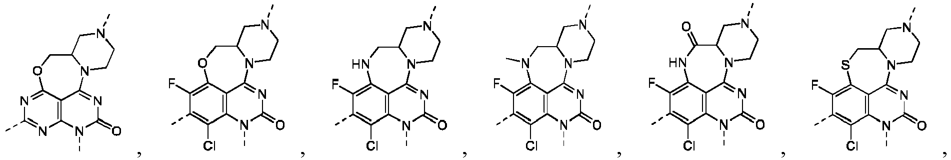

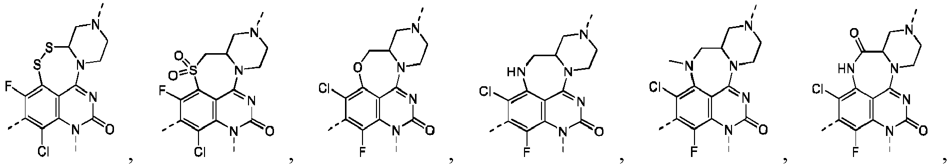

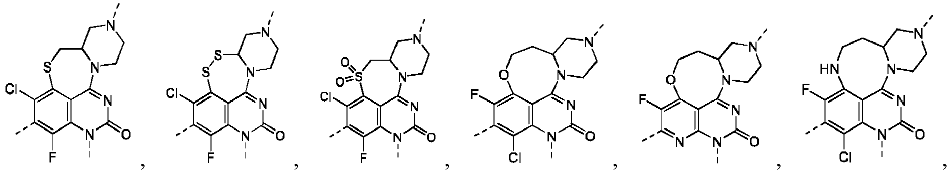

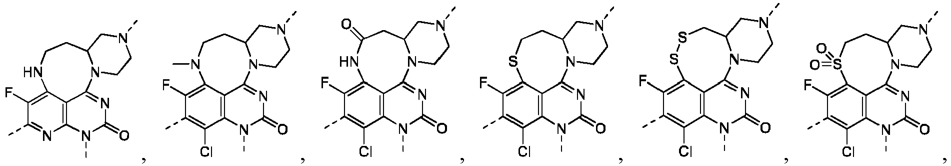

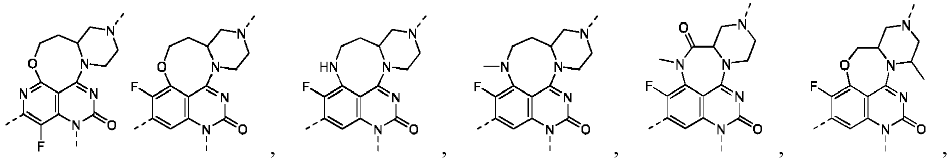

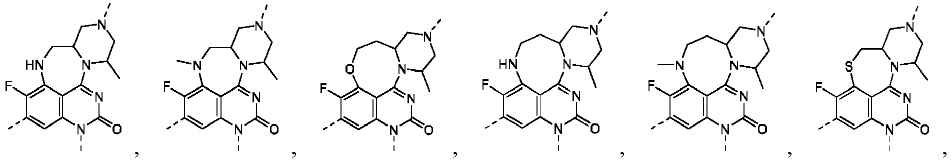

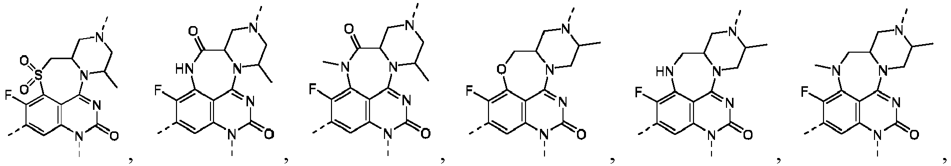

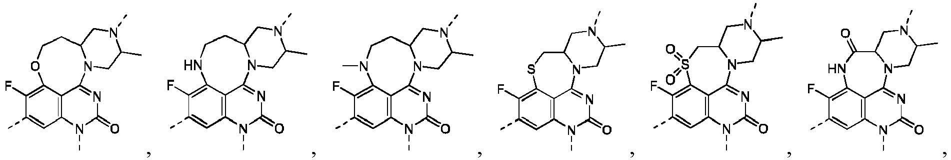

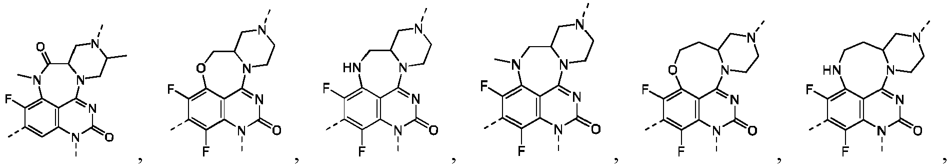

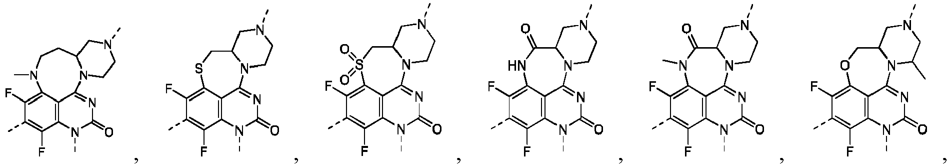

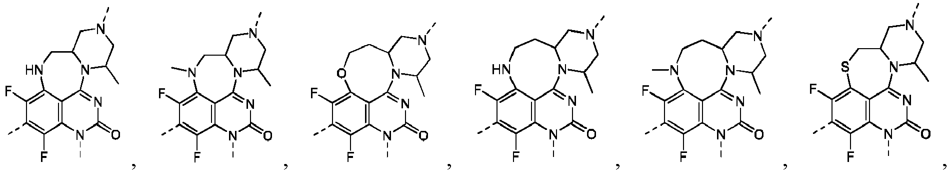

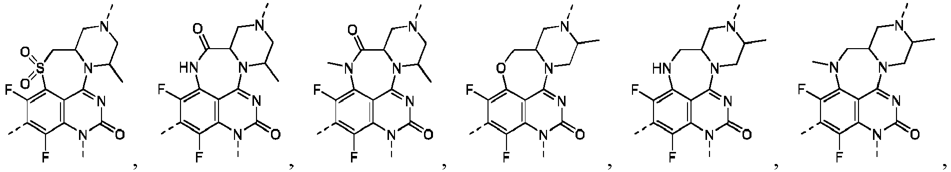

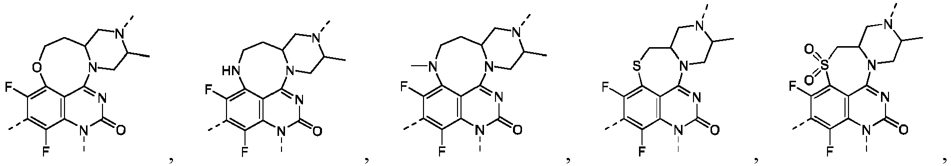

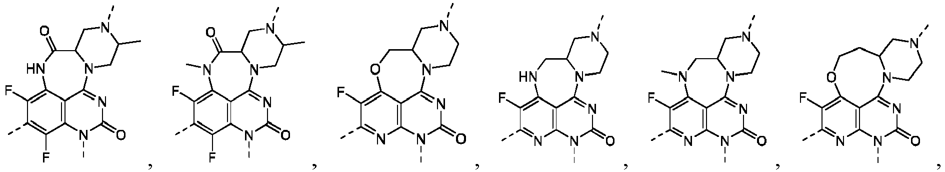

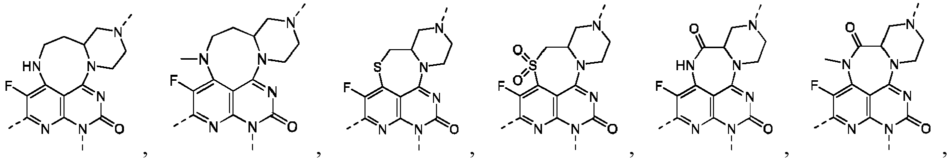

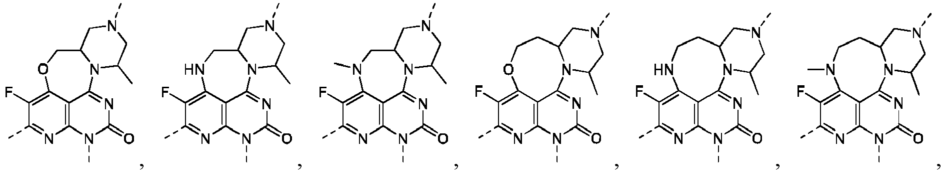

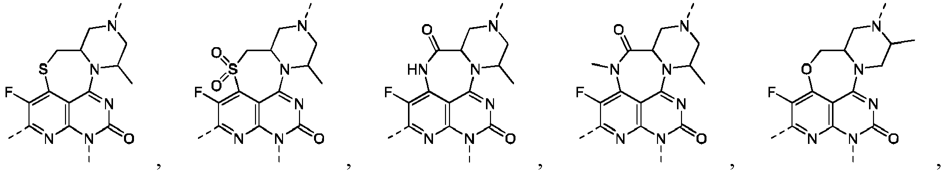

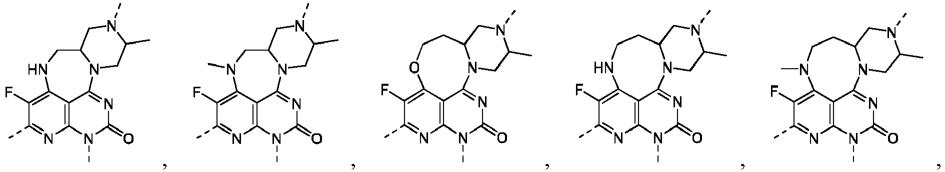

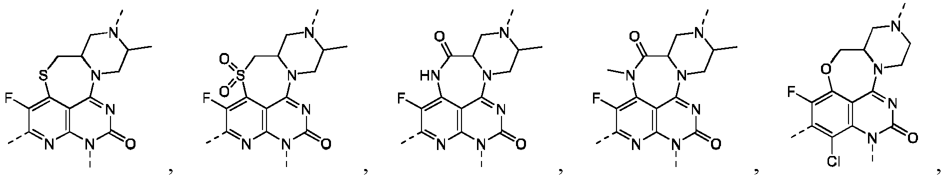

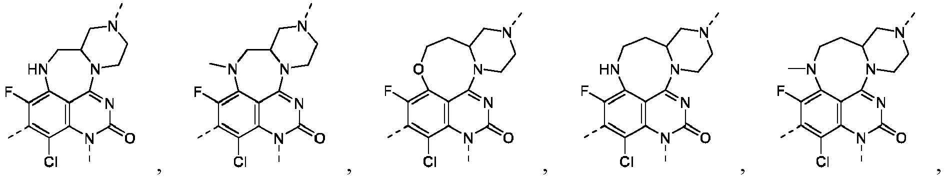

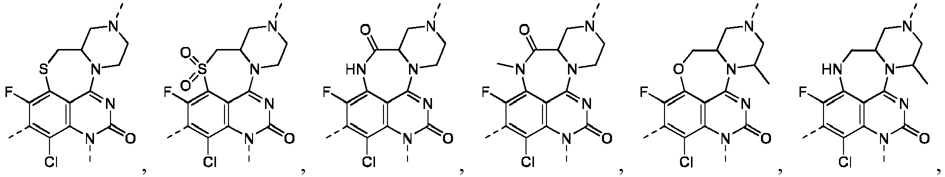

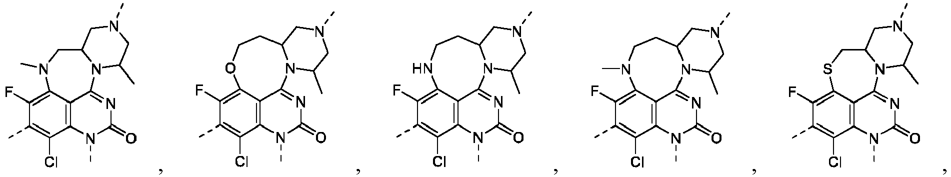

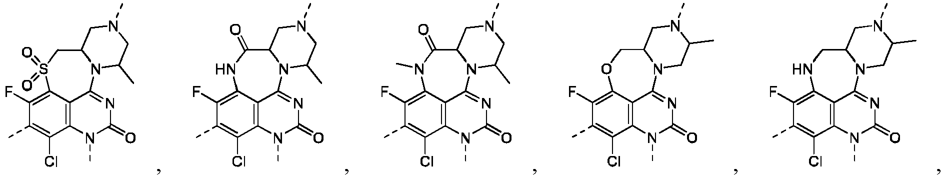

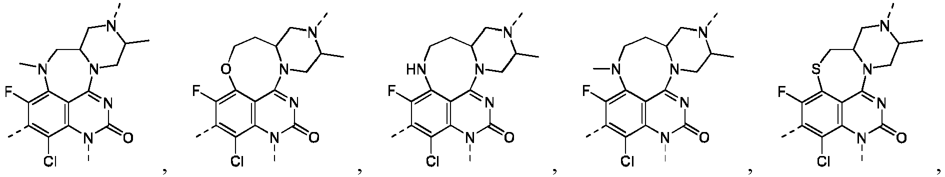

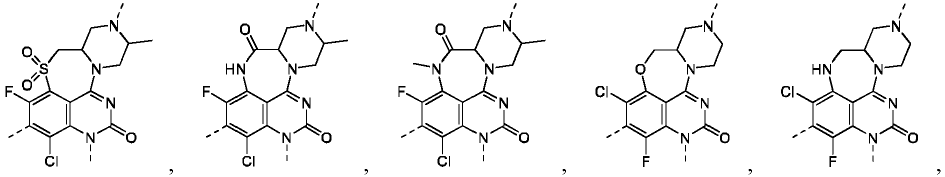

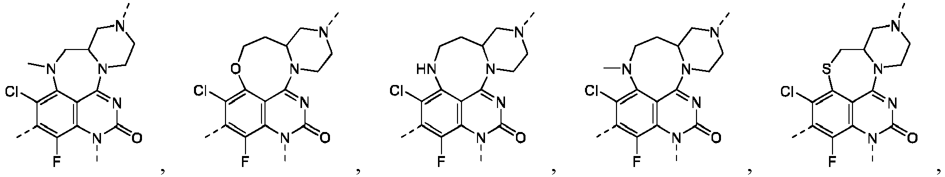

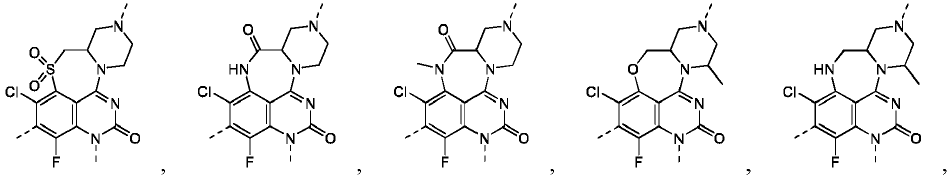

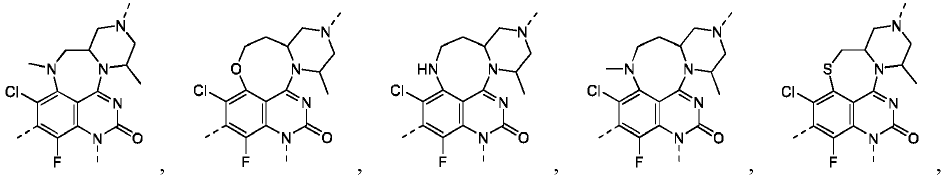

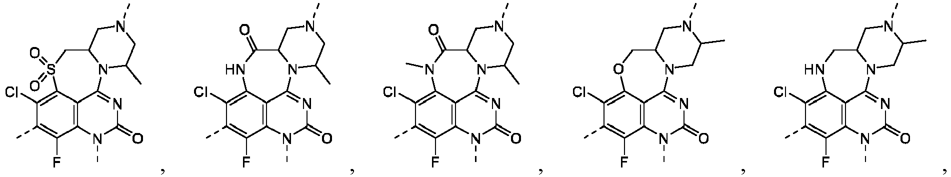

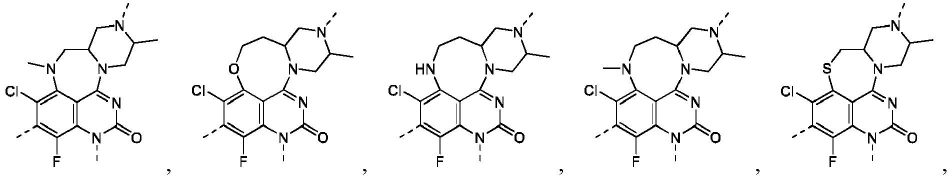

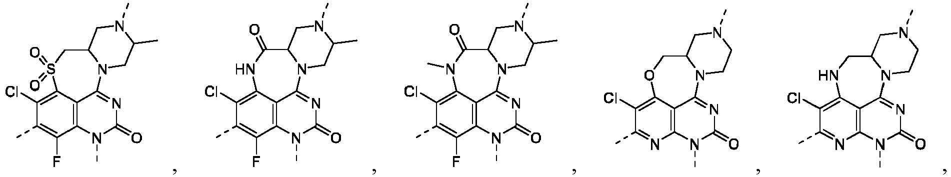

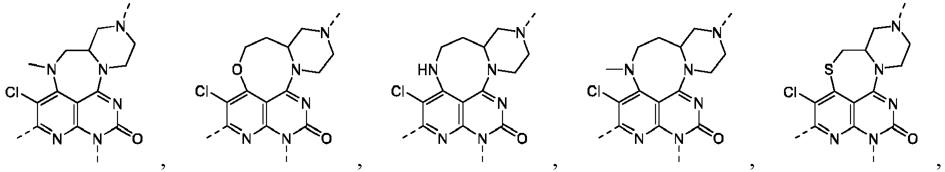

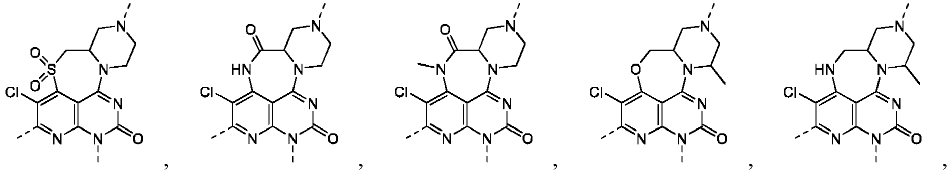

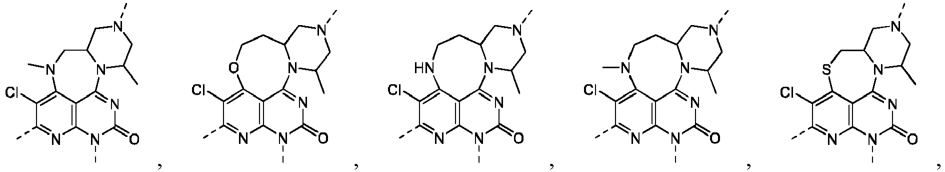

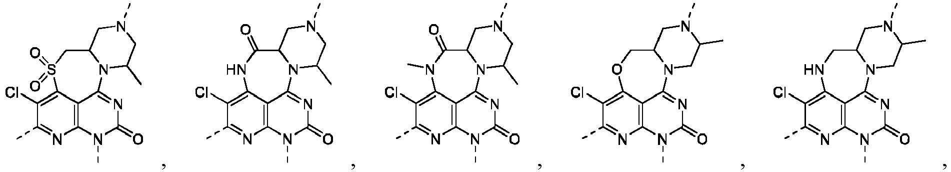

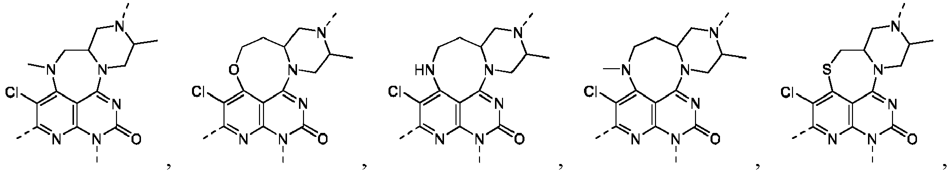

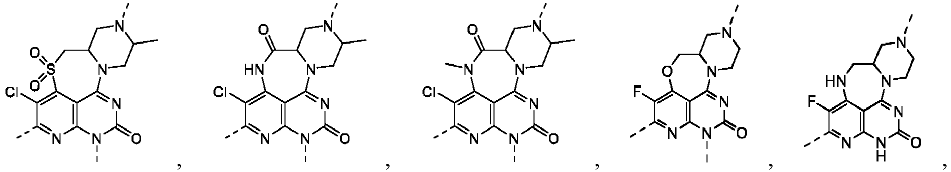

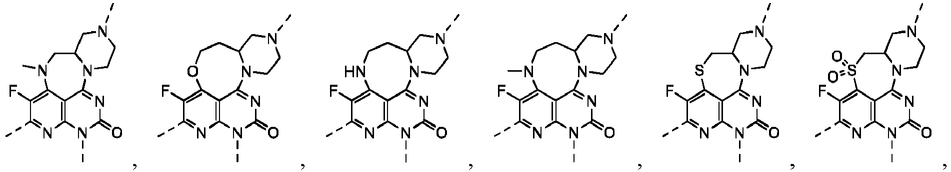

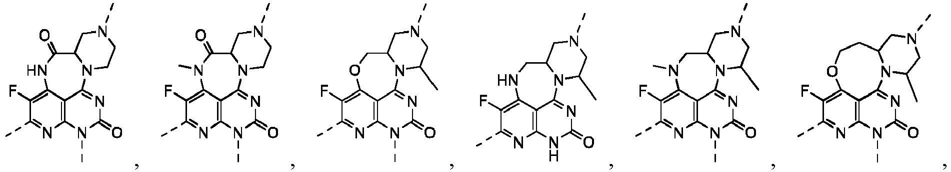

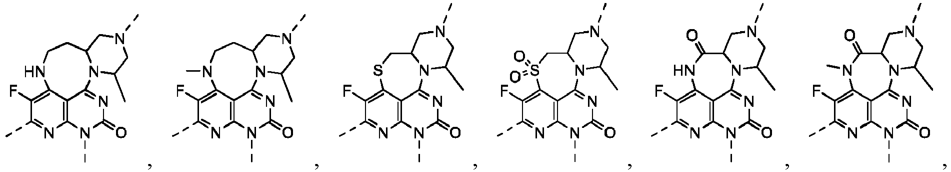

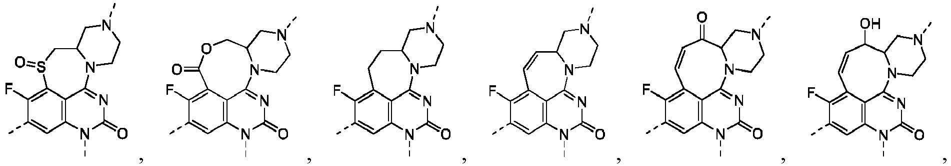

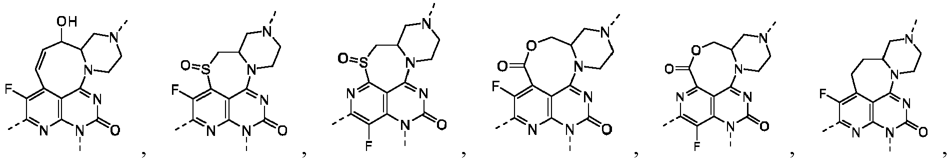

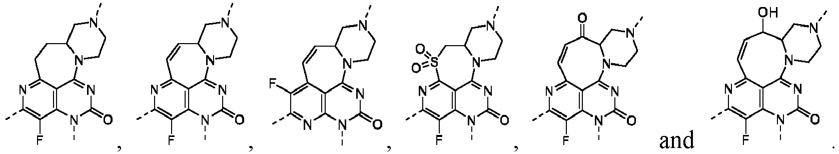

16. The compound, the optical isomer thereof and the pharmaceutically acceptable salt thereof according to claim 1, wherein R₇ is selected from H, F, Cl, Br, I, CN, Me, CF₃,

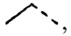

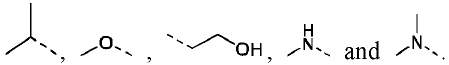

17. The compound, the optical isomer thereof and the pharmaceutically acceptable salt thereof according to claim 1, wherein R₈ and R₉ are each independently selected from H, F, Cl, Br, I, CN, Me, CF₃,

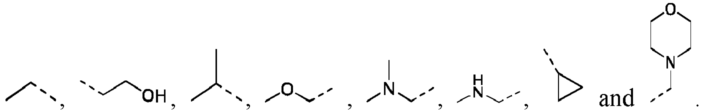

18. The compound, the optical isomer thereof and the pharmaceutically acceptable salt thereof according to any one of claims 1, 16 and 17, wherein the moiety 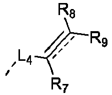 is selected from 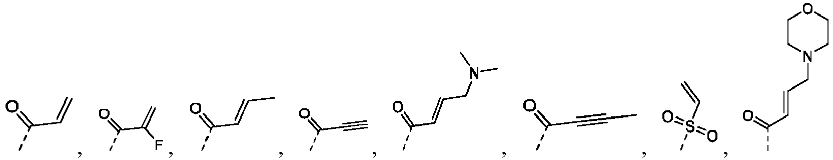 and

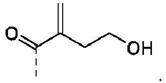

19. The compound, the optical isomer thereof and the pharmaceutically acceptable salt thereof according to any one of claims 1-18, selected from 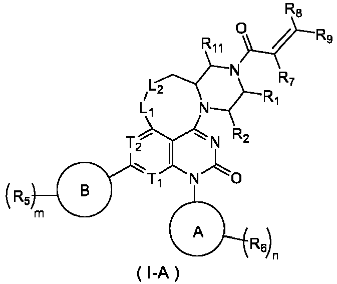 wherein,
R₁ and R₂ are as defined in claims 1, 5 and 6;
L₁ and L₂ are as defined in claim 1;
T and T₂ are as defined in claims 1 and 9;
R₅ is as defined in claims 1, 8 and 13;
R₆ is as defined in claims 1, 9 and 11;
ring A is as defined in claims 1, 10 and 11;
ring B is as defined in claims 1, 12 and 13;
R₇ is as defined in claims 1, 16 and 18;
R₈ and R₉ are as defined in claims 1, 17 and 18.

20. The compound, the optical isomer thereof and the pharmaceutically acceptable salt thereof according to any one of claims 1-18, selected from 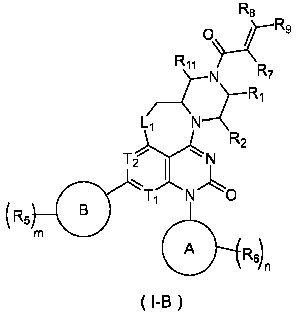 wherein,
R₁ and R₂ are as defined in claims 1, 5 and 6;
L₁ is as defined in claim 1;
T and T₂ are as defined in claims 1 and 9;
R₅ is as defined in claims 1, 8 and 13;
R₆ is as defined in claims 1, 9 and 11;
ring A is as defined in claims 1, 10 and 11;
ring B is as defined in claims 1, 12 and 13;
R₇ is as defined in claims 1, 16 and 18;
R₈ and R₉ are as defined in claims 1, 17 and 18.

21. A compound of the formula below, an optical isomer thereof and a pharmaceutically acceptable salt thereof, selected from

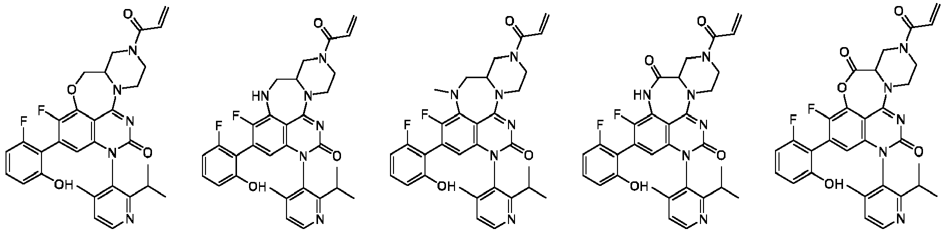

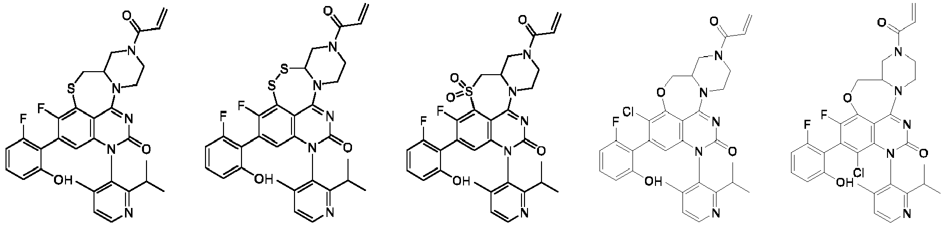

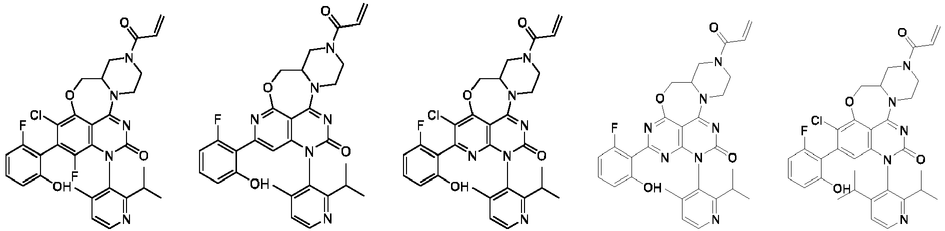

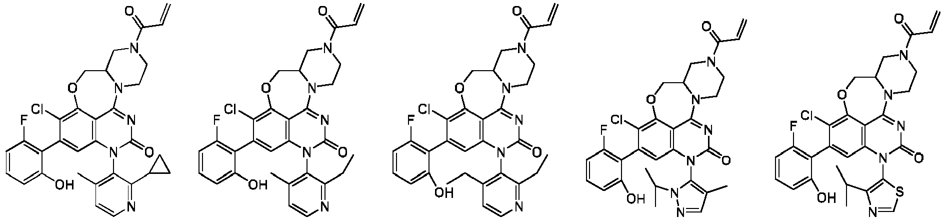

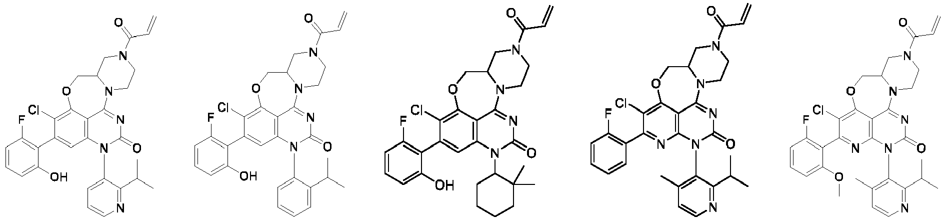

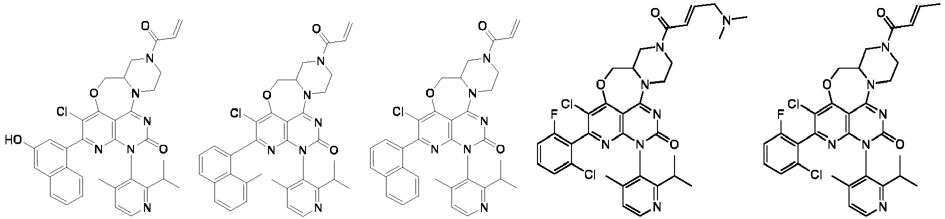

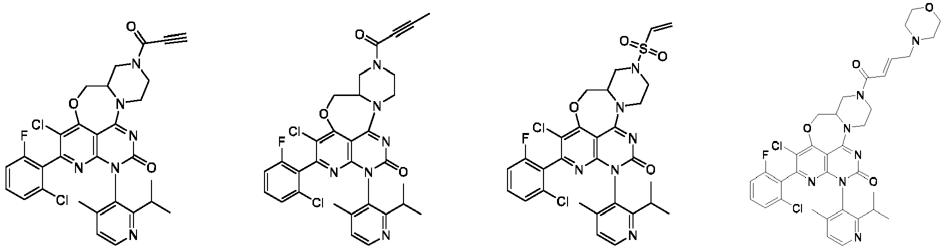

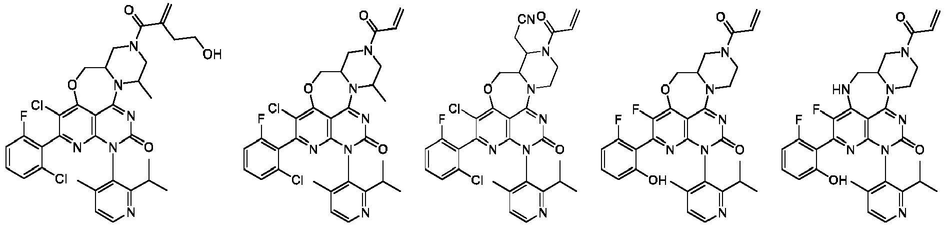

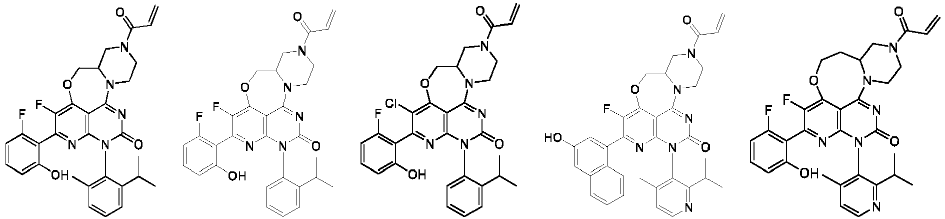

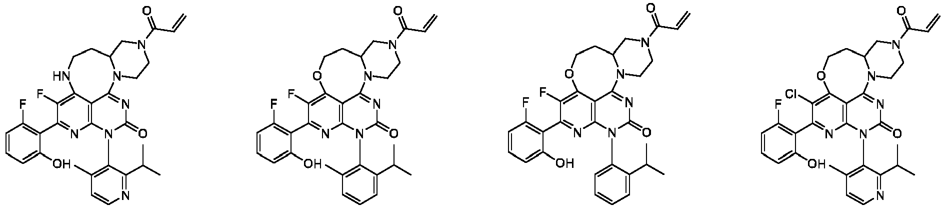

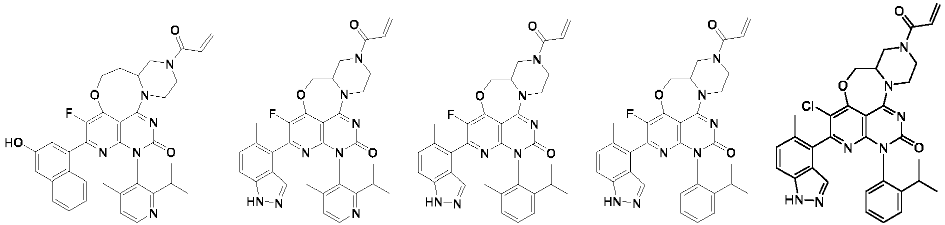

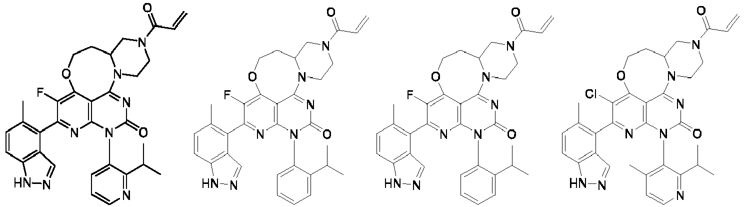

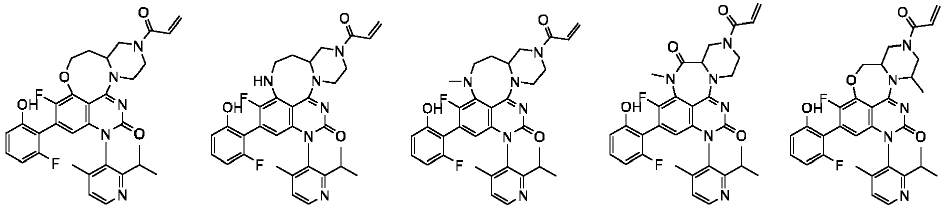

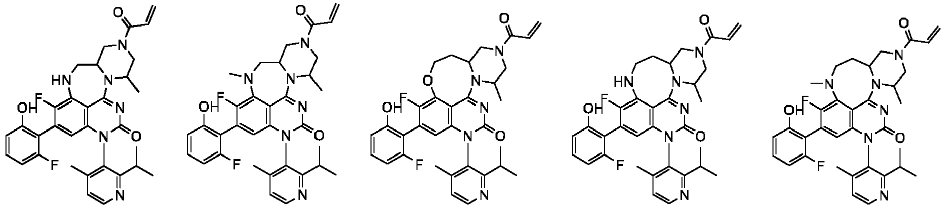

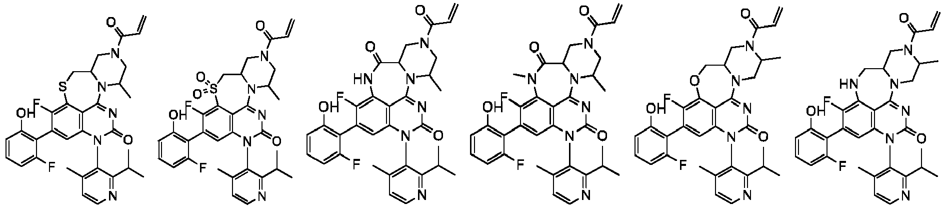

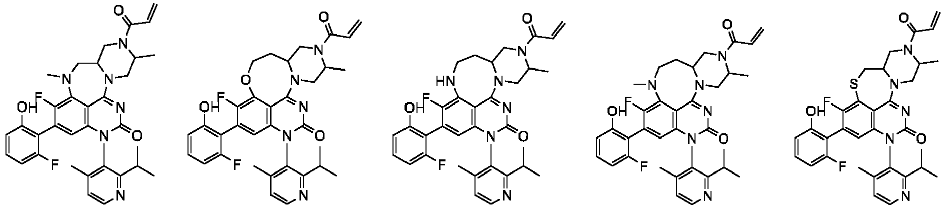

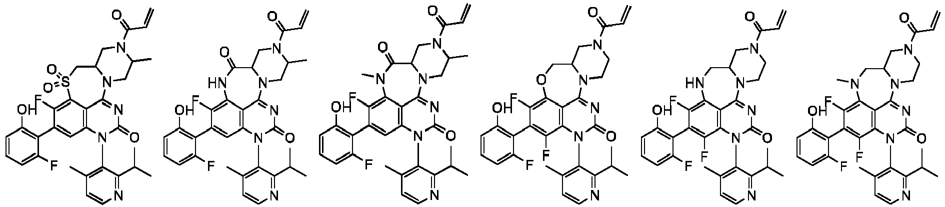

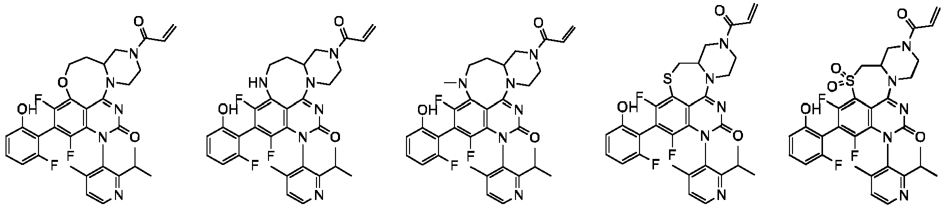

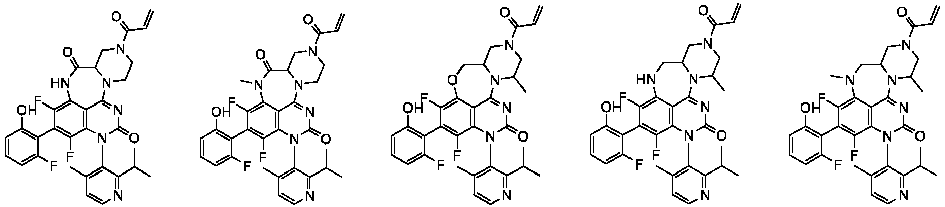

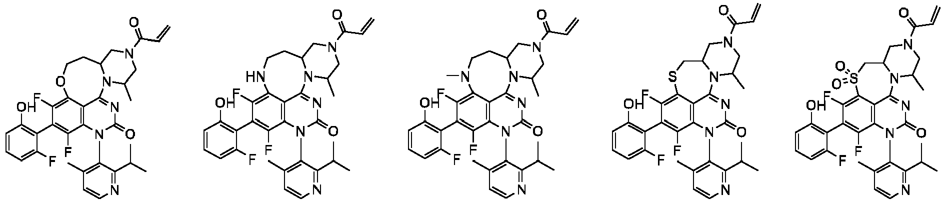

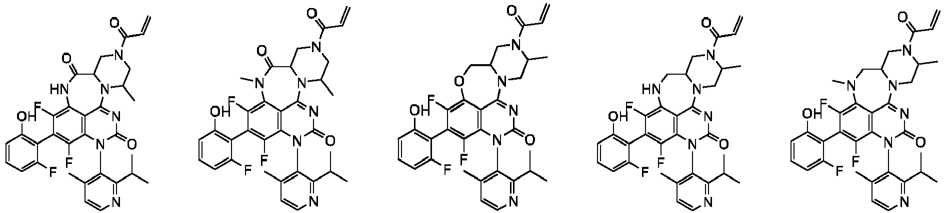

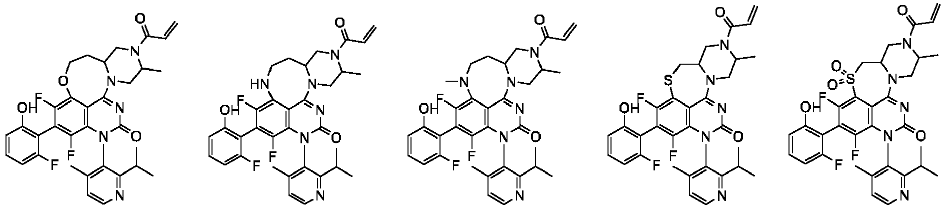

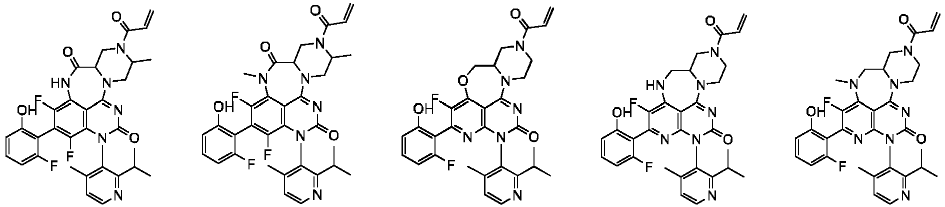

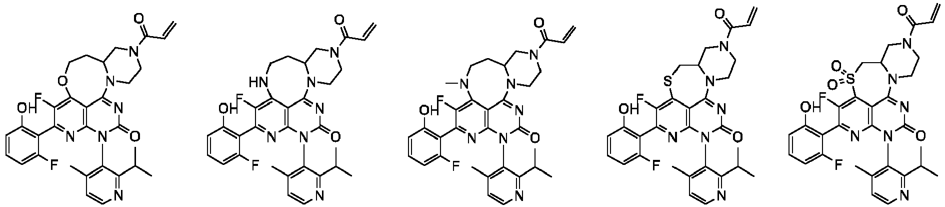

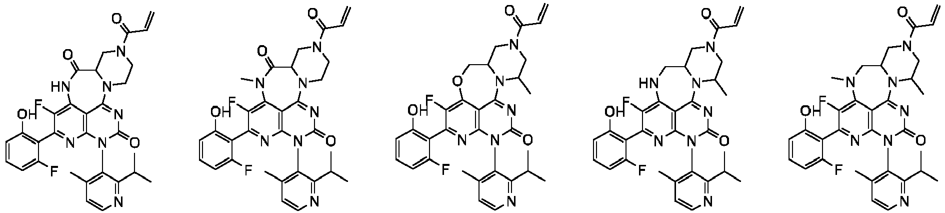

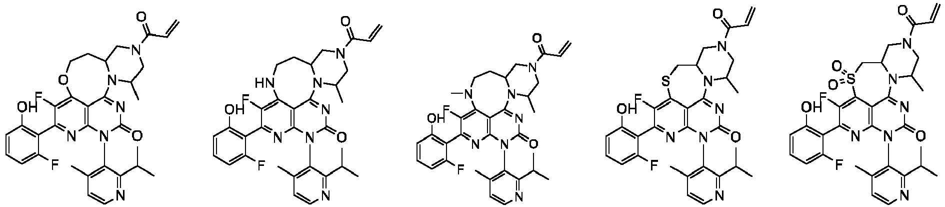

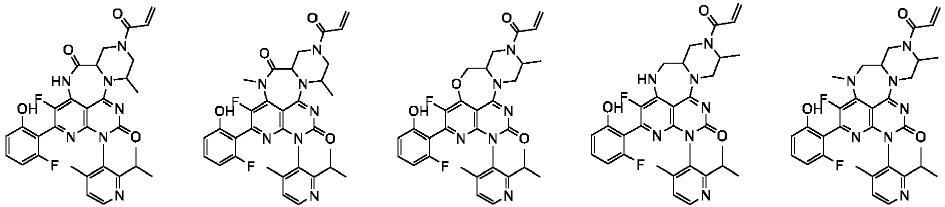

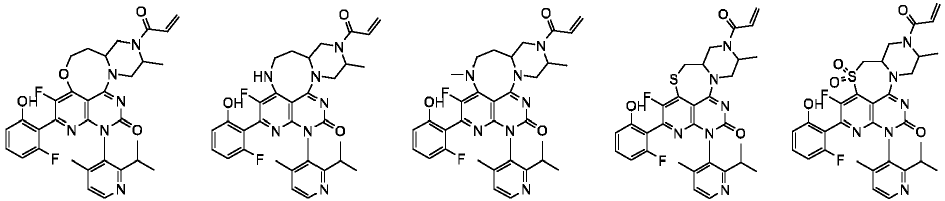

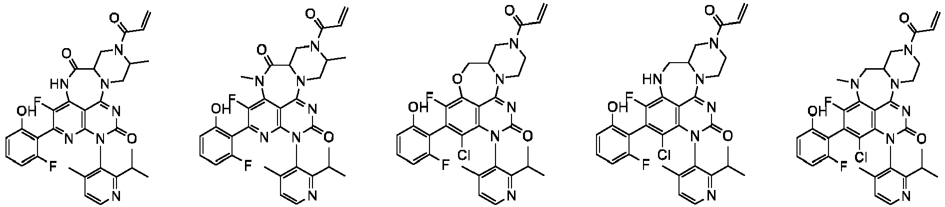

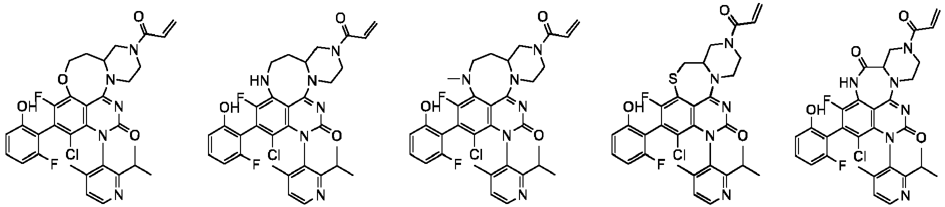

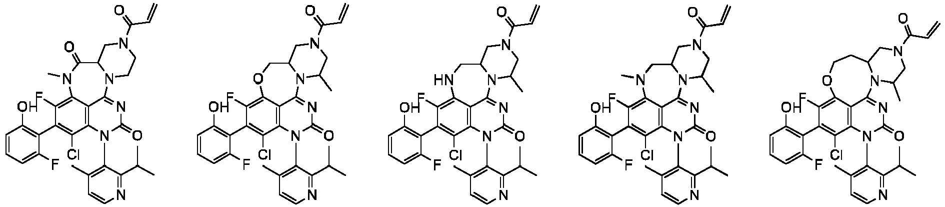

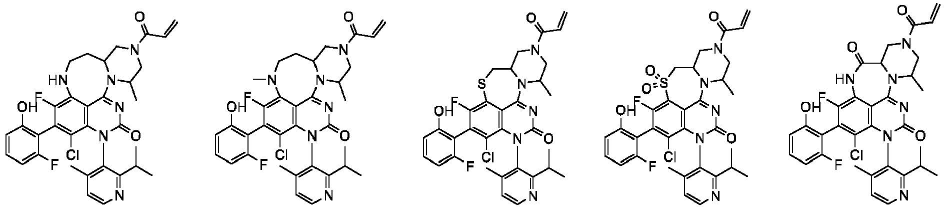

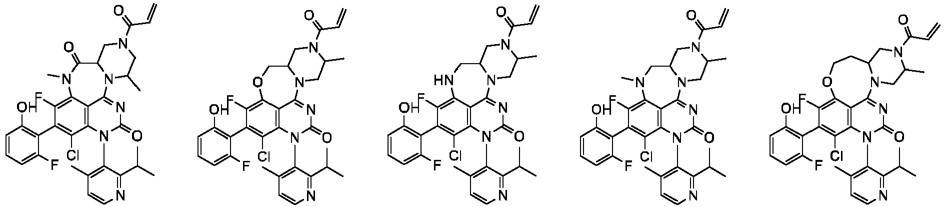

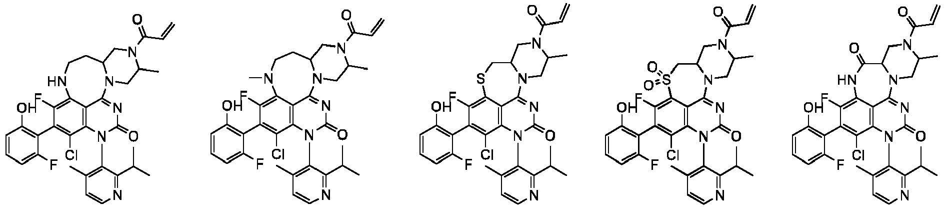

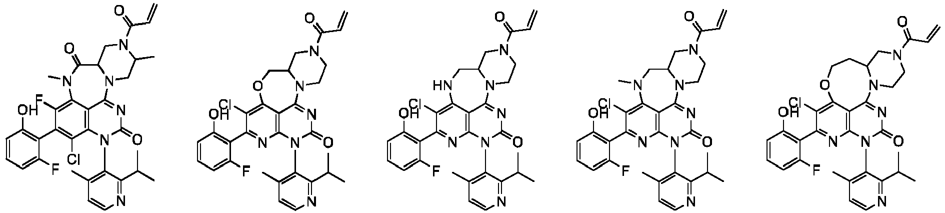

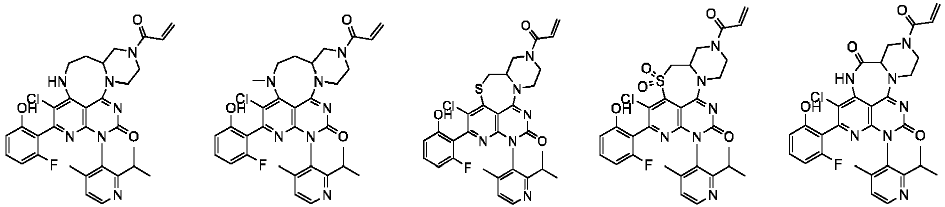

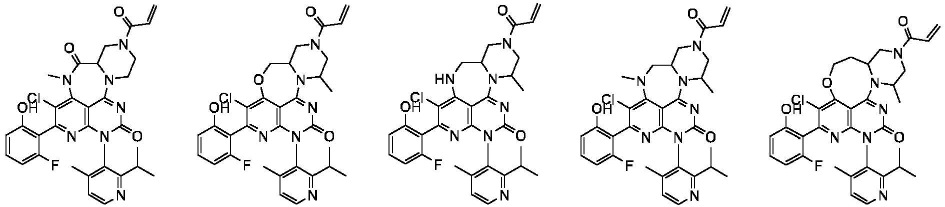

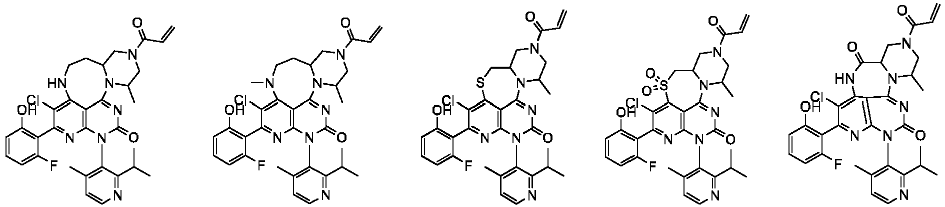

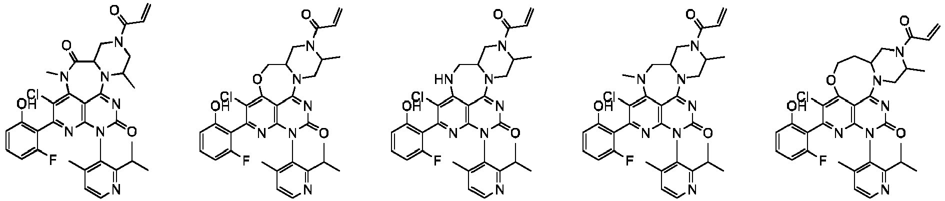

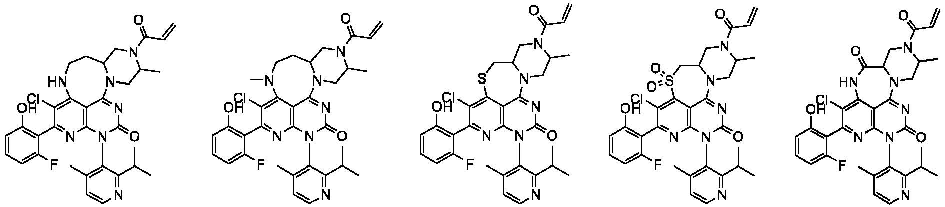

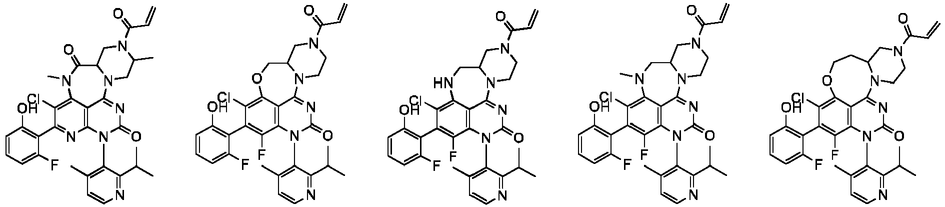

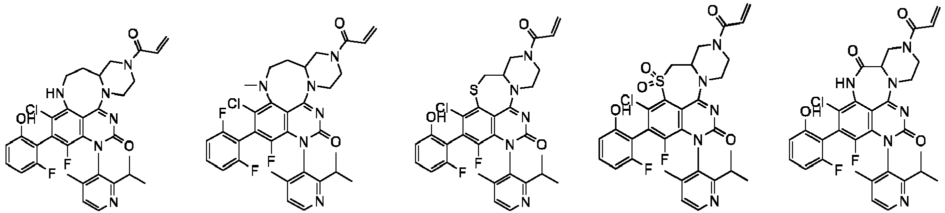

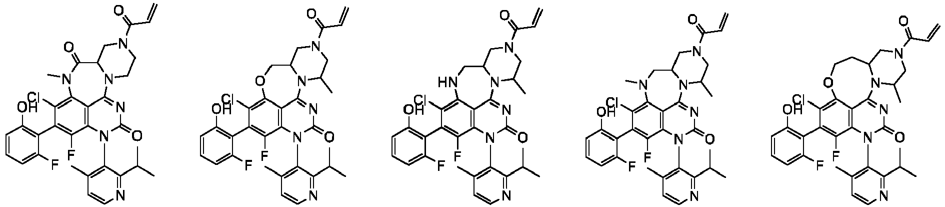

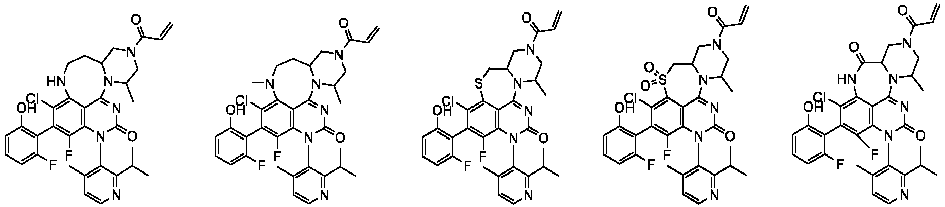

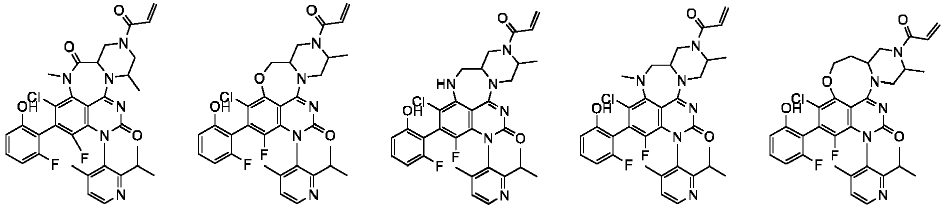

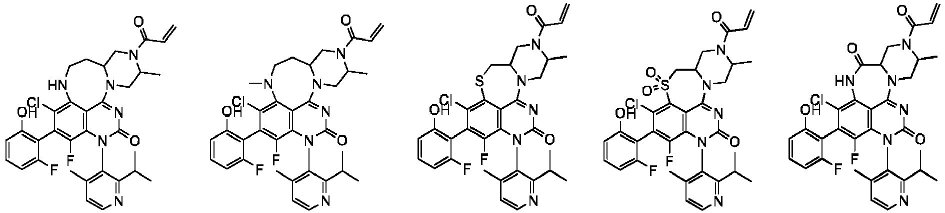

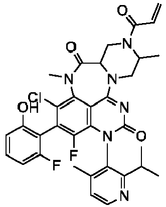

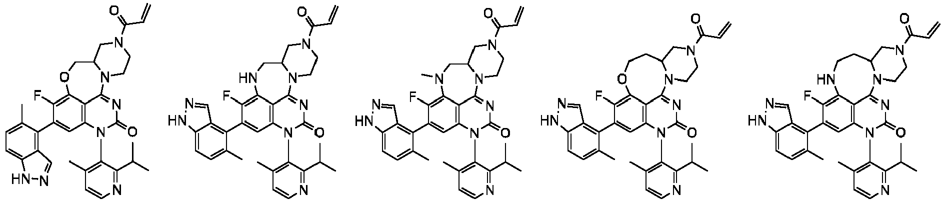

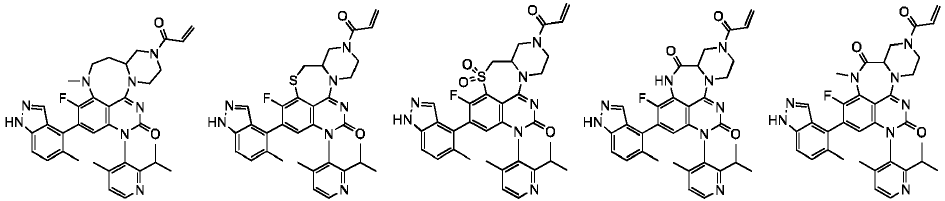

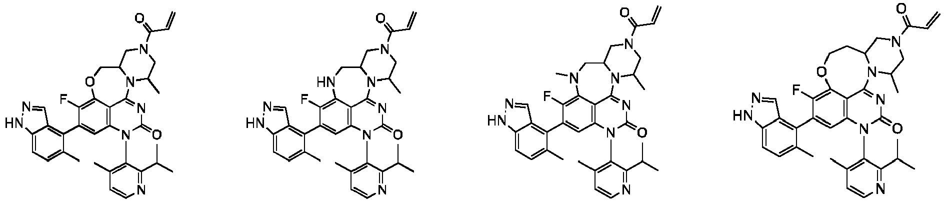

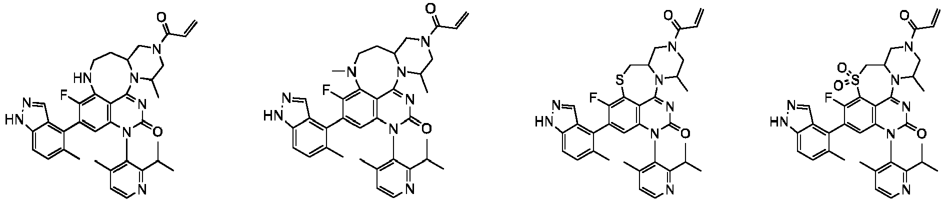

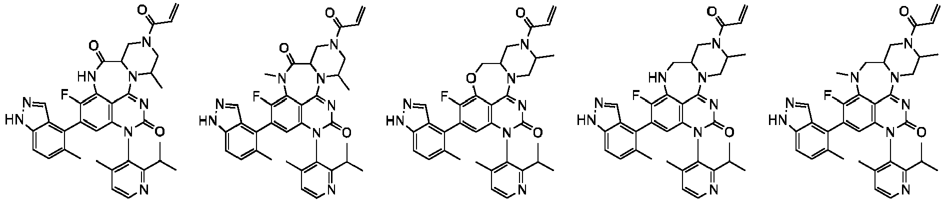

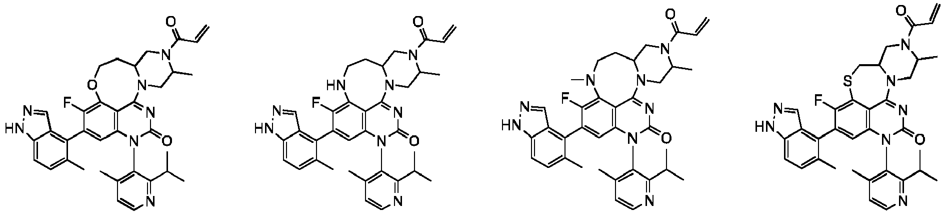

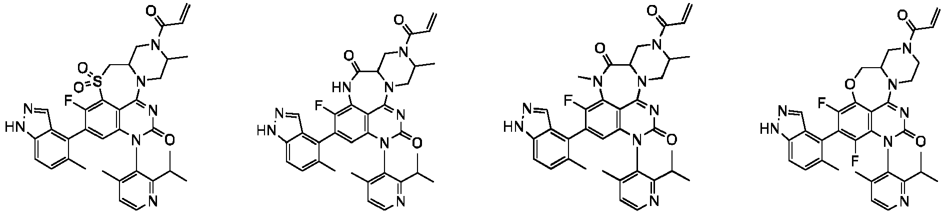

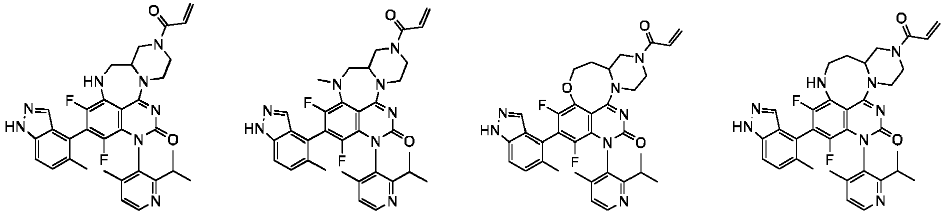

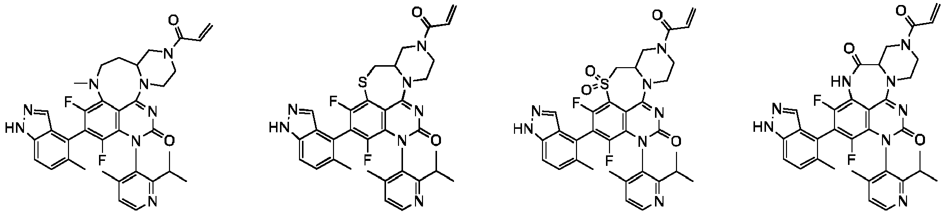

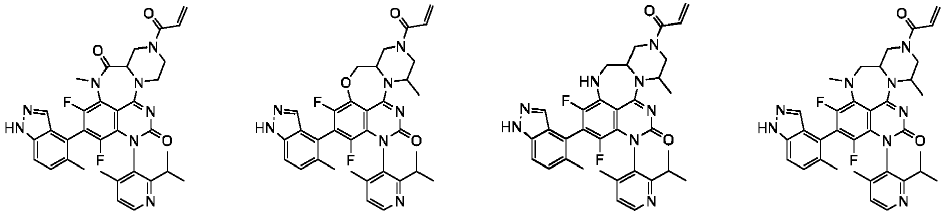

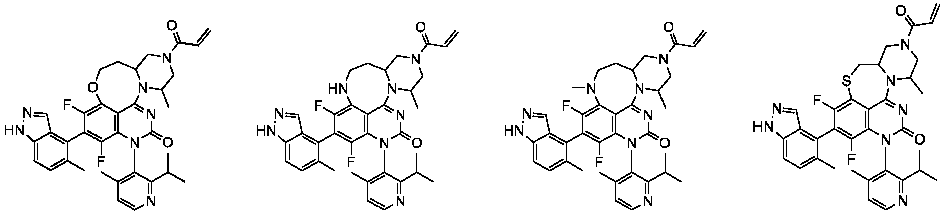

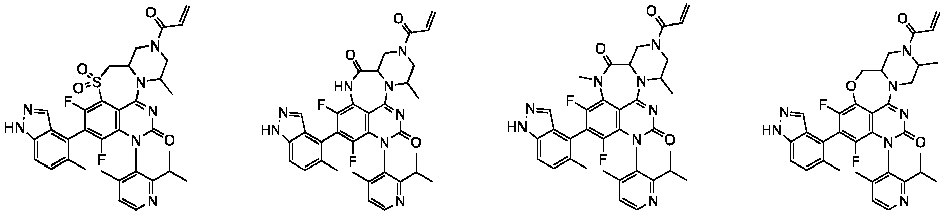

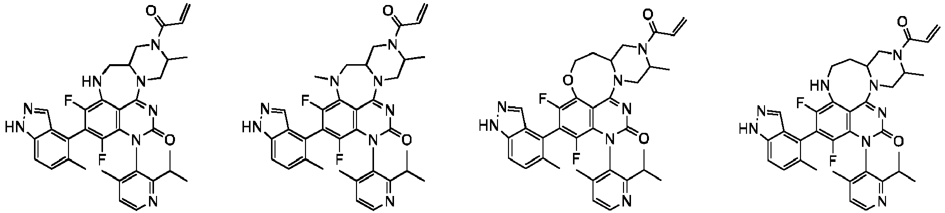

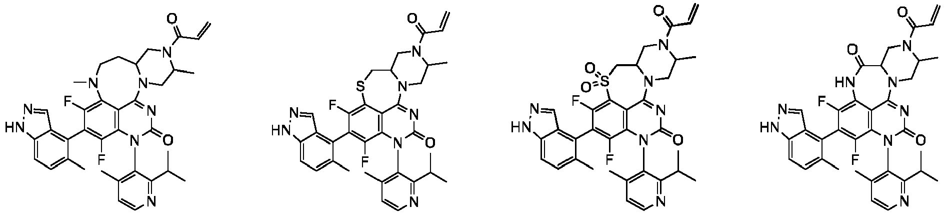

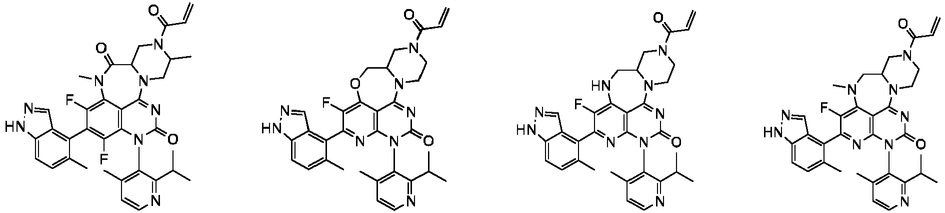

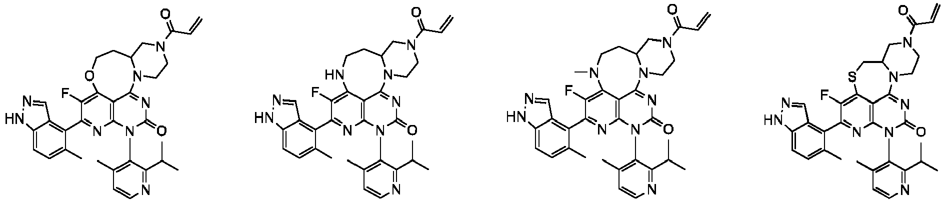

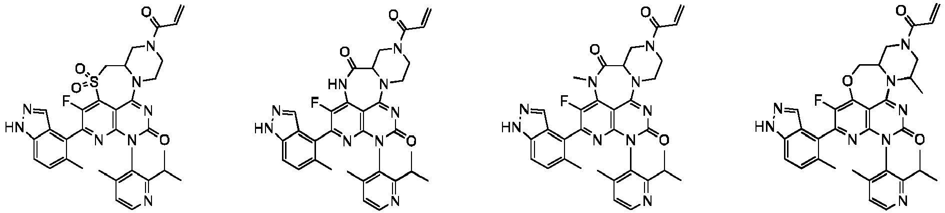

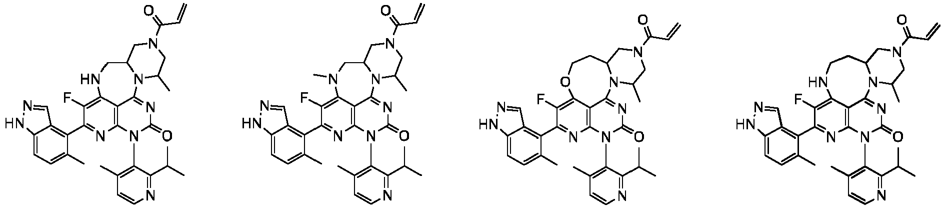

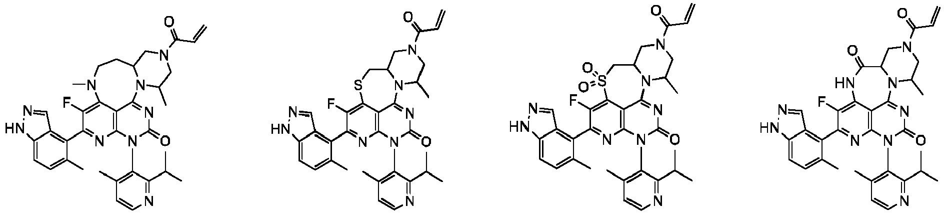

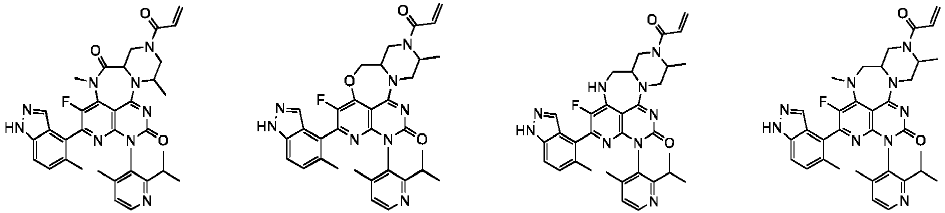

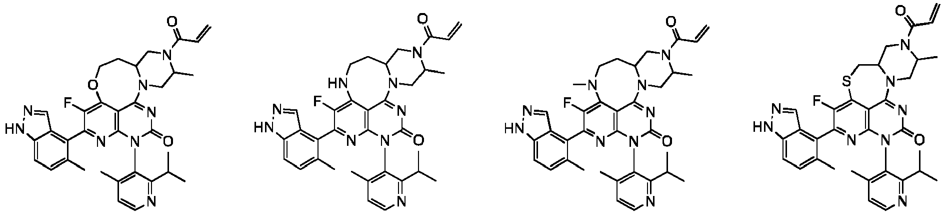

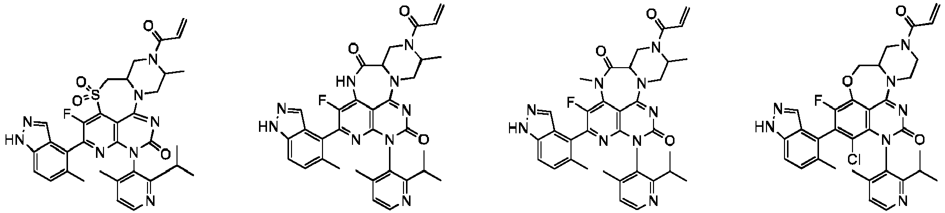

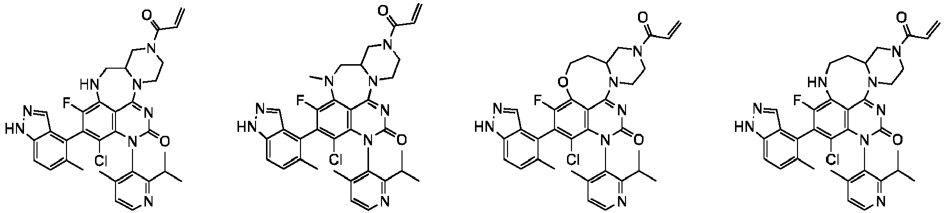

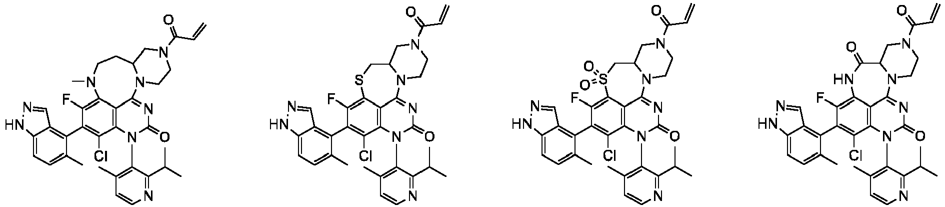

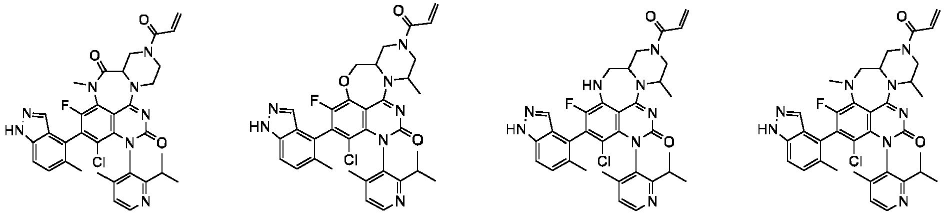

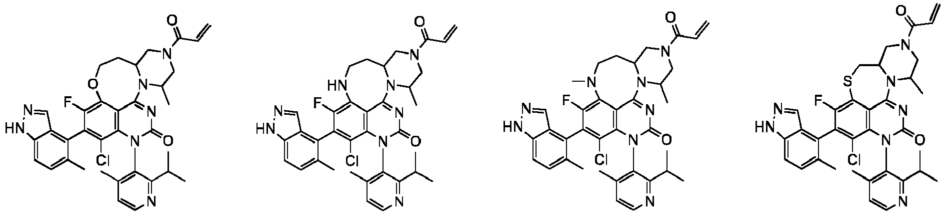

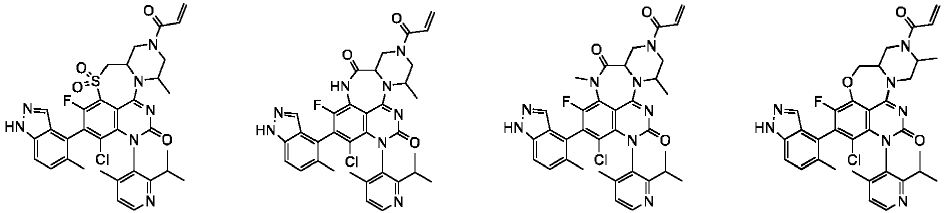

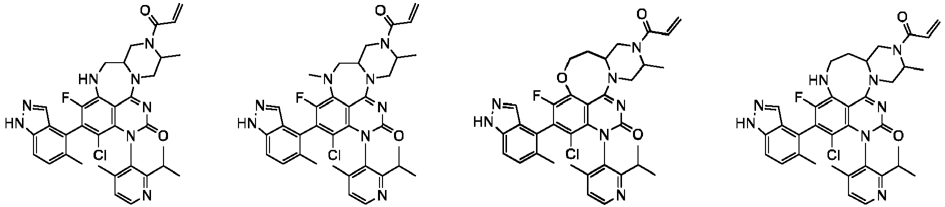

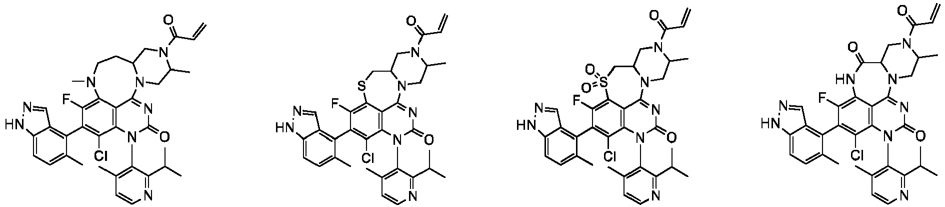

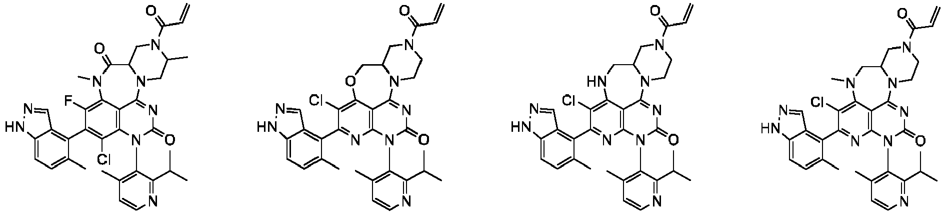

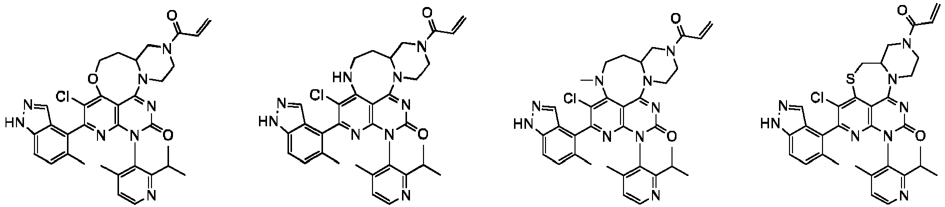

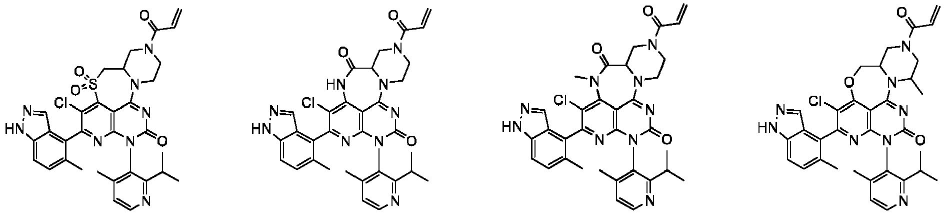

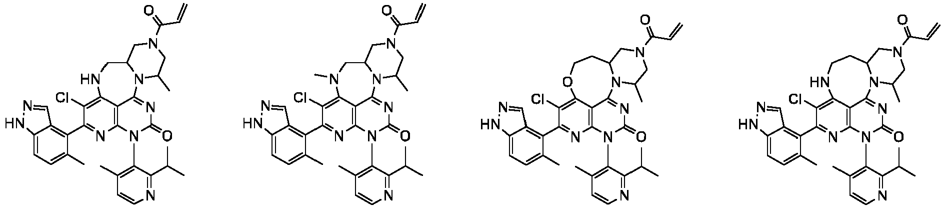

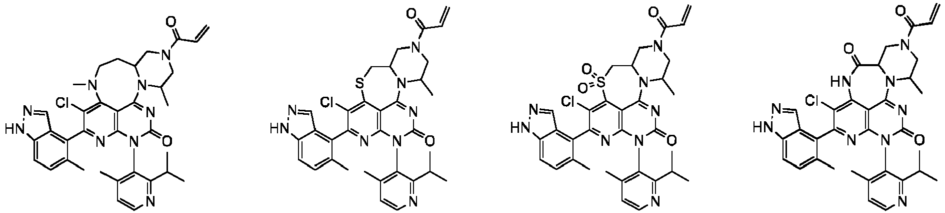

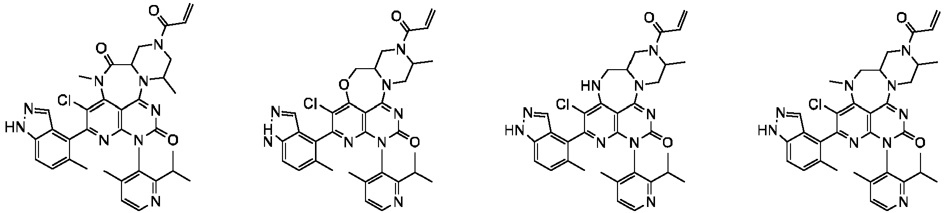

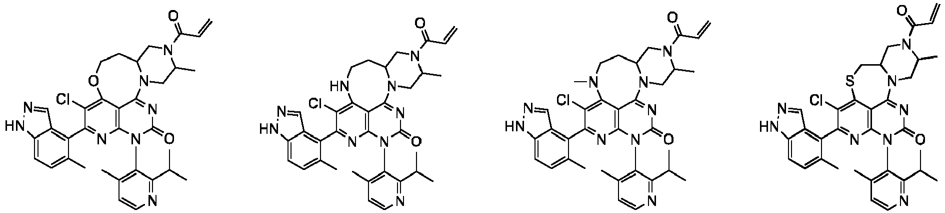

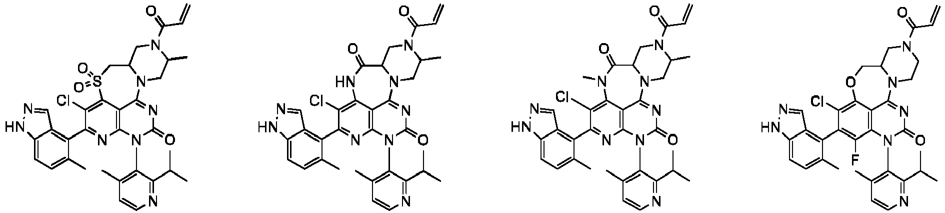

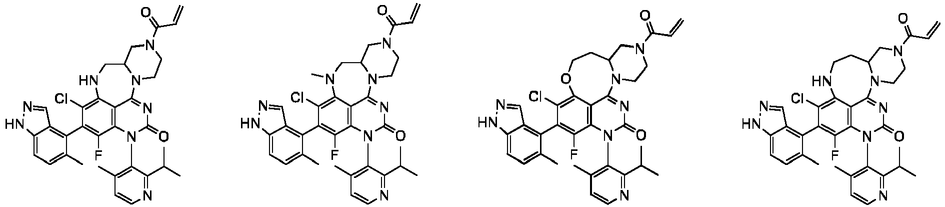

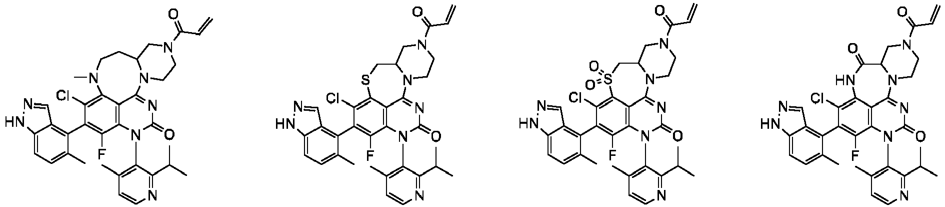

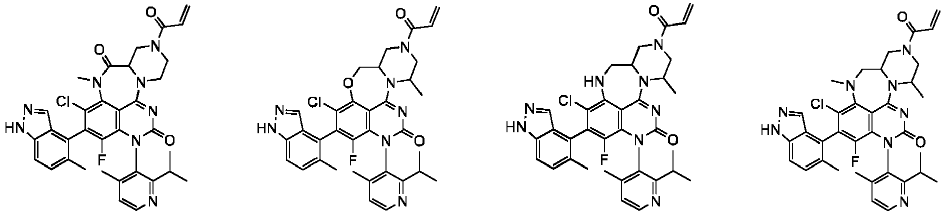

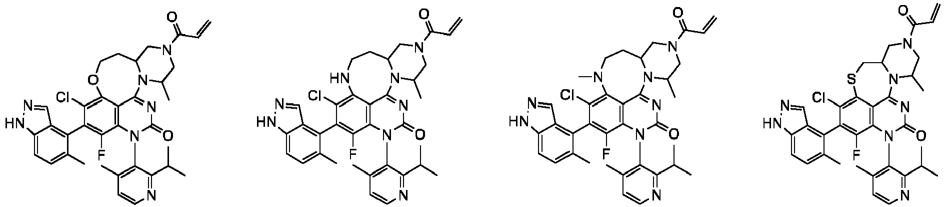

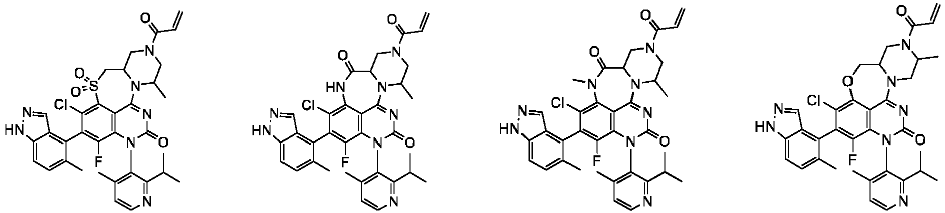

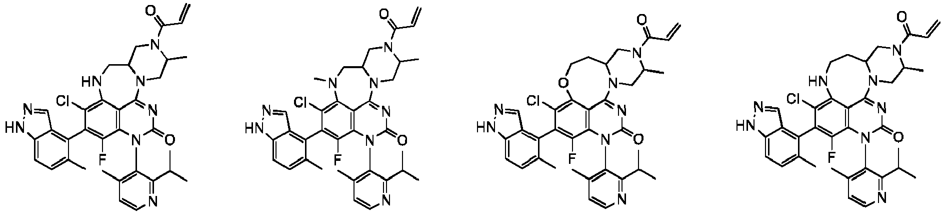

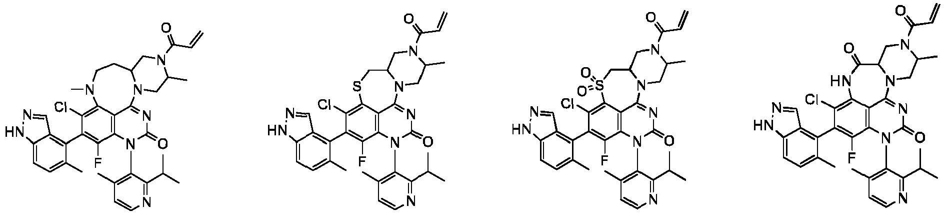

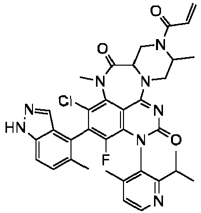

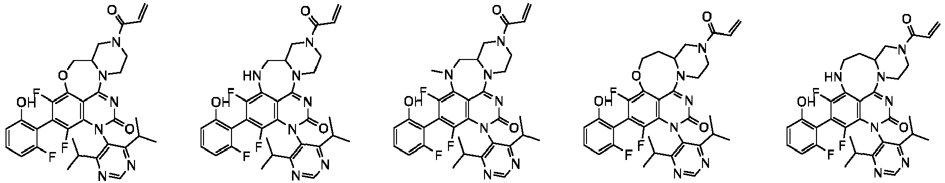

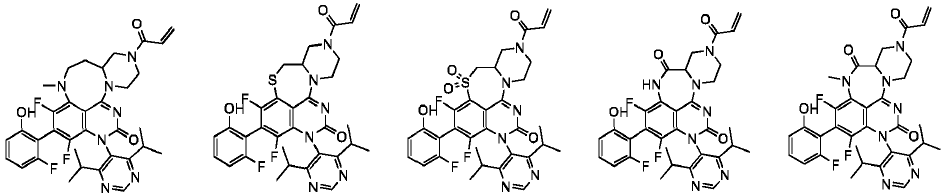

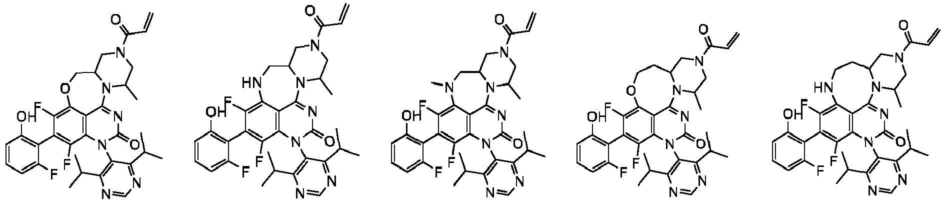

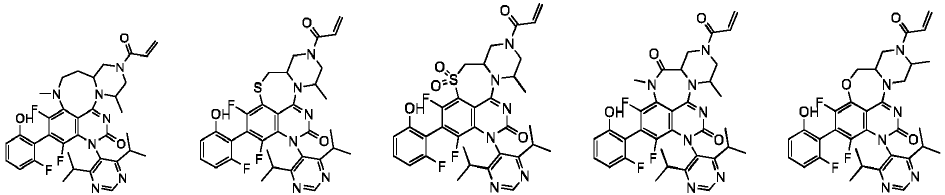

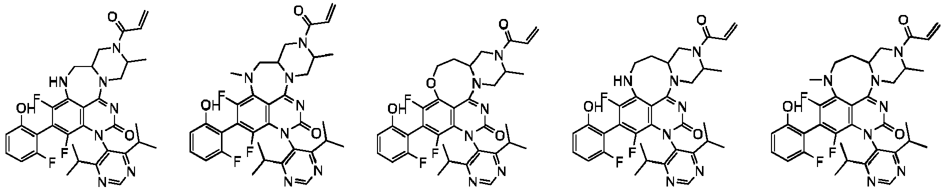

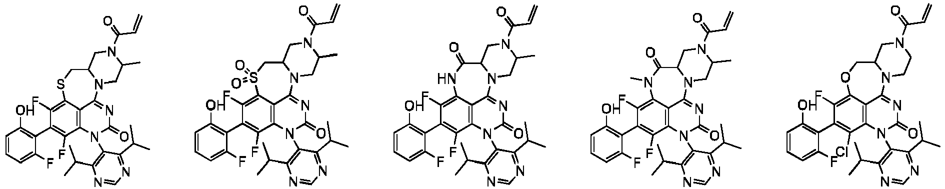

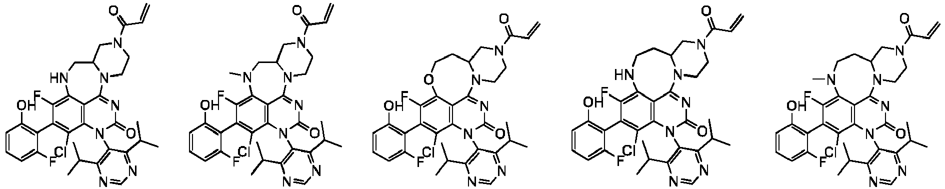

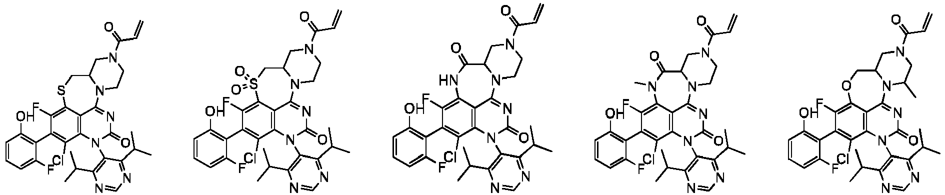

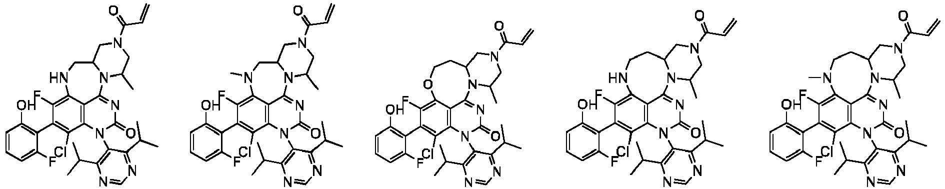

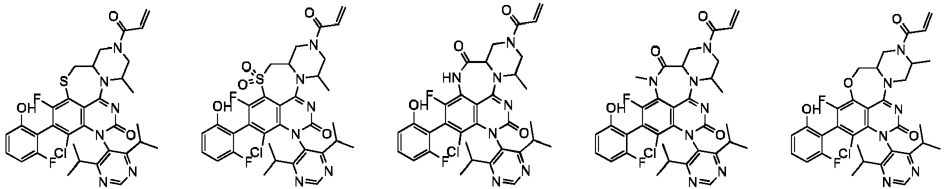

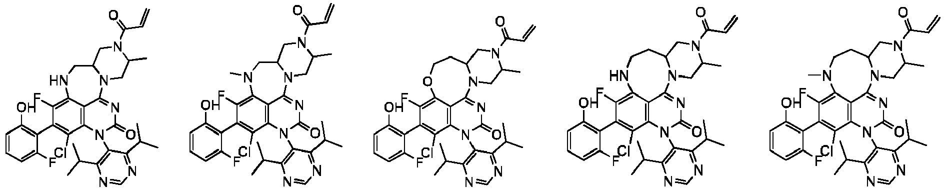

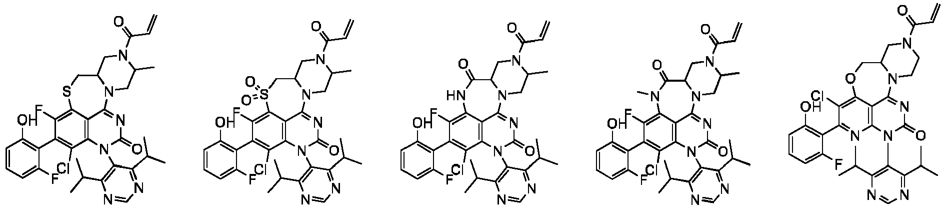

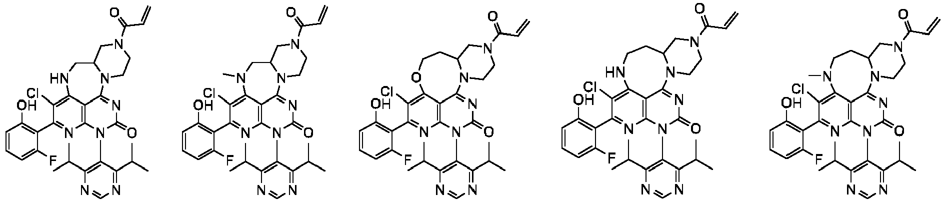

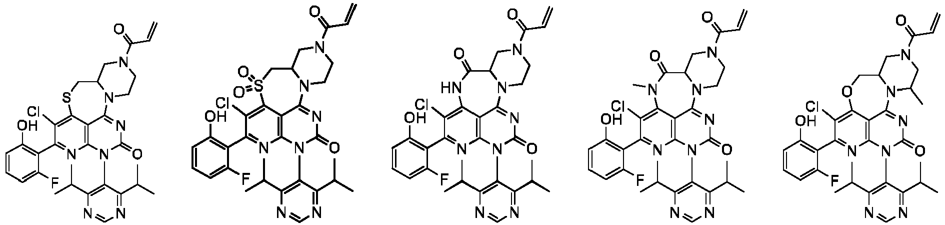

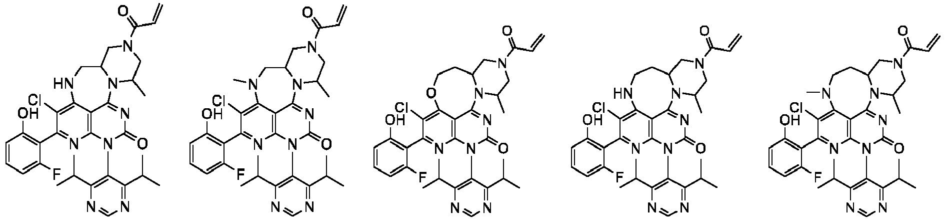

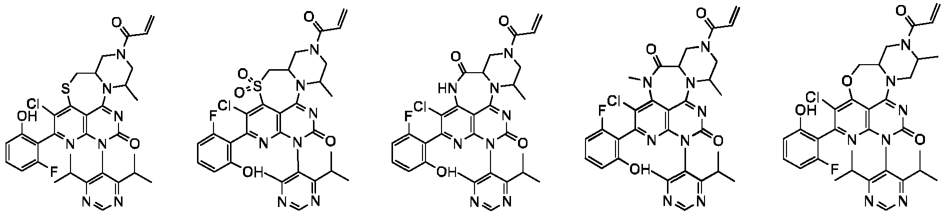

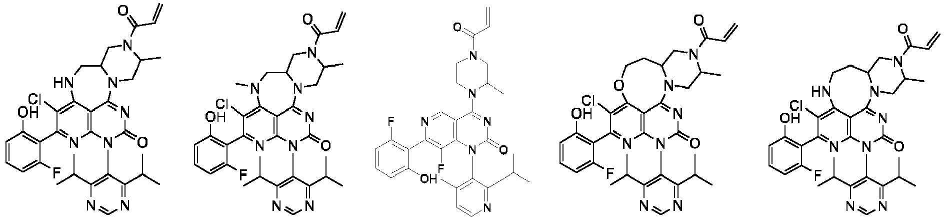

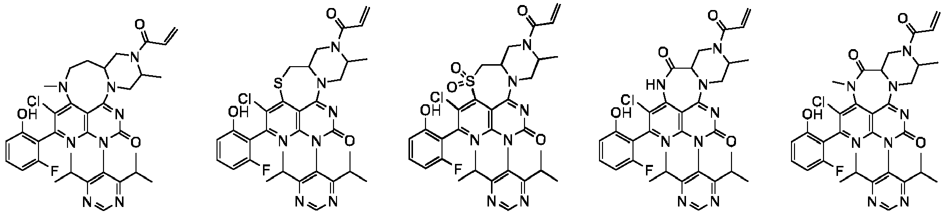

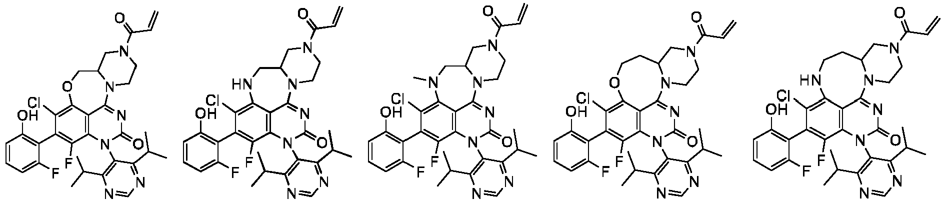

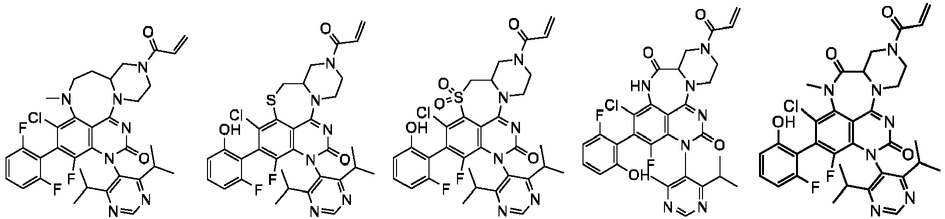

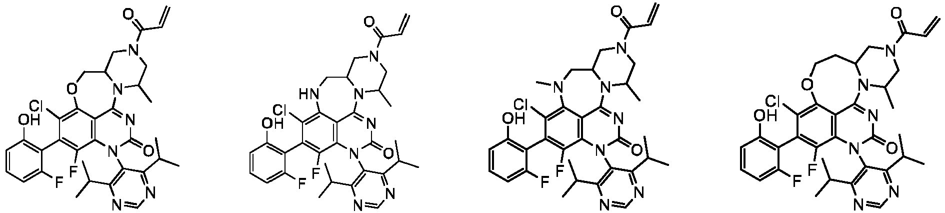

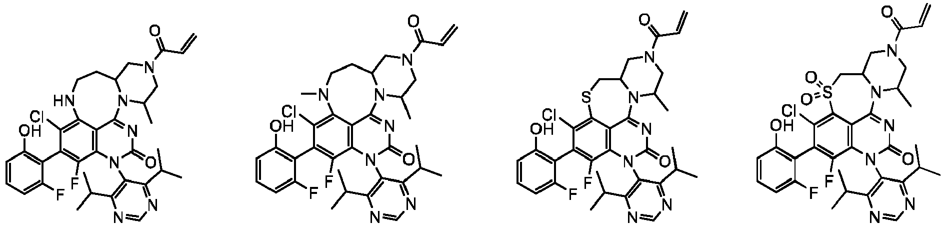

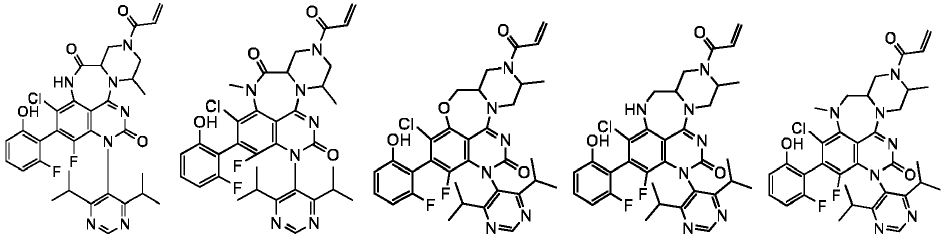

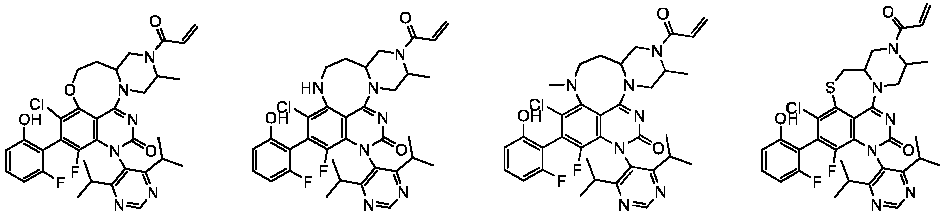

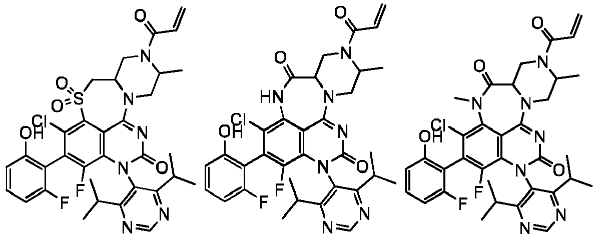

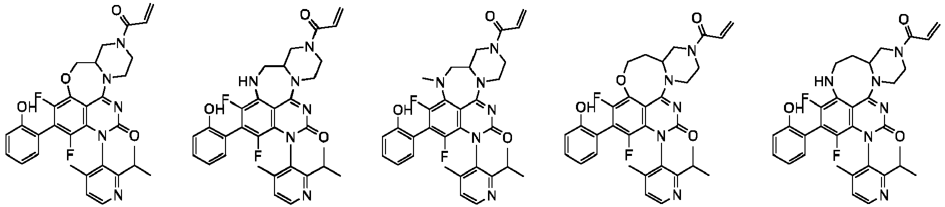

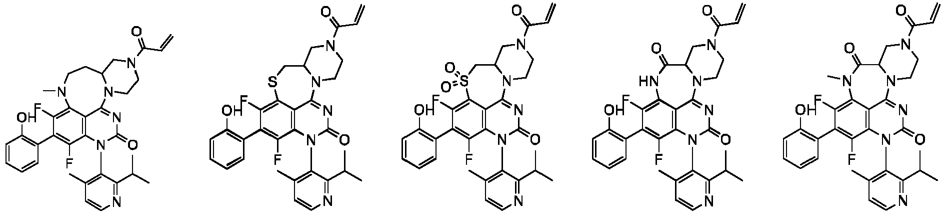

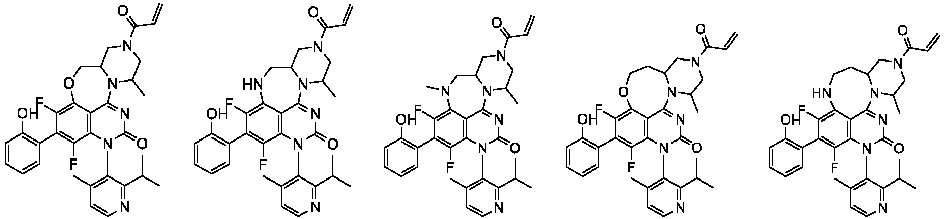

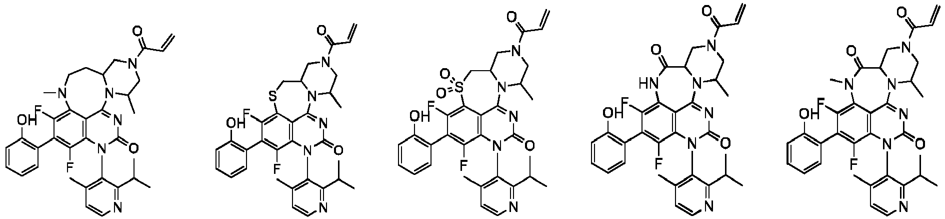

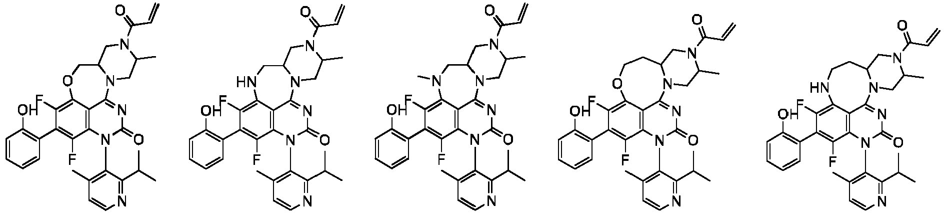

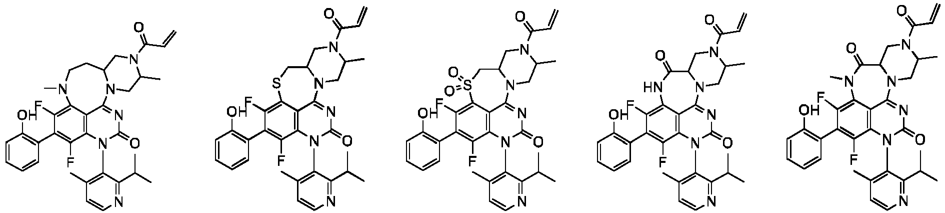

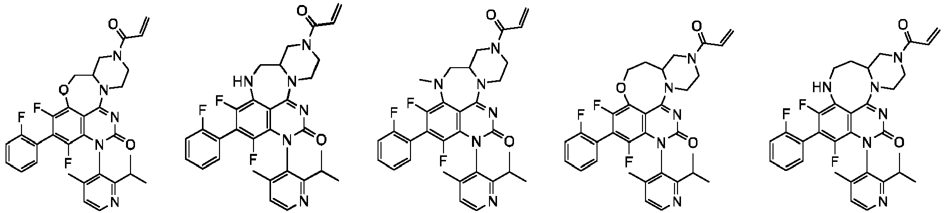

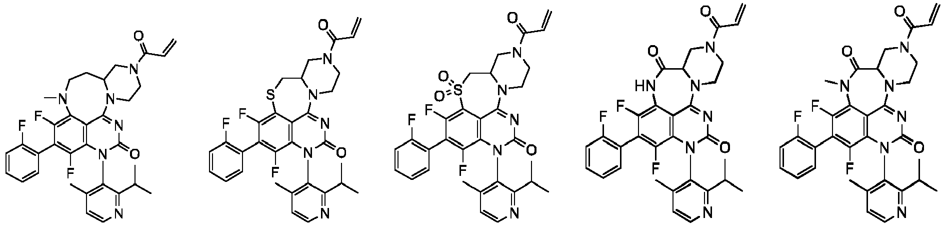

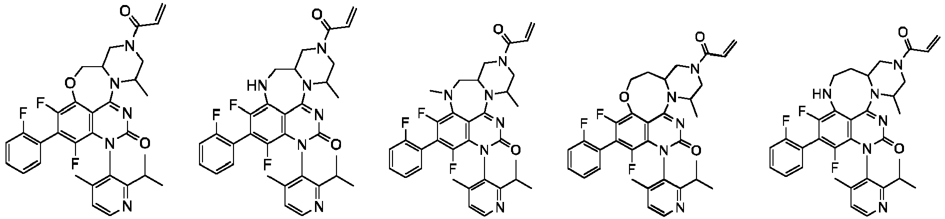

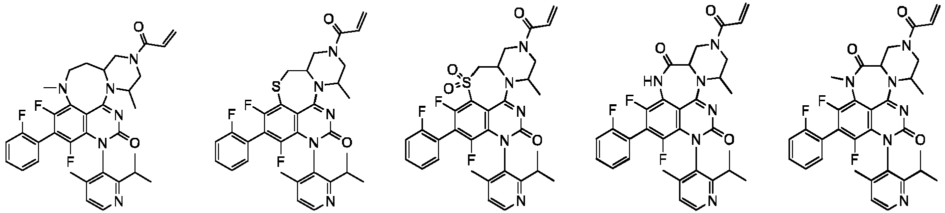

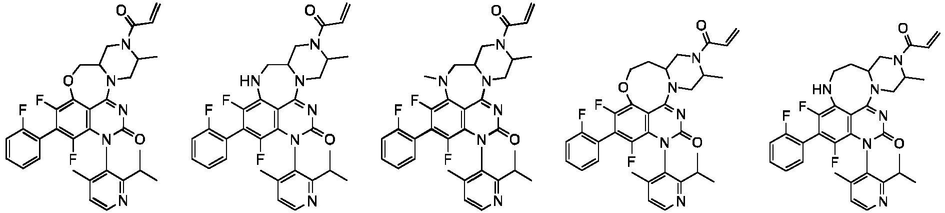

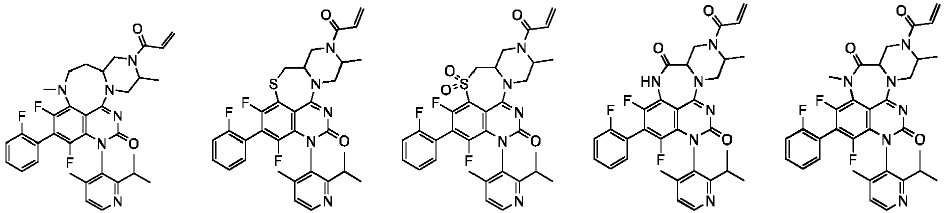

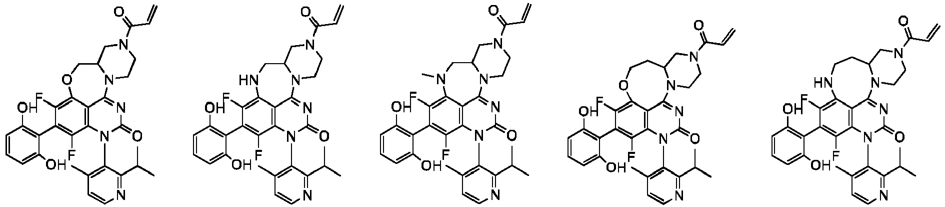

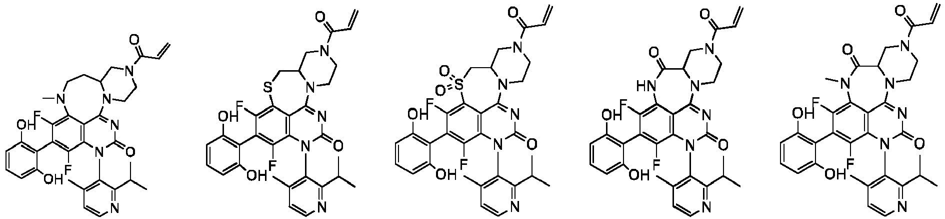

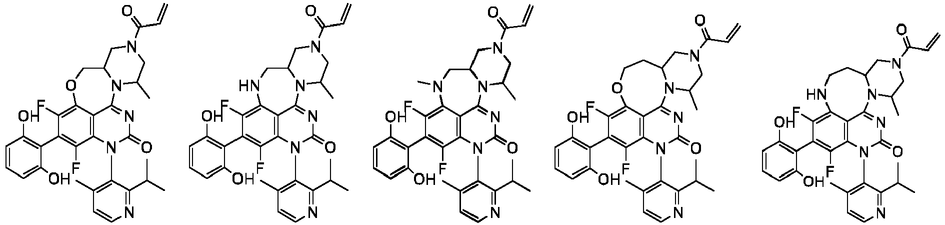

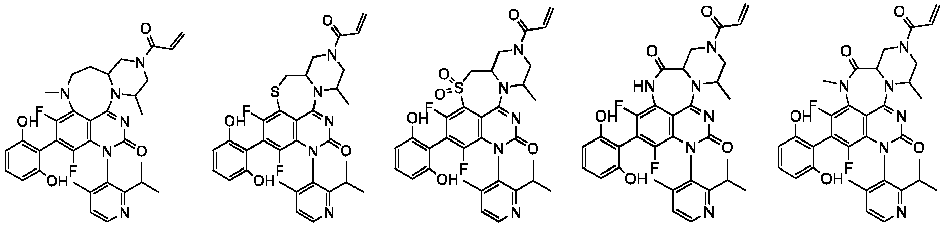

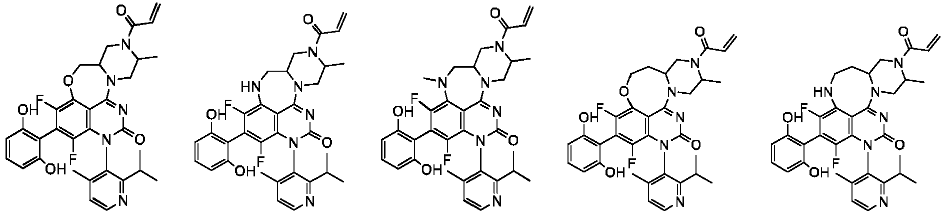

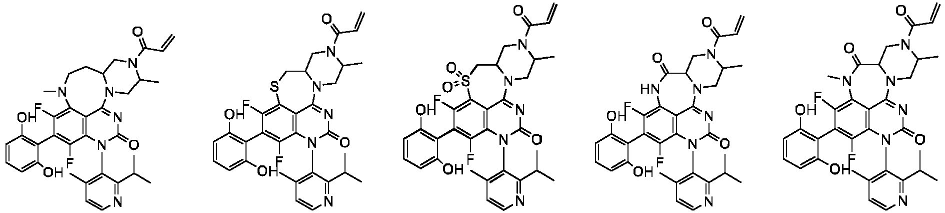

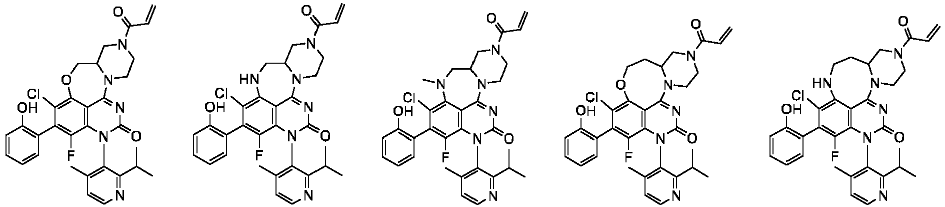

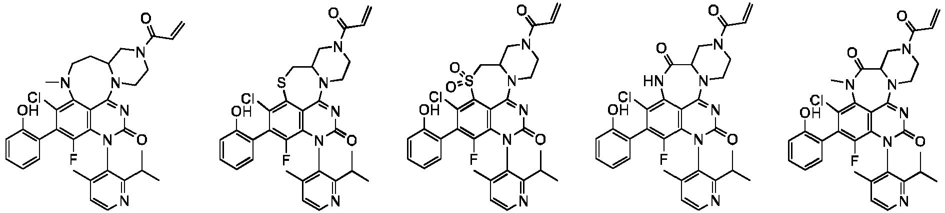

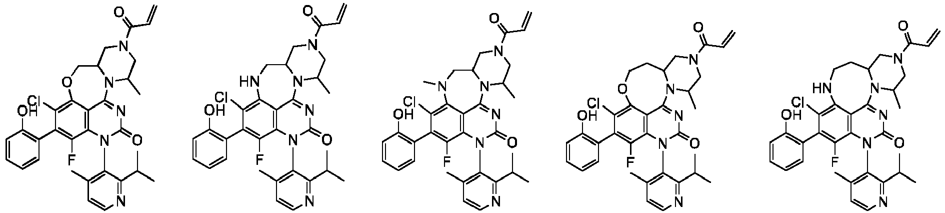

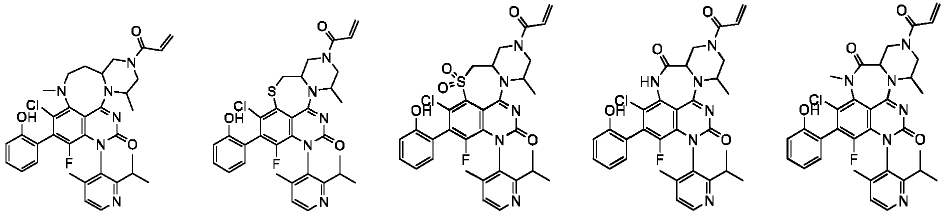

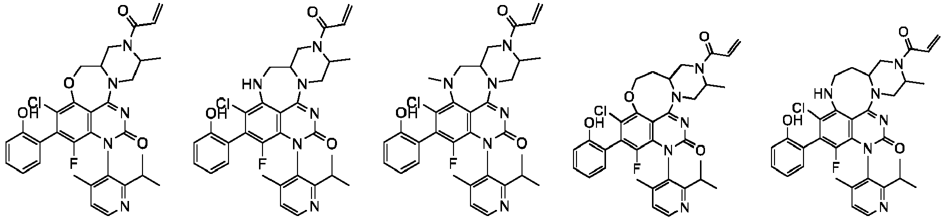

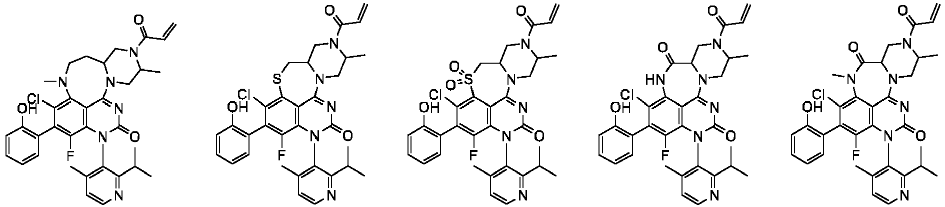

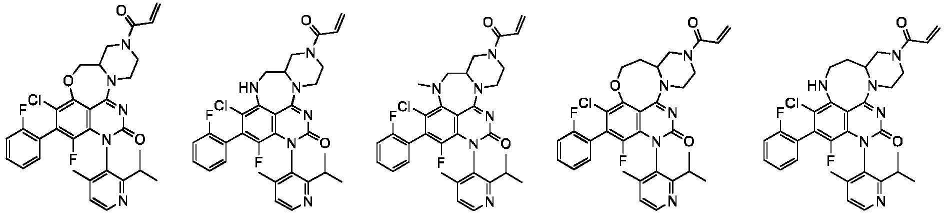

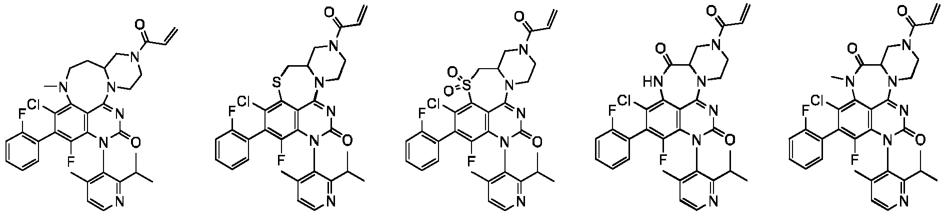

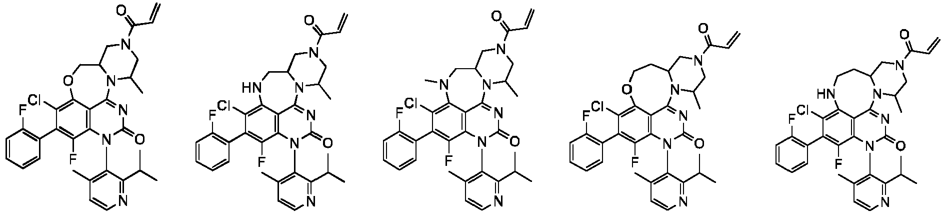

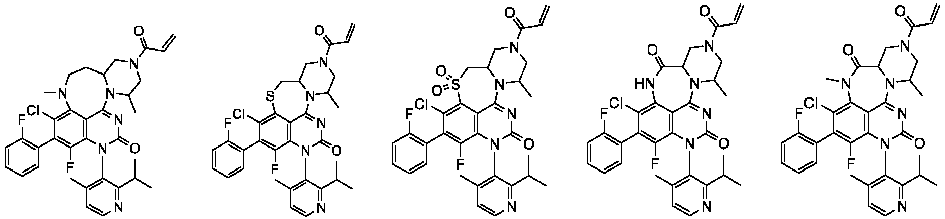

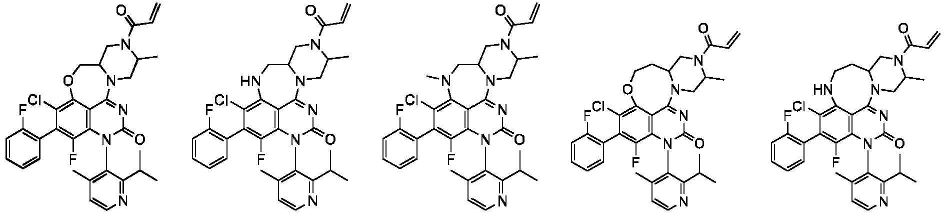

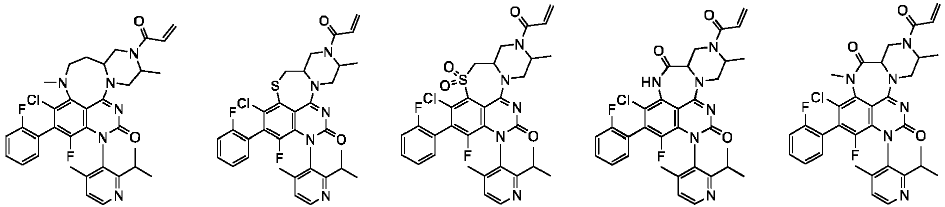

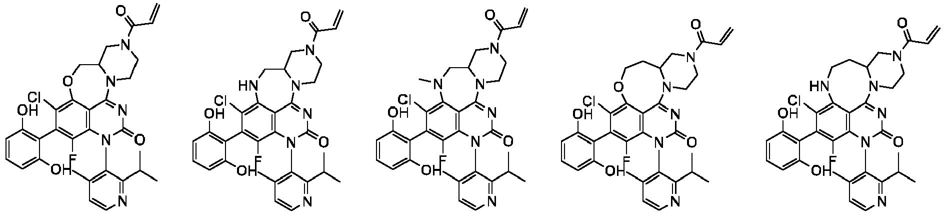

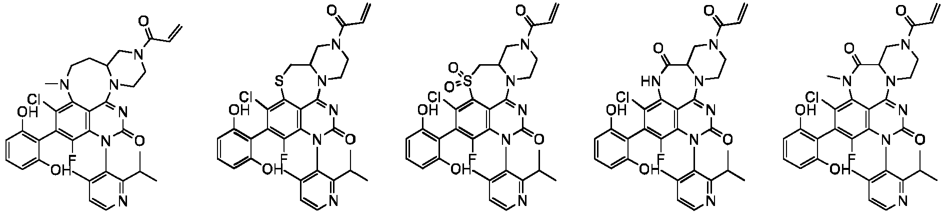

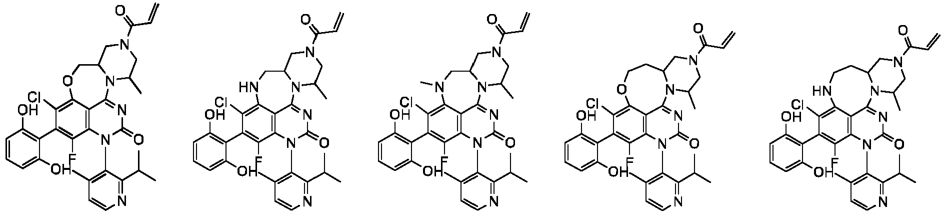

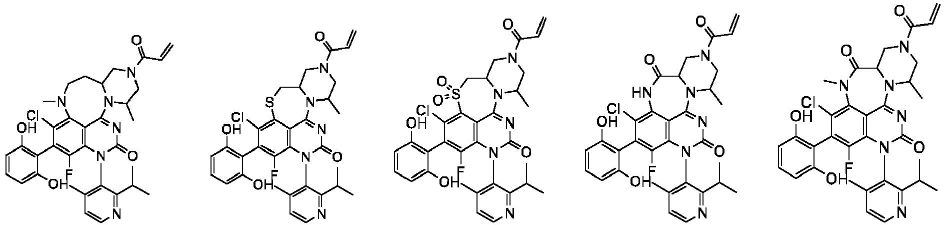

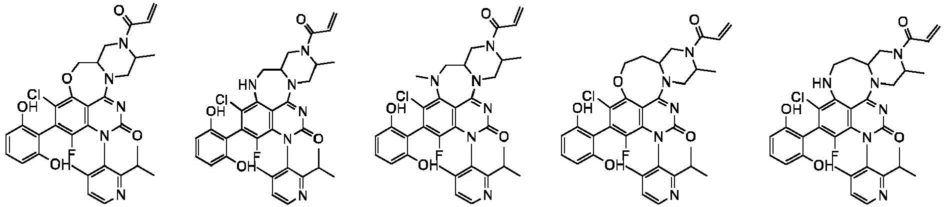

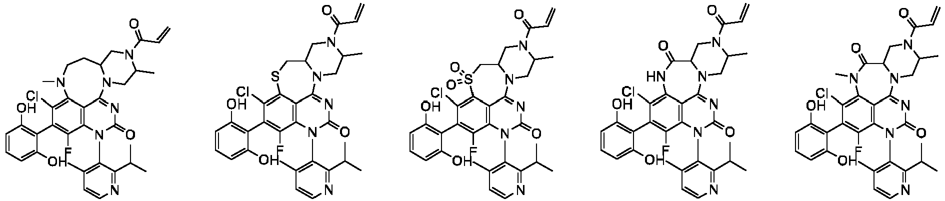

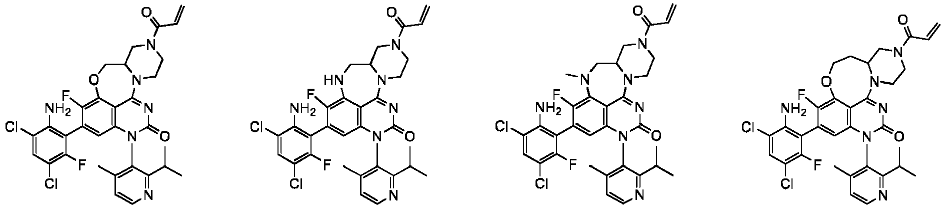

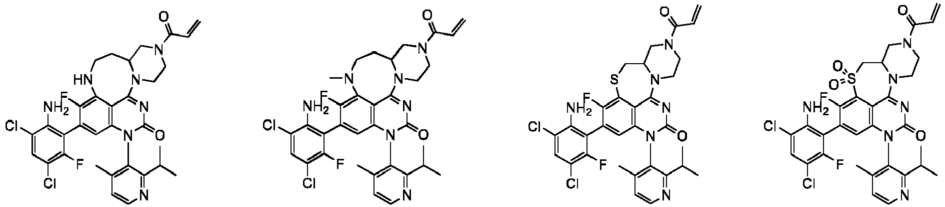

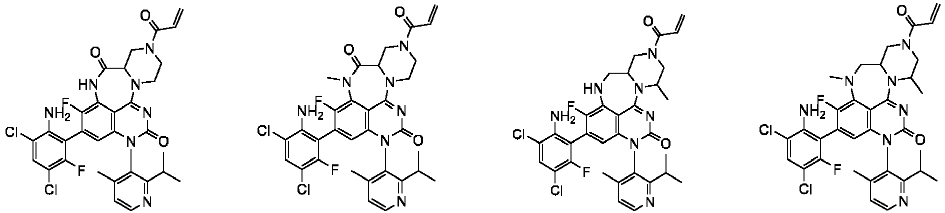

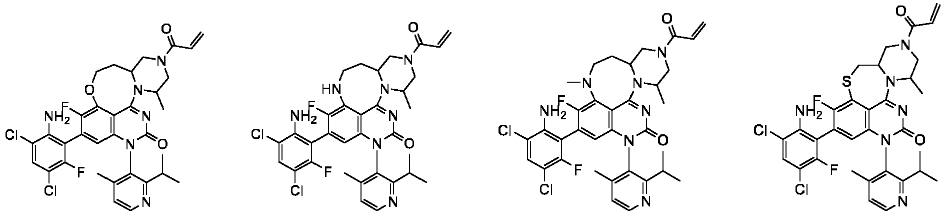

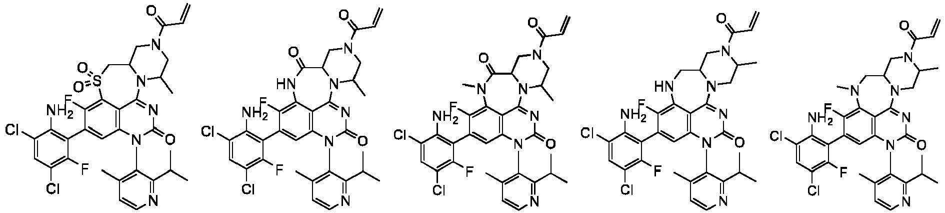

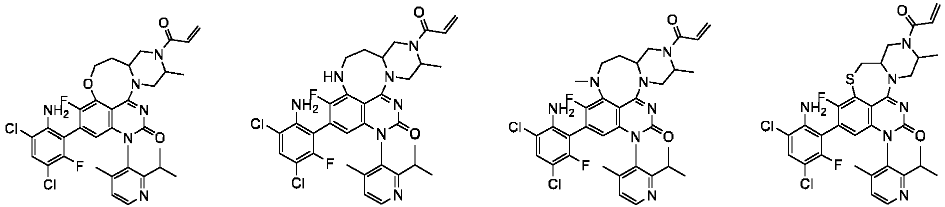

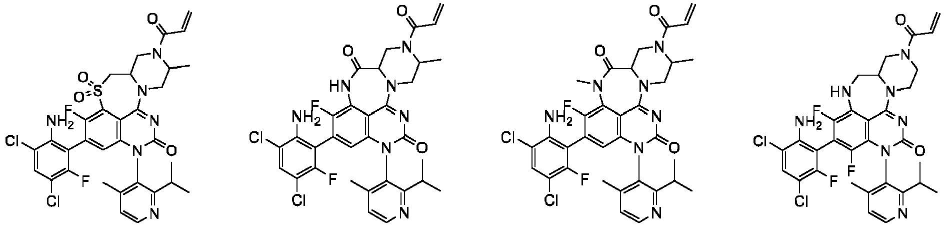

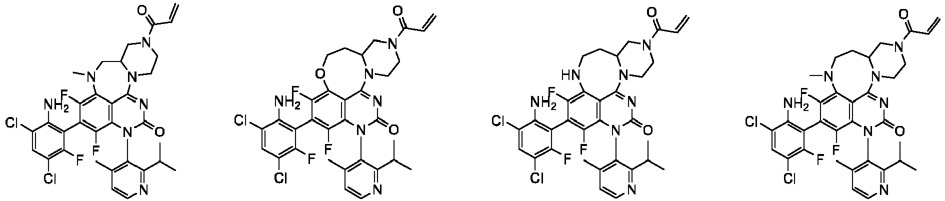

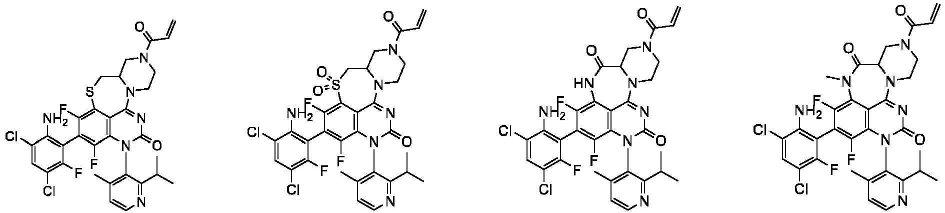

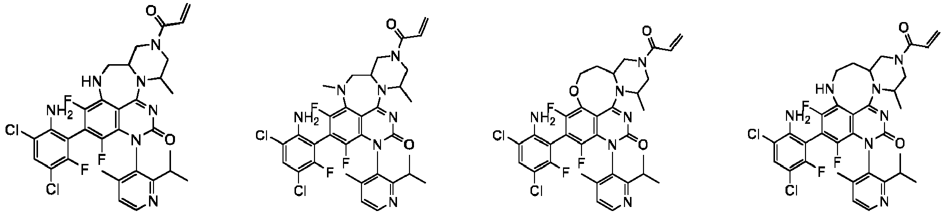

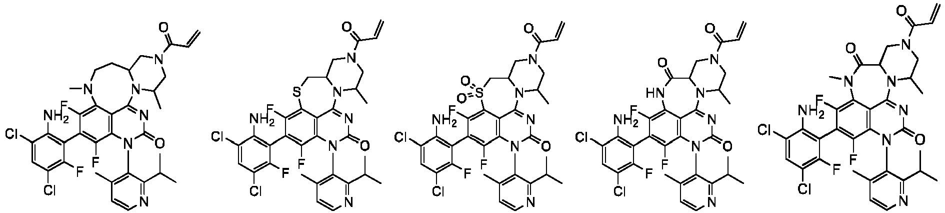

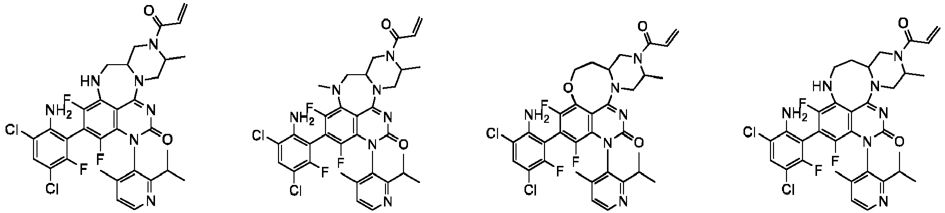

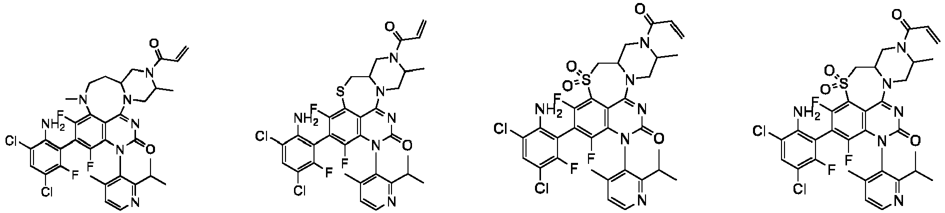

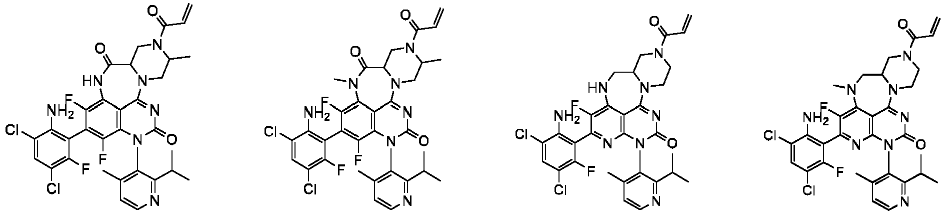

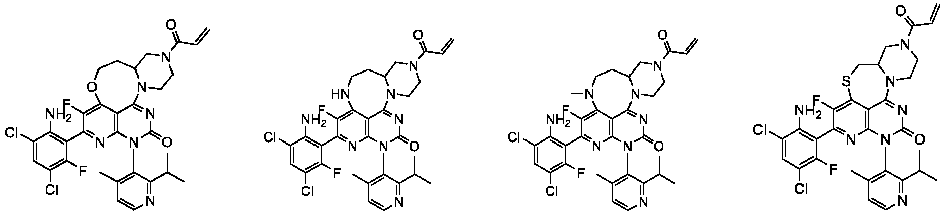

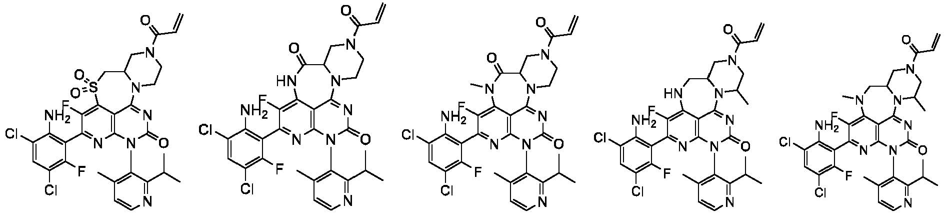

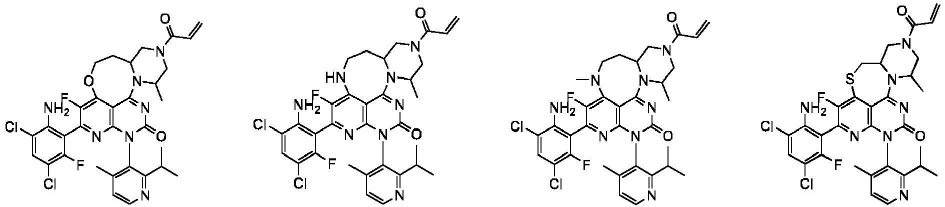

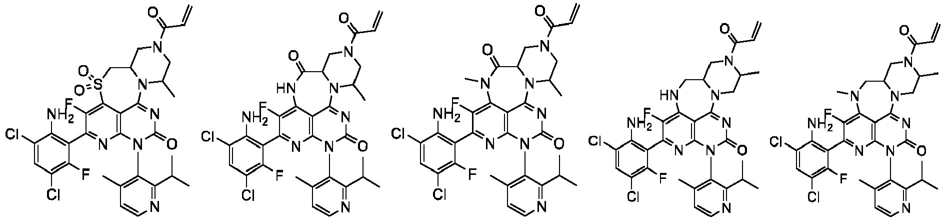

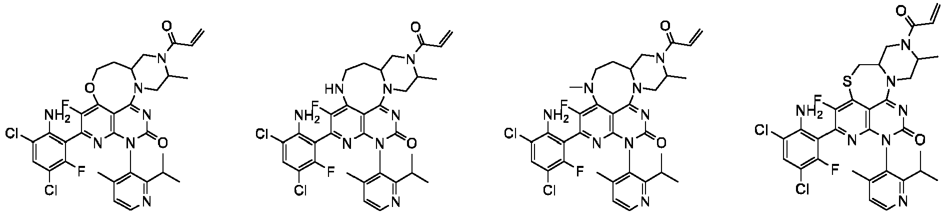

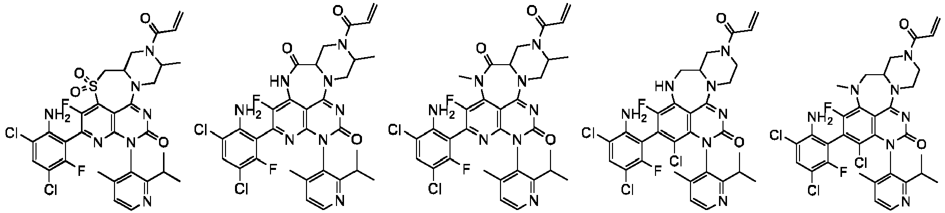

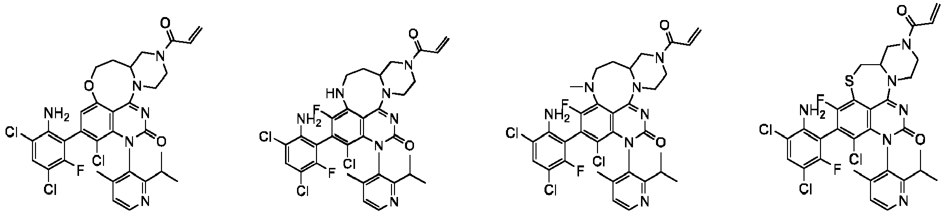

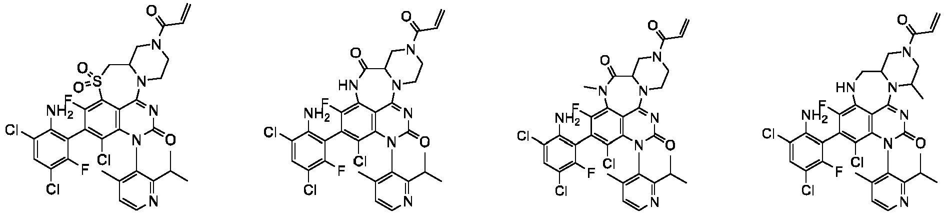

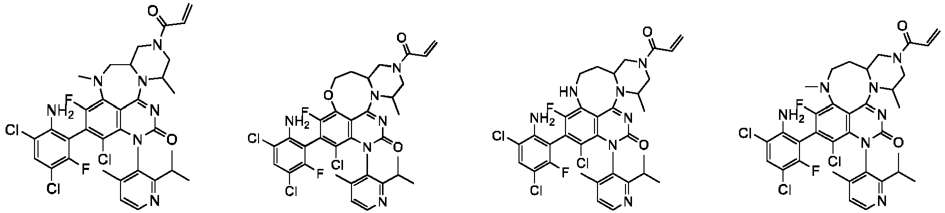

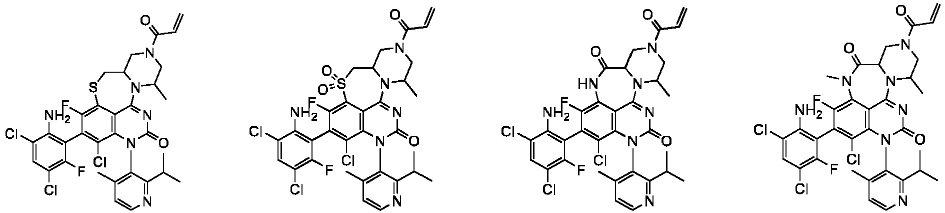

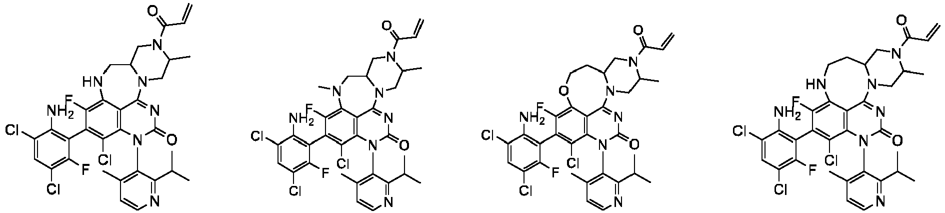

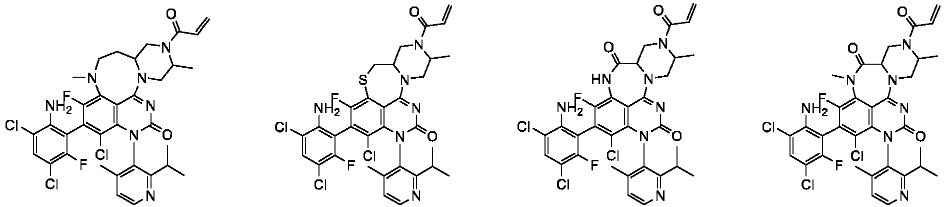

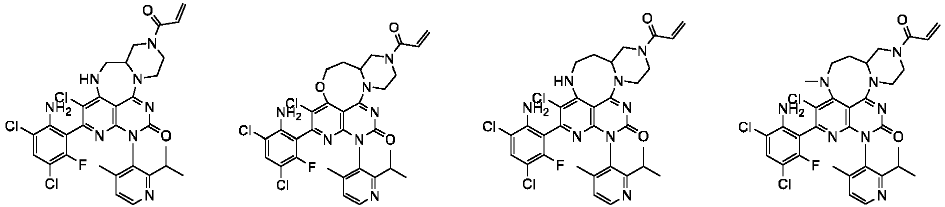

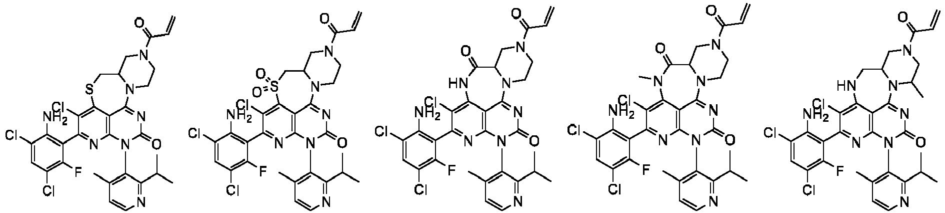

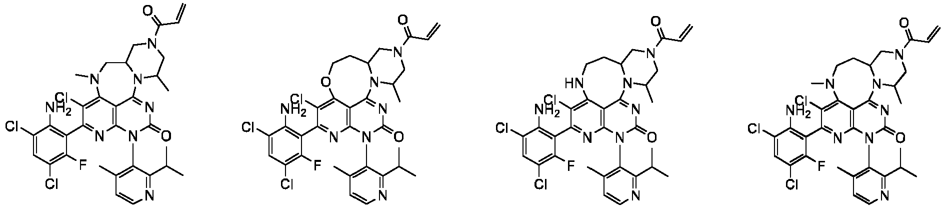

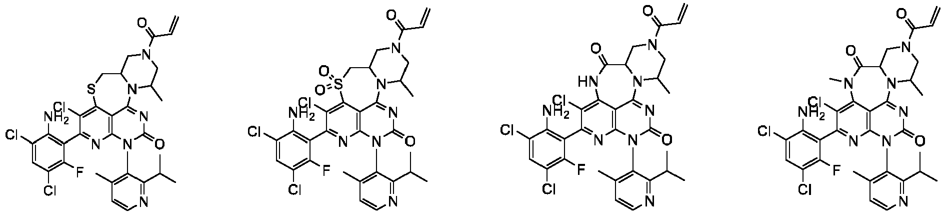

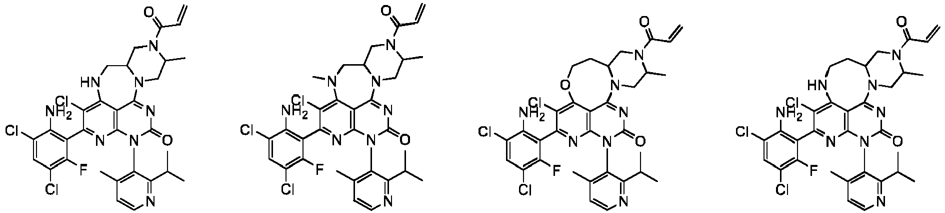

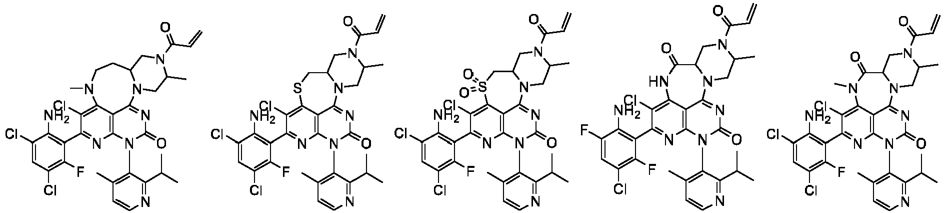

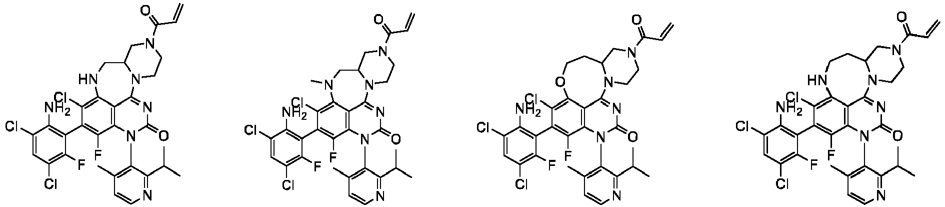

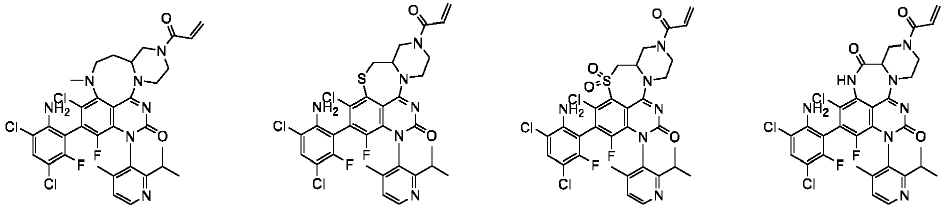

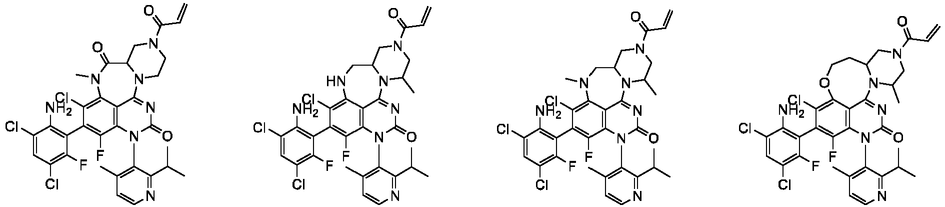

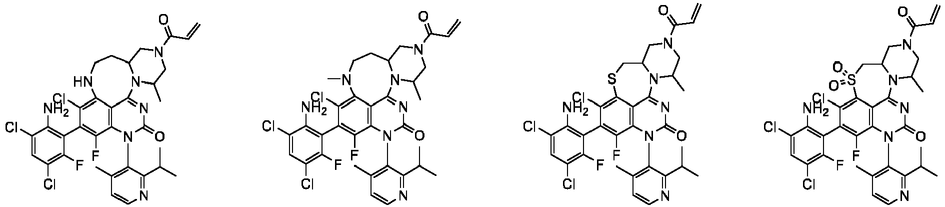

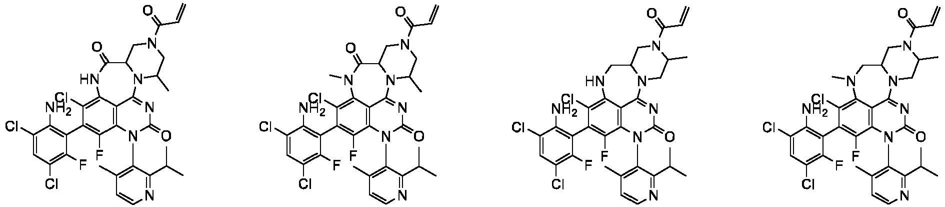

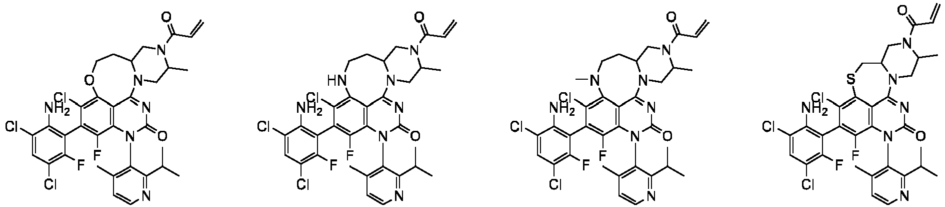

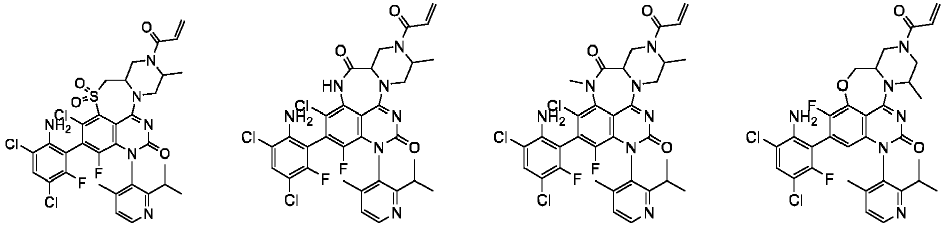

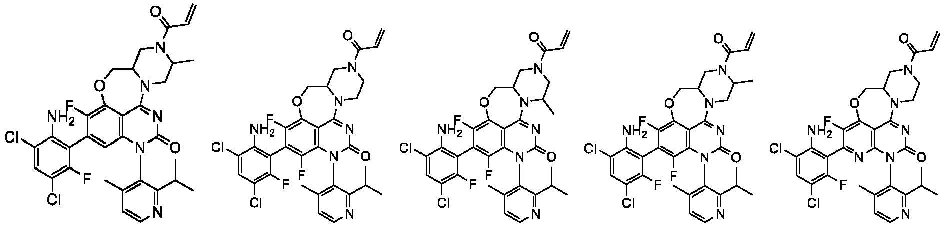

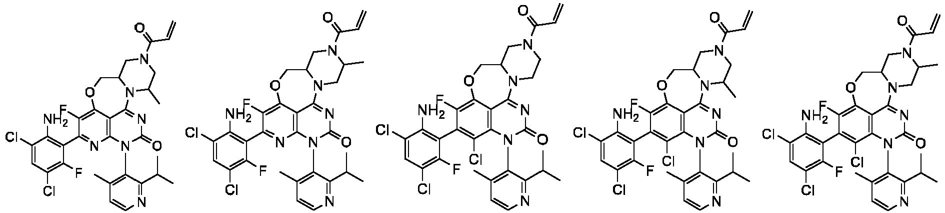

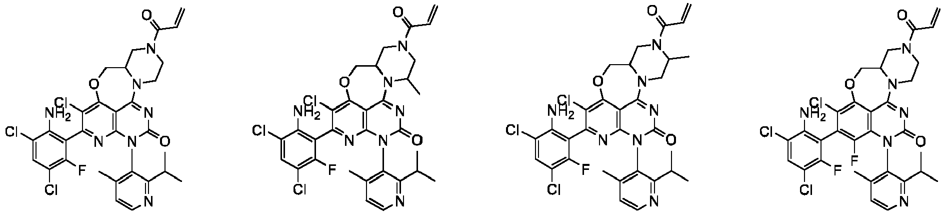

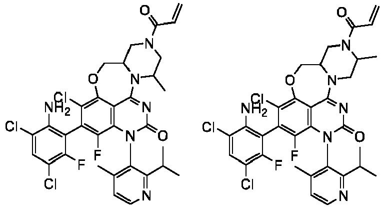

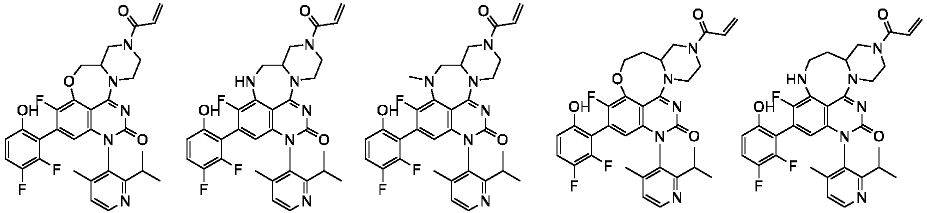

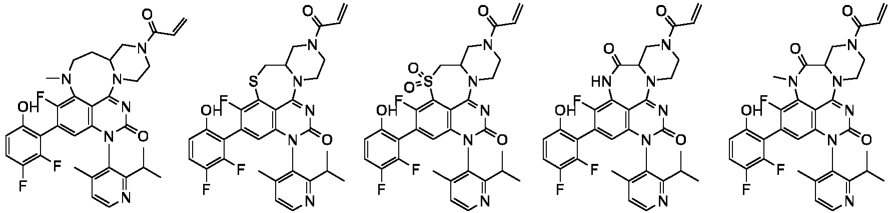

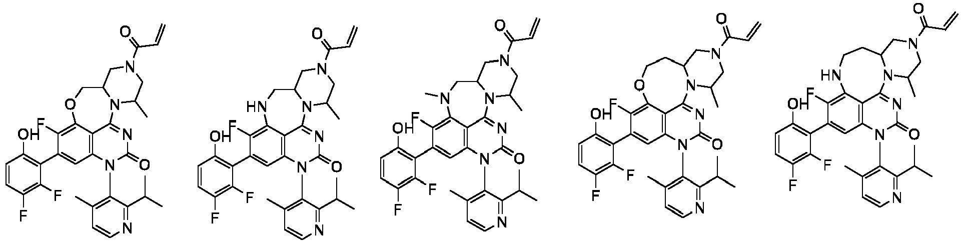

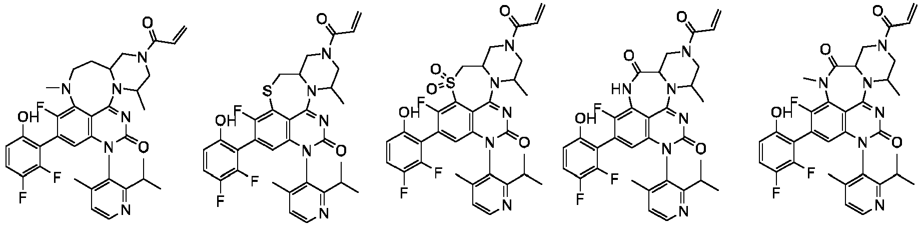

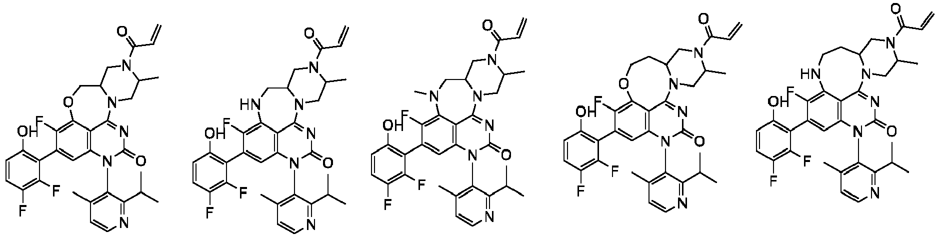

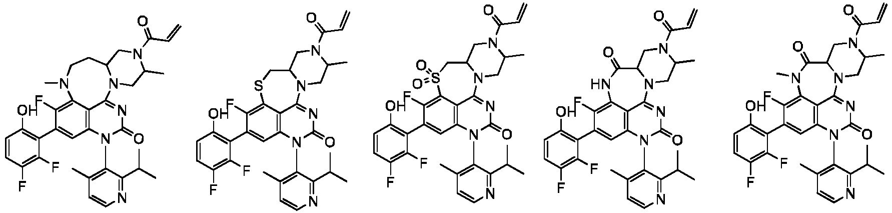

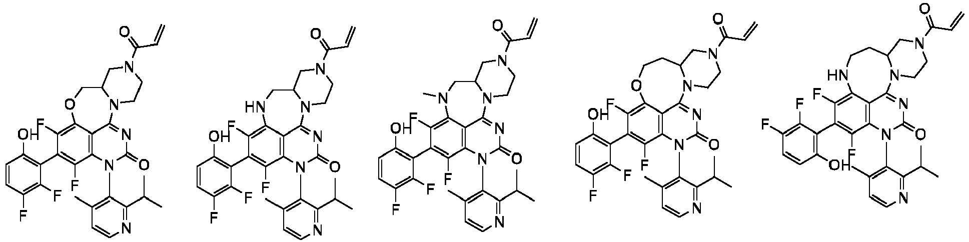

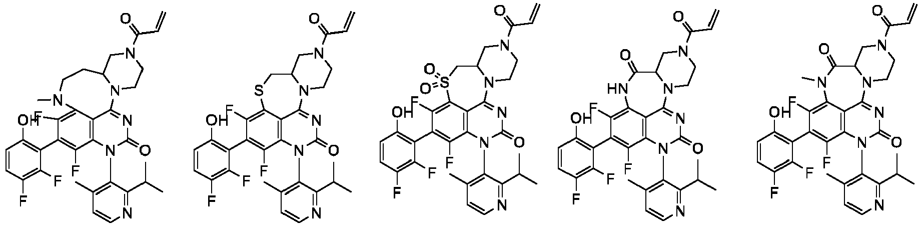

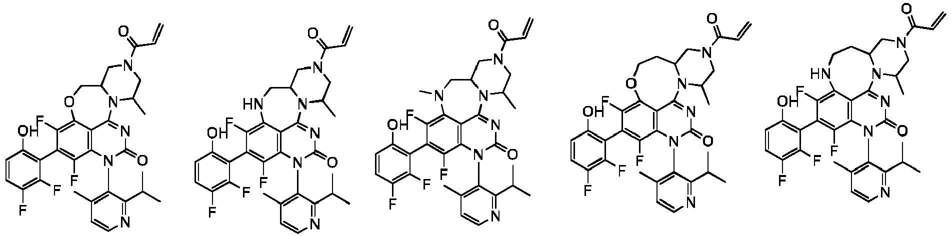

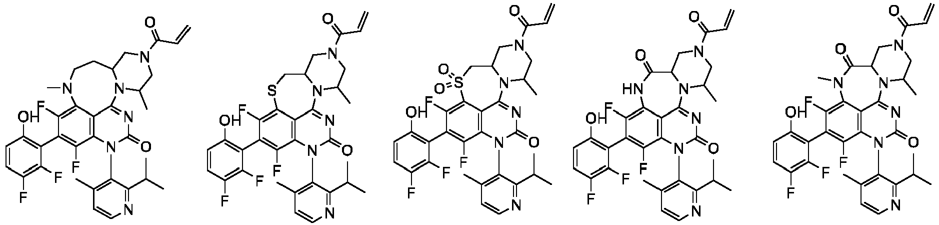

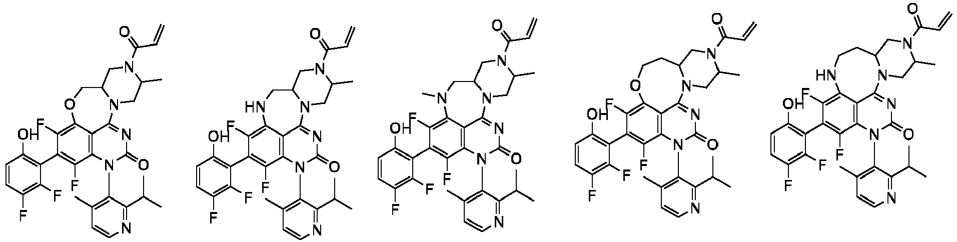

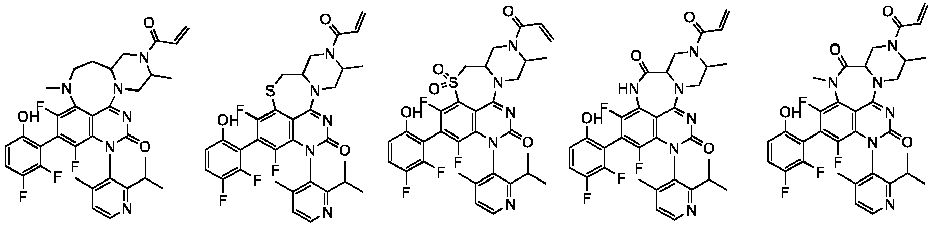

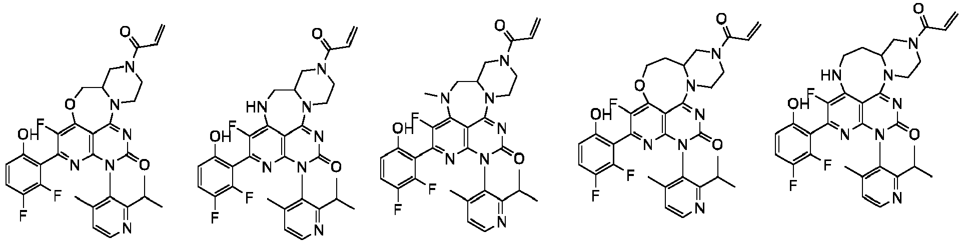

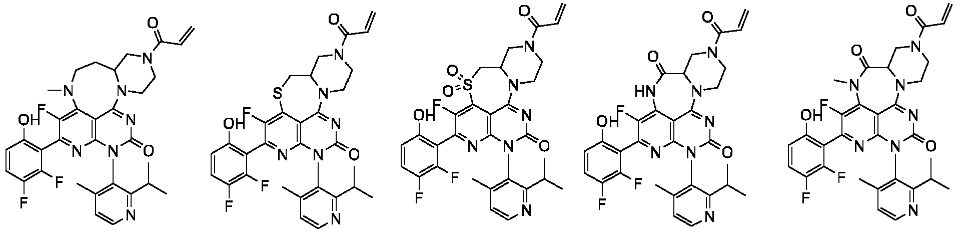

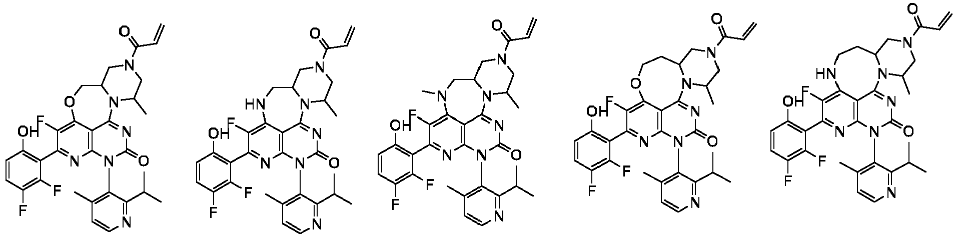

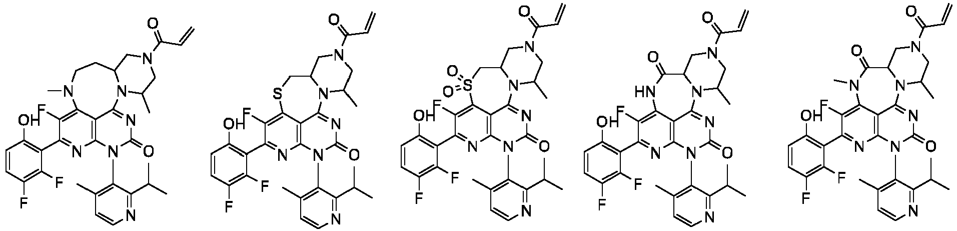

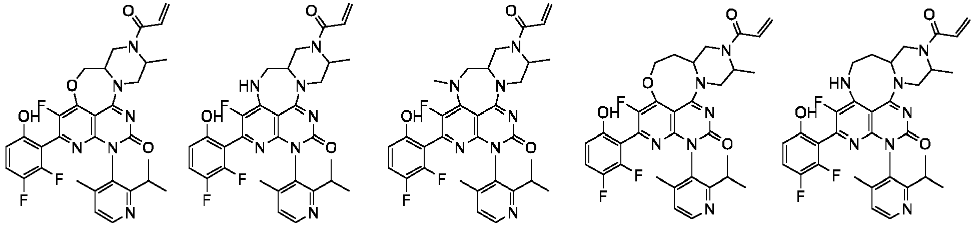

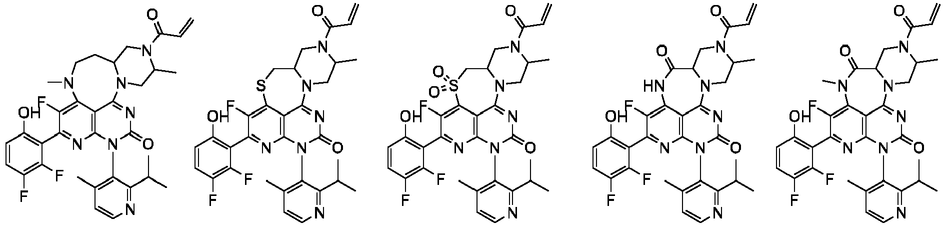

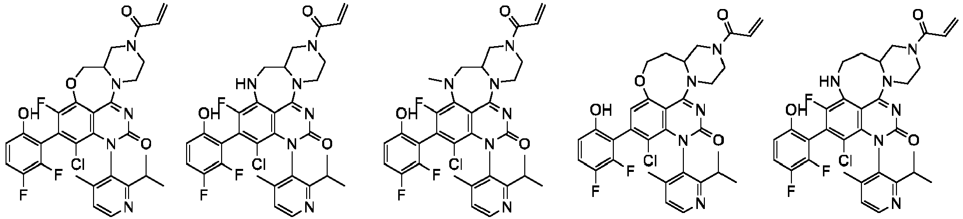

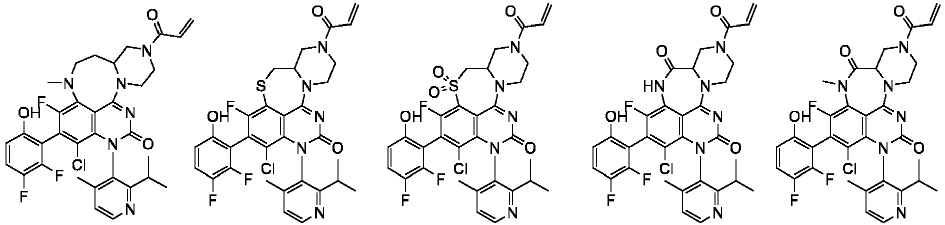

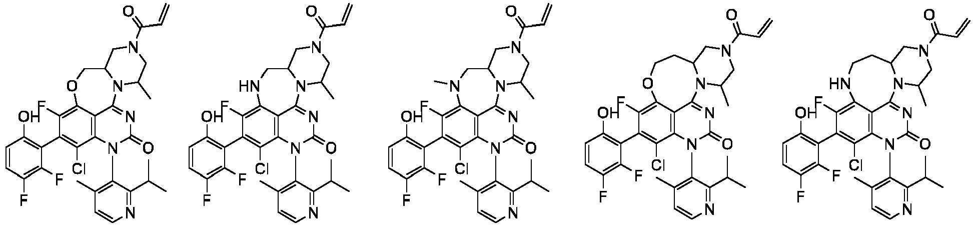

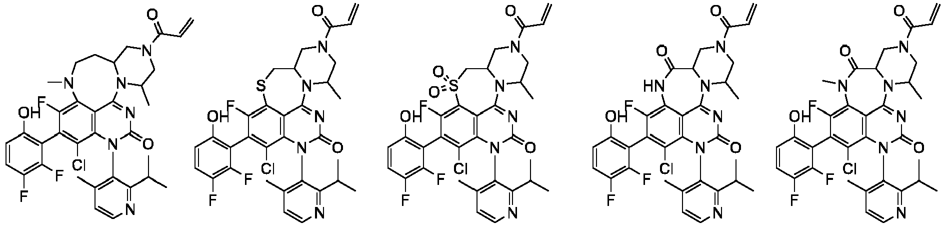

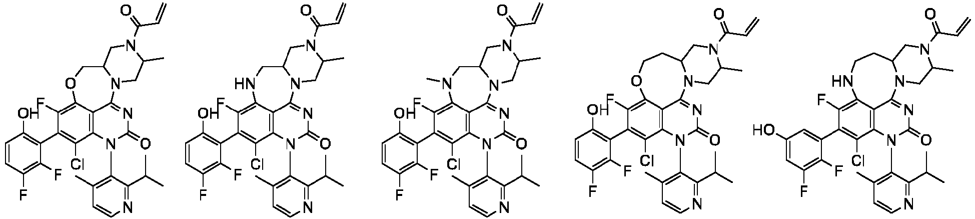

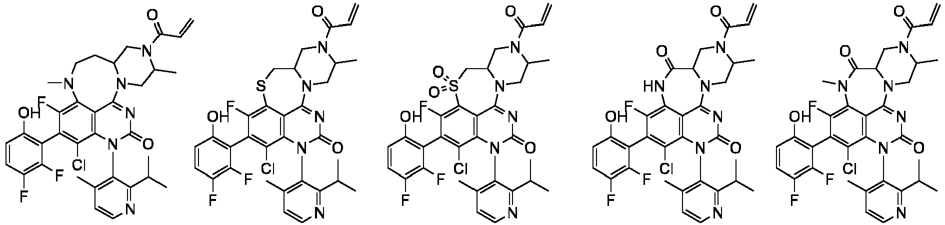

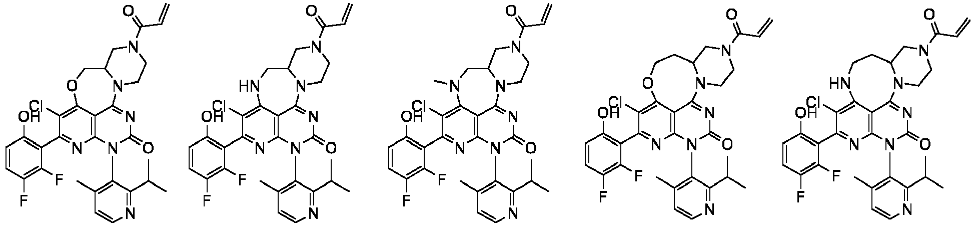

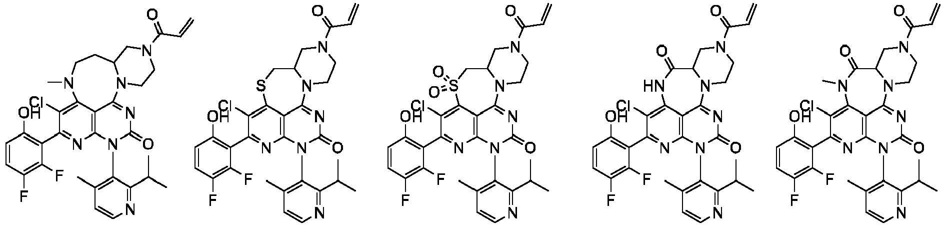

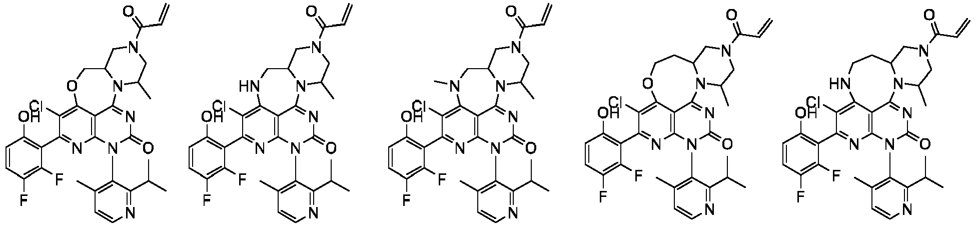

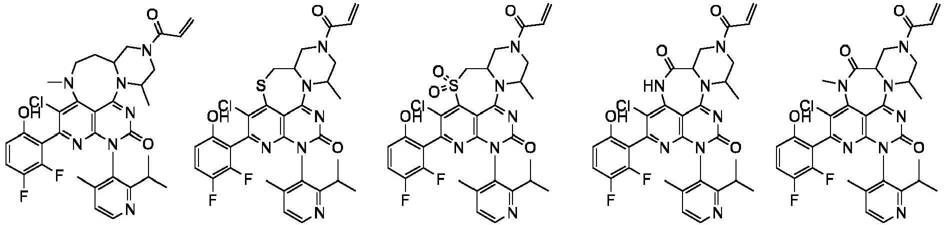

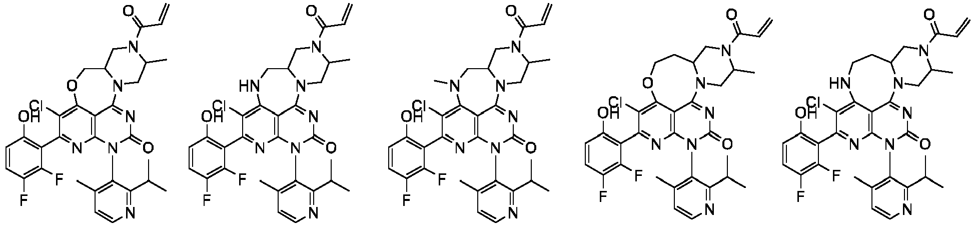

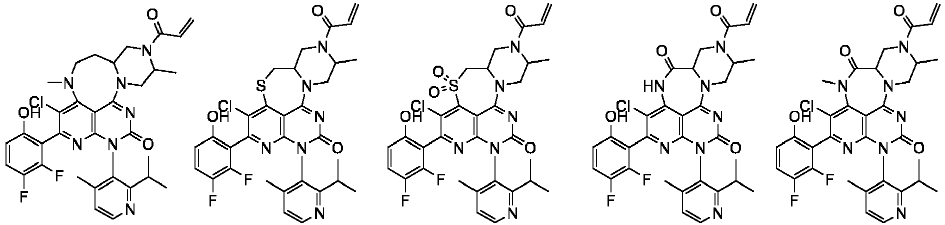

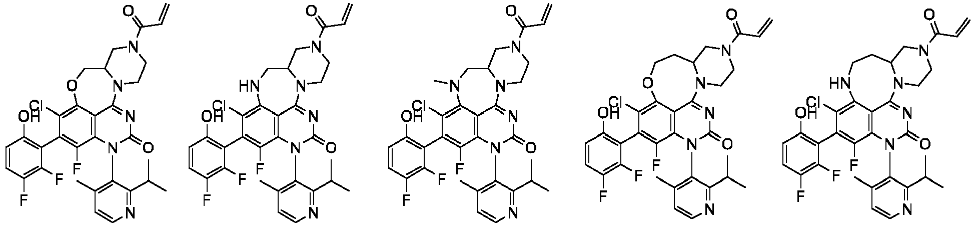

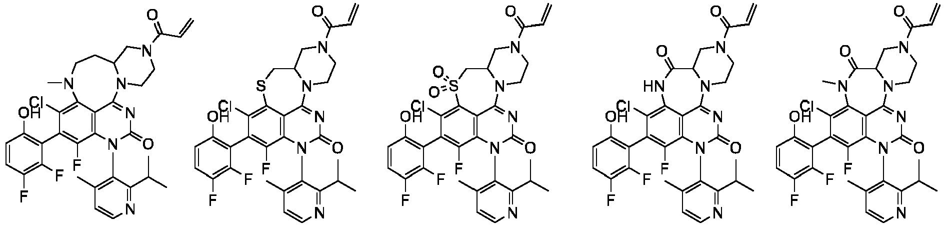

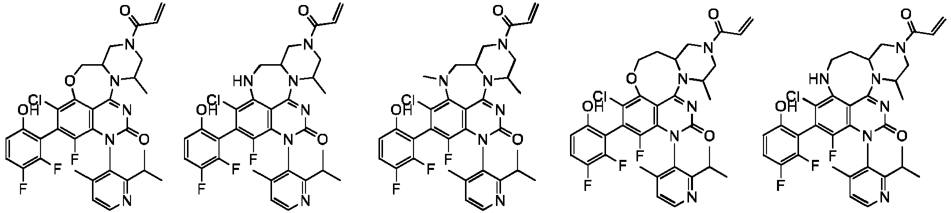

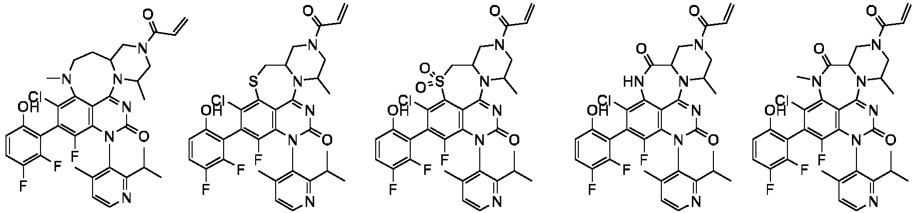

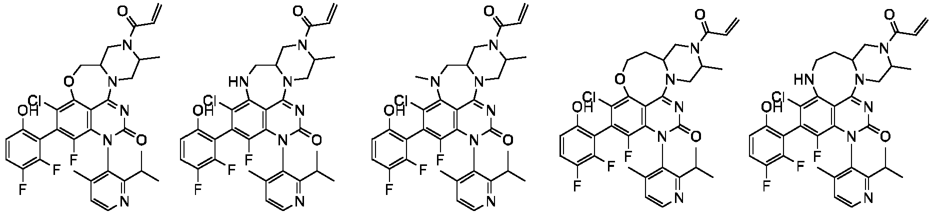

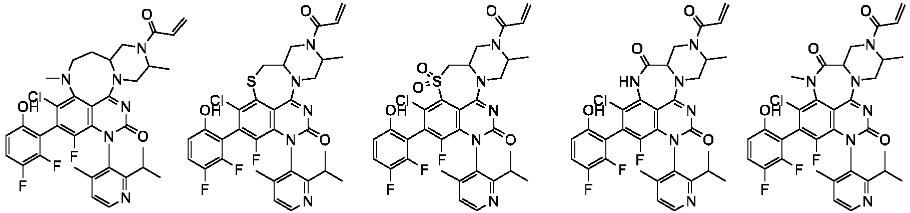

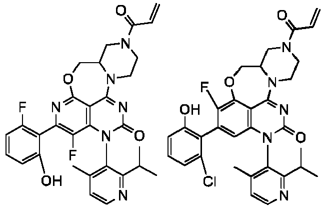

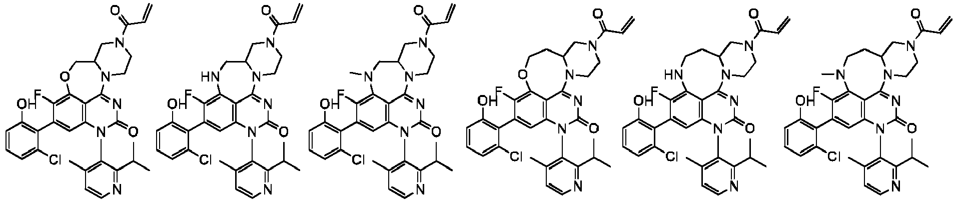

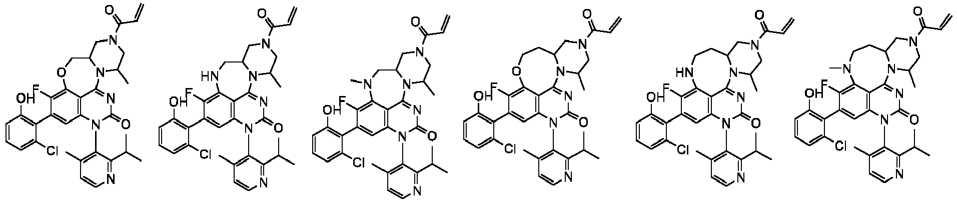

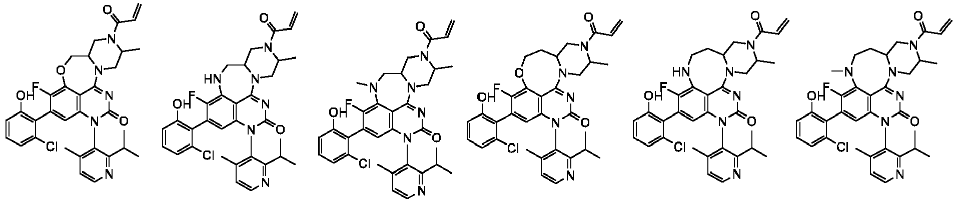

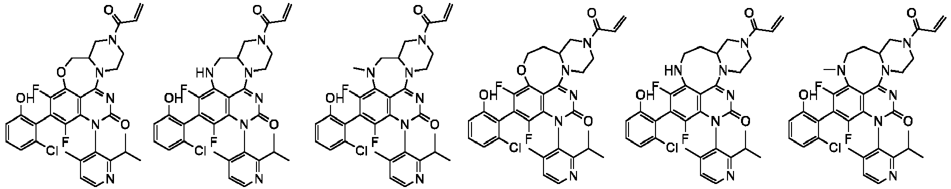

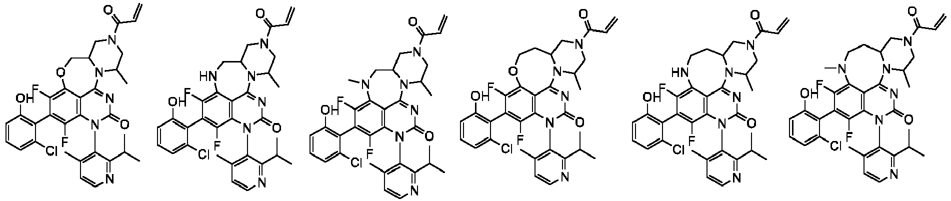

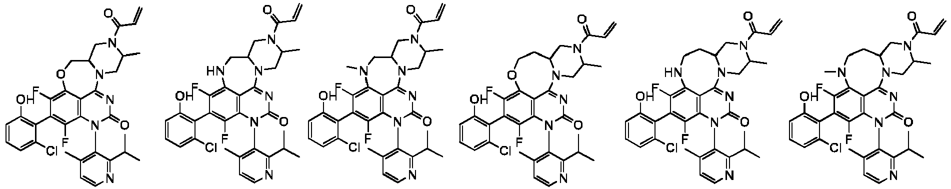

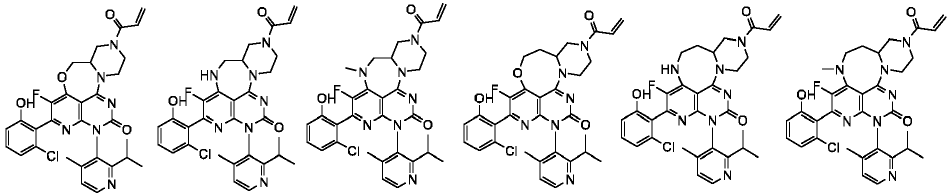

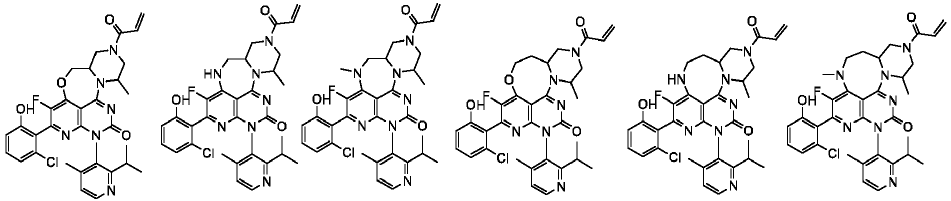

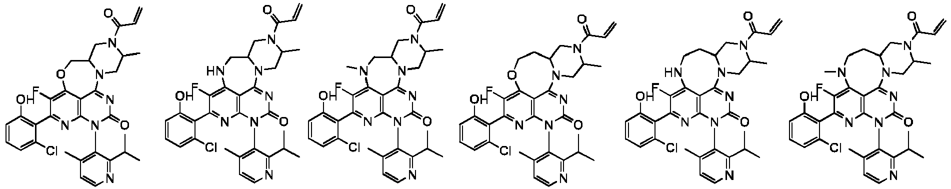

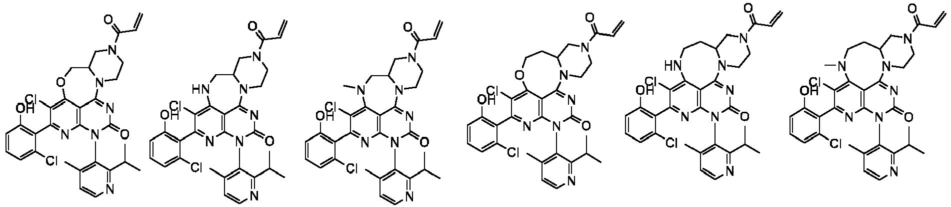

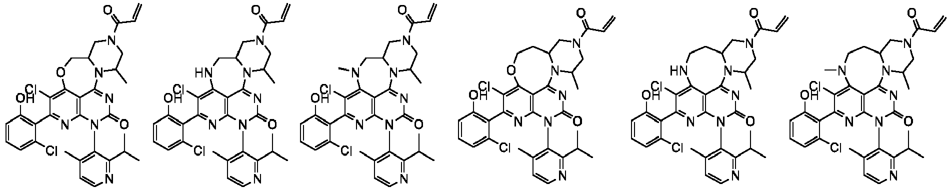

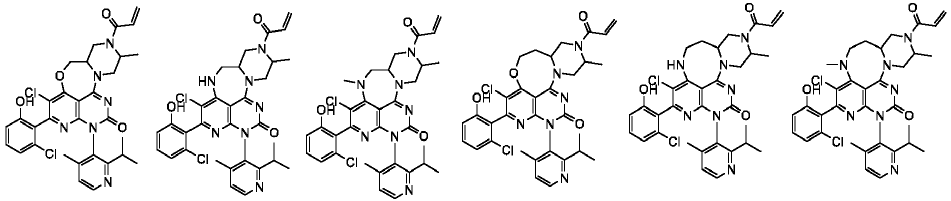

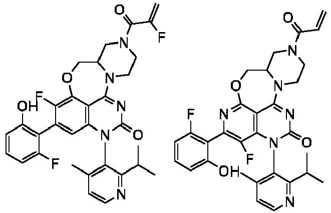

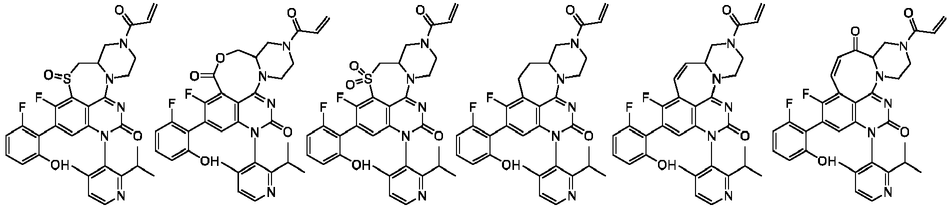

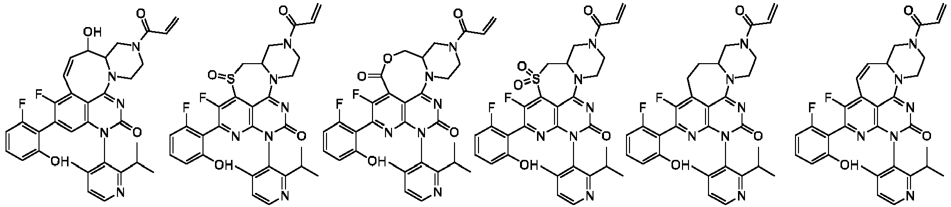

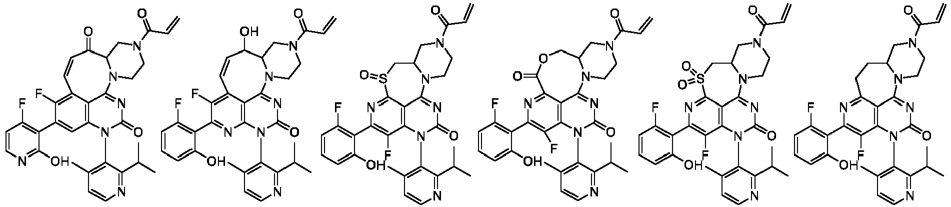

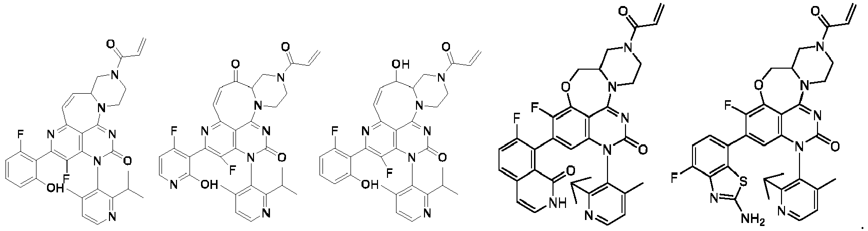

22. A pharmaceutical composition comprising a therapeutically effective amount of the compound or the pharmaceutically acceptable salt thereof according to any one of claims of 1-21, and one or more pharmaceutically acceptable carriers, diluents or excipients.

23. Use of the compound or the pharmaceutically acceptable salt thereof according to any one of claims 1-21 or the pharmaceutical composition according to claim 22 in preparing a medicament for preventing and/or treating an KRAS-G12C-related diseased.

24. The use according to claim 23, wherein the KRAS-G12C-related disease is selected from non-small cell lung cancer, colon cancer and pancreatic cancer.
